# EUROPEAN PATENT APPLICATION

(11) **EP 4 467 642 A2**
(43) Date of publication of application: **27.11.2024**
(21) Application number: 24192681.5
(22) Date of filing: 06.05.2021
(51) Int. Cl.: C12N 9/22

(54) **ENZYMES WITH RUVC DOMAINS**

(30) Priority: 08.05.2020 US 202063022320 P; 29.05.2020 US 202063032464 P; 19.11.2020 US 202063116155 P; 27.04.2021 US 202163180570 P
(62) Divisional of application: 21800930.6
(71) Applicant: Metagenomi, Inc., Emeryville, CA 94608 (US)
(72) Inventor: THOMAS, Brian, Emeryville, 94608 (US); BROWN, Christopher, Emeryville, 94608 (US); KANTOR, Rose, Emeryville, 94608 (US); DEVOTO, Audra, Emeryville, 94608 (US); BUTTERFIELD, Cristina, Emeryville, 94608 (US); ALEXANDER, Lisa, Emeryville, 94608 (US); GOLTSMAN, Daniela S.A., Emeryville, 94608 (US); LIU, Jason, Emeryville, 94608 (US); LAMOTHE, Rebecca, Emeryville, 94608 (US); ESPINOSA, Diego, Emeryville, 94608 (US); STORLIE, Meghan, Emeryville, 94608 (US); COST, Greg, Emeryville, 94608 (US)
(74) Representative: Mewburn Ellis LLP

(57) **Abstract**

The present disclosure provides for endonuclease enzymes having distinguishing domain features, as well as methods of using such enzymes or variants thereof.

## Description

### RELATED APPLICATIONS

This application claims priority to U.S. Provisional Application No. 63/022,320, filed on May 8, 2020, entitled "ENZYMES WITH RUVC DOMAINS", U.S. Provisional Application No. 63/032,464, filed 29 May 2020, entitled "ENZYMES WITH RUVC DOMAINS", and U.S. Provisional Application No. 63/116,155, filed on November 19, 2020, entitled "ENZYMES WITH RUVC DOMAINS", and U.S. Provisional Application No. 63/180,570, filed on April 27, 2021, entitled "ENZYMES WITH RUVC DOMAINS", all of which are incorporated by reference herein in their entireties.

### BACKGROUND

Cas enzymes along with their associated Clustered Regularly Interspaced Short Palindromic Repeats (CRISPR) guide ribonucleic acids (RNAs) appear to be a pervasive (-45% of bacteria, -84% of archaea) component of prokaryotic immune systems, serving to protect such microorganisms against non-self nucleic acids, such as infectious viruses and plasmids by CRISPR-RNA guided nucleic acid cleavage. While the deoxyribonucleic acid (DNA) elements encoding CRISPR RNA elements may be relatively conserved in structure and length, their CRISPR-associated (Cas) proteins are highly diverse, containing a wide variety of nucleic acid-interacting domains. While CRISPR DNA elements have been observed as early as 1987, the programmable endonuclease cleavage ability of CRISPR/Cas complexes has only been recognized relatively recently, leading to the use of recombinant CRISPR/Cas systems in diverse DNA manipulation and gene editing applications.

### SEQUENCE LISTING

The instant application contains a Sequence Listing which has been submitted electronically in ASCII format and is hereby incorporated by reference in its entirety. Said ASCII copy, created on May 29, 2020, is named 55921_712_601_SL.txt and is 24,659,439 bytes in size.

### SUMMARY

In some aspects, the present disclosure provides for an engineered nuclease system, comprising: (a) an endonuclease comprising a RuvC _III domain and an HNH domain, wherein the endonuclease is derived from an uncultivated microorganism, wherein the endonuclease is a class 2, type II Cas endonuclease; and (b) an engineered guide ribonucleic acid structure configured to form a complex with the endonuclease comprising: (i) a guide ribonucleic acid sequence configured to hybridize to a target deoxyribonucleic acid sequence; and (ii) a tracr ribonucleic acid sequence configured to bind to the endonuclease. In some embodiments, the RuvC_III domain comprises a sequence with at least 70%, at least 75%, at least 80%, at least 85%, at least 88%, at least 90%, at least 92%, at least 95%, or at least 98% sequence identity to any one of SEQ ID NOs: 1827-3637.

In some aspects, the present disclosure provides for an engineered nuclease system comprising: (a) an endonuclease comprising a RuvC _III domain having at least 70%, at least 75%, at least 80%, at least 85%, at least 88%, at least 90%, at least 92%, at least 95%, or at least 98% sequence identity to any one of SEQ ID NOs: 1827-3637; and (b) an engineered guide ribonucleic acid structure configured to form a complex with the endonuclease comprising: (i) a guide ribonucleic acid sequence configured to hybridize to a target deoxyribonucleic acid sequence; and (ii) a tracr ribonucleic acid sequence configured to bind to the endonuclease.

In some aspects, the present disclosure provides for an engineered nuclease system comprising: (a) an endonuclease configured to bind to a protospacer adjacent motif (PAM) sequence comprising SEQ ID NOs: 5512-5537, wherein the endonuclease is a class 2, type II Cas endonuclease; and (b) an engineered guide ribonucleic acid structure configured to form a complex with the endonuclease comprising: (i) a guide ribonucleic acid sequence configured to hybridize to a target deoxyribonucleic acid sequence; and (ii) a tracr ribonucleic acid sequence configured to bind to the endonuclease.

In some embodiments, the endonuclease is derived from an uncultivated microorganism. In some embodiments, the endonuclease has not been engineered to bind to a different PAM sequence. In some embodiments, the endonuclease is not a Cas9 endonuclease, a Cas14 endonuclease, a Cas12a endonuclease, a Cas12b endonuclease, a Cas 12c endonuclease, a Cas12d endonuclease, a Cas12e endonuclease, a Cas13a endonuclease, a Cas13b endonuclease, a Cas13c endonuclease, or a Cas 13d endonuclease. In some embodiments, the endonuclease has less than 80% identity to a Cas9 endonuclease. In some embodiments, the endonuclease further comprises an HNH domain. In some embodiments, the tracr ribonucleic acid sequence comprises a sequence with at least 80% sequence identity to about 60 to 90 consecutive nucleotides selected from any one of SEQ ID NOs: 5476-5511 and SEQ ID NO: 5538.

In some aspects, the present disclosure provides for an engineered nuclease system comprising, (a) an engineered guide ribonucleic acid structure comprising: (i) a guide ribonucleic acid sequence configured to hybridize to a target deoxyribonucleic acid sequence; and (ii) a tracr ribonucleic acid sequence configured to bind to an endonuclease, wherein the tracr ribonucleic acid sequence comprises a sequence with at least 80% sequence identity to about 60 to 90 consecutive nucleotides selected from any one of SEQ ID NOs: 5476-5511 and SEQ ID NO: 5538; and (b) a class 2, type II Cas endonuclease configured to bind to the engineered guide ribonucleic acid. In some embodiments, the endonuclease is configured to bind to a protospacer adjacent motif (PAM) sequence selected from the group comprising SEQ ID NOs: 5512-5537.

In some embodiments, the engineered guide ribonucleic acid structure comprises at least two ribonucleic acid polynucleotides. In some embodiments, the engineered guide ribonucleic acid structure comprises one ribonucleic acid polynucleotide comprising the guide ribonucleic acid sequence and the tracr ribonucleic acid sequence.

In some embodiments, the guide ribonucleic acid sequence is complementary to a prokaryotic, bacterial, archaeal, eukaryotic, fungal, plant, mammalian, or human genomic sequence. In some embodiments, the guide ribonucleic acid sequence is 15-24 nucleotides in length. In some embodiments, the endonuclease comprises one or more nuclear localization sequences (NLSs) proximal to an N- or C-terminus of the endonuclease. In some embodiments, the NLS comprises a sequence selected from SEQ ID NOs: 5597-5612.

In some embodiments, the engineered nuclease system further comprises a single- or double-stranded DNA repair template comprising from 5' to 3': a first homology arm comprising a sequence of at least 20 nucleotides 5' to the target deoxyribonucleic acid sequence, a synthetic DNA sequence of at least 10 nucleotides, and a second homology arm comprising a sequence of at least 20 nucleotides 3' to the target sequence. In some embodiments, the first or second homology arm comprises a sequence of at least 40, 80, 120, 150, 200, 300, 500, or 1,000 nucleotides.

In some embodiments, the system further comprises a source of Mg2+.

In some embodiments, the endonuclease and the tracr ribonucleic acid sequence are derived from distinct bacterial species within a same phylum. In some embodiments, the endonuclease is derived from a bacterium belonging to a genus Dermabacter. In some embodiments, the endonuclease is derived from a bacterium belonging to Phylum Verrucomicrobia, Phylum Candidatus Peregrinibacteria, or Phylum Candidatus Melainabacteria. In some embodiments, the endonuclease is derived from a bacterium comprising a 16S rRNA gene having at least 90% identity to any one of SEQ ID NOs: 5592-5595 .

In some embodiments, the HNH domain comprises a sequence with at least 70% or at least 80% identity to any one of SEQ ID NOs: 5638-5460. In some embodiments, the endonuclease comprises SEQ ID NOs: 1-1826 or a variant thereof having at least 55% identity thereto. In some embodiments, the endonuclease comprises a sequence at least 70%, 80%, or 90% identical to a sequence selected from the group consisting of SEQ ID NOs: 1827-1830 or SEQ ID NOs: 1827-2140.

In some embodiments, the endonuclease comprises a sequence at least 70%, 80%, or 90% identical to a sequence selected from the group consisting of SEQ ID NOs: 3638-3641 or SEQ ID NOs: 3638-3954. In some embodiments, the endonuclease comprises at least 1, at least 2, at least 3, at least 4, or at least 5 peptide motifs selected from the group consisting of SEQ ID NOs: 5615-5632. In some embodiments, the endonuclease comprises a sequence at least 70%, 80%, or 90% identical to a sequence selected from the group consisting of SEQ ID NOs: 1-4 or SEQ ID NOs: 1-319.

In some embodiments, the guide RNA structure comprises a sequence at least 70%, 80%, or 90% identical to a sequence selected from the group consisting of SEQ ID NOs: 5461-5464, SEQ ID NOs: 5476-5479, or SEQ ID NOs: 5476-5489. In some embodiments, the guide RNA structure comprises an RNA sequence predicted to comprise a hairpin consisting of a stem and a loop, wherein the stem comprises at least 10, at least 12 or at least 14 base-paired ribonucleotides, and an asymmetric bulge within 4 base pairs of the loop.

In some embodiments, the endonuclease is configured to bind to a PAM comprising a sequence selected from the group consisting of SEQ ID NOs: 5512-5515 or SEQ ID NOs: 5527-5530.

In some embodiments: (a) the endonuclease comprises a sequence at least 70%, at least 80%, or at least 90% identical to SEQ ID NO: 1827; (b) the guide RNA structure comprises a sequence at least 70%, at least 80%, or at least 90% identical to at least one of SEQ ID NO: 5461 or SEQ ID NO: 5476; and (c) the endonuclease is configured to bind to a PAM comprising SEQ ID NO: 5512 or SEQ ID NO: 5527. In some embodiments: (a) the endonuclease comprises a sequence at least 70%, at least 80%, or at least 90% identical to SEQ ID NO: 1828; (b) the guide RNA structure comprises a sequence at least 70%, at least 80%, or at least 90% identical to at least one of SEQ ID NO: 5462 or SEQ ID NO: 5477; and (c) the endonuclease is configured to bind to a PAM comprising SEQ ID NO: 5513 or SEQ ID NO: 5528. In some embodiments: (a) the endonuclease comprises a sequence at least 70%, at least 80%, or at least 90% identical to SEQ ID NO: 1829; (b) the guide RNA structure comprises a sequence at least 70%, at least 80%, or at least 90% identical to at least one of SEQ ID NO: 5463 or SEQ ID NO: 5478; and (c) the endonuclease is configured to bind to a PAM comprising SEQ ID NO: 5514 or SEQ ID NO: 5529. In some embodiments: (a) the endonuclease comprises a sequence at least 70%, at least 80%, or at least 90% identical to SEQ ID NO: 1830; (b) the guide RNA structure comprises a sequence at least 70%, at least 80%, or at least 90% identical to at least one of SEQ ID NO: 5464 or SEQ ID NO: 5479; and (c) the endonuclease is configured to bind to a PAM comprising SEQ ID NO: 5515 or SEQ ID NO: 5530.

In some embodiments, the endonuclease comprises a sequence at least 70%, 80%, or 90% identical to a sequence selected from the group consisting of SEQ ID NOs: 2141-2142 or SEQ ID NOs: 2141-2241. In some embodiments, the endonuclease comprises a sequence at least 70%, 80%, or 90% identical to a sequence selected from the group consisting of SEQ ID NOs: 3955-3956 or SEQ ID NOs: 3955-4055. In some embodiments, the endonuclease comprises at least 1, at least 2, at least 3, at least 4, or at least 5 peptide motifs selected from the group consisting of SEQ ID NOs: 5632-5638. In some embodiments, the endonuclease comprises a sequence at least 70%, 80%, or 90% identical to a sequence selected from the group consisting of SEQ ID NOs: 320-321 or SEQ ID NOs: 320-420. In some embodiments, the guide RNA structure comprises a sequence at least 70%, 80%, or 90% identical to a sequence selected from the group consisting of SEQ ID NO: 5465, SEQ ID NOs: 5490-5491 or SEQ ID NOs: 5490-5494. In some embodiments, the guide RNA structure comprises a tracr ribonucleic acid sequence comprising a hairpin comprising at least 8, at least 10, or at least 12 base-paired ribonucleotides. In some embodiments, the endonuclease is configured to bind to a PAM comprising a sequence selected from the group consisting of SEQ ID NOs: 5516 and SEQ ID NOs: 5531. In some embodiments: (a) the endonuclease comprises a sequence at least 70%, 80%, or 90% identical to SEQ ID NO: 2141; (b) the guide RNA structure comprises a sequence at least 70%, 80%, or 90% identical to SEQ ID NO: 5490; and (c) the endonuclease is configured to binding to a PAM comprising SEQ ID NO: 5531. In some embodiments: (a) the endonuclease comprises a sequence at least 70%, 80%, or 90% identical to SEQ ID NO: 2142; (b) the guide RNA structure comprises a sequence at least 70%, 80%, or 90% identical to SEQ ID NO: 5465 or SEQ ID NO: 5491; and (c) the endonuclease is configured to binding to a PAM comprising SEQ ID NO: 5516.

In some embodiments, the endonuclease comprises a sequence at least 70%, 80%, or 90% identical to a sequence selected from the group consisting of SEQ ID NOs: 2245-2246. In some embodiments, the endonuclease comprises a sequence at least 70%, 80%, or 90% identical to a sequence selected from the group consisting of SEQ ID NOs: 4059-4060. In some embodiments, the endonuclease comprises at least 1, at least 2, at least 3, at least 4, or at least 5 peptide motifs selected from the group consisting of SEQ ID NOs: 5639-5648. In some embodiments, the endonuclease comprises a sequence at least 70%, 80%, or 90% identical to a sequence selected from the group consisting of SEQ ID NOs: 424-425. In some embodiments, the guide RNA structure comprises a sequence at least 70%, 80%, or 90% identical to a sequence selected from the group consisting of SEQ ID NOs: 5498-5499 and SEQ ID NO: 5539. In some embodiments, the guide RNA structure comprises a guide ribonucleic acid sequence predicted to comprise a hairpin with an uninterrupted base-paired region comprising at least 8 nucleotides of a guide ribonucleic acid sequence and at least 8 nucleotides of a tracr ribonucleic acid sequence, and wherein the tracr ribonucleic acid sequence comprises, from 5' to 3', a first hairpin and a second hairpin, wherein the first hairpin has a longer stem than the second hairpin.

In some embodiments, the endonuclease comprises a sequence at least 70%, 80%, or 90% identical to a sequence selected from the group consisting of SEQ ID NOs: 2242-2244 or SEQ ID NOs: 2247-2249. In some embodiments, the endonuclease comprises a sequence at least 70%, 80%, or 90% identical to a sequence selected from the group consisting of SEQ ID NOs: 4056-4058 and SEQ ID NOs 4061-4063. In some embodiments, the endonuclease comprises at least 1, at least 2, at least 3, at least 4, or at least 5 peptide motifs selected from the group consisting of SEQ ID NOs: 5639-5648. In some embodiments, the endonuclease comprises a sequence at least 70%, 80%, or 90% identical to a sequence selected from the group consisting of SEQ ID NOs: 421-423 or SEQ ID NOs: 426-428. In some embodiments, the guide RNA structure comprises a sequence at least 70%, 80%, or 90% identical to a sequence selected from the group consisting of SEQ ID NOs: 5466-5467, SEQ ID NOs: 5495-5497, SEQ ID NO: 5500-5502, and SEQ ID NO: 5539. In some embodiments, the guide RNA structure comprises a guide ribonucleic acid sequence predicted to comprise a hairpin with an uninterrupted base-paired region comprising at least 8 nucleotides of a guide ribonucleic acid sequence and at least 8 nucleotides of a tracr ribonucleic acid sequence, and wherein the tracr ribonucleic acid sequence comprises, from 5' to 3', a first hairpin and a second hairpin, wherein the first hairpin has a longer stem than the second hairpin. In some embodiments, the endonuclease is configured to binding to a PAM comprising a sequence selected from the group consisting of SEQ ID NOs: 5517-5518 or SEQ ID NOs: 5532-5534. In some embodiments: (a) the endonuclease comprises a sequence at least 70%, 80%, or 90% identical to SEQ ID NO: 2247; (b) the guide RNA structure comprises a sequence at least 70%, 80%, or 90% identical to SEQ ID NO: 5500; and (c) the endonuclease is configured to binding to a PAM comprising SEQ ID NO: 5517 or SEQ ID NO: 5532. In some embodiments: (a) the endonuclease comprises a sequence at least 70%, 80%, or 90% identical to SEQ ID NO: 2248; (b) the guide RNA structure comprises a sequence at least 70%, 80%, or 90% identical to SEQ ID NO: 5501; and (c) the endonuclease is configured to binding to a PAM comprising SEQ ID NO: 5518 or SEQ ID NOs: 5533. In some embodiments: (a) the endonuclease comprises a sequence at least 70%, 80%, or 90% identical to SEQ ID NO: 2249; (b) the guide RNA structure comprises a sequence at least 70%, 80%, or 90% identical to SEQ ID NO: 5502; and (c) the endonuclease is configured to binding to a PAM comprising SEQ ID NO: 5534.

In some embodiments, the endonuclease comprises a sequence at least 70%, 80%, or 90% identical to a sequence selected from the group consisting of SEQ ID NO: 2253 or SEQ ID NOs: 2253-2481. In some embodiments, the endonuclease comprises a sequence at least 70%, 80%, or 90% identical to a sequence selected from the group consisting of SEQ ID NO: 4067 or SEQ ID NOs: 4067-4295. In some embodiments, the endonuclease comprises a peptide motif according to SEQ ID NO: 5649. In some embodiments, the endonuclease comprises a sequence at least 70%, 80%, or 90% identical to a sequence selected from the group consisting of SEQ ID NO: 432 or SEQ ID NOs: 432-660. In some embodiments, the guide RNA structure comprises a sequence at least 70%, 80%, or 90% identical to a sequence selected from the group consisting of SEQ ID NO: 5468 or SEQ ID NO: 5503. In some embodiments, the endonuclease is configured to binding to a PAM comprising a sequence selected from the group consisting of SEQ ID NOs: 5519. In some embodiments: (a) the endonuclease comprises a sequence at least 70%, 80%, or 90% identical to SEQ ID NO: 2253; (b) the guide RNA structure comprises a sequence at least 70%, 80%, or 90% identical to SEQ ID NO: 5468 or SEQ ID NO: 5503; and (c) the endonuclease is configured to binding to a PAM comprising SEQ ID NO: 5519.

In some embodiments, the endonuclease comprises a sequence at least 70%, 80%, or 90% identical to a sequence selected from the group consisting of SEQ ID NOs: 2482-2489. In some embodiments, the endonuclease comprises a sequence at least 70%, 80%, or 90% identical to a sequence selected from the group consisting of SEQ ID NOs: 4296-4303. In some embodiments, the endonuclease comprises a sequence at least 70%, 80%, or 90% identical to a sequence selected from the group consisting of or SEQ ID NOs: 661-668. In some embodiments, the endonuclease comprises a sequence at least 70%, 80%, or 90% identical to a sequence selected from the group consisting of or SEQ ID NOs: 2490-2498. In some embodiments, the endonuclease comprises a sequence at least 70%, 80%, or 90% identical to a sequence selected from the group consisting of SEQ ID NOs: 4304-4312. In some embodiments, the endonuclease comprises a sequence at least 70%, 80%, or 90% identical to a sequence selected from the group consisting of SEQ ID NOs: 669-677. In some embodiments, the guide RNA structure comprises a sequence at least 70%, 80%, or 90% identical to a sequence selected from the group consisting of SEQ ID NO: 5504.

In some embodiments, the endonuclease comprises a sequence at least 70%, 80%, or 90% identical to a sequence selected from the group consisting of SEQ ID NO: 2499 or SEQ ID NOs: 2499-2750. In some embodiments, the endonuclease comprises a sequence at least 70%, 80%, or 90% identical to a sequence selected from the group consisting of SEQ ID NO: 4313 or SEQ ID NOs: 4313-4564. In some embodiments, the endonuclease comprises at least 1, at least 2, at least 3, at least 4, or at least 5 peptide motifs selected from the group consisting of SEQ ID NOs: 5650-5667. In some embodiments, the endonuclease comprises a sequence at least 70%, 80%, or 90% identical to a sequence selected from the group consisting of SEQ ID NO: 678 or SEQ ID NOs: 678-929. In some embodiments, the guide RNA structure comprises a sequence at least 70%, 80%, or 90% identical to SEQ ID NO: 5469 or SEQ ID NO: 5505. In some embodiments, the endonuclease is configured to binding to a PAM comprising SEQ ID NOs: 5520 or SEQ ID NOs: 5535. In some embodiments: (a) the endonuclease comprises a sequence at least 70%, 80%, or 90% identical to SEQ ID NO: 2499; (b) the guide RNA structure comprises a sequence at least 70%, 80%, or 90% identical to SEQ ID NO: 5469 or SEQ ID NO: 5505; and (c) the endonuclease is configured to binding to a PAM comprising SEQ ID NO: 5520 or SEQ ID NO: 5535.

In some embodiments, the endonuclease comprises a sequence at least 70%, 80%, or 90% identical to a sequence selected from the group consisting of SEQ ID NO: 2751 or SEQ ID NOs: 2751-2913. In some embodiments, the endonuclease comprises a sequence at least 70%, 80%, or 90% identical to a sequence selected from the group consisting of SEQ ID NO: 4565 or SEQ ID NOs: 4565-4727. In some embodiments, the endonuclease comprises at least 1, at least 2, at least 3, at least 4, or at least 5 peptide motifs selected from the group consisting of SEQ ID NOs: 5668-5678. In some embodiments, the endonuclease comprises a sequence at least 70%, 80%, or 90% identical to a sequence selected from the group consisting of SEQ ID NO: 930 or SEQ ID NOs: 930-1092. In some embodiments, the guide RNA structure comprises a sequence at least 70%, 80%, or 90% identical to SEQ ID NO: 5470 or SEQ ID NOs: 5506. In some embodiments, the endonuclease is configured to binding to a PAM comprising a sequence selected from the group consisting of SEQ ID NOs: 5521 or SEQ ID NOs: 5536. In some embodiments: (a) the endonuclease comprises a sequence at least 70%, 80%, or 90% identical to SEQ ID NO: 2751; (b) the guide RNA structure comprises a sequence at least 70%, 80%, or 90% identical to SEQ ID NO: 5470 or SEQ ID NO: 5506; and (c) the endonuclease is configured to binding to a PAM comprising SEQ ID NO: 5521 or SEQ ID NO: 5536.

In some embodiments, the endonuclease comprises a sequence at least 70%, 80%, or 90% identical to a sequence selected from the group consisting of SEQ ID NO: 2914 or SEQ ID NOs: 2914-3174. In some embodiments, the endonuclease comprises a sequence at least 70%, 80%, or 90% identical to a sequence selected from the group consisting of SEQ ID NO: 4728 or SEQ ID NOs: 4728-4988. In some embodiments, the endonuclease comprises at least 1, at least 2, or at least 3 peptide motifs selected from the group consisting of SEQ ID NOs: 5676-5678. In some embodiments, the endonuclease comprises a sequence at least 70%, 80%, or 90% identical to a sequence selected from the group consisting of SEQ ID NO: 1093 or SEQ ID NOs: 1093-1353. In some embodiments, the guide RNA structure comprises a sequence at least 70%, 80%, or 90% identical to a sequence selected from the group consisting of SEQ ID NO: 5471, SEQ ID NO: 5507, and SEQ ID NOs: 5540-5542. In some embodiments, the guide RNA structure comprises a tracr ribonucleic acid sequence predicted to comprise at least two hairpins comprising less than 5 base-paired ribonucleotides. In some embodiments, the endonuclease is configured to binding to a PAM comprising SEQ ID NO: 5522. In some embodiments: (a) the endonuclease comprises a sequence at least 70%, 80%, or 90% identical to SEQ ID NO: 2914; (b) the guide RNA structure comprises a sequence at least 70%, 80%, or 90% identical to SEQ ID NO: 5471 or SEQ ID NO: 5507; and (c) the endonuclease is configured to binding to a PAM comprising SEQ ID NO: 5522.

In some embodiments, the endonuclease comprises a sequence at least 70%, 80%, or 90% identical to a sequence selected from the group consisting of SEQ ID NO: 3175 or SEQ ID NOs: 3175-3330. In some embodiments, the endonuclease comprises a sequence at least 70%, 80%, or 90% identical to a sequence selected from the group consisting of SEQ ID NO: 4989 or SEQ ID NOs: 4989-5146. In some embodiments, the endonuclease comprises at least 1, at least 2, at least 3, at least 4, or at least 5 peptide motifs selected from the group consisting of SEQ ID NOs: 5679-5686. In some embodiments, the endonuclease comprises a sequence at least 70%, 80%, or 90% identical to a sequence selected from the group consisting of SEQ ID NO: 1354 or SEQ ID NOs: 1354-1511. In some embodiments, the guide RNA structure comprises a sequence at least 70%, 80%, or 90% identical to a sequence selected from the group consisting of SEQ ID NOs: 5472 or SEQ ID NOs: 5508. In some embodiments, the endonuclease is configured to binding to a PAM comprising a sequence selected from the group consisting of SEQ ID NO: 5523 or SEQ ID NO: 5537. In some embodiments: (a) the endonuclease comprises a sequence at least 70%, 80%, or 90% identical to SEQ ID NO: 3175; (b) the guide RNA structure comprises a sequence at least 70%, 80%, or 90% identical to SEQ ID NO: 5472 or SEQ ID NO: 5508; and (c) the endonuclease is configured to binding to a PAM comprising SEQ ID NO: 5523 or SEQ ID NO: 5537.

In some embodiments, the endonuclease comprises a sequence at least 70%, 80%, or 90% identical to a sequence selected from the group consisting of SEQ ID NOs: 3331 or SEQ ID NOs: 3331-3474. In some embodiments, the endonuclease comprises a sequence at least 70%, 80%, or 90% identical to a sequence selected from the group consisting of SEQ ID NOs: 5147 or SEQ ID NOs: 5147-5290. In some embodiments, the endonuclease comprises at least 1, at least 2, at least 3, at least 4, or at least 5 peptide motifs selected from the group consisting of SEQ ID NOs: 5674-5675 and SEQ ID NOs: 5687-5693. In some embodiments, the endonuclease comprises a sequence at least 70%, 80%, or 90% identical to a sequence selected from the group consisting of SEQ ID NO: 1512 or SEQ ID NOs: 1512-1655. In some embodiments, the guide RNA structure comprises a sequence at least 70%, 80%, or 90% identical to a sequence selected from the group consisting of SEQ ID NO: 5473 or SEQ ID NO: 5509. In some embodiments, the endonuclease is configured to binding to a PAM comprising SEQ ID NO: 5524. In some embodiments: (a) the endonuclease comprises a sequence at least 70%, 80%, or 90% identical to SEQ ID NO: 3331; (b) the guide RNA structure comprises a sequence at least 70%, 80%, or 90% identical to SEQ ID NO: 5473 or SEQ ID NO: 5509; and (c) the endonuclease is configured to binding to a PAM comprising SEQ ID NO: 5524.

In some embodiments, the endonuclease comprises a sequence at least 70%, 80%, or 90% identical to a sequence selected from the group consisting of SEQ ID NO: 3475 or SEQ ID NOs: 3475-3568. In some embodiments, the endonuclease comprises a sequence at least 70%, 80%, or 90% identical to a sequence selected from the group consisting of SEQ ID NO: 5291 or SEQ ID NOs: 5291-5389. In some embodiments, the endonuclease comprises at least 1, at least 2, at least 3, at least 4, or at least 5 peptide motifs selected from the group consisting of SEQ ID NOs: 5694-5699. In some embodiments, the endonuclease comprises a sequence at least 70%, 80%, or 90% identical to a sequence selected from the group consisting of SEQ ID NO: 1656 or SEQ ID NOs: 1656-1755. In some embodiments, the guide RNA structure comprises a sequence at least 70%, 80%, or 90% identical to SEQ ID NO: 5474 or SEQ ID NO: 5510. In some embodiments, the endonuclease is configured to binding to a PAM comprising SEQ ID NOs: 5525. In some embodiments: (a) the endonuclease comprises a sequence at least 70%, 80%, or 90% identical to SEQ ID NO: 3475; (b) the guide RNA structure comprises a sequence at least 70%, 80%, or 90% identical to SEQ ID NO: 5474 or SEQ ID NO: 5510; and (c) the endonuclease is configured to binding to a PAM comprising SEQ ID NO: 5525.

In some embodiments, the endonuclease comprises a sequence at least 70%, 80%, or 90% identical to a sequence selected from the group consisting of SEQ ID NO: 3569 or SEQ ID NOs: 3569-3637. In some embodiments, the endonuclease comprises a sequence at least 70%, 80%, or 90% identical to a sequence selected from the group consisting of SEQ ID NO: 5390 or SEQ ID NOs: 5390-5460. In some embodiments, the endonuclease comprises at least 1, at least 2, at least 3, at least 4, or at least 5 peptide motifs selected from the group consisting of SEQ ID NOs: 5700-5717. In some embodiments, the endonuclease comprises a sequence at least 70%, 80%, or 90% identical to a sequence selected from the group consisting of SEQ ID NO: 1756 or SEQ ID NOs: 1756-1826. In some embodiments, the guide RNA structure comprises a sequence at least 70%, 80%, or 90% identical to SEQ ID NO: 5475 or SEQ ID NOs: 5511. In some embodiments, the endonuclease is configured to binding to a PAM comprising SEQ ID NO: 5526. In some embodiments: (a) the endonuclease comprises a sequence at least 70%, 80%, or 90% identical to SEQ ID NO: 3569; (b) the guide RNA structure comprises a sequence at least 70%, 80%, or 90% identical to SEQ ID NO: 5475 or SEQ ID NO: 5511; and (c) the endonuclease is configured to binding to a PAM comprising SEQ ID NO: 5526. In some embodiments, the sequence identity is determined by a BLASTP, CLUSTALW, MUSCLE, MAFFT, or CLUSTALW with the parameters of the Smith-Waterman homology search algorithm. In some embodiments, the sequence identity is determined by the BLASTP homology search algorithm using parameters of a wordlength (W) of 3, an expectation (E) of 10, and a BLOSUM62 scoring matrix setting gap costs at existence of 11, extension of 1, and using a conditional compositional score matrix adjustment.

In some aspects, the present disclosure provides for an engineered guide ribonucleic acid polynucleotide comprising: (a) a DNA-targeting segment comprising a nucleotide sequence that is complementary to a target sequence in a target DNA molecule; and (b) a protein-binding segment comprising two complementary stretches of nucleotides that hybridize to form a double-stranded RNA (dsRNA) duplex, wherein the two complementary stretches of nucleotides are covalently linked to one another with intervening nucleotides, and wherein the engineered guide ribonucleic acid polynucleotide is configured to forming a complex with an endonuclease comprising a RuvC_III domain having at least 70%, at least 75%, at least 80%, at least 85%, at least 88%, at least 90%, at least 92%, at least 95%, or at least 98%sequence identity to any one of SEQ ID NOs: 1827-3637 and targeting the complex to the target sequence of the target DNA molecule. In some embodiments, the DNA-targeting segment is positioned 5' of both of the two complementary stretches of nucleotides.

In some embodiments: (a) the protein binding segment comprises a sequence having at least 70%, at least 75%, at least 80%, at least 85%, at least 88%, at least 90%, at least 92%, at least 95%, or at least 98% identity to a sequence selected from the group consisting of SEQ ID NOs: 5476-5479 or SEQ ID NOs: 5476-5489; (b) the protein binding segment comprises a sequence having at least 70%, at least 80%, or at least 90% identity to a sequence selected from the group consisting of (SEQ ID NOs: 5490-5491 or SEQ ID NOs: 5490-5494) and SEQ ID NO: 5538; (c) the protein binding segment comprises a sequence having at least 70%, at least 80%, or at least 90% identity to a sequence selected from the group consisting of SEQ ID NOs: 5498-5499; (d) the protein binding segment comprises a sequence having at least 70%, at least 80%, or at least 90% identity to a sequence selected from the group consisting of SEQ ID NOs: 5495-5497 and SEQ ID NOs: 5500-5502; (e) the protein binding segment comprises a sequence having at least 70%, at least 80%, or at least 90% identity to SEQ ID NO: 5503; (f) the protein binding segment comprises a sequence having at least 70%, at least 80%, or at least 90% identity to SEQ ID NO: 5504; (g) the protein binding segment comprises a sequence having at least 70%, at least 80%, or at least 90% identity to SEQ ID NOs: 5505; (h) protein binding segment comprises a sequence having at least 70%, at least 80%, or at least 90% identity to SEQ ID NO: 5506; (i) protein binding segment comprises a sequence having at least 70%, at least 80%, or at least 90% identity to SEQ ID NO: 5507; (j) the protein binding segment comprises a sequence having at least 70%, at least 80%, or at least 90% identity to SEQ ID NO: 5508; (k) the protein binding segment comprises a sequence having at least 70%, at least 80%, or at least 90% identity to SEQ ID NO: 5509; (l) the protein binding segment comprises a sequence having at least 70%, at least 80%, or at least 90% identity to SEQ ID NO: 5510; or (m) the protein binding segment comprises a sequence having at least 70%, at least 80%, or at least 90% identity to SEQ ID NO: 5511.

In some embodiments: (a) the guide ribonucleic acid polynucleotide comprises an RNA sequence comprising a hairpin comprising a stem and a loop, wherein the stem comprises at least 10, at least 12, or at least 14 base-paired ribonucleotides, and an asymmetric bulge within 4 base pairs of the loop; (b) the guide ribonucleic acid polynucleotide comprises a tracr ribonucleic acid sequence predicted to comprise a hairpin comprising at least 8, at least 10, or at least 12 base-paired ribonucleotides; (c) the guide ribonucleic acid polynucleotide comprises a guide ribonucleic acid sequence predicted to comprise a hairpin with an uninterrupted base-paired region comprising at least 8 nucleotides of a guide ribonucleic acid sequence and at least 8 nucleotides of a tracr ribonucleic acid sequence, and wherein the tracr ribonucleic acid sequence comprises, from 5' to 3', a first hairpin and a second hairpin, wherein the first hairpin has a longer stem than the second hairpin; or (d) the guide ribonucleic acid polynucleotide comprises a tracr ribonucleic acid sequence predicted to comprise at least two hairpins comprising less than 5 base-paired ribonucleotides.

In some aspects, the present disclosure provides for a deoxyribonucleic acid polynucleotide encoding any of the engineered guide ribonucleic acid polynucleotides described herein.

In some aspects, the present disclosure provides for a nucleic acid comprising an engineered nucleic acid sequence optimized for expression in an organism, wherein the nucleic acid encodes a class 2, type II Cas endonuclease comprising a RuvC_III domain and an HNH domain, and wherein the endonuclease is derived from an uncultivated microorganism.

In some aspects, the present disclosure provides for a nucleic acid comprising an engineered nucleic acid sequence optimized for expression in an organism, wherein the nucleic acid encodes an endonuclease comprising a RuvC_III domain having at least 70% sequence identity to any one of SEQ ID NOs: 1827-3637. In some embodiments, the endonuclease comprises an HNH domain having at least 70% or at least 80% sequence identity to any one of SEQ ID NOs: 3638-5460. In some embodiments, the endonuclease comprises SEQ ID NOs: 5572-5591 or a variant thereof having at least 70% sequence identity thereto. In some embodiments, the endonuclease comprises a sequence encoding one or more nuclear localization sequences (NLSs) proximal to an N- or C-terminus of the endonuclease. In some embodiments, the NLS comprises a sequence selected from SEQ ID NOs: 5597-5612.

In some embodiments, the organism is prokaryotic, bacterial, eukaryotic, fungal, plant, mammalian, rodent, or human. In some embodiments, the organism is E. coli, and: (a) the nucleic acid sequence has at least 70%, 80%, or 90% identity to a sequence selected from the group consisting of SEQ ID NOs: 5572-5575; (b) the nucleic acid sequence has at least 70%, 80%, or 90% identity to a sequence selected from the group consisting of SEQ ID NOs: 5576-5577; (c) the nucleic acid sequence has at least 70%, 80%, or 90% identity to a sequence selected from the group consisting of SEQ ID NOs: 5578-5580; (d) the nucleic acid sequence has at least 70%, 80%, or 90% identity to SEQ ID NO: 5581; (e) the nucleic acid sequence has at least 70%, 80%, or 90% identity to SEQ ID NO: 5582; (f) the nucleic acid sequence has at least 70%, 80%, or 90% identity to SEQ ID NO: 5583; (g) the nucleic acid sequence has at least 70%, 80%, or 90% identity to SEQ ID NO: 5584; (h) the nucleic acid sequence has at least 70%, 80%, or 90% identity to SEQ ID NO: 5585; (i) the nucleic acid sequence has at least 70%, 80%, or 90% identity to SEQ ID NO: 5586; or (j) the nucleic acid sequence has at least 70%, 80%, or 90% identity to SEQ ID NO: 5587. In some embodiments, the organism is human, and: (a) the nucleic acid sequence has at least 70%, 80%, or 90% identity to SEQ ID NO: 5588 or SEQ ID NO: 5589; or (b) the nucleic acid sequence has at least 70%, 80%, or 90% identity to SEQ ID NO: 5590 or SEQ ID NO: 5591.

In some aspects, the present disclosure provides for a vector comprising a nucleic acid sequence encoding a class 2, type II Cas endonuclease comprising a RuvC_III domain and an HNH domain, wherein the endonuclease is derived from an uncultivated microorganism.

In some aspects, the present disclosure provides for a vector comprising the any of the nucleic acids described herein. In some embodiments, the vector further comprises a nucleic acid encoding an engineered guide ribonucleic acid structure configured to form a complex with the endonuclease comprising: (a) a guide ribonucleic acid sequence configured to hybridize to a target deoxyribonucleic acid sequence; and (b) a tracr ribonucleic acid sequence configured to binding to the endonuclease. In some embodiments, the vector is a plasmid, a minicircle, a CELiD, an adeno-associated virus (AAV) derived virion, or a lentivirus.

In some aspects, the present disclosure provides for a cell comprising any of the vectors described herein.

In some aspects, the present disclosure provides for a method of manufacturing an endonuclease, comprising cultivating any of the cells described herein.

In some aspects, the present disclosure provides for a method for binding, cleaving, marking, or modifying a double-stranded deoxyribonucleic acid polynucleotide, comprising: (a) contacting the double-stranded deoxyribonucleic acid polynucleotide with a class 2, type II Cas endonuclease in complex with an engineered guide ribonucleic acid structure configured to bind to the endonuclease and the double-stranded deoxyribonucleic acid polynucleotide; (b) wherein the double-stranded deoxyribonucleic acid polynucleotide comprises a protospacer adjacent motif (PAM); and (c) wherein the PAM comprises a sequence selected from the group consisting of SEQ ID NOs: 5512-5526 or SEQ ID NOs: 5527-5537. In some embodiments, the double-stranded deoxyribonucleic acid polynucleotide comprises a first strand comprising a sequence complementary to a sequence of the engineered guide ribonucleic acid structure and a second strand comprising the PAM. In some embodiments, the PAM is directly adjacent to the 3' end of the sequence complementary to the sequence of the engineered guide ribonucleic acid structure.

In some embodiments, the class 2, type II Cas endonuclease is not a Cas9 endonuclease, a Cas14 endonuclease, a Cas12a endonuclease, a Cas12b endonuclease, a Cas 12c endonuclease, a Cas12d endonuclease, a Cas12e endonuclease, a Cas13a endonuclease, a Cas13b endonuclease, a Cas13c endonuclease, or a Cas 13d endonuclease. In some embodiments, the class 2, type II Cas endonuclease is derived from an uncultivated microorganism. In some embodiments, the double-stranded deoxyribonucleic acid polynucleotide is a eukaryotic, plant, fungal, mammalian, rodent, or human double-stranded deoxyribonucleic acid polynucleotide.

In some embodiments: (a) the PAM comprises a sequence selected from the group consisting of SEQ ID NOs: 5512-5515 and SEQ ID NOs: 5527-5530; (b) the PAM comprises SEQ ID NO: 5516 or SEQ ID NO: 5531; (c) the PAM comprises SEQ ID NO: 5539; (d) the PAM comprises SEQ ID NO: 5517 or SEQ ID NO: 5518; (e) the PAM comprises SEQ ID NO: 5519; (f) the PAM comprises SEQ ID NO: 5520 or SEQ ID NO: 5535; (g) the PAM comprises SEQ ID NO: 5521 or SEQ ID NO: 5536; (h) the PAM comprises SEQ ID NO: 5522; (i) the PAM comprises SEQ ID NO: 5523 or SEQ ID NO: 5537; (j) the PAM comprises SEQ ID NO: 5524; (k) the PAM comprises SEQ ID NO: 5525; or (l) the PAM comprises SEQ ID NO: 5526.

In some aspects, the present disclosure provides for a method of modifying a target nucleic acid locus, the method comprising delivering to the target nucleic acid locus any of the engineered nuclease systems described herein, wherein the endonuclease is configured to form a complex with the engineered guide ribonucleic acid structure, and wherein the complex is configured such that upon binding of the complex to the target nucleic acid locus, the complex modifies the target nucleic locus. In some embodiments, modifying the target nucleic acid locus comprises binding, nicking, cleaving, or marking the target nucleic acid locus. In some embodiments, the target nucleic acid locus comprises deoxyribonucleic acid (DNA) or ribonucleic acid (RNA). In some embodiments, the target nucleic acid comprises genomic DNA, viral DNA, viral RNA, or bacterial DNA. In some embodiments, the target nucleic acid locus is in vitro. In some embodiments, the target nucleic acid locus is within a cell. In some embodiments, the cell is a prokaryotic cell, a bacterial cell, a eukaryotic cell, a fungal cell, a plant cell, an animal cell, a mammalian cell, a rodent cell, a primate cell, or a human cell.

In some embodiments, delivering the engineered nuclease system to the target nucleic acid locus comprises delivering any of the nucleic acids described herein or any of the vectors described herein. In some embodiments, delivering the engineered nuclease system to the target nucleic acid locus comprises delivering a nucleic acid comprising an open reading frame encoding the endonuclease. In some embodiments, the nucleic acid comprises a promoter to which the open reading frame encoding the endonuclease is operably linked. In some embodiments, the engineered nuclease system to the target nucleic acid locus comprises delivering a capped mRNA containing the open reading frame encoding the endonuclease. In some embodiments, the engineered nuclease system to the target nucleic acid locus comprises delivering a translated polypeptide. In some embodiments, the engineered nuclease system to the target nucleic acid locus comprises delivering a deoxyribonucleic acid (DNA) encoding the engineered guide ribonucleic acid structure operably linked to a ribonucleic acid (RNA) pol III promoter. In some embodiments, the endonuclease induces a single-stranded break or a double-stranded break at or proximal to the target locus.

In some aspects, the present disclosure provides for an engineered nuclease system comprising: (a) an endonuclease comprising a sequence having at least 75% sequence identity to any one of SEQ ID NOs: 5718-5846 or 6257; and (b) an engineered guide ribonucleic acid structure configured to form a complex with said endonuclease comprising: (i) a ribonucleic acid sequence configured to hybridize to a target deoxyribonucleic acid sequence; and (ii) a ribonucleic acid sequence configured to bind to said endonuclease. In some aspects, the present disclosure provides for an engineered nuclease system comprising: (a) an endonuclease configured to bind to a protospacer adjacent motif (PAM) sequence comprising SEQ ID NOs: 5847-5861 or 6258-6278, wherein said endonuclease is a class 2, type II Cas endonuclease; and (b) an engineered guide ribonucleic acid structure configured to form a complex with said endonuclease comprising: (i) a ribonucleic acid sequence configured to hybridize to a target deoxyribonucleic acid sequence; and (ii) a ribonucleic acid sequence configured to bind to said endonuclease. In some embodiments, said endonuclease is derived from an uncultivated microorganism. In some embodiments, said endonuclease has not been engineered to bind to a different PAM sequence. In some embodiments, said endonuclease is not a Cas9 endonuclease, a Cas14 endonuclease, a Cas12a endonuclease, a Cas12b endonuclease, a Cas 12c endonuclease, a Cas12d endonuclease, a Cas12e endonuclease, a Cas13a endonuclease, a Cas13b endonuclease, a Cas13c endonuclease, or a Cas 13d endonuclease. In some embodiments, said endonuclease has less than 80% identity to a Cas9 endonuclease. In some embodiments, said ribonucleic acid sequence comprises a sequence with at least 80% sequence identity to (a) any one of SEQ ID NOs: 5886-5887, 5891, 5893, or 5894; or (b) the non-degenerate nucleotides of any one of SEQ ID NOs: 5862-5885, 5888-5890, 5892, 5895-5896, or 6279-6301. In some aspects, the present disclosure provides for an engineered nuclease system comprising, (a) an engineered guide ribonucleic acid structure comprising: (i) a ribonucleic acid sequence configured to hybridize to a target deoxyribonucleic acid sequence; and (ii) a ribonucleic acid sequence configured to bind to an endonuclease, wherein said ribonucleic acid sequence comprises a sequence with at least 80% sequence identity (a) any one of SEQ ID NOs: 5886-5887, 5891, 5893, or 5894; or (b) the non-degenerate nucleotides of any one of SEQ ID NOs: 5862-5885, 5888-5890, 5892, 5895-5896, or 6279-6301; and a class 2, type II Cas endonuclease configured to bind to said engineered guide ribonucleic acid. In some embodiments, endonuclease is configured to bind to a protospacer adjacent motif (PAM) sequence selected from the group comprising SEQ ID NOs: 5847-5861 or 6258-6278. In some embodiments, said guide ribonucleic acid sequence is 15-24 nucleotides in length or 19-24 nucleotides in length. In some embodiments, said endonuclease comprises one or more nuclear localization sequences (NLSs) proximal to an N- or C-terminus of said endonuclease. In some embodiments, said NLS comprises a sequence selected from SEQ ID NOs: 5597-5612. In some embodiments, the system further comprises a single- or double-stranded DNA repair template comprising from 5' to 3': a first homology arm comprising a sequence of at least 20 nucleotides 5' to said target deoxyribonucleic acid sequence, a synthetic DNA sequence of at least 10 nucleotides, and a second homology arm comprising a sequence of at least 20 nucleotides 3' to said target sequence. In some embodiments, said first or second homology arm comprises a sequence of at least 40, 80, 120, 150, 200, 300, 500, or 1,000 nucleotides. In some embodiments, said sequence identity is determined by a BLASTP, CLUSTALW, MUSCLE, MAFFT, or CLUSTALW with the parameters of the Smith-Waterman homology search algorithm. In some embodiments, said sequence identity is determined by said BLASTP homology search algorithm using parameters of a wordlength (W) of 3, an expectation (E) of 10, and a BLOSUM62 scoring matrix setting gap costs at existence of 11, extension of 1, and using a conditional compositional score matrix adjustment.

In some aspects, the present disclosure provides for an engineered guide ribonucleic acid polynucleotide comprising: (a) a DNA-targeting segment comprising a nucleotide sequence that is complementary to a target sequence in a target DNA molecule; and (b) a protein-binding segment comprising two complementary stretches of nucleotides that hybridize to form a double-stranded RNA (dsRNA) duplex, wherein said two complementary stretches of nucleotides are covalently linked to one another with intervening nucleotides, and wherein said engineered guide ribonucleic acid polynucleotide is configured to form a complex with an endonuclease comprising sequence having at least 75% sequence identity to any one of SEQ ID NOs: 5718-5846 or 6257 and target said complex to said target sequence of said target DNA molecule. In some embodiments, said DNA-targeting segment is positioned 5' of both of said two complementary stretches of nucleotides.

In some aspects, the present disclosure provides for a deoxyribonucleic acid polynucleotide encoding any of the engineered guide ribonucleic acid polynucleotides described herein.

In some aspects, the present disclosure provides for a nucleic acid comprising an engineered nucleic acid sequence optimized for expression in an organism, wherein said nucleic acid encodes an endonuclease comprising a sequence having at least 75% sequence identity to any one of SEQ ID NOs: 5718-5846 or 6257. In some embodiments, said endonuclease comprises a sequence encoding one or more nuclear localization sequences (NLSs) proximal to an N- or C-terminus of said endonuclease. In some embodiments, said NLS comprises a sequence selected from SEQ ID NOs: 5597-5612. In some embodiments, said organism is prokaryotic, bacterial, eukaryotic, fungal, plant, mammalian, rodent, or human.

In some aspects, the present disclosure provides for a vector comprising any of the nucleic acids described herein. In some embodiments, the vector further comprises a nucleic acid encoding an engineered guide ribonucleic acid structure configured to form a complex with said endonuclease comprising: (a) a ribonucleic acid sequence configured to hybridize to a target deoxyribonucleic acid sequence; and (b) a ribonucleic acid sequence configured to bind to said endonuclease. In some embodiments, the vector is a plasmid, a minicircle, a CELiD, an adeno-associated virus (AAV) derived virion, or a lentivirus.

In some aspects, the present disclosure provides for a cell comprising any of the vectors described herein

In some aspects, the present disclosure provides for a method of manufacturing an endonuclease, comprising cultivating any of the cells described herein.

In some aspects, the present disclosure provides for a method for binding, cleaving, marking, or modifying a double-stranded deoxyribonucleic acid polynucleotide, comprising: contacting said double-stranded deoxyribonucleic acid polynucleotide with a class 2, type II Cas endonuclease in complex with an engineered guide ribonucleic acid structure configured to bind to said endonuclease and said double-stranded deoxyribonucleic acid polynucleotide; wherein said double-stranded deoxyribonucleic acid polynucleotide comprises a protospacer adjacent motif (PAM); and wherein said PAM comprises a sequence selected from the group consisting of SEQ ID NOs: 5847-5861 or 6258-6278. In some embodiments, said double-stranded deoxyribonucleic acid polynucleotide comprises a first strand comprising a sequence complementary to a sequence of said engineered guide ribonucleic acid structure and a second strand comprising said PAM. In some embodiments, said PAM is directly adjacent to the 3' end of said sequence complementary to said sequence of said engineered guide ribonucleic acid structure. In some embodiments, said class 2, type II Cas endonuclease is not a Cas9 endonuclease, a Cas14 endonuclease, a Cas12a endonuclease, a Cas12b endonuclease, a Cas 12c endonuclease, a Cas12d endonuclease, a Cas12e endonuclease, a Cas13a endonuclease, a Cas13b endonuclease, a Cas13c endonuclease, or a Cas 13d endonuclease. In some embodiments, said double-stranded deoxyribonucleic acid polynucleotide is a eukaryotic, plant, fungal, mammalian, rodent, or human double-stranded deoxyribonucleic acid polynucleotide.

In some aspects, the present disclosure provides for a method of modifying a target nucleic acid locus, said method comprising delivering to said target nucleic acid locus any of the engineered nuclease systems described herein, wherein said endonuclease is configured to form a complex with said engineered guide ribonucleic acid structure, and wherein said complex is configured such that upon binding of said complex to said target nucleic acid locus, said complex modifies said target nucleic locus. In some embodiments, said target nucleic acid locus comprises binding, nicking, cleaving, or marking said target nucleic acid locus. In some embodiments, said target nucleic acid locus comprises deoxyribonucleic acid (DNA) or ribonucleic acid (RNA). In some embodiments, said target nucleic acid comprises genomic DNA, viral DNA, viral RNA, or bacterial DNA. In some embodiments, said target nucleic acid locus is in vitro. In some embodiments, said target nucleic acid locus is within a cell. In some embodiments, said cell is a prokaryotic cell, a bacterial cell, a eukaryotic cell, a fungal cell, a plant cell, an animal cell, a mammalian cell, a rodent cell, a primate cell, or a human cell. In some embodiments, said engineered nuclease system to said target nucleic acid locus comprises delivering any of the nucleic acids described herein or any of the vectors described herein. In some embodiments, delivering said engineered nuclease system to said target nucleic acid locus comprises delivering a nucleic acid comprising an open reading frame encoding said endonuclease. In some embodiments, said nucleic acid comprises a promoter to which said open reading frame encoding said endonuclease is operably linked. In some embodiments, delivering said engineered nuclease system to said target nucleic acid locus comprises delivering a capped mRNA containing said open reading frame encoding said endonuclease. In some embodiments, delivering said engineered nuclease system to said target nucleic acid locus comprises delivering a translated polypeptide. In some embodiments, delivering said engineered nuclease system to said target nucleic acid locus comprises delivering a deoxyribonucleic acid (DNA) encoding said engineered guide ribonucleic acid structure operably linked to a ribonucleic acid (RNA) pol III promoter. In some embodiments, said endonuclease induces a single-stranded break or a double-stranded break at or proximal to said target locus.

In some aspects, the present disclosure provides for a method of editing a TRAC locus in a cell, comprising contacting to said cell (a) an RNA-guided endonuclease; and (b) an engineered guide RNA, wherein said engineered guide RNA is configured to form a complex with said endonuclease and said engineered guide RNA comprises a spacer sequence configured to hybridize to a region of said TRAC locus, wherein said engineered guide RNA comprises a targeting sequence having at least 80% identity, at least 82% identity, at least 84% identity, at least 86% identity, at least 88% identity, at least 90% identity, at least 91% identity, at least 92% identity, at least 93% identity, at least 94% identity, at least 95% identity, at least 96% identity, at least 97% identity, at least 98% identity, at least 99% identity, or at least 100% identity to at least 19, at least 20, at least 21, at least 22, at least 23, or at least 24 consecutive nucleotides of any one of SEQ ID NOs: 5950-5958 or 5959-5965. In some embodiments, said RNA-guided endonuclease is a class II, type II Cas endonuclease. In some embodiments, said RNA-guided endonuclease comprises a RuvCIII domain comprising a sequence having at least 75% identity, at least 80% identity, at least 82% identity, at least 84% identity, at least 86% identity, at least 88% identity, at least 90% identity, at least 91% identity, at least 92% identity, at least 93% identity, at least 94% identity, at least 95% identity, at least 96% identity, at least 97% identity, at least 98% identity, at least 99% identity, or at least 100% identity to SEQ ID NO: 2242 or SEQ ID NO: 2244. In some embodiments, said RNA-guided endonuclease further comprises an HNH domain. In some embodiments, said RNA-guided endonuclease comprises a sequence having at least 75% identity, at least 80% identity, at least 82% identity, at least 84% identity, at least 86% identity, at least 88% identity, at least 90% identity, at least 91% identity, at least 92% identity, at least 93% identity, at least 94% identity, at least 95% identity, at least 96% identity, at least 97% identity, at least 98% identity, at least 99% identity, or at least 100% identity to SEQ ID NO: 421 or SEQ ID NO: 423. In some embodiments, said engineered guide RNA comprises a targeting sequence having at least 85% identity to at least 18 consecutive nucleotides of any one of SEQ ID NOs: 5950-5958 and said endonuclease comprises a sequence having at least 75% identity to SEQ ID NO:421. In some embodiments, said engineered guide RNA comprises a targeting sequence having at least 85% identity to at least 18 consecutive nucleotides of any one of SEQ ID NOs: 5959-5965 and said endonuclease comprises a sequence having at least 75% identity to SEQ ID NO: 423. In some embodiments, said engineered guide RNA comprises a targeting sequence having at least 85% identity to at least 18 consecutive nucleotides of any one of SEQ ID NOs: 5953-5957. In some embodiments, said engineered guide RNA comprises a targeting sequence having at least 85% identity to at least 18 consecutive nucleotides of any one of SEQ ID NOs: 5960-5961 or 5963-5964.

In some aspects, the present disclosure provides for a method of editing a TRBC locus in a cell, comprising contacting to said cell (a) an RNA-guided endonuclease; and (b) an engineered guide RNA, wherein said engineered guide RNA is configured to form a complex with said endonuclease and said engineered guide RNA comprises a spacer sequence configured to hybridize to a region of said TRBC locus, wherein said engineered guide RNA comprises a targeting sequence having at least 80% identity, at least 82% identity, at least 84% identity, at least 86% identity, at least 88% identity, at least 90% identity, at least 91% identity, at least 92% identity, at least 93% identity, at least 94% identity, at least 95% identity, at least 96% identity, at least 97% identity, at least 98% identity, at least 99% identity, or at least 100% identity to at least 19, at least 20, at least 21, at least 22, at least 23, or at least 24 consecutive nucleotides of any one of SEQ ID NOs: 5966-6004 or 6005-6025. In some embodiments, said RNA-guided endonuclease is a class II, type II Cas endonuclease. In some embodiments, said RNA-guided endonuclease comprises a RuvCIII domain comprising a sequence having at least 75% identity, at least 80% identity, at least 82% identity, at least 84% identity, at least 86% identity, at least 88% identity, at least 90% identity, at least 91% identity, at least 92% identity, at least 93% identity, at least 94% identity, at least 95% identity, at least 96% identity, at least 97% identity, at least 98% identity, at least 99% identity, or at least 100% identity to SEQ ID NO: 2242 or SEQ ID NO: 2244. In some embodiments, said RNA-guided endonuclease further comprises an HNH domain. In some embodiments, said RNA-guided endonuclease comprises a sequence having at least 75% identity, at least 80% identity, at least 82% identity, at least 84% identity, at least 86% identity, at least 88% identity, at least 90% identity, at least 91% identity, at least 92% identity, at least 93% identity, at least 94% identity, at least 95% identity, at least 96% identity, at least 97% identity, at least 98% identity, at least 99% identity, or at least 100% identity to SEQ ID NO: 421 or SEQ ID NO: 423. In some embodiments, said engineered guide RNA comprises a targeting sequence having at least 85% identity to at least 18 consecutive nucleotides of any one of SEQ ID NOs: 5966-6004 and said endonuclease comprises a sequence having at least 75% identity to SEQ ID NO: 421. In some embodiments, said engineered guide RNA comprises a targeting sequence having at least 85% identity to at least 18 consecutive nucleotides of any one of SEQ ID NOs: 6005-6025 and said endonuclease comprises a sequence having at least 75% identity to SEQ ID NO: 423. In some embodiments, said engineered guide RNA comprises a targeting sequence having at least 85% identity to at least 18 consecutive nucleotides of any one of SEQ ID NOs: 5970, 5971, 5983, or 5984. In some embodiments, said engineered guide RNA comprises a targeting sequence having at least 85% identity to at least 18 consecutive nucleotides of any one of SEQ ID NOs: 6006, 6010, 6011, or 6012.

In some aspects, the present disclosure provides for a method of editing a GR (NR3C1) locus in a cell, comprising contacting to said cell (a) an RNA-guided endonuclease; and (b) an engineered guide RNA, wherein said engineered guide RNA is configured to form a complex with said endonuclease and said engineered guide RNA comprises a spacer sequence configured to hybridize to a region of said GR (NR3C1) locus, wherein said engineered guide RNA comprises a targeting sequence having at least 80% identity, at least 82% identity, at least 84% identity, at least 86% identity, at least 88% identity, at least 90% identity, at least 91% identity, at least 92% identity, at least 93% identity, at least 94% identity, at least 95% identity, at least 96% identity, at least 97% identity, at least 98% identity, at least 99% identity, or at least 100% identity to at least 19, at least 20, at least 21, at least 22, at least 23, or at least 24 consecutive nucleotides consecutive nucleotides of any one of SEQ ID NOs: 6026-6090 or 6091-6121. In some embodiments, said RNA-guided endonuclease is a class II, type II Cas endonuclease. In some embodiments, said RNA-guided endonuclease comprises a RuvCIII domain comprising a sequence having at least 75% identity, at least 80% identity, at least 82% identity, at least 84% identity, at least 86% identity, at least 88% identity, at least 90% identity, at least 91% identity, at least 92% identity, at least 93% identity, at least 94% identity, at least 95% identity, at least 96% identity, at least 97% identity, at least 98% identity, at least 99% identity, or at least 100% identity to SEQ ID NO: 2242 or SEQ ID NO: 2244. In some embodiments, said RNA-guided endonuclease further comprises an HNH domain. In some embodiments, said RNA-guided endonuclease comprises a sequence having at least 75% identity to SEQ ID NO: 421 or SEQ ID NO: 423. In some embodiments, said engineered guide RNA comprises a targeting sequence having at least 85% identity to at least 18 consecutive nucleotides of any one of SEQ ID NOs: 6026-6090 and said endonuclease comprises a sequence having at least 75% identity to SEQ ID NO: 421. In some embodiments, said engineered guide RNA comprises a targeting sequence having at least 85% identity to at least 18 consecutive nucleotides of any one of SEQ ID NOs: 6091-6121 and said endonuclease comprises a sequence having at least 75% identity to SEQ ID NO: 423. In some embodiments, said engineered guide RNA comprises a targeting sequence having at least 85% identity to at least 18 consecutive nucleotides of any one of SEQ ID NOs: 6027-6028, 6029, 6038, 6043, 6049, 6076, 6080, 6081, or 6086. In some embodiments, said engineered guide RNA comprises a targeting sequence having at least 85% identity to at least 18 consecutive nucleotides of any one of SEQ ID NOs: 6092, 6115, or 6119.

In some aspects, the present disclosure provides for a method of editing an AAVS1 locus in a cell, comprising contacting to said cell (a) an RNA-guided endonuclease; and (b) an engineered guide RNA, wherein said engineered guide RNA is configured to form a complex with said endonuclease and said engineered guide RNA comprises a spacer sequence configured to hybridize to a region of said AAVS1 locus, wherein said engineered guide RNA comprises a targeting sequence having at least 80% identity, at least 82% identity, at least 84% identity, at least 86% identity, at least 88% identity, at least 90% identity, at least 91% identity, at least 92% identity, at least 93% identity, at least 94% identity, at least 95% identity, at least 96% identity, at least 97% identity, at least 98% identity, at least 99% identity, or at least 100% identity to at least 19, at least 20, at least 21, at least 22, at least 23, or at least 24 consecutive nucleotides of any one of SEQ ID NOs: 6122-6152. In some embodiments, said RNA-guided endonuclease is a class II, type II Cas endonuclease. In some embodiments, said RNA-guided endonuclease comprises a RuvCIII domain comprising a sequence having at least 75% identity, at least 80% identity, at least 82% identity, at least 84% identity, at least 86% identity, at least 88% identity, at least 90% identity, at least 91% identity, at least 92% identity, at least 93% identity, at least 94% identity, at least 95% identity, at least 96% identity, at least 97% identity, at least 98% identity, at least 99% identity, or at least 100% identity to SEQ ID NO: 2242 or SEQ ID NO: 2244. In some embodiments, said RNA-guided endonuclease further comprises an HNH domain. In some embodiments, said RNA-guided endonuclease comprises a sequence having at least 75% identity, at least 80% identity, at least 82% identity, at least 84% identity, at least 86% identity, at least 88% identity, at least 90% identity, at least 91% identity, at least 92% identity, at least 93% identity, at least 94% identity, at least 95% identity, at least 96% identity, at least 97% identity, at least 98% identity, at least 99% identity, or at least 100% identity to SEQ ID NO: 421 or SEQ ID NO: 423. In some embodiments, said engineered guide RNA comprises a targeting sequence having at least 85% identity to at least 18 consecutive nucleotides of any one of SEQ ID NOs: 6122, 6125-6126, 6128, 6131, 6133, 6136, 6141, 6143, or 6148.

In some aspects, the present disclosure provides for a method of editing an TIGIT locus in a cell, comprising contacting to said cell (a) an RNA-guided endonuclease; and (b) an engineered guide RNA, wherein said engineered guide RNA is configured to form a complex with said endonuclease and said engineered guide RNA comprises a spacer sequence configured to hybridize to a region of said TIGIT locus, wherein said engineered guide RNA comprises a targeting sequence having at least 80% identity, at least 82% identity, at least 84% identity, at least 86% identity, at least 88% identity, at least 90% identity, at least 91% identity, at least 92% identity, at least 93% identity, at least 94% identity, at least 95% identity, at least 96% identity, at least 97% identity, at least 98% identity, at least 99% identity, or at least 100% identity to at least 19, at least 20, at least 21, at least 22, at least 23, or at least 24 consecutive nucleotides of any one of SEQ ID NOs: 6153-6181. In some embodiments, said RNA-guided endonuclease is a class II, type II Cas endonuclease. In some embodiments, said RNA-guided endonuclease comprises a sequence having at least 75% identity, at least 80% identity, at least 82% identity, at least 84% identity, at least 86% identity, at least 88% identity, at least 90% identity, at least 91% identity, at least 92% identity, at least 93% identity, at least 94% identity, at least 95% identity, at least 96% identity, at least 97% identity, at least 98% identity, at least 99% identity, or at least 100% identity to SEQ ID NO: 421 or SEQ ID NO: 423. In some embodiments, said RNA-guided endonuclease comprises a RuvCIII domain comprising a sequence having at least 75% identity, at least 80% identity, at least 82% identity, at least 84% identity, at least 86% identity, at least 88% identity, at least 90% identity, at least 91% identity, at least 92% identity, at least 93% identity, at least 94% identity, at least 95% identity, at least 96% identity, at least 97% identity, at least 98% identity, at least 99% identity, or at least 100% identity to SEQ ID NO: 2242 or SEQ ID NO: 2244. In some embodiments, said RNA-guided endonuclease further comprises an HNH domain. In some embodiments, said engineered guide RNA comprises a targeting sequence having at least 85% identity to at least 18 consecutive nucleotides of any one of SEQ ID NOs: 66155, 6159, 616, or 6172.

In some aspects, the present disclosure provides for a method of editing an CD38 locus in a cell, comprising contacting to said cell (a) an RNA-guided endonuclease; and (b) an engineered guide RNA, wherein said engineered guide RNA is configured to form a complex with said endonuclease and said engineered guide RNA comprises a spacer sequence configured to hybridize to a region of said CD38 locus, wherein said engineered guide RNA comprises a targeting sequence having at least 80% identity, at least 82% identity, at least 84% identity, at least 86% identity, at least 88% identity, at least 90% identity, at least 91% identity, at least 92% identity, at least 93% identity, at least 94% identity, at least 95% identity, at least 96% identity, at least 97% identity, at least 98% identity, at least 99% identity, or at least 100% identity to at least 19, at least 20, at least 21, at least 22, at least 23, or at least 24 consecutive nucleotides of any one of SEQ ID NOs: 6182-6248 or 6249-6256. In some embodiments, said RNA-guided endonuclease is a class II, type II Cas endonuclease. In some embodiments, said RNA-guided endonuclease comprises a RuvCIII domain comprising a sequence having at least 75% identity, at least 80% identity, at least 82% identity, at least 84% identity, at least 86% identity, at least 88% identity, at least 90% identity, at least 91% identity, at least 92% identity, at least 93% identity, at least 94% identity, at least 95% identity, at least 96% identity, at least 97% identity, at least 98% identity, at least 99% identity, or at least 100% identity to SEQ ID NO: 2242 or SEQ ID NO: 2244. In some embodiments, said RNA-guided endonuclease further comprises an HNH domain. In some embodiments, said RNA-guided endonuclease comprises a sequence having at least 75% identity, at least 80% identity, at least 82% identity, at least 84% identity, at least 86% identity, at least 88% identity, at least 90% identity, at least 91% identity, at least 92% identity, at least 93% identity, at least 94% identity, at least 95% identity, at least 96% identity, at least 97% identity, at least 98% identity, at least 99% identity, or at least 100% identity to SEQ ID NO: 421 or SEQ ID NO: 423. In some embodiments, said engineered guide RNA comprises a targeting sequence having at least 85% identity to at least 18 consecutive nucleotides of any one of SEQ ID NOs: 6182-6248 and said endonuclease comprises a sequence having at least 75% identity to SEQ ID NO: 421. In some embodiments, said engineered guide RNA comprises a targeting sequence having at least 85% identity to at least 18 consecutive nucleotides of any one of SEQ ID NOs: 6249-6256 and said endonuclease comprises a sequence having at least 75% identity to SEQ ID NO: 423. In some embodiments, said engineered guide RNA comprises a targeting sequence having at least 85% identity to at least 18 consecutive nucleotides of any one of SEQ ID NOs: 6182-6183, 6189, 6191, 6208, 6210, 6211, or 6215. In some embodiments, said engineered guide RNA comprises a targeting sequence having at least 85% identity to at least 18 consecutive nucleotides of SEQ ID NO: 6251.

In some embodiments of any of the methods for editing particular loci in cells above, said cell is a peripheral blood mononuclear cell, a T-cell, an NK cell, a hematopoietic stem cell (HSCT), or a B-cell, or any combination thereof.

In some aspects, the present disclosure provides for an engineered guide ribonucleic acid polynucleotide comprising: (a) a DNA-targeting segment comprising a nucleotide sequence that is complementary to a target sequence in a target DNA molecule; and (b) a protein-binding segment comprising two complementary stretches of nucleotides that hybridize to form a double-stranded RNA (dsRNA) duplex, wherein said two complementary stretches of nucleotides are covalently linked to one another with intervening nucleotides, and wherein said engineered guide ribonucleic acid polynucleotide is configured to form a complex with a class 2, type II Cas endonuclease and target said complex to said target sequence of said target DNA molecule, wherein said DNA-targeting segment comprises a sequence having at least 80% identity, at least 82% identity, at least 84% identity, at least 86% identity, at least 88% identity, at least 90% identity, at least 91% identity, at least 92% identity, at least 93% identity, at least 94% identity, at least 95% identity, at least 96% identity, at least 97% identity, at least 98% identity, at least 99% identity, or at least 100% identity to at least 19, at least 20, at least 21, at least 22, at least 23, or at least 24 consecutive nucleotides of any one of SEQ ID NOs: 5950-5965, 5966-6025, 6026-6121, 6122-6152, 6153-6181, or 6182-6256. In some embodiments, said protein-binding segment comprises a sequence having at least 85% identity to any one of SEQ ID NOs: 5466 or 6304.

In some aspects, the present disclosure provides for a system for generating an edited immune cell, comprising: (a) an RNA-guided endonuclease; (b) an engineered guide ribonucleic acid polynucleotide according to claim 97 configured to bind said RNA-guided endonuclease; and (c) a single- or double-stranded DNA repair template comprising first and second homology arms flanking a sequence encoding a chimeric antigen receptor (CAR). In some embodiments, said cell is a peripheral blood mononuclear cell, a T-cell, an NK cell, a hematopoietic stem cell (HSCT), or a B-cell, or any combination thereof. In some aspects, said RNA-guided endonuclease is a class II, type II Cas endonuclease. In some aspects, said RNA-guided endonuclease comprises a RuvCIII domain comprising a sequence having at least 75% identity, at least 80% identity, at least 82% identity, at least 84% identity, at least 86% identity, at least 88% identity, at least 90% identity, at least 91% identity, at least 92% identity, at least 93% identity, at least 94% identity, at least 95% identity, at least 96% identity, at least 97% identity, at least 98% identity, at least 99% identity, or at least 100% identity to SEQ ID NO: 2242 or SEQ ID NO: 2244. In some aspects, said RNA-guided endonuclease further comprises an HNH domain. In some aspects, said RNA-guided endonuclease comprises a sequence having at least 75% identity, at least 80% identity, at least 82% identity, at least 84% identity, at least 86% identity, at least 88% identity, at least 90% identity, at least 91% identity, at least 92% identity, at least 93% identity, at least 94% identity, at least 95% identity, at least 96% identity, at least 97% identity, at least 98% identity, at least 99% identity, or at least 100% identity to SEQ ID NO: 421 or SEQ ID NO: 423.

Additional aspects and advantages of the present disclosure will become readily apparent to those skilled in this art from the following detailed description, wherein only illustrative embodiments of the present disclosure are shown and described. As will be realized, the present disclosure is capable of other and different embodiments, and its several details are capable of modifications in various obvious respects, all without departing from the disclosure. Accordingly, the drawings and description are to be regarded as illustrative in nature, and not as restrictive.

### INCORPORATION BY REFERENCE

All publications, patents, and patent applications mentioned in this specification are herein incorporated by reference to the same extent as if each individual publication, patent, or patent application was specifically and individually indicated to be incorporated by reference.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings (also "Figure" and "FIG." herein), of which:

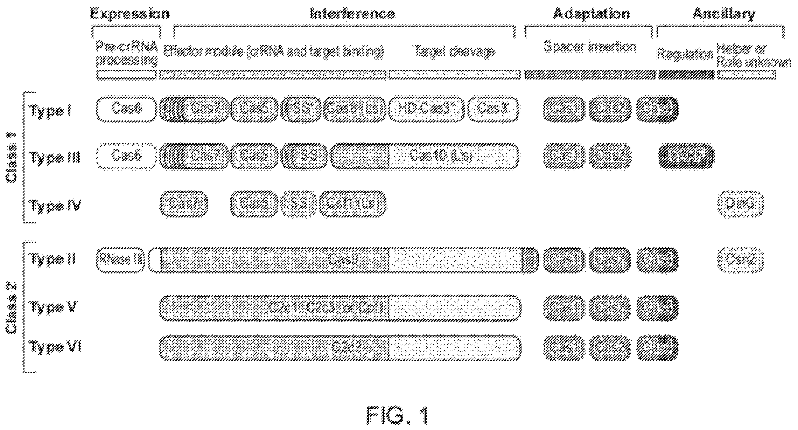

FIGURE 1 depicts typical organizations of CRISPR/Cas loci of different classes and types.
FIGURE 2 depicts the architecture of a natural Class2/Type II crRNA/tracrRNA pair, compared to a hybrid sgRNA wherein both are joined.

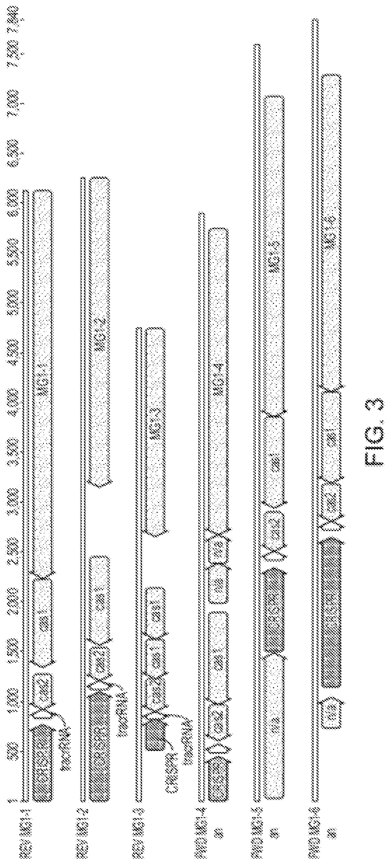

FIGURE 3 depicts schematics showing organization of CRISPR loci encoding enzymes from the MG1 family.

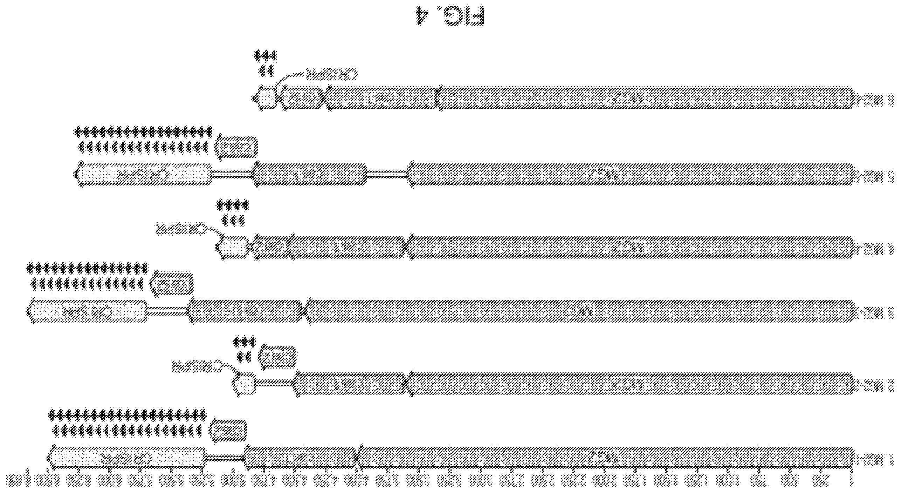

FIGURE 4 depicts schematics showing organization of CRISPR loci encoding enzymes from the MG2 family.

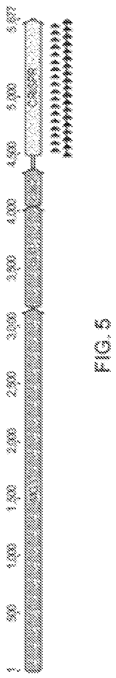

FIGURE 5 depicts schematics showing organization of CRISPR loci encoding enzymes from the MG3 family.
FIGURE 6 depicts a structure-based alignment of an enzyme of the present disclosure (MG1-1) versus Cas9 from Staphylococcus aureus (SEQ ID NO:5613). Predicted essential residues for function are called out below the sequence; conserved residues are highlighted in black.
FIGURE 7 depicts a structure-based alignment of an enzyme of the present disclosure (MG2-1) versus Cas9 from Staphylococcus aureus (SEQ ID NO:5613). Predicted essential residues for function are called out below the sequence; conserved residues are highlighted in black.
FIGURE 8 depicts a structure-based alignment of an enzyme of the present disclosure (MG3-1) versus Cas9 from Actinomyces naeslundii (SEQ ID NO: 5614). Predicted essential residues for function are called out below the sequence; conserved residues are highlighted in black.
FIGURES 9A, 9B, 9C, 9D, 9E, 9F, 9G, and 9H depicts a structure-based alignment of MG1 family enzymes MG1-1 through MG1-6 (SEQ ID NOs: 5, 6, 9, 1, 2, and 3). Predicted essential residues for function are called out below the sequence; conserved residues are highlighted in black.
FIGURE 10 depicts in vitro cleavage of DNA by MG1-4 in complex with its corresponding sgRNA containing targeting sequences of varying lengths.
FIGURE 11 depicts in cell cleavage of E. coli genomic DNA using MG1-4 along with its corresponding sgRNA. Shown are dilution series of cells transformed with MG1-4 along with target or non-target spacer (top); bottom panel shows the data quantitated, where the left bar represents non-target sgRNA and the right bar represents target sgRNA.
FIGURE 12 depicts in cell indel formation generated by transfection of HEK cells with MG1-4 or MG1-6 constructs described in Example 11 alongside their corresponding sgRNAs containing various different targeting sequences targeting various locations in the human genome.
FIGURE 13 depicts vitro cleavage of DNA by MG3-6 in complex with its corresponding sgRNA containing targeting sequences of varying lengths.
FIGURE 14 depicts in cell cleavage of E. coli genomic DNA using MG3-7 along with its corresponding sgRNA. Shown are dilution series of cells transformed with MG3-7 along with target or non-target spacer (top); bottom panel shows the data quantitated, where the left bar represents non-target sgRNA and the right bar represents target sgRNA.
FIGURE 15 depicts in cell indel formation generated by transfection of HEK cells with MG3-7 constructs described in Example 13 alongside their corresponding sgRNAs containing various different targeting sequences targeting various locations in the human genome.
FIGURE 16 depicts in vitro cleavage of DNA by MG15-1 in complex with its corresponding sgRNA containing targeting sequences of varying lengths.
FIGURES 17, 18, 19, and 20 depict agarose gels showing the results of PAM vector library cleavage in the presence of TXTL extracts containing various MG family nucleases and their corresponding tracrRNAs or sgRNAs.
FIGURES 21, 22, 23, 24, 25 and 26 depict predicted structures (predicted e.g., as in Example 7) of corresponding sgRNAs of MG enzymes described herein.

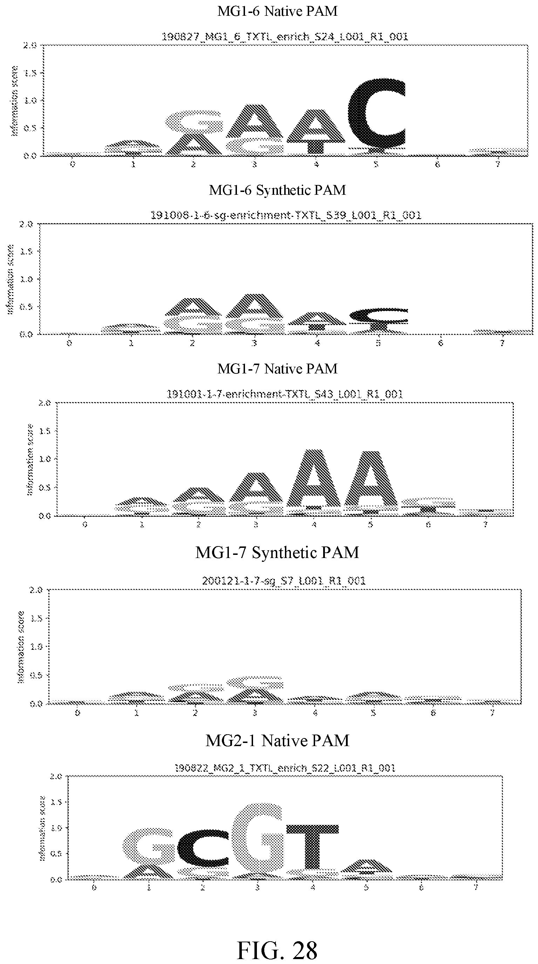

FIGURES 27, 28, 29, 30, 31, 32 and 33 depict seqLogo representations of PAM sequences derived via NGS as described herein (e.g. as described in Example 6).
FIGURE 34 depicts in cell cleavage of E. coli genomic DNA using MG2-7 along with its corresponding sgRNA. Shown are dilution series of cells transformed with MG2-7 along with target or non-target spacer (top); bottom panel shows the data quantitated, where the right bar represents non-target sgRNA and the left bar represents target sgRNA.
FIGURE 35 depicts in cell cleavage of E. coli genomic DNA using MG14-1 along with its corresponding sgRNA. Shown are dilution series of cells transformed with MG14-1 along with target or non-target spacer (top); bottom panel shows the data quantitated, where the right bar represents non-target sgRNA and the left bar represents target sgRNA.

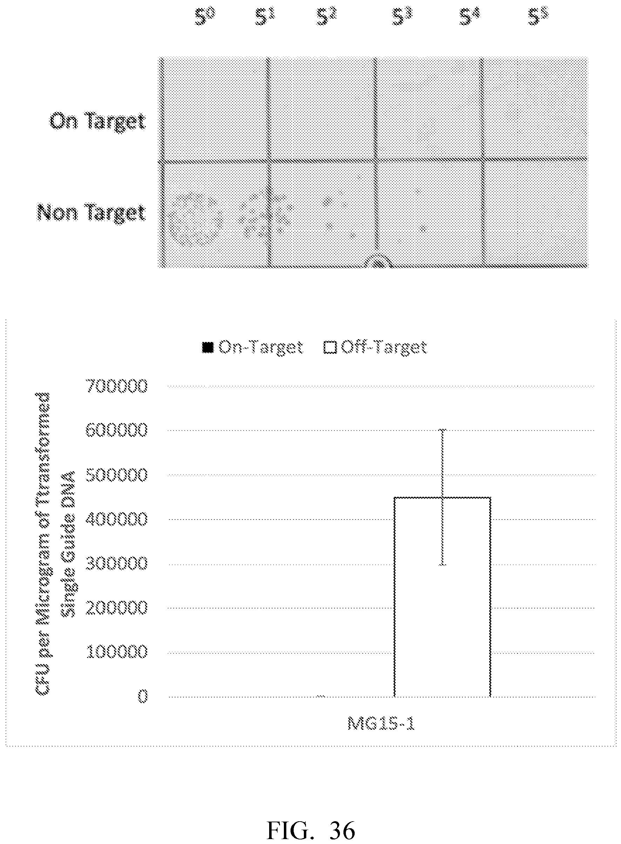

FIGURE 36 depicts in cell cleavage of E. coli genomic DNA using MG15-1 along with its corresponding sgRNA. Shown are dilution series of cells transformed with MG15-1 along with target or non-target spacer (top); bottom panel shows the data quantitated, where the right bar represents non-target sgRNA and the left bar represents target sgRNA.
FIGURE 37-39 depicts in cell indel formation generated by transfection of HEK cells with MG1-4, MG1-6 and MG1-7 constructs described in Example 11 alongside their corresponding sgRNAs containing various different targeting sequences targeting various locations in the human genome.
FIGURE 40-42 depicts in cell indel formation generated by transfection of HEK cells with MG3-6, MG3-7 and MG3-8 constructs described in Example 13 alongside their corresponding sgRNAs containing various different targeting sequences targeting various locations in the human genome.
FIGURE 43 depicts in cell indel formation generated by transfection of HEK cells with MG14-1 constructs described in Example 14 alongside their corresponding sgRNAs containing various different targeting sequences targeting various locations in the human genome.
FIGURE 44 depicts in cell indel formation generated by transfection of HEK cells with MG18-1 constructs described in Example 17 alongside their corresponding sgRNAs containing various different targeting sequences targeting various locations in the human genome.
FIGURE 45 depicts environmental distribution of nucleases described herein. Protein length is shown for representatives of selected protein families. Colors indicate the environment or environment type from which each protein was identified.
FIGURE 46 depicts predicted catalytic residues of nucleases described herein. Protein length is shown for representatives of selected protein families. Colors indicate the number of catalytic residues that were predicted for each protein. For the effector enzymes described herein, six catalytic residues were searched that correspond with the HNH and RuvC domains.

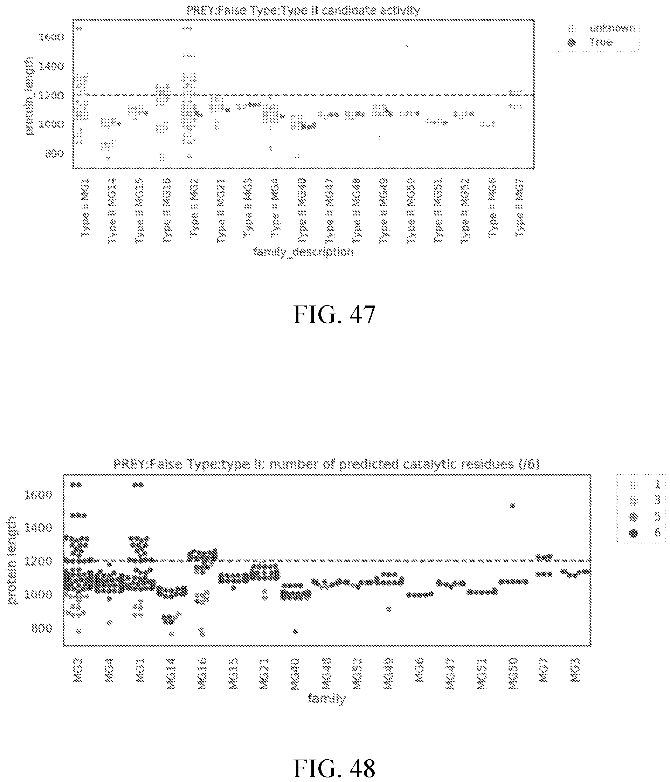

FIGURE 47 depicts candidate activity of nucleases described herein versus protein length.
FIGURE 48 depicts the number of catalytic residues predicted for nucleases described herein.

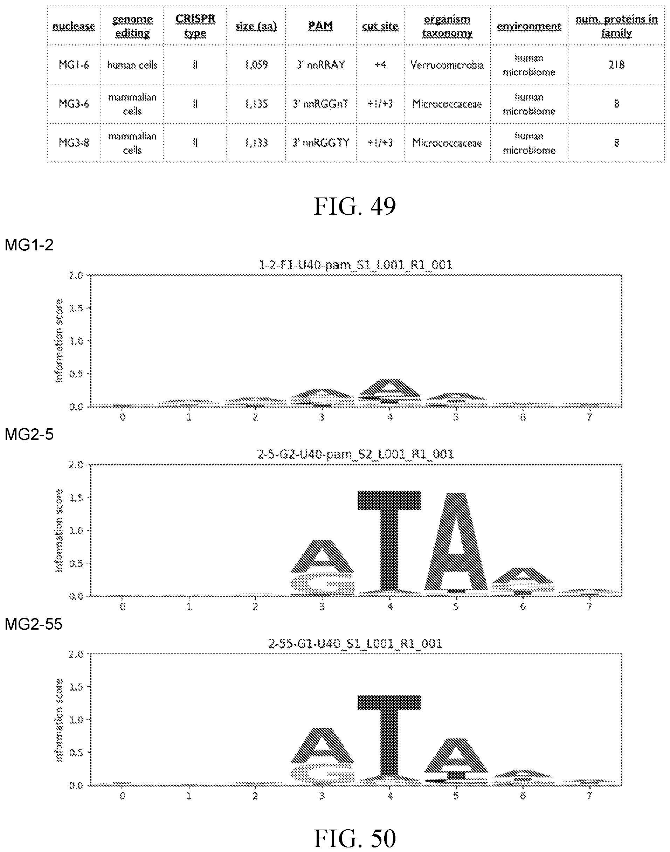

FIGURES 49 shows a table of various characteristic information of select nucleases described herein.

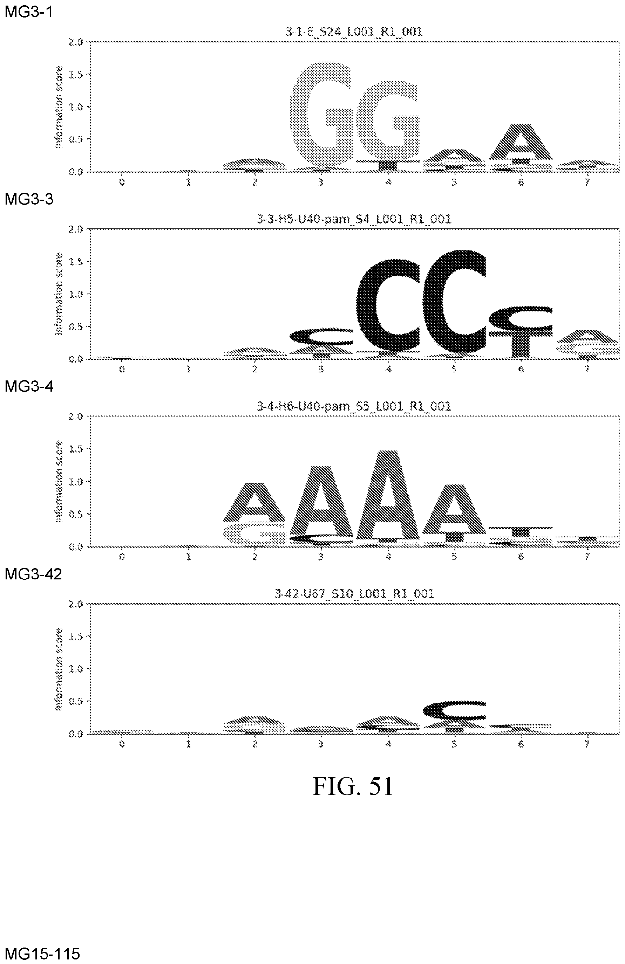

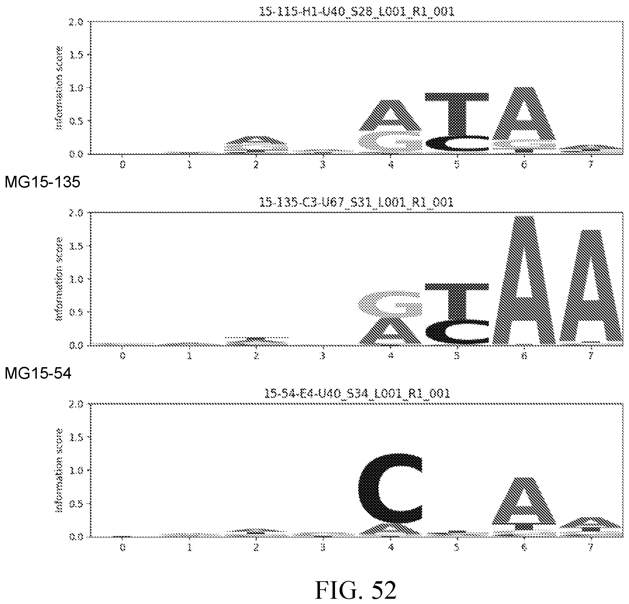

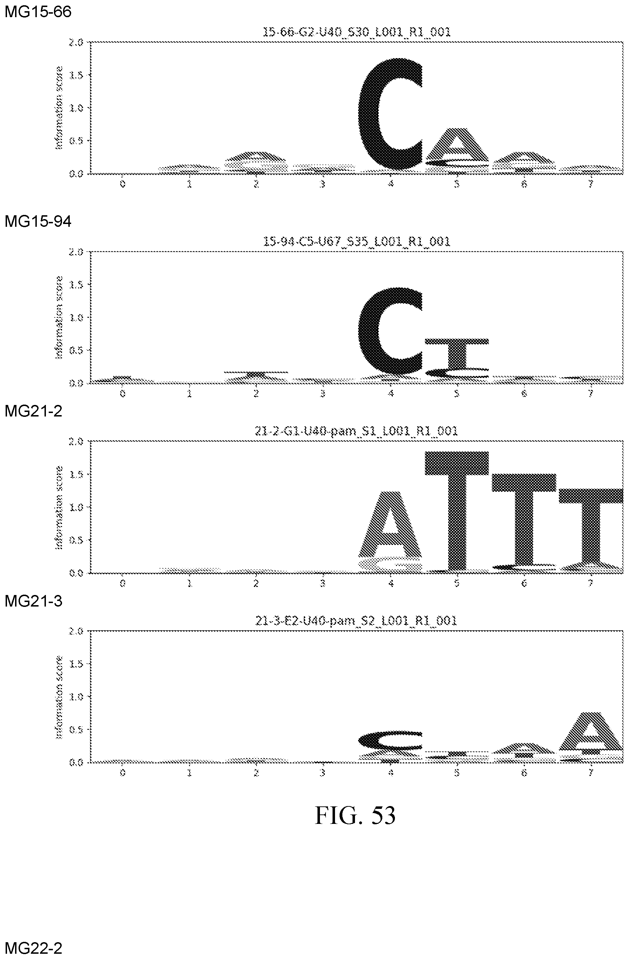

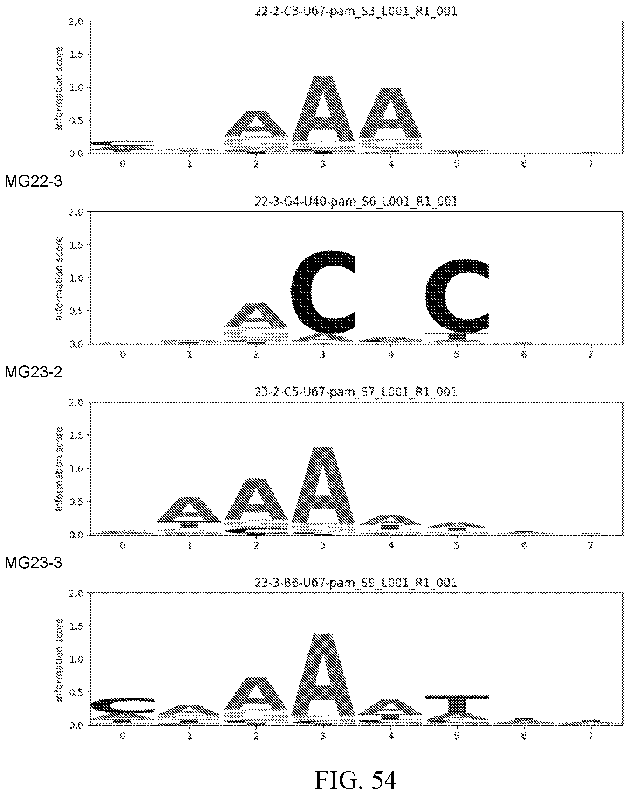

FIGURES 50-54 depict seqLogo representations of PAM sequences derived via NGS as described herein (e.g., as described in Example 6).

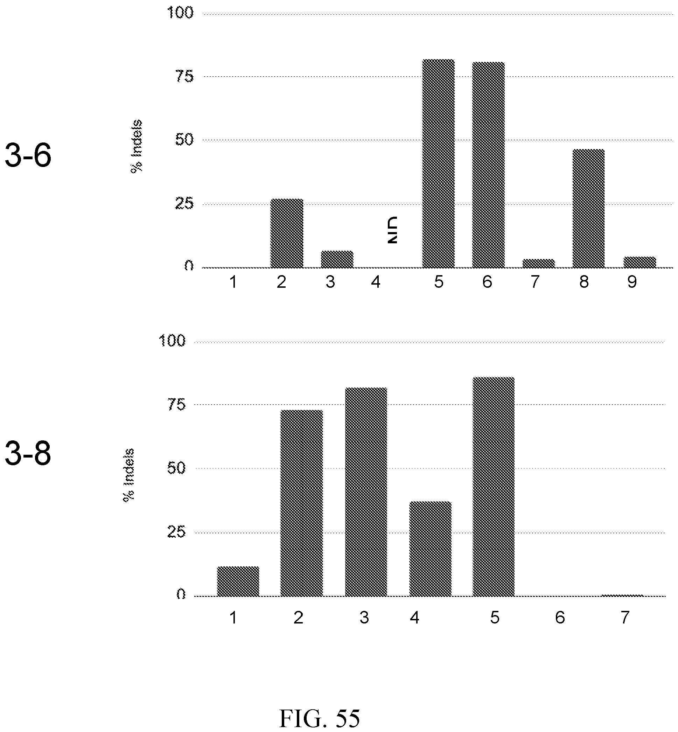

FIGURE 55 shows a guide RNA screen at TRAC with MG3-6 and MG3-8. For the top panel (MG3-6) the x-axis numbers refer to the spacers corresponding to SEQ ID NOs: 5950-5958; for the bottom panel (MG3-8) the x-axis numbers refer to the spacers corresponding to SEQ ID NOs: 5959-5965.
FIGURE 56 shows the activity (% indels) of MG3-6 with guide RNAs of various core sequences, lengths and doses.
FIGURE 57 shows the activity (% indels) of MG3-8 with guide RNAs of various sequences and lengths.
FIGURE 58 shows the activity (% indels) of MG3-6 with TRAC Guide 6 and MG3-8 with TRAC guide 8.
FIGURE 59 show the effect of MG3-6 with a TRAC6 guide RNA on T-cell receptor expression by flow cytometry. There were no changes in viability post-editing.
FIGURE 60 shows increased TRAC editing efficiency with higher amounts of gRNA.
FIGURE 61 shows how TCR expression may be eliminated and replaced with CAR expression.
FIGURE 62 shows targeted CAR integration with MG3-6.
FIGURE 63 shows GR (NR3Cl) editing by MG3-6 with various guide RNAs targeting various exons of the NR3Cl gene.
FIGURE 64 shows GR (NR3Cl) editing by MG3-8 with various guide RNAs targeting various exons of the NR3Cl gene.

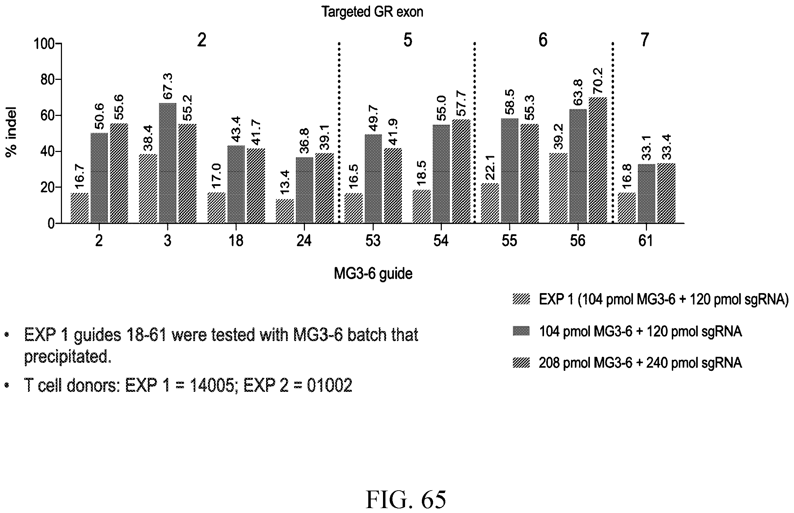

FIGURE 65 compares GR editing with two MG3-6 batches and various guide RNAs.
FIGURE 66 shows the process of how gene editing may be used to create an allogeneic CAR-NK cell.
FIGURE 67 shows TRAC editing using MG3-6 with a TRAC 6 guide RNA.
FIGURE 68 shows CAR expression (Y-axis) by MG3-6 in CD56+ NK cells by flow cytometry.
FIGURE 69 shows CD38 editing in primary NK cells using MG3-6 and MG3-8 with various guide RNAs.
FIGURE 70 shows TRAC editing in hematopoietic stem cells by MG3-6 and MG3-8 with various guide RNAs.
FIGURE 71 shows TRAC editing in B-cells by MG3-6 with TRAC guide 6 using two different buffers.
FIGURE 72 shows consensus PAM sequences for MG48-1 (A) and MG48-3 (B) determined by the method of Example 25.
FIGURE 73 illustrates RNAseq mapping with the sequenced tracr region highlighted, as performed by the method of Example 25 for MG48-1 (A) and MG48-3 (B).

### BRIEF DESCRIPTION OF THE SEQUENCE LISTING

The Sequence Listing filed herewith provides exemplary polynucleotide and polypeptide sequences for use in methods, compositions and systems according to the disclosure. Below are exemplary descriptions of sequences therein.
**MG1**
   SEQ ID NOs: 1-319 show the full-length peptide sequences of MG1 nucleases.
   SEQ ID NOs: 1827-2140 show the peptide sequences of RuvC_III domains of MG1 nucleases above.
   SEQ ID NOs: 3638-3955 show the peptide of HNH domains of MG1 nucleases above.
   SEQ ID NOs: 5476-5479 show the nucleotide sequences of MG1 tracrRNAs derived from the same loci as MG1 nucleases above (e.g., same loci as SEQ ID NO:1-4, respectively).
   SEQ ID NOs: 5461-5464 show the nucleotide sequences of sgRNAs engineered to function with an MG1 nuclease (e.g., SEQ ID NO:1-4, respectively), where Ns denote nucleotides of a targeting sequence.
   SEQ ID NOs: 5572-5575 show nucleotide sequences for E. coli codon-optimized coding sequences for MG1 family enzymes (SEQ ID NOs: 1-4).
   SEQ ID NOs: 5588-5589 show nucleotide sequences for human codon-optimized coding sequences for MG1 family enzymes (SEQ ID NOs: 1 and 3).
   SEQ ID NOs: 5616-5632 show peptide motifs characteristic of MG1 family enzymes.
**MG2**
   SEQ ID NOs: 320-420 show the full-length peptide sequences of MG2 nucleases.
   SEQ ID NOs: 2141-2241 show the peptide sequences of RuvC_III domains of MG2 nucleases above.
   SEQ ID NOs: 3955-4055 show the peptide of HNH domains of MG2 nucleases above.
   SEQ ID NOs: 5490-5494 show the nucleotide sequences of MG2 tracrRNAs derived from the same loci as MG2 nucleases above (e.g., same loci as SEQ ID NOs: 320, 321, 323, 325, and 326, respectively).
   SEQ ID NO: 5465 shows the nucleotide sequence of an sgRNA engineered to function with an MG2 nuclease (e.g., SEQ ID NO: 321 above).
   SEQ ID NOs: 5572-5575 show nucleotide sequences for E. coli codon-optimized coding sequences for MG2 family enzymes.
   SEQ ID NOs: 5631-5638 show peptide sequences characteristic of MG2 family enzymes.
**MG3**
   SEQ ID NOs: 421-431 show the full-length peptide sequences of MG3 nucleases.
   SEQ ID NOs: 2242-2252 show the peptide sequences of RuvC_III domains of MG3 nucleases above.
   SEQ ID NOs: 4056-4066 show the peptide of HNH domains of MG3 nucleases above.
   SEQ ID NOs: 5495-5502 show the nucleotide sequences of MG3 tracrRNAs derived from the same loci as MG3 nucleases above (e.g., same loci as SEQ ID NOs: 421-428, respectively).
   SEQ ID NOs: 5466-5467 show the nucleotide sequence of sgRNAs engineered to function with an MG3 nuclease (e.g., SEQ ID NOs: 421 - 423).
   SEQ ID NOs: 5578-5580 show nucleotide sequences for E. coli codon-optimized coding sequences for MG3 family enzymes.
   SEQ ID NOs: 5639-5648 show peptide sequences characteristic of MG3 family enzymes.
**MG4**
   SEQ ID NOs: 432-660 show the full-length peptide sequences of MG4 nucleases.
   SEQ ID NOs: 2253-2481 show the peptide sequences of RuvC_III domains of MG4 nucleases above.
   SEQ ID NOs: 4067-4295 show the peptide of HNH domains of MG4 nucleases above.
   SEQ ID NO: 5503 shows the nucleotide sequences of an MG4 tracrRNA derived from the same loci as MG4 nucleases above.
   SEQ ID NO: 5468 shows the nucleotide sequence of sgRNAs engineered to function with an MG4 nuclease.
   SEQ ID NO: 5649 shows a peptide sequence characteristic of MG4 family enzymes.
**MG6**
   SEQ ID NOs: 661-668 show the full-length peptide sequences of MG6 nucleases.
   SEQ ID NOs: 2482-2489 show the peptide sequences of RuvC_III domains of MG6 nucleases above.
   SEQ ID NOs: 4296-4303 show the peptide of HNH domains of MG3 nucleases above.
**MG7**
   SEQ ID NOs: 669-677 show the full-length peptide sequences of MG7 nucleases.
   SEQ ID NOs: 2490-2498 show the peptide sequences of RuvC_III domains of MG7 nucleases above.
   SEQ ID NOs: 4304-4312 show the peptide of HNH domains of MG3 nucleases above.
   SEQ ID NO: 5504 shows the nucleotide sequence of an MG7 tracrRNA derived from the same loci as MG7 nucleases above.
**MG14**
   SEQ ID NOs: 678-929 show the full-length peptide sequences of MG14 nucleases.
   SEQ ID NOs: 2499-2750 show the peptide sequences of RuvC_III domains of MG14 nucleases above.
   SEQ ID NOs: 4313-4564 show the peptide of HNH domains of MG14 nucleases above.
   SEQ ID NO: 5505 shows the nucleotide sequences of MG14 tracrRNA derived from the same loci as MG14 nucleases above.
   SEQ ID NO: 5581 shows a nucleotide sequence for an E. coli codon-optimized coding sequences for an MG14 family enzyme.
   SEQ ID NOs: 5650-5667 show peptide sequences characteristic of MG14 family enzymes.
**MG15**
   SEQ ID NOs: 930-1092 show the full-length peptide sequences of MG15 nucleases.
   SEQ ID NOs: 2751-2913 show the peptide sequences of RuvC_III domains of MG15 nucleases above.
   SEQ ID NOs: 4565-4727 show the peptide of HNH domains of MG15 nucleases above.
   SEQ ID NO: 5506 shows the nucleotide sequences of MG15 tracrRNA derived from the same loci as MG15 nucleases above.
   SEQ ID NOs: 5470 shows the nucleotide sequence of an sgRNA engineered to function with an MG15 nuclease.
   SEQ ID NO: 5582 shows a nucleotide sequence for an E. coli codon-optimized coding sequences for an MG15 family enzyme.
   SEQ ID NOs: 5668-5675 show peptide sequences characteristic of MG15 family enzymes.
**MG16**
   SEQ ID NOs: 1093-1353 show the full-length peptide sequences of MG16 nucleases.
   SEQ ID NOs: 2914-3174 show the peptide sequences of RuvC_III domains of MG16 nucleases above.
   SEQ ID NOs: 4728-4988 show the peptide of HNH domains of MG16 nucleases above.
   SEQ ID NOs: 5507 show the nucleotide sequences of an MG16 tracrRNA derived from the same loci as MG3 nucleases above.
   SEQ ID NOs: 5471 shows the nucleotide sequence of sgRNAs engineered to function with an MG16 nuclease.
   SEQ ID NO: 5583 shows a nucleotide sequence for an E. coli codon-optimized coding sequences for an MG16 family enzyme.
   SEQ ID NOs: 5676-5678 show peptide sequences characteristic of MG16 family enzymes.
**MG18**
   SEQ ID NOs: 1354-1511 show the full-length peptide sequences of MG18 nucleases.
   SEQ ID NOs: 3175-3330 show the peptide sequences of RuvC_III domains of MG18 nucleases above.
   SEQ ID NOs: 4989-5146 show the peptide of HNH domains of MG18 nucleases above.
   SEQ ID NO: 5508 shows the nucleotide sequences of MG18 tracrRNA derived from the same loci as MG18 nucleases above.
   SEQ ID NOs: 5472 shows the nucleotide sequence of an sgRNA engineered to function with an MG18 nuclease.
   SEQ ID NO: 5584 shows a nucleotide sequence for an E. coli codon-optimized coding sequences for an MG18 family enzyme.
   SEQ ID NOs: 5679-5686 show peptide sequences characteristic of MG18 family enzymes.
**MG21**
   SEQ ID NOs: 1512-1655 show the full-length peptide sequences of MG21 nucleases.
   SEQ ID NOs: 3331-3474 show the peptide sequences of RuvC_III domains of MG21 nucleases above.
   SEQ ID NOs: 5147-5290 show the peptide of HNH domains of MG21 nucleases above.
   SEQ ID NOs: 5509 show the nucleotide sequence of an MG21 tracrRNA derived from the same loci as MG21 nucleases above.
   SEQ ID NOs: 5473 shows the nucleotide sequence of an sgRNA engineered to function with an MG21 nuclease.
   SEQ ID NO: 5585 shows a nucleotide sequence for an E. coli codon-optimized coding sequences for an MG21 family enzyme.
   SEQ ID NOs: 5687-5692 and 5674-5675 show peptide sequences characteristic of MG21 family enzymes.
**MG22**
   SEQ ID NOs: 1656-1755 show the full-length peptide sequences of MG22 nucleases.
   SEQ ID NOs: 3475-3568 show the peptide sequences of RuvC_III domains of MG22 nucleases above.
   SEQ ID NOs: 5291-5389 show the peptide of HNH domains of MG22 nucleases above.
   SEQ ID NO: 5510 show the nucleotide sequence of an MG22 tracrRNA derived from the same loci as MG22 nucleases above.
   SEQ ID NOs: 5474 shows the nucleotide sequence of an sgRNAs engineered to function with an MG22 nuclease.
   SEQ ID NO: 5586 shows a nucleotide sequence for an E. coli codon-optimized coding sequences for an MG22 family enzyme.
   SEQ ID NOs: 5694-5699 show peptide sequences characteristic of MG22 family enzymes.
**MG23**
   SEQ ID NOs: 1756-1826 show the full-length peptide sequences of MG23 nucleases.
   SEQ ID NOs: 3569-3637 show the peptide sequences of RuvC_III domains of MG23 nucleases above.
   SEQ ID NOs: 5390-5460 show the peptide of HNH domains of MG23 nucleases above.
   SEQ ID NO: 5511 shows the nucleotide sequences of an MG23 tracrRNA derived from the same loci as MG23 nucleases above.
   SEQ ID NOs: 5475 shows the nucleotide sequence of an sgRNA engineered to function with an MG23 nuclease.
   SEQ ID NO: 5587 shows a nucleotide sequence for an E. coli codon-optimized coding sequences for an MG23 family enzyme.
   SEQ ID NOs: 5700-5717 show peptide sequences characteristic of MG23 family enzymes.
**MG40**
   SEQ ID NOs: 5718-5750 show the full-length peptide sequences of MG40 nucleases.
   SEQ ID NOs: 5847-5852 show protospacer adjacent motifs associated with MG 40 nucleases.
   SEQ ID NOs: 5862-5873 show the nucleotide sequence of an sgRNA engineered to function with an MG40 nuclease.
**MG47**
   SEQ ID NOs: 5751-5768 show the full-length peptide sequences of MG47 nucleases.
   SEQ ID NOs: 5853-5854 show protospacer adjacent motifs associated with MG47 nucleases.
   SEQ ID NOs: 5878-5881 show the nucleotide sequence of an sgRNA engineered to function with an MG47 nuclease.
**MG48**
   SEQ ID NOs: 5769-5804 show the full-length peptide sequences of MG48 nucleases.
   SEQ ID NOs: 5855-5856 show protospacer adjacent motifs associated with MG48 nucleases.
   SEQ ID NOs: 5886, 5890 and 5893 show the nucleotide sequences of MG48 tracrRNA derived from the same loci as MG48 nucleases above
   SEQ ID NOs: 5887, 5891 and 5894 show CRISPR repeats associated with MG48 nucleases described herein.
   SEQ ID NOs: 5888-5889, 5892 and 5895-5896 show putative sgRNA designed to function with an MG48 nuclease.
**MG49**
   SEQ ID NOs: 5805-5823 show the full-length peptide sequences of MG49 nucleases.
   SEQ ID NOs: 5857-5858 show protospacer adjacent motifs associated with MG49 nucleases.
   SEQ ID NOs: 5862-5873 show the nucleotide sequence of an sgRNA engineered to function with an MG40 nuclease.
   SEQ ID NOs: 5876-5877 show the nucleotide sequence of an sgRNA engineered to function with an MG49 nuclease.
**MG50**
   SEQ ID NOs: 5824-5826 show the full-length peptide sequences of MG50 nucleases.
   SEQ ID NO: 5859 shows a protospacer adjacent motif associated with MG50 nucleases.
   SEQ ID NOs: 5884-5885 show the nucleotide sequence of an sgRNA engineered to function with an MG50 nuclease.
**MG51**
   SEQ ID NOs: 5827-5830 show the full-length peptide sequences of MG51 nucleases.
   SEQ ID NO: 5860 shows a protospacer adjacent motif associated with MG51 nucleases.
   SEQ ID NOs: 5882-5883 show the nucleotide sequence of an sgRNA engineered to function with an MG51 nuclease.
**MG52**
   SEQ ID NOs: 5831-5846 show the full-length peptide sequences of MG52 nucleases.
   SEQ ID NO: 5861 shows a protospacer adjacent motif associated with MG52 nucleases.
   SEQ ID NOs: 5874-5875 show the nucleotide sequence of an sgRNA engineered to function with an MG42 nuclease.

### DETAILED DESCRIPTION

While various embodiments of the invention have been shown and described herein, it will be obvious to those skilled in the art that such embodiments are provided by way of example only. Numerous variations, changes, and substitutions may occur to those skilled in the art without departing from the invention. It should be understood that various alternatives to the embodiments of the invention described herein may be employed.

The practice of some methods disclosed herein employ, unless otherwise indicated, techniques of immunology, biochemistry, chemistry, molecular biology, microbiology, cell biology, genomics and recombinant DNA. See for example Sambrook and Green, Molecular Cloning: A Laboratory Manual, 4th Edition (2012); the series Current Protocols in Molecular Biology (F. M. Ausubel, et al. eds.); the series Methods In Enzymology (Academic Press, Inc.), PCR 2: A Practical Approach (M.J. MacPherson, B.D. Hames and G.R. Taylor eds. (1995)), Harlow and Lane, eds. (1988) Antibodies, A Laboratory Manual, and Culture of Animal Cells: A Manual of Basic Technique and Specialized Applications, 6th Edition (R.I. Freshney, ed. (2010)) (which is entirely incorporated by reference herein).

As used herein, the singular forms "a", "an" and "the" are intended to include the plural forms as well, unless the context clearly indicates otherwise. Furthermore, to the extent that the terms "including", "includes", "having", "has", "with", or variants thereof are used in either the detailed description and/or the claims, such terms are intended to be inclusive in a manner similar to the term "comprising".

The term "about" or "approximately" means within an acceptable error range for the particular value as determined by one of ordinary skill in the art, which will depend in part on how the value is measured or determined, i.e., the limitations of the measurement system. For example, "about" can mean within one or more than one standard deviation, per the practice in the art. Alternatively, "about" can mean a range of up to 20%, up to 15%, up to 10%, up to 5%, or up to 1% of a given value.

As used herein, a "cell" generally refers to a biological cell. A cell may be the basic structural, functional and/or biological unit of a living organism. A cell may originate from any organism having one or more cells. Some non-limiting examples include: a prokaryotic cell, eukaryotic cell, a bacterial cell, an archaeal cell, a cell of a single-cell eukaryotic organism, a protozoa cell, a cell from a plant (e.g., cells from plant crops, fruits, vegetables, grains, soy bean, corn, maize, wheat, seeds, tomatoes, rice, cassava, sugarcane, pumpkin, hay, potatoes, cotton, cannabis, tobacco, flowering plants, conifers, gymnosperms, ferns, clubmosses, hornworts, liverworts, mosses), an algal cell, (e.g.,, Botryococcus braunii, Chlamydomonas reinhardtii, Nannochloropsis gaditana, Chlorella pyrenoidosa, Sargassum patens C. Agardh, and the like), seaweeds (e.g., kelp), a fungal cell (e.g.,, a yeast cell, a cell from a mushroom), an animal cell, a cell from an invertebrate animal (e.g., fruit fly, cnidarian, echinoderm, nematode, etc.), a cell from a vertebrate animal (e.g., fish, amphibian, reptile, bird, mammal), a cell from a mammal (e.g., a pig, a cow, a goat, a sheep, a rodent, a rat, a mouse, a non-human primate, a human, etc.), and etcetera. Sometimes a cell is not originating from a natural organism (e.g., a cell can be a synthetically made, sometimes termed an artificial cell).

The term "nucleotide," as used herein, generally refers to a base-sugar-phosphate combination. A nucleotide may comprise a synthetic nucleotide. A nucleotide may comprise a synthetic nucleotide analog. Nucleotides may be monomeric units of a nucleic acid sequence (e.g., deoxyribonucleic acid (DNA) and ribonucleic acid (RNA)). The term nucleotide may include ribonucleoside triphosphates adenosine triphosphate (ATP), uridine triphosphate (UTP), cytosine triphosphate (CTP), guanosine triphosphate (GTP) and deoxyribonucleoside triphosphates such as dATP, dCTP, dITP, dUTP, dGTP, dTTP, or derivatives thereof. Such derivatives may include, for example, [αS]dATP, 7-deaza-dGTP and 7-deaza-dATP, and nucleotide derivatives that confer nuclease resistance on the nucleic acid molecule containing them. The term nucleotide as used herein may refer to dideoxyribonucleoside triphosphates (ddNTPs) and their derivatives. Illustrative examples of dideoxyribonucleoside triphosphates may include, but are not limited to, ddATP, ddCTP, ddGTP, ddITP, and ddTTP. A nucleotide may be unlabeled or detectably labeled, such as using moieties comprising optically detectable moieties (e.g., fluorophores). Labeling may also be carried out with quantum dots. Detectable labels may include, for example, radioactive isotopes, fluorescent labels, chemiluminescent labels, bioluminescent labels and enzyme labels. Fluorescent labels of nucleotides may include but are not limited fluorescein, 5-carboxyfluorescein (FAM), 2'7'-dimethoxy-4'5-dichloro-6-carboxyfluorescein (JOE), rhodamine, 6-carboxyrhodamine (R6G), N,N,N',N'-tetramethyl-6-carboxyrhodamine (TAMRA), 6-carboxy-X-rhodamine (ROX), 4-(4'dimethylaminophenylazo) benzoic acid (DABCYL), Cascade Blue, Oregon Green, Texas Red, Cyanine and 5-(2'-aminoethyl)aminonaphthalene-1-sulfonic acid (EDANS). Specific examples of fluorescently labeled nucleotides can include [R6G]dUTP, [TAMRA]dUTP, [R110]dCTP, [R6G]dCTP, [TAMRA]dCTP, [JOE]ddATP, [R6G]ddATP, [FAM]ddCTP, [R110]ddCTP, [TAMRA]ddGTP, [ROX]ddTTP, [dR6G]ddATP, [dR110]ddCTP, [dTAMRA]ddGTP, and [dROX]ddTTP available from Perkin Elmer, Foster City, Calif; FluoroLink DeoxyNucleotides, FluoroLink Cy3-dCTP, FluoroLink Cy5-dCTP, FluoroLink Fluor X-dCTP, FluoroLink Cy3-dUTP, and FluoroLink Cy5-dUTP available from Amersham, Arlington Heights, Ill.; Fluorescein-15-dATP, Fluorescein-12-dUTP, Tetramethyl-rodamine-6-dUTP, IR770-9-dATP, Fluorescein-12-ddUTP, Fluorescein-12-UTP, and Fluorescein-15-2'-dATP available from Boehringer Mannheim, Indianapolis, Ind.; and Chromosome Labeled Nucleotides, BODIPY-FL-14-UTP, BODIPY-FL-4-UTP, BODIPY-TMR-14-UTP, BODIPY-TMR-14-dUTP, BODIPY-TR-14-UTP, BODIPY-TR-14-dUTP, Cascade Blue-7-UTP, Cascade Blue-7-dUTP, fluorescein-12-UTP, fluorescein-12-dUTP, Oregon Green 488-5-dUTP, Rhodamine Green-5-UTP, Rhodamine Green-5-dUTP, tetramethylrhodamine-6-UTP, tetramethylrhodamine-6-dUTP, Texas Red-5-UTP, Texas Red-5-dUTP, and Texas Red-12-dUTP available from Molecular Probes, Eugene, Oreg. Nucleotides can also be labeled or marked by chemical modification. A chemically-modified single nucleotide can be biotin-dNTP. Some non-limiting examples of biotinylated dNTPs can include, biotin-dATP (e.g., bio-N6-ddATP, biotin-14-dATP), biotin-dCTP (e.g., biotin-11-dCTP, biotin-14-dCTP), and biotin-dUTP (e.g., biotin-11-dUTP, biotin-16-dUTP, biotin-20-dUTP).

The terms "polynucleotide," "oligonucleotide," and "nucleic acid" are used interchangeably to generally refer to a polymeric form of nucleotides of any length, either deoxyribonucleotides or ribonucleotides, or analogs thereof, either in single-, double-, or multi-stranded form. A polynucleotide may be exogenous or endogenous to a cell. A polynucleotide may exist in a cell-free environment. A polynucleotide may be a gene or fragment thereof. A polynucleotide may be DNA. A polynucleotide may be RNA. A polynucleotide may have any three-dimensional structure and may perform any function. A polynucleotide may comprise one or more analogs (e.g., altered backbone, sugar, or nucleobase). If present, modifications to the nucleotide structure may be imparted before or after assembly of the polymer. Some non-limiting examples of analogs include: 5-bromouracil, peptide nucleic acid, xeno nucleic acid, morpholinos, locked nucleic acids, glycol nucleic acids, threose nucleic acids, dideoxynucleotides, cordycepin, 7-deaza-GTP, fluorophores (e.g., rhodamine or fluorescein linked to the sugar), thiol containing nucleotides, biotin linked nucleotides, fluorescent base analogs, CpG islands, methyl-7-guanosine, methylated nucleotides, inosine, thiouridine, pseudourdine, dihydrouridine, queuosine, and wyosine. Non-limiting examples of polynucleotides include coding or non-coding regions of a gene or gene fragment, loci (locus) defined from linkage analysis, exons, introns, messenger RNA (mRNA), transfer RNA (tRNA), ribosomal RNA (rRNA), short interfering RNA (siRNA), short-hairpin RNA (shRNA), micro-RNA (miRNA), ribozymes, cDNA, recombinant polynucleotides, branched polynucleotides, plasmids, vectors, isolated DNA of any sequence, isolated RNA of any sequence, cell-free polynucleotides including cell-free DNA (cfDNA) and cell-free RNA (cfRNA), nucleic acid probes, and primers. The sequence of nucleotides may be interrupted by non-nucleotide components.

The terms "transfection" or "transfected" generally refer to introduction of a nucleic acid into a cell by non-viral or viral-based methods. The nucleic acid molecules may be gene sequences encoding complete proteins or functional portions thereof. See, e.g., Sambrook et al., 1989, Molecular Cloning: A Laboratory Manual, 18.1-18.88.

The terms "peptide," "polypeptide," and "protein" are used interchangeably herein to generally refer to a polymer of at least two amino acid residues joined by peptide bond(s). This term does not connote a specific length of polymer, nor is it intended to imply or distinguish whether the peptide is produced using recombinant techniques, chemical or enzymatic synthesis, or is naturally occurring. The terms apply to naturally occurring amino acid polymers as well as amino acid polymers comprising at least one modified amino acid. In some cases, the polymer may be interrupted by non-amino acids. The terms include amino acid chains of any length, including full length proteins, and proteins with or without secondary and/or tertiary structure (e.g., domains). The terms also encompass an amino acid polymer that has been modified, for example, by disulfide bond formation, glycosylation, lipidation, acetylation, phosphorylation, oxidation, and any other manipulation such as conjugation with a labeling component. The terms "amino acid" and "amino acids," as used herein, generally refer to natural and non-natural amino acids, including, but not limited to, modified amino acids and amino acid analogues. Modified amino acids may include natural amino acids and non-natural amino acids, which have been chemically modified to include a group or a chemical moiety not naturally present on the amino acid. Amino acid analogues may refer to amino acid derivatives. The term "amino acid" includes both D-amino acids and L-amino acids.

As used herein, the "non-native" can generally refer to a nucleic acid or polypeptide sequence that is not found in a native nucleic acid or protein. Non-native may refer to affinity tags. Non-native may refer to fusions. Non-native may refer to a naturally occurring nucleic acid or polypeptide sequence that comprises mutations, insertions and/or deletions. A non-native sequence may exhibit and/or encode for an activity (e.g., enzymatic activity, methyltransferase activity, acetyltransferase activity, kinase activity, ubiquitinating activity, etc.) that may also be exhibited by the nucleic acid and/or polypeptide sequence to which the non-native sequence is fused. A non-native nucleic acid or polypeptide sequence may be linked to a naturally-occurring nucleic acid or polypeptide sequence (or a variant thereof) by genetic engineering to generate a chimeric nucleic acid and/or polypeptide sequence encoding a chimeric nucleic acid and/or polypeptide.

The term "promoter", as used herein, generally refers to the regulatory DNA region which controls transcription or expression of a gene and which may be located adjacent to or overlapping a nucleotide or region of nucleotides at which RNA transcription is initiated. A promoter may contain specific DNA sequences which bind protein factors, often referred to as transcription factors, which facilitate binding of RNA polymerase to the DNA leading to gene transcription. A `basal promoter', also referred to as a `core promoter', may generally refer to a promoter that contains all the basic necessary elements to promote transcriptional expression of an operably linked polynucleotide. Eukaryotic basal promoters typically, though not necessarily, contain a TATA-box and/or a CAAT box.

The term "expression", as used herein, generally refers to the process by which a nucleic acid sequence or a polynucleotide is transcribed from a DNA template (such as into mRNA or other RNA transcript) and/or the process by which a transcribed mRNA is subsequently translated into peptides, polypeptides, or proteins. Transcripts and encoded polypeptides may be collectively referred to as "gene product." If the polynucleotide is derived from genomic DNA, expression may include splicing of the mRNA in a eukaryotic cell.

As used herein, "operably linked", "operable linkage", "operatively linked", or grammatical equivalents thereof generally refer to juxtaposition of genetic elements, e.g., a promoter, an enhancer, a polyadenylation sequence, etc., wherein the elements are in a relationship permitting them to operate in the expected manner. For instance, a regulatory element, which may comprise promoter and/or enhancer sequences, is operatively linked to a coding region if the regulatory element helps initiate transcription of the coding sequence. There may be intervening residues between the regulatory element and coding region so long as this functional relationship is maintained.

A "vector" as used herein, generally refers to a macromolecule or association of macromolecules that comprises or associates with a polynucleotide and which may be used to mediate delivery of the polynucleotide to a cell. Examples of vectors include plasmids, viral vectors, liposomes, and other gene delivery vehicles. The vector generally comprises genetic elements, e.g., regulatory elements, operatively linked to a gene to facilitate expression of the gene in a target.

As used herein, "an expression cassette" and "a nucleic acid cassette" are used interchangeably generally to refer to a combination of nucleic acid sequences or elements that are expressed together or are operably linked for expression. In some cases, an expression cassette refers to the combination of regulatory elements and a gene or genes to which they are operably linked for expression.

A "functional fragment" of a DNA or protein sequence generally refers to a fragment that retains a biological activity (either functional or structural) that is substantially similar to a biological activity of the full-length DNA or protein sequence. A biological activity of a DNA sequence may be its ability to influence expression in a manner known to be attributed to the full-length sequence.

As used herein, an "engineered" object generally indicates that the object has been modified by human intervention. According to non-limiting examples: a nucleic acid may be modified by changing its sequence to a sequence that does not occur in nature; a nucleic acid may be modified by ligating it to a nucleic acid that it does not associate with in nature such that the ligated product possesses a function not present in the original nucleic acid; an engineered nucleic acid may synthesized in vitro with a sequence that does not exist in nature; a protein may be modified by changing its amino acid sequence to a sequence that does not exist in nature; an engineered protein may acquire a new function or property. An "engineered" system comprises at least one engineered component.

As used herein, "synthetic" and "artificial" are used interchangeably to refer to a protein or a domain thereof that has low sequence identity (e.g., less than 50% sequence identity, less than 25% sequence identity, less than 10% sequence identity, less than 5% sequence identity, less than 1% sequence identity) to a naturally occurring human protein. For example, VPR and VP64 domains are synthetic transactivation domains.

The term "tracrRNA" or "tracr sequence", as used herein, can generally refer to a nucleic acid with at least about 5%, 10%, 20%, 30%, 40%, 50%, 60%, 70%, 80%, 90%, 95%, or 100% sequence identity and/or sequence similarity to a wild type exemplary tracrRNA sequence (e.g., a tracrRNA from S. pyogenes S. aureus, etc or SEQ ID NOs: 5476-5511). tracrRNA can refer to a nucleic acid with at most about 5%, 10%, 20%, 30%, 40%, 50%, 60%, 70%, 80%, 90%, or 100% sequence identity and/or sequence similarity to a wild type exemplary tracrRNA sequence (e.g., a tracrRNA from S. pyogenes S. aureus, etc). tracrRNA may refer to a modified form of a tracrRNA that can comprise a nucleotide change such as a deletion, insertion, or substitution, variant, mutation, or chimera. A tracrRNA may refer to a nucleic acid that can be at least about 60% identical to a wild type exemplary tracrRNA (e.g., a tracrRNA from S. pyogenes S. aureus, etc) sequence over a stretch of at least 6 contiguous nucleotides. For example, a tracrRNA sequence can be at least about 60% identical, at least about 65% identical, at least about 70% identical, at least about 75% identical, at least about 80% identical, at least about 85% identical, at least about 90% identical, at least about 95% identical, at least about 98% identical, at least about 99% identical, or 100 % identical to a wild type exemplary tracrRNA (e.g., a tracrRNA from S. pyogenes S. aureus, etc) sequence over a stretch of at least 6 contiguous nucleotides. Type II tracrRNA sequences can be predicted on a genome sequence by identifying regions with complementarity to part of the repeat sequence in an adjacent CRISPR array.

As used herein, a "guide nucleic acid" can generally refer to a nucleic acid that may hybridize to another nucleic acid. A guide nucleic acid may be RNA. A guide nucleic acid may be DNA. The guide nucleic acid may be programmed to bind to a sequence of nucleic acid site-specifically. The nucleic acid to be targeted, or the target nucleic acid, may comprise nucleotides. The guide nucleic acid may comprise nucleotides. A portion of the target nucleic acid may be complementary to a portion of the guide nucleic acid. The strand of a double-stranded target polynucleotide that is complementary to and hybridizes with the guide nucleic acid may be called the complementary strand. The strand of the double-stranded target polynucleotide that is complementary to the complementary strand, and therefore may not be complementary to the guide nucleic acid may be called noncomplementary strand. A guide nucleic acid may comprise a polynucleotide chain and can be called a "single guide nucleic acid." A guide nucleic acid may comprise two polynucleotide chains and may be called a "double guide nucleic acid." If not otherwise specified, the term "guide nucleic acid" may be inclusive, referring to both single guide nucleic acids and double guide nucleic acids. A guide nucleic acid may comprise a segment that can be referred to as a "nucleic acid-targeting segment" or a "nucleic acid-targeting sequence." A nucleic acid-targeting segment may comprise a sub-segment that may be referred to as a "protein binding segment" or "protein binding sequence" or "Cas protein binding segment".

The term "sequence identity" or "percent identity" in the context of two or more nucleic acids or polypeptide sequences, generally refers to two (e.g., in a pairwise alignment) or more (e.g., in a multiple sequence alignment) sequences that are the same or have a specified percentage of amino acid residues or nucleotides that are the same, when compared and aligned for maximum correspondence over a local or global comparison window, as measured using a sequence comparison algorithm. Suitable sequence comparison algorithms for polypeptide sequences include, e.g., BLASTP using parameters of a wordlength (W) of 3, an expectation I of 10, and the BLOSUM62 scoring matrix setting gap costs at existence of 11, extension of 1, and using a conditional compositional score matrix adjustment for polypeptide sequences longer than 30 residues; BLASTP using parameters of a wordlength (W) of 2, an expectation(E) of 1000000, and the PAM30 scoring matrix setting gap costs at 9 to open gaps and 1 to extend gaps for sequences of less than 30 residues (these are the default parameters for BLASTP in the BLAST suite available at https://blast.ncbi.nlm.nih.gov); CLUSTALW with parameters of; the Smith-Waterman homology search algorithm with parameters of a match of 2, a mismatch of-1, and a gap of -1; MUSCLE with default parameters; MAFFT with parameters retree of 2 and maxiterations of 1000; Novafold with default parameters; HMMER hmmalign with default parameters.

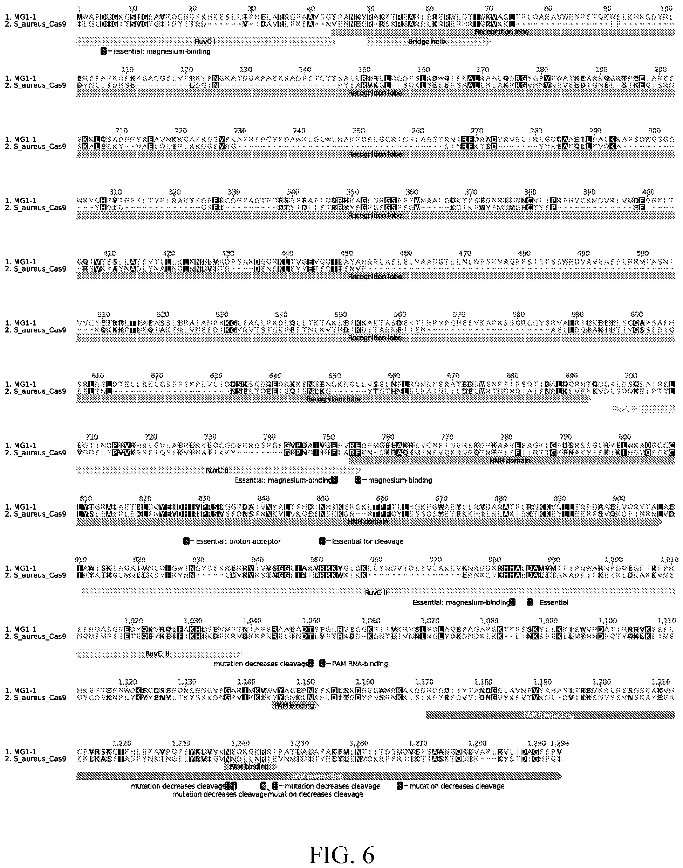

Included in the current disclosure are variants of any of the enzyme described herein with one or more conservative amino acid substitutions. Such conservative substitutions can be made in the amino acid sequence of a polypeptide without disrupting the three-dimensional structure or function of the polypeptide. Conservative substitutions can be accomplished by substituting amino acids with similar hydrophobicity, polarity, and R chain length for one another. Additionally or alternatively, by comparing aligned sequences of homologous proteins from different species, conservative substitutions can be identified by locating amino acid residues that have been mutated between species (e.g. non-conserved residues) without altering the basic functions of the encoded proteins. Such conservatively substituted variants may include variants with at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99% identity to any one of the endonuclease protein sequences described herein (e.g. MG1, MG2, MG3, MG4, MG6, MG7, MG14, MG15, MG16, MG18, MG21, MG22, or MG23 family endonucleases described herein). In some embodiments, such conservatively substituted variants are functional variants. Such functional variants can encompass sequences with substitutions such that the activity of critical active site residues of the endonuclease are not disrupted. In some embodiments, a functional variant of any of the proteins described herein lacks substitution of at least one of the conserved or functional residues called out in FIGURES 6, 7, 8, 9A, 9B, 9C, 9D, 9E, 9F, 9G, or 9H.. In some embodiments, a functional variant of any of the proteins described herein lacks substitution of all of the conserved or functional residues called out in in FIGURES 6, 7, 8, 9A, 9B, 9C, 9D, 9E, 9F, 9G, or 9H.

Conservative substitution tables providing functionally similar amino acids are available from a variety of references (see, for e.g., Creighton, Proteins: Structures and Molecular Properties (W H Freeman & Co.; 2nd edition (December 1993)). The following eight groups each contain amino acids that are conservative substitutions for one another:
1) Alanine (A), Glycine (G);
2) Aspartic acid (D), Glutamic acid (E);
3) Asparagine (N), Glutamine (Q);
4) Arginine (R), Lysine (K);
5) Isoleucine (I), Leucine (L), Methionine (M), Valine (V);
6) Phenylalanine (F), Tyrosine (Y), Tryptophan (W);
7) Serine (S), Threonine (T); and
8) Cysteine (C), Methionine (M)

As used herein, the term "RuvC_III domain" generally refers to a third discontinuous segment of a RuvC endonuclease domain (the RuvC nuclease domain being comprised of three discontiguous segments, RuvC_I, RuvC_II, and RuvC _III). A RuvC domain or segments thereof can generally be identified by alignment to known domain sequences, structural alignment to proteins with annotated domains, or by comparison to Hidden Markov Models (HMMs) built based on known domain sequences (e.g., Pfam HMM PF18541 for RuvC_III).

As used herein, the term "HNH domain" generally refers to an endonuclease domain having characteristic histidine and asparagine residues. An HNH domain can generally be identified by alignment to known domain sequences, structural alignment to proteins with annotated domains, or by comparison to Hidden Markov Models (HMMs) built based on known domain sequences (e.g., Pfam HMM PF01844 for domain HNH).

### Overview

The discovery of new Cas enzymes with unique functionality and structure may offer the potential to further disrupt deoxyribonucleic acid (DNA) editing technologies, improving speed, specificity, functionality, and ease of use. Relative to the predicted prevalence of Clustered Regularly Interspaced Short Palindromic Repeats (CRISPR) systems in microbes and the sheer diversity of microbial species, relatively few functionally characterized CRISPR/Cas enzymes exist in the literature. This is partly because a huge number of microbial species may not be readily cultivated in laboratory conditions. Metagenomic sequencing from natural environmental niches that represent large numbers of microbial species may offer the potential to drastically increase the number of new CRISPR/Cas systems known and speed the discovery of new oligonucleotide editing functionalities. A recent example of the fruitfulness of such an approach is demonstrated by the 2016 discovery of CasX/CasY CRISPR systems from metagenomic analysis of natural microbial communities.

CRISPR/Cas systems are RNA-directed nuclease complexes that have been described to function as an adaptive immune system in microbes. In their natural context, CRISPR/Cas systems occur in CRISPR (clustered regularly interspaced short palindromic repeats) operons or loci, which generally comprise two parts: (i) an array of short repetitive sequences (30-40bp) separated by equally short spacer sequences, which encode the RNA-based targeting element; and (ii) ORFs encoding the Cas encoding the nuclease polypeptide directed by the RNA-based targeting element alongside accessory proteins/enzymes. Efficient nuclease targeting of a particular target nucleic acid sequence generally requires both (i) complementary hybridization between the first 6-8 nucleic acids of the target (the target seed) and the crRNA guide; and (ii) the presence of a protospacer-adjacent motif (PAM) sequence within a defined vicinity of the target seed (the PAM usually being a sequence not commonly represented within the host genome). Depending on the exact function and organization of the system, CRISPR-Cas systems are commonly organized into 2 classes, 5 types and 16 subtypes based on shared functional characteristics and evolutionary similarity.

Class I CRISPR-Cas systems have large, multisubunit effector complexes, and comprise Types I, III, and IV.

Type I CRISPR-Cas systems are considered of moderate complexity in terms of components. In Type I CRISPR-Cas systems, the array of RNA-targeting elements is transcribed as a long precursor crRNA (pre-crRNA) that is processed at repeat elements to liberate short, mature crRNAs that direct the nuclease complex to nucleic acid targets when they are followed by a suitable short consensus sequence called a protospacer-adjacent motif (PAM). This processing occurs via an endoribonuclease subunit (Cas6) of a large endonuclease complex called Cascade, which also comprises a nuclease (Cas3) protein component of the crRNA-directed nuclease complex. Cas I nucleases function primarily as DNA nucleases.

Type III CRISPR systems may be characterized by the presence of a central nuclease, known as Cas10, alongside a repeat-associated mysterious protein (RAMP) that comprises Csm or Cmr protein subunits. Like in Type I systems, the mature crRNA is processed from a pre-crRNA using a Cas6-like enzyme. Unlike type I and II systems, type III systems appear to target and cleave DNA-RNA duplexes (such as DNA strands being used as templates for an RNA polymerase).

Type IV CRISPR-Cas systems possess an effector complex that consists of a highly reduced large subunit nuclease (csf1), two genes for RAMP proteins of the Cas5 (csf3) and Cas7 (csf2) groups, and, in some cases, a gene for a predicted small subunit; such systems are commonly found on endogenous plasmids.

Class II CRISPR-Cas systems generally have single-polypeptide multidomain nuclease effectors, and comprise Types II, V and VI.

Type II CRISPR-Cas systems are considered the simplest in terms of components. In Type II CRISPR-Cas systems, the processing of the CRISPR array into mature crRNAs does not require the presence of a special endonuclease subunit, but rather a small trans-encoded crRNA (tracrRNA) with a region complementary to the array repeat sequence; the tracrRNA interacts with both its corresponding effector nuclease (e.g. Cas9) and the repeat sequence to form a precursor dsRNA structure, which is cleaved by endogenous RNAse III to generate a mature effector enzyme loaded with both tracrRNA and crRNA. Cas II nucleases are known as DNA nucleases. Type 2 effectors generally exhibit a structure consisting of a RuvC-like endonuclease domain that adopts the RNase H fold with an unrelated HNH nuclease domain inserted within the folds of the RuvC-like nuclease domain. The RuvC-like domain is responsible for the cleavage of the target (e.g., crRNA complementary) DNA strand, while the HNH domain is responsible for cleavage of the displaced DNA strand.

Type V CRISPR-Cas systems are characterized by a nuclease effector (e.g. Cas12) structure similar to that of Type II effectors, comprising a RuvC-like domain. Similar to Type II, most (but not all) Type V CRISPR systems use a tracrRNA to process pre-crRNAs into mature crRNAs; however, unlike Type II systems which requires RNAse III to cleave the pre-crRNA into multiple crRNAs, type V systems are capable of using the effector nuclease itself to cleave pre-crRNAs. Like Type-II CRISPR-Cas systems, Type V CRISPR-Cas systems are again known as DNA nucleases. Unlike Type II CRISPR-Cas systems, some Type V enzymes (e.g., Cas12a) appear to have a robust single-stranded nonspecific deoxyribonuclease activity that is activated by the first crRNA directed cleavage of a double-stranded target sequence.

Type VI CRIPSR-Cas systems have RNA-guided RNA endonucleases. Instead of RuvC-like domains, the single polypeptide effector of Type VI systems (e.g. Cas13) comprises two HEPN ribonuclease domains. Differing from both Type II and V systems, Type VI systems also appear to not need a tracrRNA for processing of pre-crRNA into crRNA. Similar to type V systems, however, some Type VI systems (e.g., C2C2) appear to possess robust single-stranded nonspecific nuclease (ribonuclease) activity activated by the first crRNA directed cleavage of a target RNA.

Because of their simpler architecture, Class II CRISPR-Cas have been most widely adopted for engineering and development as designer nuclease/genome editing applications.

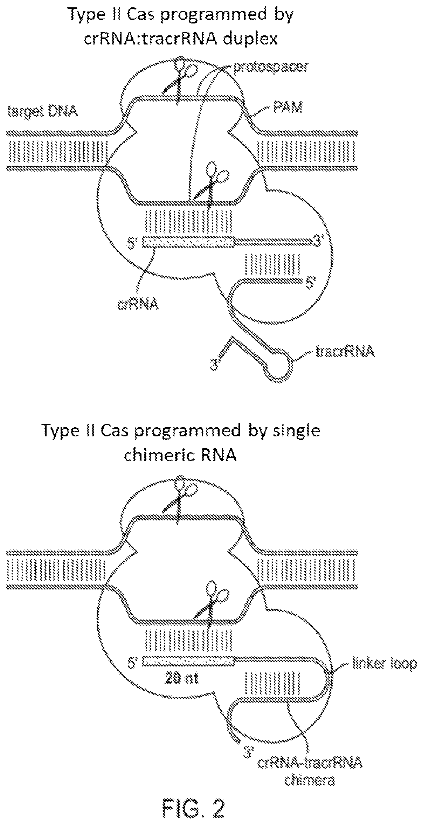

One of the early adaptations of such a system for in vitro use can be found in Jinek et al. (Science. 2012 Aug 17;337(6096):816-21, which is entirely incorporated herein by reference). The Jinek study first described a system that involved (i) recombinantly-expressed, purified full-length Cas9 (e.g., a Class II, Type II Cas enzyme) isolated from *S. pyogenes* SF370, (ii) purified mature ~42 nt crRNA bearing a ~20 nt 5' sequence complementary to the target DNA sequence desired to be cleaved followed by a 3' tracr-binding sequence (the whole crRNA being in vitro transcribed from a synthetic DNA template carrying a T7 promoter sequence); (iii) purified tracrRNA in vitro transcribed from a synthetic DNA template carrying a T7 promoter sequence, and (iv) Mg2+. Jinek later described an improved, engineered system wherein the crRNA of (ii) is joined to the 5' end of (iii) by a linker (e.g., GAAA) to form a single fused synthetic guide RNA (sgRNA) capable of directing Cas9 to a target by itself (compare top and bottom panel of FIGURE 2).

Mali et al. (Science. 2013 Feb 15; 339(6121): 823-826.), which is entirely incorporated herein by reference, later adapted this system for use in mammalian cells by providing DNA vectors encoding (i) an ORF encoding codon-optimized Cas9 (e.g., a Class II, Type II Cas enzyme) under a suitable mammalian promoter with a C-terminal nuclear localization sequence (e.g., SV40 NLS) and a suitable polyadenylation signal (e.g., TK pA signal); and (ii) an ORF encoding an sgRNA (having a 5' sequence beginning with G followed by 20 nt of a complementary targeting nucleic acid sequence joined to a 3' tracr-binding sequence, a linker, and the tracrRNA sequence) under a suitable Polymerase III promoter (e.g., the U6 promoter).

### MG Enzymes

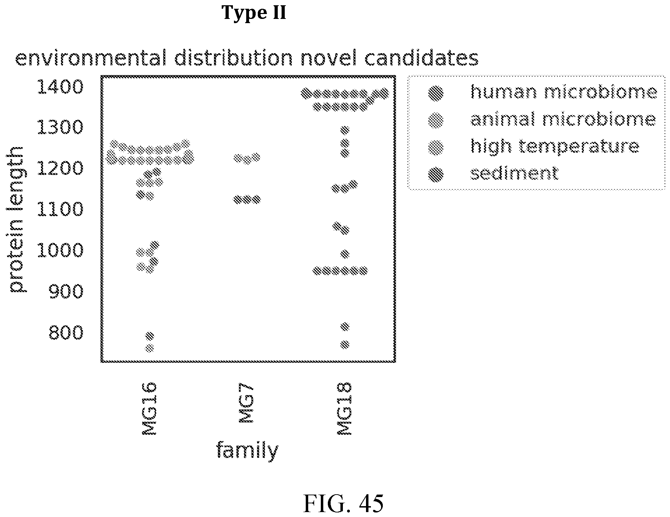

In one aspect, the present disclosure provides for an engineered nuclease system discovered through metagenomic sequencing. In some cases, the metagenomic sequencing is conducted on samples. In some cases, the samples may be collected by a variety of environments. Such environments may be a human microbiome, an animal microbiome, environments with high temperatures, environments with low temperatures. Such environments may include sediment. An example of the types of such environments of the engineered nuclease systems described herein may be found in Figure 45.

### MG1 Enzymes

In one aspect, the present disclosure provides for an engineered nuclease system comprising (a) an endonuclease. In some cases, the endonuclease is a Cas endonuclease. In some cases, the endonuclease is a Type II, Class II Cas endonuclease. The endonuclease may comprise a RuvC_III domain, wherein said RuvC_III domain has at least about 70% sequence identity to any one of SEQ ID NOs: 1827-2140. In some cases, the endonuclease may comprise a RuvC_III domain, wherein the RuvC_III domain has at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99% identity to any one of SEQ ID NOs: 1827-2140. In some cases, the endonuclease may comprise a RuvC_III domain, wherein the substantially identical to any one of SEQ ID NOs: 1827-2140. The endonuclease may comprise a RuvC_III domain having at least about 70% sequence identity to any one of SEQ ID NOs: 1827-1831. In some cases, the endonuclease may comprise a RuvC_III domain having at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99% identity to any one of SEQ ID NOs: 1827-1831. In some cases, the endonuclease may comprise a RuvC_III domain substantially identical to any one of SEQ ID NOs: 1827-1831. In some cases, the endonuclease may comprise a RuvC_III domain having at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99% identity to SEQ ID NO: 1827. In some cases, the endonuclease may comprise a RuvC_III domain having at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99% identity to SEQ ID NO: 1828. In some cases, the endonuclease may comprise a RuvC_III domain having at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99% identity to SEQ ID NO: 1829. In some cases, the endonuclease may comprise a RuvC_III domain having at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99% identity to SEQ ID NO: 1830. In some cases, the endonuclease may comprise a RuvC_III domain having at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99% identity to SEQ ID NO: 1831.

The endonuclease may comprise an HNH domain having at least about 70% identity to any one of SEQ ID NOs: 3638-3955. In some cases, the endonuclease may comprise an HNH domain having at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99% identical to any one of SEQ ID NOs: 3638-3955. The endonuclease may comprise an HNH domain substantially identical to any one of SEQ ID NOs: 3638-3955. The endonuclease may comprise an HNH domain having at least about 70% identity to any one of SEQ ID NOs: 3638-3955. In some cases, the endonuclease may comprise an HNH domain having at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99% identical to any one of SEQ ID NOs: 3638-3955. The endonuclease may comprise an HNH domain substantially identical to any one of SEQ ID NOs: 3638-3955. The endonuclease may comprise an HNH domain having at least about 70% identity to any one of SEQ ID NOs: 3638-3641. In some cases, the endonuclease may comprise an HNH domain having at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99% identical to any one of SEQ ID NOs: 3638-3641. The endonuclease may comprise an HNH domain substantially identical to any one of SEQ ID NOs: 3638-3641. The endonuclease may comprise an HNH domain having at least about 70% identity to any one of SEQ ID NOs: 3638. In some cases, the endonuclease may comprise an HNH domain having at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99% identical to any one of SEQ ID NOs: 3638. The endonuclease may comprise an HNH domain substantially identical to any one of SEQ ID NOs: 3638. The endonuclease may comprise an HNH domain having at least about 70% identity to any one of SEQ ID NOs: 3639. In some cases, the endonuclease may comprise an HNH domain having at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99% identical to any one of SEQ ID NOs: 3639. The endonuclease may comprise an HNH domain substantially identical to any one of SEQ ID NOs: 3639. The endonuclease may comprise an HNH domain having at least about 70% identity to any one of SEQ ID NOs: 3640. In some cases, the endonuclease may comprise an HNH domain having at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99% identical to any one of SEQ ID NOs: 3640. The endonuclease may comprise an HNH domain substantially identical to any one of SEQ ID NOs: 3640. The endonuclease may comprise an HNH domain having at least about 70% identity to any one of SEQ ID NOs: 3641. In some cases, the endonuclease may comprise an HNH domain having at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99% identical to any one of SEQ ID NOs: 3641. The endonuclease may comprise an HNH domain substantially identical to any one of SEQ ID NOs: 3641.

In some cases, the endonuclease may comprise a variant having at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% identity to any one of SEQ ID NOs: 1-6 or 9-319. In some cases, the endonuclease may be substantially identical to any one of SEQ ID NOs: 1-6 or 9-319. In some cases, the endonuclease may comprise a variant having at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% identity to any one of SEQ ID NOs: 1-4. In some cases, the endonuclease may be substantially identical to any one of SEQ ID NOs: 1-4. In some cases, the endonuclease may comprise a peptide motif substantially identical to any one of SEQ ID NOs: 5615, 5616, or 5617.

In some cases, the endonuclease may comprise a variant having one or more nuclear localization sequences (NLSs). The NLS may be proximal to the N- or C-terminus of said endonuclease. The NLS may be appended N-terminal or C-terminal to any one of SEQ ID NOs: 1-6 or 9-319, or to a variant having at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% identity to any one of SEQ ID NOs: 1-319. The NLS may be an SV40 large T antigen NLS. The NLS may be a c-myc NLS. The NLS can comprise a sequence with at least about 80%, at least about 85%, at least about 90%, at least about 95%, at least about 99% identity to any one of SEQ ID NOs: 5593-5608. The NLS can comprise a sequence substantially identical to any one of SEQ ID NOs: 5593-5608. The NLS can comprise any of the sequences in Table 1 below, or a combination thereof:

**Table 1: Example NLS Sequences that can be used with Cas Effectors According to the Disclosure**

| **Source** | **NLS amino acid sequence** | **SEQ ID NO:** |
|---|---|---|
| SV40 | PKKKRKV | 5597 |
| nucleoplasmin bipartite NLS | KRPAATKKAGQAKKKK | 5598 |
| c-myc NLS | PAAKRVKLD | 5599 |
| c-myc NLS | RQRRNELKRSP | 5600 |
| hRNPA1 M9 NLS | NQSSNFGPMKGGNFGGRSSGPYGGGGQYFA KPRNQGGY | 5601 |
| Importin-alpha IBB domain | RMRIZFKNKGKDTAELRRRRVEVSVELRKAK KDEQILKRRNV | 5602 |
| Myoma T protein | VSRKRPRP | 5603 |
| Myoma T protein | PPKKARED | 5604 |
| p53 | PQPKKKPL | 5605 |
| mouse c-abl IV | SALIKKKKKMAP | 5606 |
| influenza virus NS1 | DRLRR | 5607 |
| influenza virus NS1 | PKQKKRK | 5608 |
| Hepatitis virus delta antigen | RKLKKKIKKL | 5609 |
| mouse Mx1 protein | REKKKFLKRR | 5610 |
| human poly(ADP-ribose) polymerase | KRKGDEVDGVDEVAKKKSKK | 5611 |
| steroid hormone receptor (human) glucocorticoid | RKCLQAGMNLEARKTKK | 5612 |

In some cases, the endonuclease may be recombinant (e.g., cloned, expressed, and purified by a suitable method such as expression in E. coli followed by epitope-tag purification). In some cases, the endonuclease may be derived from a bacterium with a 16S rRNA gene having at least about 90% identity to any one of SEQ ID NOs: 5592-5595. The endonuclease may be derived from a species having a 16S rRNA gene at least about 80%, at least about 82%, at least about 83%, at least about 84%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, or at least about 99% identity to any one of SEQ ID NOs: 5592-5595. The endonuclease may be derived from a species having a 16S rRNA gene substantially identical to any one of SEQ ID NOs: 5592-5595. The endonuclease may be derived from a bacterium belonging to the Phylum Verrucomicrobia or the Phylum Candidatus Peregrinibacteria.

In some cases, sequence identity may be determined by the BLASTP, CLUSTALW, MUSCLE, MAFFT, Novafold, or CLUSTALW with the parameters of the Smith-Waterman homology search algorithm. The sequence identity may be determined by the BLASTP algorithm using parameters of a wordlength (W) of 3, an expectation (E) of 10, and using a BLOSUM62 scoring matrix setting gap costs at existence of 11, extension of 1, and using a conditional compositional score matrix adjustment.

In some cases, the system above may comprise (b) at least one engineered synthetic guide ribonucleic acid (sgRNA) capable of forming a complex with the endonuclease bearing a 5' targeting region complementary to a desired cleavage sequence. In some cases, the 5' targeting region may comprises a PAM sequence compatible with the endonuclease. In some cases, the 5' most nucleotide of the targeting region may be G. In some cases, the 5' targeting region may be 15-23 nucleotides in length. The guide sequence and the tracr sequence may be supplied as separate ribonucleic acids (RNAs) or a single ribonucleic acid (RNA). The guide RNA may comprise a crRNA tracrRNA binding sequence 3' to the targeting region. The guide RNA may comprise a tracrRNA sequence preceded by a 4-nucleotide linker 3' to the crRNA tracrRNA binding region. The sgRNA may comprise, from 5' to 3': a non-natural guide nucleic acid sequence capable of hybridizing to a target sequence in a cell; and a tracr sequence. In some cases, the non-natural guide nucleic acid sequence and the tracr sequence are covalently linked.

In some cases, the tracr sequence may have a particular sequence. The tracr sequence may have at least about 80% to at least about 60-100 (e.g., at least about 60, at least about 65, at least about 70, at least about 75, at least about 80, at least about 85, or at least about 90) consecutive nucleotides of a natural tracrRNA sequence. The tracr sequence may have at least about 80% sequence identity to at least about 60-100 (e.g., at least about 60, at least about 65, at least about 70, at least about 75, at least about 80, at least about 85, or at least about 90) consecutive nucleotides of any one of SEQ ID NOs: 5476-5489. In some cases, the tracrRNA may have at least about 80%, at least about 85%, at least about 90%, at least about 91%, at least about 92%, at least about 93%, at least about 94%, at least about 95%, at least about 96%, at least about 97%, at least about 98%, or at least about 99% identity to at least about 60-90 (e.g., at least about 60, at least about 65, at least about 70, at least about 75, at least about 80, at least about 85, or at least about 90) consecutive nucleotides of any one of SEQ ID NOs: 5476-5489. In some cases, the tracrRNA may be substantially identical to at least about 60-100 (e.g., at least about 60, at least about 65, at least about 70, at least about 75, at least about 80, at least about 85, or at least about 90) consecutive nucleotides of any one of SEQ ID NOs: 5476-5489. The tracrRNA may comprise any of SEQ ID NOs: 5476-5489.

In some cases, the at least one engineered synthetic guide ribonucleic acid (sgRNA) capable of forming a complex with the endonuclease may comprise a sequence having at least about 80% identity to any one of SEQ ID NOs: 5461-5464. The sgRNA may comprise a sequence having at least about 80%, at least about 85%, at least about 90%, at least about 91%, at least about 92%, at least about 93%, at least about 94%, at least about 95%, at least about 96%, at least about 97%, at least about 98%, or at least about 99% identity to any one of SEQ ID NOs: 5461-5464. The sgRNA may comprise a sequence substantially identical to any one of SEQ ID NOs: 5461-5464.

In some cases, the system above may comprise two different sgRNAs targeting a first region and a second region for cleavage in a target DNA locus, wherein the second region is 3' to the first region. In some cases, the system above may comprise a single- or double-stranded DNA repair template comprising from 5' to 3': a first homology arm comprising a sequence of at least about 20 (e.g., at least about 40, 80, 120, 150, 200, 300, 500, or 1kb) nucleotides 5' to the first region, a synthetic DNA sequence of at least about 10 nucleotides, and a second homology arm comprising a sequence of at least about 20 (e.g., at least about 40, 80, 120, 150, 200, 300, 500, or 1kb) nucleotides 3' to the second region.

In another aspect, the present disclosure provides a method for modifying a target nucleic acid locus. The method may comprise delivering to the target nucleic acid locus any of the non-natural systems disclosed herein, including an enzyme and at least one synthetic guide RNA (sgRNA) disclosed herein. The enzyme may form a complex with the at least one sgRNA, and upon binding of the complex to the target nucleic acid locus, may modify the target nucleic acid locus. Delivering the enzyme to said locus may comprise transfecting a cell with the system or nucleic acids encoding the system. Delivering the nuclease to said locus may comprise electroporating a cell with the system or nucleic acids encoding the system. Delivering the nuclease to said locus may comprise incubating the system in a buffer with a nucleic acid comprising the locus of interest. In some cases, the target nucleic acid locus comprises deoxyribonucleic acid (DNA) or ribonucleic acid (RNA). The target nucleic acid locus may comprise genomic DNA, viral DNA, viral RNA, or bacterial DNA. The target nucleic acid locus may be within a cell. The target nucleic acid locus may be in vitro. The target nucleic acid locus may be within a eukaryotic cell or a prokaryotic cell. The cell may be an animal cell, a human cell, bacterial cell, archaeal cell, or a plant cell. The enzyme may induce a single or double-stranded break at or proximal to the target locus of interest.

In cases where the target nucleic acid locus may be within a cell, the enzyme may be supplied as a nucleic acid containing an open reading frame encoding the enzyme having a RuvC_III domain having at least about 75% (e.g., at least about 90%, at least about 91%, at least about 92%, at least about 93%, at least about 94%, at least about 95%, at least about 96%, at least about 97%, at least about 98%, at least about 99%) identity to any one of SEQ ID NOs: 1827-2140. The deoxyribonucleic acid (DNA) containing an open reading frame encoding said endonuclease may comprise a sequence substantially identical to any of SEQ ID NOs: 5572-5575 or at variant having at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% identity to any one of SEQ ID NOs: 5572-5575. In some cases, the nucleic acid comprises a promoter to which the open reading frame encoding the endonuclease is operably linked. The promoter may be a CMV, EF1a, SV40, PGK1, Ubc, human beta actin, CAG, TRE, or CaMKIIa promoter. The endonuclease may be supplied as a capped mRNA containing said open reading frame encoding said endonuclease. The endonuclease may be supplied as a translated polypeptide. The at least one engineered sgRNA may be supplied as deoxyribonucleic acid (DNA) containing a gene sequence encoding said at least one engineered sgRNA operably linked to a ribonucleic acid (RNA) pol III promoter. In some cases, the organism may be eukaryotic. In some cases, the organism may be fungal. In some cases, the organism may be human.

In some cases, the present disclosure may provide for an expression cassette comprising the system disclosed herein, or the nucleic acid described herein. In some cases, the expression cassette or nucleic acid may be supplied as a vector. In some cases, the expression cassette, nucleic acid, or vector may be supplied in a cell. In some cases, the cell is a cell of a bacterium with a 16S rRNA gene having at least about 90% (e.g., at least about 99%) identity to any one of SEQ ID NOs: 5592-5595.

### MG2 Enzymes

In one aspect, the present disclosure provides for an engineered nuclease system comprising (a) an endonuclease. In some cases, the endonuclease is a Cas endonuclease. In some cases, the endonuclease is a Type II, Class II Cas endonuclease. The endonuclease may comprise a RuvC_III domain, wherein said RuvC_III domain has at least about 70% sequence identity to any one of SEQ ID NOs: 2141-2241. In some cases, the endonuclease may comprise a RuvC_III domain, wherein the RuvC_III domain has at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99% identity to any one of SEQ ID NOs: 2141-2241. In some cases, the endonuclease may comprise a RuvC_III domain, wherein the substantially identical to any one of SEQ ID NOs: 2141-2142. The endonuclease may comprise a RuvC_III domain having at least about 70% sequence identity to any one of SEQ ID NOs: 2141-2142. In some cases, the endonuclease may comprise a RuvC_III domain having at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99% identity to any one of SEQ ID NOs: 2141-2142. In some cases, the endonuclease may comprise a RuvC_III domain substantially identical to any one of SEQ ID NOs: 2141-2142.

The endonuclease may comprise an HNH domain having at least about 70% identity to any one of SEQ ID NOs: 3955-4055. In some cases, the endonuclease may comprise an HNH domain having at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99% identical to any one of SEQ ID NOs: 3955-4055. The endonuclease may comprise an HNH domain substantially identical to any one of SEQ ID NOs: 3955-4055. The endonuclease may comprise an HNH domain having at least about 70% identity to any one of SEQ ID NOs: 3955-3956. In some cases, the endonuclease may comprise an HNH domain having at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99% identical to any one of SEQ ID NOs: 3955-3956. The endonuclease may comprise an HNH domain substantially identical to any one of SEQ ID NOs: 3955-3956.

In some cases, the endonuclease may comprise a variant having at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% identity to any one of SEQ ID NOs: 320-420 . In some cases, the endonuclease may be substantially identical to any one of SEQ ID NOs: 320-420. In some cases, the endonuclease may comprise a variant having at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% identity to any one of SEQ ID NOs:320-321. In some cases, the endonuclease may be substantially identical to any one of SEQ ID NOs: 320-321.

In some cases, the endonuclease may comprise a variant having one or more nuclear localization sequences (NLSs). The NLS may be proximal to the N- or C-terminus of said endonuclease. The NLS may be appended N-terminal or C-terminal to any one of SEQ ID NOs: 320-420, or to a variant having at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% identity to any one of SEQ ID NOs: 320-420. The NLS may be an SV40 large T antigen NLS. The NLS may be a c-myc NLS. The NLS can comprise a sequence with at least about 80%, at least about 85%, at least about 90%, at least about 95%, at least about 99% identity to any one of SEQ ID NOs: 5593-5608. The NLS can comprise a sequence substantially identical to any one of SEQ ID NOs: 5593-5608. The NLS can comprise any of the sequences in Table 1 or a combination thereof:

In some cases, sequence identity may be determined by the BLASTP, CLUSTALW, MUSCLE, MAFFT, Novafold, or CLUSTALW with the parameters of the Smith-Waterman homology search algorithm. The sequence identity may be determined by the BLASTP algorithm using parameters of a wordlength (W) of 3, an expectation (E) of 10, and using a BLOSUM62 scoring matrix setting gap costs at existence of 11, extension of 1, and using a conditional compositional score matrix adjustment.

In some cases, the system above may comprise (b) at least one engineered synthetic guide ribonucleic acid (sgRNA) capable of forming a complex with the endonuclease bearing a 5' targeting region complementary to a desired cleavage sequence. In some cases, the 5' targeting region may comprises a PAM sequence compatible with the endonuclease. In some cases, the 5' most nucleotide of the targeting region may be G. In some cases, the 5' targeting region may be 15-23 nucleotides in length. The guide sequence and the tracr sequence may be supplied as separate ribonucleic acids (RNAs) or a single ribonucleic acid (RNA). The guide RNA may comprise a crRNA tracrRNA binding sequence 3' to the targeting region. The guide RNA may comprise a tracrRNA sequence preceded by a 4-nucleotide linker 3' to the crRNA tracrRNA binding region. The sgRNA may comprise, from 5' to 3': a non-natural guide nucleic acid sequence capable of hybridizing to a target sequence in a cell; and a tracr sequence. In some cases, the non-natural guide nucleic acid sequence and the tracr sequence are covalently linked.

In some cases, the tracr sequence may have a particular sequence. The tracr sequence may have at least about 80% to at least about 60-100 (e.g., at least about 60, at least about 65, at least about 70, at least about 75, at least about 80, at least about 85, or at least about 90) consecutive nucleotides of a natural tracrRNA sequence. The tracr sequence may have at least about 80% sequence identity to at least about 60-100 (e.g., at least about 60, at least about 65, at least about 70, at least about 75, at least about 80, at least about 85, or at least about 90) consecutive nucleotides of any one of SEQ ID NOs: 5490-5494. In some cases, the tracrRNA may have at least about 80%, at least about 85%, at least about 90%, at least about 91%, at least about 92%, at least about 93%, at least about 94%, at least about 95%, at least about 96%, at least about 97%, at least about 98%, or at least about 99% identity to at least about 60-90 (e.g., at least about 60, at least about 65, at least about 70, at least about 75, at least about 80, at least about 85, or at least about 90) consecutive nucleotides of any one of SEQ ID NOs: 5490-5494. In some cases, the tracrRNA may be substantially identical to at least about 60-100 (e.g., at least about 60, at least about 65, at least about 70, at least about 75, at least about 80, at least about 85, or at least about 90) consecutive nucleotides of any one of SEQ ID NOs: 5490-5494. The tracrRNA may comprise any of SEQ ID NOs: 5490-5494.

In some cases, the at least one engineered synthetic guide ribonucleic acid (sgRNA) capable of forming a complex with the endonuclease may comprise a sequence having at least about 80% identity to SEQ ID NO: 5465. The sgRNA may comprise a sequence having at least about 80%, at least about 85%, at least about 90%, at least about 91%, at least about 92%, at least about 93%, at least about 94%, at least about 95%, at least about 96%, at least about 97%, at least about 98%, or at least about 99% identity to SEQ ID NO: 5465. The sgRNA may comprise a sequence substantially identical to SEQ ID NO: 5465.

In some cases, the system above may comprise two different sgRNAs targeting a first region and a second region for cleavage in a target DNA locus, wherein the second region is 3' to the first region. In some cases, the system above may comprise a single- or double-stranded DNA repair template comprising from 5' to 3': a first homology arm comprising a sequence of at least about 20 (e.g., at least about 40, 80, 120, 150, 200, 300, 500, or 1kb) nucleotides 5' to the first region, a synthetic DNA sequence of at least about 10 nucleotides, and a second homology arm comprising a sequence of at least about 20 (e.g., at least about 40, 80, 120, 150, 200, 300, 500, or 1kb) nucleotides 3' to the second region.

In another aspect, the present disclosure provides a method for modifying a target nucleic acid locus of interest. The method may comprise delivering to the target nucleic acid locus any of the non-natural systems disclosed herein, including an enzyme and at least one synthetic guide RNA (sgRNA) disclosed herein. The enzyme may form a complex with the at least one sgRNA, and upon binding of the complex to the target nucleic acid locus of interest, may modify the target nucleic acid locus of interest. Delivering the enzyme to said locus may comprise transfecting a cell with the system or nucleic acids encoding the system. Delivering the nuclease to said locus may comprise electroporating a cell with the system or nucleic acids encoding the system. Delivering the nuclease to said locus may comprise incubating the system in a buffer with a nucleic acid comprising the locus of interest. In some cases, the target nucleic acid locus comprises deoxyribonucleic acid (DNA) or ribonucleic acid (RNA). The target nucleic acid locus may comprise genomic DNA, viral DNA, viral RNA, or bacterial DNA. The target nucleic acid locus may be within a cell. The target nucleic acid locus may be in vitro. The target nucleic acid locus may be within a eukaryotic cell or a prokaryotic cell. The cell may be an animal cell, a human cell, bacterial cell, archaeal cell, or a plant cell. The enzyme may induce a single or double-stranded break at or proximal to the target locus of interest.

In cases where the target nucleic acid locus may be within a cell, the enzyme may be supplied as a nucleic acid containing an open reading frame encoding the enzyme having a RuvC_III domain having at least about 75% (e.g., at least about 90%, at least about 91%, at least about 92%, at least about 93%, at least about 94%, at least about 95%, at least about 96%, at least about 97%, at least about 98%, at least about 99%) identity to any one of SEQ ID NOs: 2141-2241. The deoxyribonucleic acid (DNA) containing an open reading frame encoding said endonuclease may comprise a sequence substantially identical to any of SEQ ID NOs: 5576-5577 or at variant having at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% identity to any one of SEQ ID NOs: 5576-5577. In some cases, the nucleic acid comprises a promoter to which the open reading frame encoding the endonuclease is operably linked. The promoter may be a CMV, EF1a, SV40, PGK1, Ubc, human beta actin, CAG, TRE, or CaMKIIa promoter. The endonuclease may be supplied as a capped mRNA containing said open reading frame encoding said endonuclease. The endonuclease may be supplied as a translated polypeptide. The at least one engineered sgRNA may be supplied as deoxyribonucleic acid (DNA) containing a gene sequence encoding said at least one engineered sgRNA operably linked to a ribonucleic acid (RNA) pol III promoter. In some cases, the organism may be eukaryotic. In some cases, the organism may be fungal. In some cases, the organism may be human.

### MG3 Enzymes

In one aspect, the present disclosure provides for an engineered nuclease system comprising (a) an endonuclease. In some cases, the endonuclease is a Cas endonuclease. In some cases, the endonuclease is a Type II, Class II Cas endonuclease. The endonuclease may comprise a RuvC_III domain, wherein said RuvC_III domain has at least about 70% sequence identity to any one of SEQ ID NOs: 2242-2251. In some cases, the endonuclease may comprise a RuvC_III domain, wherein the RuvC_III domain has at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99% identity to any one of SEQ ID NOs: 2242-2251. In some cases, the endonuclease may comprise a RuvC_III domain, wherein the substantially identical to any one of SEQ ID NOs: 2242-2251. The endonuclease may comprise a RuvC_III domain having at least about 70% sequence identity to any one of SEQ ID NOs: 2242-2244. In some cases, the endonuclease may comprise a RuvC_III domain having at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99% identity to any one of SEQ ID NOs: 2242-2244. In some cases, the endonuclease may comprise a RuvC_III domain substantially identical to any one of SEQ ID NOs: 2242-2244.

The endonuclease may comprise an HNH domain having at least about 70% identity to any one of SEQ ID NOs: 4056-4066. In some cases, the endonuclease may comprise an HNH domain having at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99% identical to any one of SEQ ID NOs: 4056-4066. The endonuclease may comprise an HNH domain substantially identical to any one of SEQ ID NOs: 4056-4066. The endonuclease may comprise an HNH domain having at least about 70% identity to any one of SEQ ID NOs: 4056-4058. In some cases, the endonuclease may comprise an HNH domain having at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99% identical to any one of SEQ ID NOs: 4056-4058. The endonuclease may comprise an HNH domain substantially identical to any one of SEQ ID NOs: 4056-4058.

In some cases, the endonuclease may comprise a variant having at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% identity to any one of SEQ ID NOs: 421-431. In some cases, the endonuclease may be substantially identical to any one of SEQ ID NOs: 421-431. In some cases, the endonuclease may comprise a variant having at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% identity to any one of SEQ ID NOs:421-423. In some cases, the endonuclease may be substantially identical to any one of SEQ ID NOs: 421-423.

In some cases, the endonuclease may comprise a variant having one or more nuclear localization sequences (NLSs). The NLS may be proximal to the N- or C-terminus of said endonuclease. The NLS may be appended N-terminal or C-terminal to any one of SEQ ID NOs: 421-431, or to a variant having at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% identity to any one of SEQ ID NOs: 421-431. The NLS may be an SV40 large T antigen NLS. The NLS may be a c-myc NLS. The NLS can comprise a sequence with at least about 80%, at least about 85%, at least about 90%, at least about 95%, at least about 99% identity to any one of SEQ ID NOs: 5593-5608. The NLS can comprise a sequence substantially identical to any one of SEQ ID NOs: 5593-5608. The NLS can comprise any of the sequences in Table 1 or a combination thereof:

In some cases, sequence identity may be determined by the BLASTP, CLUSTALW, MUSCLE, MAFFT, Novafold, or CLUSTALW with the parameters of the Smith-Waterman homology search algorithm. The sequence identity may be determined by the BLASTP algorithm using parameters of a wordlength (W) of 3, an expectation (E) of 10, and using a BLOSUM62 scoring matrix setting gap costs at existence of 11, extension of 1, and using a conditional compositional score matrix adjustment.

In some cases, the system above may comprise (b) at least one engineered synthetic guide ribonucleic acid (sgRNA) capable of forming a complex with the endonuclease bearing a 5' targeting region complementary to a desired cleavage sequence. In some cases, the 5' targeting region may comprises a PAM sequence compatible with the endonuclease. In some cases, the 5' most nucleotide of the targeting region may be G. In some cases, the 5' targeting region may be 15-23 nucleotides in length. The guide sequence and the tracr sequence may be supplied as separate ribonucleic acids (RNAs) or a single ribonucleic acid (RNA). The guide RNA may comprise a crRNA tracrRNA binding sequence 3' to the targeting region. The guide RNA may comprise a tracrRNA sequence preceded by a 4-nucleotide linker 3' to the crRNA tracrRNA binding region. The sgRNA may comprise, from 5' to 3': a non-natural guide nucleic acid sequence capable of hybridizing to a target sequence in a cell; and a tracr sequence. In some cases, the non-natural guide nucleic acid sequence and the tracr sequence are covalently linked.

In some cases, the tracr sequence may have a particular sequence. The tracr sequence may have at least about 80% to at least about 60-100 (e.g., at least about 60, at least about 65, at least about 70, at least about 75, at least about 80, at least about 85, or at least about 90) consecutive nucleotides of a natural tracrRNA sequence. The tracr sequence may have at least about 80% sequence identity to at least about 60-100 (e.g., at least about 60, at least about 65, at least about 70, at least about 75, at least about 80, at least about 85, or at least about 90) consecutive nucleotides of any one of SEQ ID NOs: 5495-5502. In some cases, the tracrRNA may have at least about 80%, at least about 85%, at least about 90%, at least about 91%, at least about 92%, at least about 93%, at least about 94%, at least about 95%, at least about 96%, at least about 97%, at least about 98%, or at least about 99% identity to at least about 60-90 (e.g., at least about 60, at least about 65, at least about 70, at least about 75, at least about 80, at least about 85, or at least about 90) consecutive nucleotides of any one of SEQ ID NOs: 5495-5502. In some cases, the tracrRNA may be substantially identical to at least about 60-100 (e.g., at least about 60, at least about 65, at least about 70, at least about 75, at least about 80, at least about 85, or at least about 90) consecutive nucleotides of any one of SEQ ID NOs: 5495-5502. The tracrRNA may comprise any of SEQ ID NOs: 5495-5502.

In some cases, the at least one engineered synthetic guide ribonucleic acid (sgRNA) capable of forming a complex with the endonuclease may comprise a sequence having at least about 80% identity to any one of SEQ ID NOs: 5466-5467. The sgRNA may comprise a sequence having at least about 80%, at least about 85%, at least about 90%, at least about 91%, at least about 92%, at least about 93%, at least about 94%, at least about 95%, at least about 96%, at least about 97%, at least about 98%, or at least about 99% identity to any one of SEQ ID NOs: 5466-5467. The sgRNA may comprise a sequence substantially identical to any one of SEQ ID NOs: 5466-5467.

In some cases, the system above may comprise two different sgRNAs targeting a first region and a second region for cleavage in a target DNA locus, wherein the second region is 3' to the first region. In some cases, the system above may comprise a single- or double-stranded DNA repair template comprising from 5' to 3': a first homology arm comprising a sequence of at least about 20 (e.g., at least about 40, 80, 120, 150, 200, 300, 500, or 1kb) nucleotides 5' to the first region, a synthetic DNA sequence of at least about 10 nucleotides, and a second homology arm comprising a sequence of at least about 20 (e.g., at least about 40, 80, 120, 150, 200, 300, 500, or 1kb) nucleotides 3' to the second region.

In another aspect, the present disclosure provides a method for modifying a target nucleic acid locus of interest. The method may comprise delivering to the target nucleic acid locus any of the non-natural systems disclosed herein, including an enzyme and at least one synthetic guide RNA (sgRNA) disclosed herein. The enzyme may form a complex with the at least one sgRNA, and upon binding of the complex to the target nucleic acid locus of interest, may modify the target nucleic acid locus of interest. Delivering the enzyme to said locus may comprise transfecting a cell with the system or nucleic acids encoding the system. Delivering the nuclease to said locus may comprise electroporating a cell with the system or nucleic acids encoding the system. Delivering the nuclease to said locus may comprise incubating the system in a buffer with a nucleic acid comprising the locus of interest. In some cases, the target nucleic acid locus comprises deoxyribonucleic acid (DNA) or ribonucleic acid (RNA). The target nucleic acid locus may comprise genomic DNA, viral DNA, viral RNA, or bacterial DNA. The target nucleic acid locus may be within a cell. The target nucleic acid locus may be in vitro. The target nucleic acid locus may be within a eukaryotic cell or a prokaryotic cell. The cell may be an animal cell, a human cell, bacterial cell, archaeal cell, or a plant cell. The enzyme may induce a single or double-stranded break at or proximal to the target locus of interest.

In cases where the target nucleic acid locus may be within a cell, the enzyme may be supplied as a nucleic acid containing an open reading frame encoding the enzyme having a RuvC_III domain having at least about 75% (e.g., at least about 90%, at least about 91%, at least about 92%, at least about 93%, at least about 94%, at least about 95%, at least about 96%, at least about 97%, at least about 98%, at least about 99%) identity to any one of SEQ ID NOs: 2242-2251. The deoxyribonucleic acid (DNA) containing an open reading frame encoding said endonuclease may comprise a sequence substantially identical to any of SEQ ID NOs: 5578-5580 or at variant having at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about

99% identity to any one of SEQ ID NOs: 5578-5580. In some cases, the nucleic acid comprises a promoter to which the open reading frame encoding the endonuclease is operably linked. The promoter may be a CMV, EF1a, SV40, PGK1, Ubc, human beta actin, CAG, TRE, or CaMKIIa promoter. The endonuclease may be supplied as a capped mRNA containing said open reading frame encoding said endonuclease. The endonuclease may be supplied as a translated polypeptide. The at least one engineered sgRNA may be supplied as deoxyribonucleic acid (DNA) containing a gene sequence encoding said at least one engineered sgRNA operably linked to a ribonucleic acid (RNA) pol III promoter. In some cases, the organism may be eukaryotic. In some cases, the organism may be fungal. In some cases, the organism may be human.

### MG4 Enzymes

In one aspect, the present disclosure provides for an engineered nuclease system comprising (a) an endonuclease. In some cases, the endonuclease is a Cas endonuclease. In some cases, the endonuclease is a Type II, Class II Cas endonuclease. The endonuclease may comprise a RuvC_III domain, wherein said RuvC_III domain has at least about 70% sequence identity to any one of SEQ ID NOs: 2253-2481. In some cases, the endonuclease may comprise a RuvC_III domain, wherein the RuvC_III domain has at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99% identity to any one of SEQ ID NOs: 2253-2481. In some cases, the endonuclease may comprise a RuvC_III domain, wherein the substantially identical to any one of SEQ ID NOs: 2253-2481. The endonuclease may comprise a RuvC_III domain having at least about 70% sequence identity to any one of SEQ ID NOs: 2253-2481. In some cases, the endonuclease may comprise a RuvC_III domain having at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99% identity to any one of SEQ ID NOs: 2253-2481. In some cases, the endonuclease may comprise a RuvC_III domain substantially identical to any one of SEQ ID NOs: 2253-2481.

The endonuclease may comprise an HNH domain having at least about 70% identity to any one of SEQ ID NOs: 4067-4295. In some cases, the endonuclease may comprise an HNH domain having at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99% identical to any one of SEQ ID NOs: 4067-4295. The endonuclease may comprise an HNH domain substantially identical to any one of SEQ ID NOs: 4067-4295. The endonuclease may comprise an HNH domain having at least about 70% identity to any one of SEQ ID NOs: 4067-4295. In some cases, the endonuclease may comprise an HNH domain having at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99% identical to any one of SEQ ID NOs: 4067-4295. The endonuclease may comprise an HNH domain substantially identical to any one of SEQ ID NOs: 4067-4295.

In some cases, the endonuclease may comprise a variant having at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% identity to any one of SEQ ID NOs: 432-660. In some cases, the endonuclease may be substantially identical to any one of SEQ ID NOs: 432-660. In some cases, the endonuclease may comprise a variant having at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% identity to any one of SEQ ID NOs: 432-660. In some cases, the endonuclease may be substantially identical to any one of SEQ ID NOs: 432-660.

In some cases, the endonuclease may comprise a variant having one or more nuclear localization sequences (NLSs). The NLS may be proximal to the N- or C-terminus of said endonuclease. The NLS may be appended N-terminal or C-terminal to any one of SEQ ID NOs: 432-660, or to a variant having at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% identity to any one of SEQ ID NOs: 432-660. The NLS may be an SV40 large T antigen NLS. The NLS may be a c-myc NLS. The NLS can comprise a sequence with at least about 80%, at least about 85%, at least about 90%, at least about 95%, at least about 99% identity to any one of SEQ ID NOs: 5593-5608. The NLS can comprise a sequence substantially identical to any one of SEQ ID NOs: 5593-5608. The NLS can comprise any of the sequences in Table 1 or a combination thereof:

In some cases, sequence identity may be determined by the BLASTP, CLUSTALW, MUSCLE, MAFFT, Novafold, or CLUSTALW with the parameters of the Smith-Waterman homology search algorithm. The sequence identity may be determined by the BLASTP algorithm using parameters of a wordlength (W) of 3, an expectation (E) of 10, and using a BLOSUM62 scoring matrix setting gap costs at existence of 11, extension of 1, and using a conditional compositional score matrix adjustment.

In some cases, the system above may comprise (b) at least one engineered synthetic guide ribonucleic acid (sgRNA) capable of forming a complex with the endonuclease bearing a 5' targeting region complementary to a desired cleavage sequence. In some cases, the 5' targeting region may comprises a PAM sequence compatible with the endonuclease. In some cases, the 5' most nucleotide of the targeting region may be G. In some cases, the 5' targeting region may be 15-23 nucleotides in length. The guide sequence and the tracr sequence may be supplied as separate ribonucleic acids (RNAs) or a single ribonucleic acid (RNA). The guide RNA may comprise a crRNA tracrRNA binding sequence 3' to the targeting region. The guide RNA may comprise a tracrRNA sequence preceded by a 4-nucleotide linker 3' to the crRNA tracrRNA binding region. The sgRNA may comprise, from 5' to 3': a non-natural guide nucleic acid sequence capable of hybridizing to a target sequence in a cell; and a tracr sequence. In some cases, the non-natural guide nucleic acid sequence and the tracr sequence are covalently linked.

In some cases, the tracr sequence may have a particular sequence. The tracr sequence may have at least about 80% to at least about 60-100 (e.g., at least about 60, at least about 65, at least about 70, at least about 75, at least about 80, at least about 85, or at least about 90) consecutive nucleotides of a natural tracrRNA sequence. The tracr sequence may have at least about 80% sequence identity to at least about 60-100 (e.g., at least about 60, at least about 65, at least about 70, at least about 75, at least about 80, at least about 85, or at least about 90) consecutive nucleotides of SEQ ID NO: 5503. In some cases, the tracrRNA may have at least about 80%, at least about 85%, at least about 90%, at least about 91%, at least about 92%, at least about 93%, at least about 94%, at least about 95%, at least about 96%, at least about 97%, at least about 98%, or at least about 99% identity to at least about 60-90 (e.g., at least about 60, at least about 65, at least about 70, at least about 75, at least about 80, at least about 85, or at least about 90) consecutive nucleotides of SEQ ID NO: 5503. In some cases, the tracrRNA may be substantially identical to at least about 60-100 (e.g., at least about 60, at least about 65, at least about 70, at least about 75, at least about 80, at least about 85, or at least about 90) consecutive nucleotides of SEQ ID NO: 5503. The tracrRNA may comprise SEQ ID NO: 5503.

In some cases, the at least one engineered synthetic guide ribonucleic acid (sgRNA) capable of forming a complex with the endonuclease may comprise a sequence having at least about 80% identity to SEQ ID NO: 5468. The sgRNA may comprise a sequence having at least about 80%, at least about 85%, at least about 90%, at least about 91%, at least about 92%, at least about 93%, at least about 94%, at least about 95%, at least about 96%, at least about 97%, at least about 98%, or at least about 99% identity to SEQ ID NO: 5468. The sgRNA may comprise a sequence substantially identical to SEQ ID NO: 5468.

In some cases, the system above may comprise two different sgRNAs targeting a first region and a second region for cleavage in a target DNA locus, wherein the second region is 3' to the first region. In some cases, the system above may comprise a single- or double-stranded DNA repair template comprising from 5' to 3': a first homology arm comprising a sequence of at least about 20 (e.g., at least about 40, 80, 120, 150, 200, 300, 500, or 1kb) nucleotides 5' to the first region, a synthetic DNA sequence of at least about 10 nucleotides, and a second homology arm comprising a sequence of at least about 20 (e.g., at least about 40, 80, 120, 150, 200, 300, 500, or 1kb) nucleotides 3' to the second region.

In another aspect, the present disclosure provides a method for modifying a target nucleic acid locus of interest. The method may comprise delivering to the target nucleic acid locus any of the non-natural systems disclosed herein, including an enzyme and at least one synthetic guide RNA (sgRNA) disclosed herein. The enzyme may form a complex with the at least one sgRNA, and upon binding of the complex to the target nucleic acid locus of interest, may modify the target nucleic acid locus of interest. Delivering the enzyme to said locus may comprise transfecting a cell with the system or nucleic acids encoding the system. Delivering the nuclease to said locus may comprise electroporating a cell with the system or nucleic acids encoding the system. Delivering the nuclease to said locus may comprise incubating the system in a buffer with a nucleic acid comprising the locus of interest. In some cases, the target nucleic acid locus comprises deoxyribonucleic acid (DNA) or ribonucleic acid (RNA). The target nucleic acid locus may comprise genomic DNA, viral DNA, viral RNA, or bacterial DNA. The target nucleic acid locus may be within a cell. The target nucleic acid locus may be in vitro. The target nucleic acid locus may be within a eukaryotic cell or a prokaryotic cell. The cell may be an animal cell, a human cell, bacterial cell, archaeal cell, or a plant cell. The enzyme may induce a single or double-stranded break at or proximal to the target locus of interest.

In cases where the target nucleic acid locus may be within a cell, the enzyme may be supplied as a nucleic acid containing an open reading frame encoding the enzyme having a RuvC_III domain having at least about 75% (e.g., at least about 90%, at least about 91%, at least about 92%, at least about 93%, at least about 94%, at least about 95%, at least about 96%, at least about 97%, at least about 98%, at least about 99%) identity to any one of SEQ ID NOs: 2253-2481. In some cases, the nucleic acid comprises a promoter to which the open reading frame encoding the endonuclease is operably linked. The promoter may be a CMV, EF1a, SV40, PGK1, Ubc, human beta actin, CAG, TRE, or CaMKIIa promoter. The endonuclease may be supplied as a capped mRNA containing said open reading frame encoding said endonuclease. The endonuclease may be supplied as a translated polypeptide. The at least one engineered sgRNA may be supplied as deoxyribonucleic acid (DNA) containing a gene sequence encoding said at least one engineered sgRNA operably linked to a ribonucleic acid (RNA) pol III promoter. In some cases, the organism may be eukaryotic. In some cases, the organism may be fungal. In some cases, the organism may be human.

### MG6 Enzymes

In one aspect, the present disclosure provides for an engineered nuclease system comprising (a) an endonuclease. In some cases, the endonuclease is a Cas endonuclease. In some cases, the endonuclease is a Type II, Class II Cas endonuclease. The endonuclease may comprise a RuvC_III domain, wherein said RuvC_III domain has at least about 70% sequence identity to any one of SEQ ID NOs: 2482-2489. In some cases, the endonuclease may comprise a RuvC_III domain, wherein the RuvC_III domain has at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99% identity to any one of SEQ ID NOs: 2482-2489. In some cases, the endonuclease may comprise a RuvC_III domain, wherein the substantially identical to any one of SEQ ID NOs: 2482-2489.

The endonuclease may comprise an HNH domain having at least about 70% identity to any one of SEQ ID NOs: 4296-4303. In some cases, the endonuclease may comprise an HNH domain having at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99% identical to any one of SEQ ID NOs: 4296-4303. The endonuclease may comprise an HNH domain substantially identical to any one of SEQ ID NOs: 4056-4066.

In some cases, the endonuclease may comprise a variant having at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% identity to any one of SEQ ID NOs: 661-668. In some cases, the endonuclease may be substantially identical to any one of SEQ ID NOs: 661-668.

In some cases, the endonuclease may comprise a variant having one or more nuclear localization sequences (NLSs). The NLS may be proximal to the N- or C-terminus of said endonuclease. The NLS may be appended N-terminal or C-terminal to any one of SEQ ID NOs: 661-668, or to a variant having at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% identity to any one of SEQ ID NOs: 661-668. The NLS may be an SV40 large T antigen NLS. The NLS may be a c-myc NLS. The NLS can comprise a sequence with at least about 80%, at least about 85%, at least about 90%, at least about 95%, at least about 99% identity to any one of SEQ ID NOs: 5593-5608. The NLS can comprise a sequence substantially identical to any one of SEQ ID NOs: 5593-5608. The NLS can comprise any of the sequences in Table 1 or a combination thereof:

In some cases, sequence identity may be determined by the BLASTP, CLUSTALW, MUSCLE, MAFFT, Novafold, or CLUSTALW with the parameters of the Smith-Waterman homology search algorithm. The sequence identity may be determined by the BLASTP algorithm using parameters of a wordlength (W) of 3, an expectation (E) of 10, and using a BLOSUM62 scoring matrix setting gap costs at existence of 11, extension of 1, and using a conditional compositional score matrix adjustment.

In some cases, the system above may comprise (b) at least one engineered synthetic guide ribonucleic acid (sgRNA) capable of forming a complex with the endonuclease bearing a 5' targeting region complementary to a desired cleavage sequence. In some cases, the 5' targeting region may comprises a PAM sequence compatible with the endonuclease. In some cases, the 5' most nucleotide of the targeting region may be G. In some cases, the 5' targeting region may be 15-23 nucleotides in length. The guide sequence and the tracr sequence may be supplied as separate ribonucleic acids (RNAs) or a single ribonucleic acid (RNA). The guide RNA may comprise a crRNA tracrRNA binding sequence 3' to the targeting region. The guide RNA may comprise a tracrRNA sequence preceded by a 4-nucleotide linker 3' to the crRNA tracrRNA binding region. The sgRNA may comprise, from 5' to 3': a non-natural guide nucleic acid sequence capable of hybridizing to a target sequence in a cell; and a tracr sequence. In some cases, the non-natural guide nucleic acid sequence and the tracr sequence are covalently linked.

In some cases, the tracr sequence may have a particular sequence. The tracr sequence may have at least about 80% to at least about 60-100 (e.g., at least about 60, at least about 65, at least about 70, at least about 75, at least about 80, at least about 85, or at least about 90) consecutive nucleotides of a natural tracrRNA sequence.

In some cases, the system above may comprise two different guide RNAs targeting a first region and a second region for cleavage in a target DNA locus, wherein the second region is 3' to the first region. In some cases, the system above may comprise a single- or double-stranded DNA repair template comprising from 5' to 3': a first homology arm comprising a sequence of at least about 20 (e.g., at least about 40, 80, 120, 150, 200, 300, 500, or 1kb) nucleotides 5' to the first region, a synthetic DNA sequence of at least about 10 nucleotides, and a second homology arm comprising a sequence of at least about 20 (e.g., at least about 40, 80, 120, 150, 200, 300, 500, or 1kb) nucleotides 3' to the second region.

In another aspect, the present disclosure provides a method for modifying a target nucleic acid locus of interest. The method may comprise delivering to the target nucleic acid locus any of the non-natural systems disclosed herein, including an enzyme and at least one synthetic guide RNA (sgRNA) disclosed herein. The enzyme may form a complex with the at least one sgRNA, and upon binding of the complex to the target nucleic acid locus of interest, may modify the target nucleic acid locus of interest. Delivering the enzyme to said locus may comprise transfecting a cell with the system or nucleic acids encoding the system. Delivering the nuclease to said locus may comprise electroporating a cell with the system or nucleic acids encoding the system. Delivering the nuclease to said locus may comprise incubating the system in a buffer with a nucleic acid comprising the locus of interest. In some cases, the target nucleic acid locus comprises deoxyribonucleic acid (DNA) or ribonucleic acid (RNA). The target nucleic acid locus may comprise genomic DNA, viral DNA, viral RNA, or bacterial DNA. The target nucleic acid locus may be within a cell. The target nucleic acid locus may be in vitro. The target nucleic acid locus may be within a eukaryotic cell or a prokaryotic cell. The cell may be an animal cell, a human cell, bacterial cell, archaeal cell, or a plant cell. The enzyme may induce a single or double-stranded break at or proximal to the target locus of interest.

In cases where the target nucleic acid locus may be within a cell, the enzyme may be supplied as a nucleic acid containing an open reading frame encoding the enzyme having a RuvC_III domain having at least about 75% (e.g., at least about 90%, at least about 91%, at least about 92%, at least about 93%, at least about 94%, at least about 95%, at least about 96%, at least about 97%, at least about 98%, at least about 99%) identity to any one of SEQ ID NOs: 2482-2489. In some cases, the nucleic acid comprises a promoter to which the open reading frame encoding the endonuclease is operably linked. The promoter may be a CMV, EF1a, SV40, PGK1, Ubc, human beta actin, CAG, TRE, or CaMKIIa promoter. The endonuclease may be supplied as a capped mRNA containing said open reading frame encoding said endonuclease. The endonuclease may be supplied as a translated polypeptide. The at least one engineered sgRNA may be supplied as deoxyribonucleic acid (DNA) containing a gene sequence encoding said at least one engineered sgRNA operably linked to a ribonucleic acid (RNA) pol III promoter. In some cases, the organism may be eukaryotic. In some cases, the organism may be fungal. In some cases, the organism may be human.

### MG7 Enzymes

In one aspect, the present disclosure provides for an engineered nuclease system comprising (a) an endonuclease. In some cases, the endonuclease is a Cas endonuclease. In some cases, the endonuclease is a Type II, Class II Cas endonuclease. The endonuclease may comprise a RuvC_III domain, wherein said RuvC_III domain has at least about 70% sequence identity to any one of SEQ ID NOs: 2490-2498. In some cases, the endonuclease may comprise a RuvC_III domain, wherein the RuvC_III domain has at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99% identity to any one of SEQ ID NOs: 2490-2498. In some cases, the endonuclease may comprise a RuvC_III domain, wherein the substantially identical to any one of SEQ ID NOs: 2490-2498. The endonuclease may comprise a RuvC_III domain having at least about 70% sequence identity to any one of SEQ ID NOs: 2490-2498. In some cases, the endonuclease may comprise a RuvC_III domain having at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99% identity to any one of SEQ ID NOs: 2490-2498. In some cases, the endonuclease may comprise a RuvC_III domain substantially identical to any one of SEQ ID NOs: 2490-2498.

The endonuclease may comprise an HNH domain having at least about 70% identity to any one of SEQ ID NOs: 4304-4312. In some cases, the endonuclease may comprise an HNH domain having at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99% identical to any one of SEQ ID NOs: 4304-4312. The endonuclease may comprise an HNH domain substantially identical to any one of SEQ ID NOs: 4304-4312. The endonuclease may comprise an HNH domain having at least about 70% identity to any one of SEQ ID NOs: 4304-4312. In some cases, the endonuclease may comprise an HNH domain having at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99% identical to any one of SEQ ID NOs: 4304-4312. The endonuclease may comprise an HNH domain substantially identical to any one of SEQ ID NOs: 4304-4312.

In some cases, the endonuclease may comprise a variant having at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% identity to any one of SEQ ID NOs: 669-677. In some cases, the endonuclease may be substantially identical to any one of SEQ ID NOs: 669-677. In some cases, the endonuclease may comprise a variant having at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% identity to any one of SEQ ID NOs: 669-677. In some cases, the endonuclease may be substantially identical to any one of SEQ ID NOs: 669-677.

In some cases, the endonuclease may comprise a variant having one or more nuclear localization sequences (NLSs). The NLS may be proximal to the N- or C-terminus of said endonuclease. The NLS may be appended N-terminal or C-terminal to any one of SEQ ID NOs: 669-677, or to a variant having at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% identity to any one of SEQ ID NOs: 669-677. The NLS may be an SV40 large T antigen NLS. The NLS may be a c-myc NLS. The NLS can comprise a sequence with at least about 80%, at least about 85%, at least about 90%, at least about 95%, at least about 99% identity to any one of SEQ ID NOs: 5593-5608. The NLS can comprise a sequence substantially identical to any one of SEQ ID NOs: 5593-5608. The NLS can comprise any of the sequences in Table 1 or a combination thereof:

In some cases, sequence identity may be determined by the BLASTP, CLUSTALW, MUSCLE, MAFFT, Novafold, or CLUSTALW with the parameters of the Smith-Waterman homology search algorithm. The sequence identity may be determined by the BLASTP algorithm using parameters of a wordlength (W) of 3, an expectation (E) of 10, and using a BLOSUM62 scoring matrix setting gap costs at existence of 11, extension of 1, and using a conditional compositional score matrix adjustment.

In some cases, the system above may comprise (b) at least one engineered synthetic guide ribonucleic acid (sgRNA) capable of forming a complex with the endonuclease bearing a 5' targeting region complementary to a desired cleavage sequence. In some cases, the 5' targeting region may comprises a PAM sequence compatible with the endonuclease. In some cases, the 5' most nucleotide of the targeting region may be G. In some cases, the 5' targeting region may be 15-23 nucleotides in length. The guide sequence and the tracr sequence may be supplied as separate ribonucleic acids (RNAs) or a single ribonucleic acid (RNA). The guide RNA may comprise a crRNA tracrRNA binding sequence 3' to the targeting region. The guide RNA may comprise a tracrRNA sequence preceded by a 4-nucleotide linker 3' to the crRNA tracrRNA binding region. The sgRNA may comprise, from 5' to 3': a non-natural guide nucleic acid sequence capable of hybridizing to a target sequence in a cell; and a tracr sequence. In some cases, the non-natural guide nucleic acid sequence and the tracr sequence are covalently linked.

In some cases, the tracr sequence may have a particular sequence. The tracr sequence may have at least about 80% to at least about 60-100 (e.g., at least about 60, at least about 65, at least about 70, at least about 75, at least about 80, at least about 85, or at least about 90) consecutive nucleotides of a natural tracrRNA sequence. The tracr sequence may have at least about 80% sequence identity to at least about 60-100 (e.g., at least about 60, at least about 65, at least about 70, at least about 75, at least about 80, at least about 85, or at least about 90) consecutive nucleotides of SEQ ID NO: 5504. In some cases, the tracrRNA may have at least about 80%, at least about 85%, at least about 90%, at least about 91%, at least about 92%, at least about 93%, at least about 94%, at least about 95%, at least about 96%, at least about 97%, at least about 98%, or at least about 99% identity to at least about 60-90 (e.g., at least about 60, at least about 65, at least about 70, at least about 75, at least about 80, at least about 85, or at least about 90) consecutive nucleotides of SEQ ID NO: 5504. In some cases, the tracrRNA may be substantially identical to at least about 60-100 (e.g., at least about 60, at least about 65, at least about 70, at least about 75, at least about 80, at least about 85, or at least about 90) consecutive nucleotides of SEQ ID NO: 5504. The tracrRNA may comprise SEQ ID NO: 5504.

In some cases, the system above may comprise two different sgRNAs targeting a first region and a second region for cleavage in a target DNA locus, wherein the second region is 3' to the first region. In some cases, the system above may comprise a single- or double-stranded DNA repair template comprising from 5' to 3': a first homology arm comprising a sequence of at least about 20 (e.g., at least about 40, 80, 120, 150, 200, 300, 500, or 1kb) nucleotides 5' to the first region, a synthetic DNA sequence of at least about 10 nucleotides, and a second homology arm comprising a sequence of at least about 20 (e.g., at least about 40, 80, 120, 150, 200, 300, 500, or 1kb) nucleotides 3' to the second region.

In another aspect, the present disclosure provides a method for modifying a target nucleic acid locus of interest. The method may comprise delivering to the target nucleic acid locus any of the non-natural systems disclosed herein, including an enzyme and at least one synthetic guide RNA (sgRNA) disclosed herein. The enzyme may form a complex with the at least one sgRNA, and upon binding of the complex to the target nucleic acid locus of interest, may modify the target nucleic acid locus of interest. Delivering the enzyme to said locus may comprise transfecting a cell with the system or nucleic acids encoding the system. Delivering the nuclease to said locus may comprise electroporating a cell with the system or nucleic acids encoding the system. Delivering the nuclease to said locus may comprise incubating the system in a buffer with a nucleic acid comprising the locus of interest. In some cases, the target nucleic acid locus comprises deoxyribonucleic acid (DNA) or ribonucleic acid (RNA). The target nucleic acid locus may comprise genomic DNA, viral DNA, viral RNA, or bacterial DNA. The target nucleic acid locus may be within a cell. The target nucleic acid locus may be in vitro. The target nucleic acid locus may be within a eukaryotic cell or a prokaryotic cell. The cell may be an animal cell, a human cell, bacterial cell, archaeal cell, or a plant cell. The enzyme may induce a single or double-stranded break at or proximal to the target locus of interest.

In cases where the target nucleic acid locus may be within a cell, the enzyme may be supplied as a nucleic acid containing an open reading frame encoding the enzyme having a RuvC_III domain having at least about 75% (e.g., at least about 90%, at least about 91%, at least about 92%, at least about 93%, at least about 94%, at least about 95%, at least about 96%, at least about 97%, at least about 98%, at least about 99%) identity to any one of SEQ ID NOs: 2490-2498. In some cases, the nucleic acid comprises a promoter to which the open reading frame encoding the endonuclease is operably linked. The promoter may be a CMV, EF1a, SV40, PGK1, Ubc, human beta actin, CAG, TRE, or CaMKIIa promoter. The endonuclease may be supplied as a capped mRNA containing said open reading frame encoding said endonuclease. The endonuclease may be supplied as a translated polypeptide. The at least one engineered sgRNA may be supplied as deoxyribonucleic acid (DNA) containing a gene sequence encoding said at least one engineered sgRNA operably linked to a ribonucleic acid (RNA) pol III promoter. In some cases, the organism may be eukaryotic. In some cases, the organism may be fungal. In some cases, the organism may be human.

### MG14 Enzymes

In one aspect, the present disclosure provides for an engineered nuclease system comprising (a) an endonuclease. In some cases, the endonuclease is a Cas endonuclease. In some cases, the endonuclease is a Type II, Class II Cas endonuclease. The endonuclease may comprise a RuvC_III domain, wherein said RuvC_III domain has at least about 70% sequence identity to any one of SEQ ID NOs: 2499-2750. In some cases, the endonuclease may comprise a RuvC_III domain, wherein the RuvC_III domain has at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99% identity to any one of SEQ ID NOs: 2499-2750. In some cases, the endonuclease may comprise a RuvC_III domain, wherein the substantially identical to any one of SEQ ID NOs: 2499-2750. The endonuclease may comprise a RuvC_III domain having at least about 70% sequence identity to any one of SEQ ID NOs: 2499-2750. In some cases, the endonuclease may comprise a RuvC_III domain having at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99% identity to any one of SEQ ID NOs: 2499-2750. In some cases, the endonuclease may comprise a RuvC_III domain substantially identical to any one of SEQ ID NOs: 2499-2750.

The endonuclease may comprise an HNH domain having at least about 70% identity to any one of SEQ ID NOs: 4313-4564. In some cases, the endonuclease may comprise an HNH domain having at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99% identical to any one of SEQ ID NOs: 4313-4564. The endonuclease may comprise an HNH domain substantially identical to any one of SEQ ID NOs: 4313-4564. The endonuclease may comprise an HNH domain having at least about 70% identity to any one of SEQ ID NOs: 4313-4564. In some cases, the endonuclease may comprise an HNH domain having at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99% identical to any one of SEQ ID NOs: 4067-4295. The endonuclease may comprise an HNH domain substantially identical to any one of SEQ ID NOs: 4313-4564.

In some cases, the endonuclease may comprise a variant having at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% identity to any one of SEQ ID NOs: 678-929. In some cases, the endonuclease may be substantially identical to any one of SEQ ID NOs: 678-929. In some cases, the endonuclease may comprise a variant having at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% identity to any one of SEQ ID NOs: 678-929. In some cases, the endonuclease may be substantially identical to any one of SEQ ID NOs: 678-929.

In some cases, the endonuclease may comprise a variant having one or more nuclear localization sequences (NLSs). The NLS may be proximal to the N- or C-terminus of said endonuclease. The NLS may be appended N-terminal or C-terminal to any one of SEQ ID NOs: 678-929, or to a variant having at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% identity to any one of SEQ ID NOs: 678-929. The NLS may be an SV40 large T antigen NLS. The NLS may be a c-myc NLS. The NLS can comprise a sequence with at least about 80%, at least about 85%, at least about 90%, at least about 95%, at least about 99% identity to any one of SEQ ID NOs: 5593-5608. The NLS can comprise a sequence substantially identical to any one of SEQ ID NOs: 5593-5608. The NLS can comprise any of the sequences in Table 1 or a combination thereof:

In some cases, sequence identity may be determined by the BLASTP, CLUSTALW, MUSCLE, MAFFT, Novafold, or CLUSTALW with the parameters of the Smith-Waterman homology search algorithm. The sequence identity may be determined by the BLASTP algorithm using parameters of a wordlength (W) of 3, an expectation (E) of 10, and using a BLOSUM62 scoring matrix setting gap costs at existence of 11, extension of 1, and using a conditional compositional score matrix adjustment.

In some cases, the system above may comprise (b) at least one engineered synthetic guide ribonucleic acid (sgRNA) capable of forming a complex with the endonuclease bearing a 5' targeting region complementary to a desired cleavage sequence. In some cases, the 5' targeting region may comprises a PAM sequence compatible with the endonuclease. In some cases, the 5' most nucleotide of the targeting region may be G. In some cases, the 5' targeting region may be 15-23 nucleotides in length. The guide sequence and the tracr sequence may be supplied as separate ribonucleic acids (RNAs) or a single ribonucleic acid (RNA). The guide RNA may comprise a crRNA tracrRNA binding sequence 3' to the targeting region. The guide RNA may comprise a tracrRNA sequence preceded by a 4-nucleotide linker 3' to the crRNA tracrRNA binding region. The sgRNA may comprise, from 5' to 3': a non-natural guide nucleic acid sequence capable of hybridizing to a target sequence in a cell; and a tracr sequence. In some cases, the non-natural guide nucleic acid sequence and the tracr sequence are covalently linked.

In some cases, the tracr sequence may have a particular sequence. The tracr sequence may have at least about 80% to at least about 60-100 (e.g., at least about 60, at least about 65, at least about 70, at least about 75, at least about 80, at least about 85, or at least about 90) consecutive nucleotides of a natural tracrRNA sequence. The tracr sequence may have at least about 80% sequence identity to at least about 60-100 (e.g., at least about 60, at least about 65, at least about 70, at least about 75, at least about 80, at least about 85, or at least about 90) consecutive nucleotides of SEQ ID NO: 5505. In some cases, the tracrRNA may have at least about 80%, at least about 85%, at least about 90%, at least about 91%, at least about 92%, at least about 93%, at least about 94%, at least about 95%, at least about 96%, at least about 97%, at least about 98%, or at least about 99% identity to at least about 60-90 (e.g., at least about 60, at least about 65, at least about 70, at least about 75, at least about 80, at least about 85, or at least about 90) consecutive nucleotides of SEQ ID NO: 5505. In some cases, the tracrRNA may be substantially identical to at least about 60-100 (e.g., at least about 60, at least about 65, at least about 70, at least about 75, at least about 80, at least about 85, or at least about 90) consecutive nucleotides of SEQ ID NO: 5505. The tracrRNA may comprise SEQ ID NO: 5505.

In some cases, the at least one engineered synthetic guide ribonucleic acid (sgRNA) capable of forming a complex with the endonuclease may comprise a sequence having at least about 80% identity to SEQ ID NO: 5469. The sgRNA may comprise a sequence having at least about 80%, at least about 85%, at least about 90%, at least about 91%, at least about 92%, at least about 93%, at least about 94%, at least about 95%, at least about 96%, at least about 97%, at least about 98%, or at least about 99% identity to SEQ ID NO: 5469. The sgRNA may comprise a sequence substantially identical to SEQ ID NO: 5469.

In some cases, the system above may comprise two different sgRNAs targeting a first region and a second region for cleavage in a target DNA locus, wherein the second region is 3' to the first region. In some cases, the system above may comprise a single- or double-stranded DNA repair template comprising from 5' to 3': a first homology arm comprising a sequence of at least about 20 (e.g., at least about 40, 80, 120, 150, 200, 300, 500, or 1kb) nucleotides 5' to the first region, a synthetic DNA sequence of at least about 10 nucleotides, and a second homology arm comprising a sequence of at least about 20 (e.g., at least about 40, 80, 120, 150, 200, 300, 500, or 1kb) nucleotides 3' to the second region.

In another aspect, the present disclosure provides a method for modifying a target nucleic acid locus of interest. The method may comprise delivering to the target nucleic acid locus any of the non-natural systems disclosed herein, including an enzyme and at least one synthetic guide RNA (sgRNA) disclosed herein. The enzyme may form a complex with the at least one sgRNA, and upon binding of the complex to the target nucleic acid locus of interest, may modify the target nucleic acid locus of interest. Delivering the enzyme to said locus may comprise transfecting a cell with the system or nucleic acids encoding the system. Delivering the nuclease to said locus may comprise electroporating a cell with the system or nucleic acids encoding the system. Delivering the nuclease to said locus may comprise incubating the system in a buffer with a nucleic acid comprising the locus of interest. In some cases, the target nucleic acid locus comprises deoxyribonucleic acid (DNA) or ribonucleic acid (RNA). The target nucleic acid locus may comprise genomic DNA, viral DNA, viral RNA, or bacterial DNA. The target nucleic acid locus may be within a cell. The target nucleic acid locus may be in vitro. The target nucleic acid locus may be within a eukaryotic cell or a prokaryotic cell. The cell may be an animal cell, a human cell, bacterial cell, archaeal cell, or a plant cell. The enzyme may induce a single or double-stranded break at or proximal to the target locus of interest.

In cases where the target nucleic acid locus may be within a cell, the enzyme may be supplied as a nucleic acid containing an open reading frame encoding the enzyme having a RuvC_III domain having at least about 75% (e.g., at least about 90%, at least about 91%, at least about 92%, at least about 93%, at least about 94%, at least about 95%, at least about 96%, at least about 97%, at least about 98%, at least about 99%) identity to any one of SEQ ID NOs: 2499-2750. The deoxyribonucleic acid (DNA) containing an open reading frame encoding said endonuclease may comprise a sequence substantially identical to SEQ ID NO: 5581 or at variant having at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% identity to SEQ ID NO: 5581. In some cases, the nucleic acid comprises a promoter to which the open reading frame encoding the endonuclease is operably linked. The promoter may be a CMV, EF1a, SV40, PGK1, Ubc, human beta actin, CAG, TRE, or CaMKIIa promoter. The endonuclease may be supplied as a capped mRNA containing said open reading frame encoding said endonuclease. The endonuclease may be supplied as a translated polypeptide. The at least one engineered sgRNA may be supplied as deoxyribonucleic acid (DNA) containing a gene sequence encoding said at least one engineered sgRNA operably linked to a ribonucleic acid (RNA) pol III promoter. In some cases, the organism may be eukaryotic. In some cases, the organism may be fungal. In some cases, the organism may be human.

### MG15 Enzymes

In one aspect, the present disclosure provides for an engineered nuclease system comprising (a) an endonuclease. In some cases, the endonuclease is a Cas endonuclease. In some cases, the endonuclease is a Type II, Class II Cas endonuclease. The endonuclease may comprise a RuvC_III domain, wherein said RuvC_III domain has at least about 70% sequence identity to any one of SEQ ID NOs: 2751-2913. In some cases, the endonuclease may comprise a RuvC_III domain, wherein the RuvC_III domain has at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99% identity to any one of SEQ ID NOs: 2751-2913. In some cases, the endonuclease may comprise a RuvC_III domain, wherein the substantially identical to any one of SEQ ID NOs: 2751-2913. The endonuclease may comprise a RuvC_III domain having at least about 70% sequence identity to any one of SEQ ID NOs: 2751-2913. In some cases, the endonuclease may comprise a RuvC_III domain having at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99% identity to any one of SEQ ID NOs: 2751-2913. In some cases, the endonuclease may comprise a RuvC_III domain substantially identical to any one of SEQ ID NOs: 2751-2913.

The endonuclease may comprise an HNH domain having at least about 70% identity to any one of SEQ ID NOs: 4565-4727. In some cases, the endonuclease may comprise an HNH domain having at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99% identical to any one of SEQ ID NOs: 4565-4727. The endonuclease may comprise an HNH domain substantially identical to any one of SEQ ID NOs: 4565-4727. The endonuclease may comprise an HNH domain having at least about 70% identity to any one of SEQ ID NOs: 4565-4727. In some cases, the endonuclease may comprise an HNH domain having at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99% identical to any one of SEQ ID NOs: 4565-4727. The endonuclease may comprise an HNH domain substantially identical to any one of SEQ ID NOs: 4565-4727.

In some cases, the endonuclease may comprise a variant having at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% identity to any one of SEQ ID NOs: 930-1092. In some cases, the endonuclease may be substantially identical to any one of SEQ ID NOs: 930-1092. In some cases, the endonuclease may comprise a variant having at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% identity to any one of SEQ ID NOs: 930-1092. In some cases, the endonuclease may be substantially identical to any one of SEQ ID NOs: 930-1092.

In some cases, the endonuclease may comprise a variant having one or more nuclear localization sequences (NLSs). The NLS may be proximal to the N- or C-terminus of said endonuclease. The NLS may be appended N-terminal or C-terminal to any one of SEQ ID NOs: 930-1092, or to a variant having at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% identity to any one of SEQ ID NOs: 930-1092. The NLS may be an SV40 large T antigen NLS. The NLS may be a c-myc NLS. The NLS can comprise a sequence with at least about 80%, at least about 85%, at least about 90%, at least about 95%, at least about 99% identity to any one of SEQ ID NOs: 5593-5608. The NLS can comprise a sequence substantially identical to any one of SEQ ID NOs: 5593-5608. The NLS can comprise any of the sequences in Table 1 or a combination thereof:

In some cases, sequence identity may be determined by the BLASTP, CLUSTALW, MUSCLE, MAFFT, Novafold, or CLUSTALW with the parameters of the Smith-Waterman homology search algorithm. The sequence identity may be determined by the BLASTP algorithm using parameters of a wordlength (W) of 3, an expectation (E) of 10, and using a BLOSUM62 scoring matrix setting gap costs at existence of 11, extension of 1, and using a conditional compositional score matrix adjustment.

In some cases, the system above may comprise (b) at least one engineered synthetic guide ribonucleic acid (sgRNA) capable of forming a complex with the endonuclease bearing a 5' targeting region complementary to a desired cleavage sequence. In some cases, the 5' targeting region may comprises a PAM sequence compatible with the endonuclease. In some cases, the 5' most nucleotide of the targeting region may be G. In some cases, the 5' targeting region may be 15-23 nucleotides in length. The guide sequence and the tracr sequence may be supplied as separate ribonucleic acids (RNAs) or a single ribonucleic acid (RNA). The guide RNA may comprise a crRNA tracrRNA binding sequence 3' to the targeting region. The guide RNA may comprise a tracrRNA sequence preceded by a 4-nucleotide linker 3' to the crRNA tracrRNA binding region. The sgRNA may comprise, from 5' to 3': a non-natural guide nucleic acid sequence capable of hybridizing to a target sequence in a cell; and a tracr sequence. In some cases, the non-natural guide nucleic acid sequence and the tracr sequence are covalently linked.

In some cases, the tracr sequence may have a particular sequence. The tracr sequence may have at least about 80% to at least about 60-100 (e.g., at least about 60, at least about 65, at least about 70, at least about 75, at least about 80, at least about 85, or at least about 90) consecutive nucleotides of a natural tracrRNA sequence. The tracr sequence may have at least about 80% sequence identity to at least about 60-100 (e.g., at least about 60, at least about 65, at least about 70, at least about 75, at least about 80, at least about 85, or at least about 90) consecutive nucleotides of SEQ ID NO: 5506. In some cases, the tracrRNA may have at least about 80%, at least about 85%, at least about 90%, at least about 91%, at least about 92%, at least about 93%, at least about 94%, at least about 95%, at least about 96%, at least about 97%, at least about 98%, or at least about 99% identity to at least about 60-90 (e.g., at least about 60, at least about 65, at least about 70, at least about 75, at least about 80, at least about 85, or at least about 90) consecutive nucleotides of SEQ ID NO: 5506. In some cases, the tracrRNA may be substantially identical to at least about 60-100 (e.g., at least about 60, at least about 65, at least about 70, at least about 75, at least about 80, at least about 85, or at least about 90) consecutive nucleotides of SEQ ID NO: 5506. The tracrRNA may comprise SEQ ID NO: 5506.

In some cases, the at least one engineered synthetic guide ribonucleic acid (sgRNA) capable of forming a complex with the endonuclease may comprise a sequence having at least about 80% identity to SEQ ID NO: 5470. The sgRNA may comprise a sequence having at least about 80%, at least about 85%, at least about 90%, at least about 91%, at least about 92%, at least about 93%, at least about 94%, at least about 95%, at least about 96%, at least about 97%, at least about 98%, or at least about 99% identity to SEQ ID NO: 5470. The sgRNA may comprise a sequence substantially identical to SEQ ID NO: 5470.

In some cases, the system above may comprise two different sgRNAs targeting a first region and a second region for cleavage in a target DNA locus, wherein the second region is 3' to the first region. In some cases, the system above may comprise a single- or double-stranded DNA repair template comprising from 5' to 3': a first homology arm comprising a sequence of at least about 20 (e.g., at least about 40, 80, 120, 150, 200, 300, 500, or 1kb) nucleotides 5' to the first region, a synthetic DNA sequence of at least about 10 nucleotides, and a second homology arm comprising a sequence of at least about 20 (e.g., at least about 40, 80, 120, 150, 200, 300, 500, or 1kb) nucleotides 3' to the second region.

In another aspect, the present disclosure provides a method for modifying a target nucleic acid locus of interest. The method may comprise delivering to the target nucleic acid locus any of the non-natural systems disclosed herein, including an enzyme and at least one synthetic guide RNA (sgRNA) disclosed herein. The enzyme may form a complex with the at least one sgRNA, and upon binding of the complex to the target nucleic acid locus of interest, may modify the target nucleic acid locus of interest. Delivering the enzyme to said locus may comprise transfecting a cell with the system or nucleic acids encoding the system. Delivering the nuclease to said locus may comprise electroporating a cell with the system or nucleic acids encoding the system. Delivering the nuclease to said locus may comprise incubating the system in a buffer with a nucleic acid comprising the locus of interest. In some cases, the target nucleic acid locus comprises deoxyribonucleic acid (DNA) or ribonucleic acid (RNA). The target nucleic acid locus may comprise genomic DNA, viral DNA, viral RNA, or bacterial DNA. The target nucleic acid locus may be within a cell. The target nucleic acid locus may be in vitro. The target nucleic acid locus may be within a eukaryotic cell or a prokaryotic cell. The cell may be an animal cell, a human cell, bacterial cell, archaeal cell, or a plant cell. The enzyme may induce a single or double-stranded break at or proximal to the target locus of interest.

In cases where the target nucleic acid locus may be within a cell, the enzyme may be supplied as a nucleic acid containing an open reading frame encoding the enzyme having a RuvC_III domain having at least about 75% (e.g., at least about 90%, at least about 91%, at least about 92%, at least about 93%, at least about 94%, at least about 95%, at least about 96%, at least about 97%, at least about 98%, at least about 99%) identity to any one of SEQ ID NOs: 2751-2913. The deoxyribonucleic acid (DNA) containing an open reading frame encoding said endonuclease may comprise a sequence substantially identical to SEQ ID NO: 5582 or at variant having at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% identity to SEQ ID NO: 5582. In some cases, the nucleic acid comprises a promoter to which the open reading frame encoding the endonuclease is operably linked. The promoter may be a CMV, EF1a, SV40, PGK1, Ubc, human beta actin, CAG, TRE, or CaMKIIa promoter. The endonuclease may be supplied as a capped mRNA containing said open reading frame encoding said endonuclease. The endonuclease may be supplied as a translated polypeptide. The at least one engineered sgRNA may be supplied as deoxyribonucleic acid (DNA) containing a gene sequence encoding said at least one engineered sgRNA operably linked to a ribonucleic acid (RNA) pol III promoter. In some cases, the organism may be eukaryotic. In some cases, the organism may be fungal. In some cases, the organism may be human.

### MG 16 Enzymes

In one aspect, the present disclosure provides for an engineered nuclease system comprising (a) an endonuclease. In some cases, the endonuclease is a Cas endonuclease. In some cases, the endonuclease is a Type II, Class II Cas endonuclease. The endonuclease may comprise a RuvC_III domain, wherein said RuvC_III domain has at least about 70% sequence identity to any one of SEQ ID NOs: 2914-3174. In some cases, the endonuclease may comprise a RuvC_III domain, wherein the RuvC_III domain has at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99% identity to any one of SEQ ID NOs: 2914-3174. In some cases, the endonuclease may comprise a RuvC_III domain, wherein the substantially identical to any one of SEQ ID NOs: 2914-3174. The endonuclease may comprise a RuvC_III domain having at least about 70% sequence identity to any one of SEQ ID NOs: 2914-3174. In some cases, the endonuclease may comprise a RuvC_III domain having at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99% identity to any one of SEQ ID NOs: 2914-3174. In some cases, the endonuclease may comprise a RuvC_III domain substantially identical to any one of SEQ ID NOs: 2914-3174.

The endonuclease may comprise an HNH domain having at least about 70% identity to any one of SEQ ID NOs: 4728-4988. In some cases, the endonuclease may comprise an HNH domain having at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99% identical to any one of SEQ ID NOs: 4728-4988. The endonuclease may comprise an HNH domain substantially identical to any one of SEQ ID NOs: 4728-4988. The endonuclease may comprise an HNH domain having at least about 70% identity to any one of SEQ ID NOs: 4728-4988. In some cases, the endonuclease may comprise an HNH domain having at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99% identical to any one of SEQ ID NOs: 4728-4988. The endonuclease may comprise an HNH domain substantially identical to any one of SEQ ID NOs: 4728-4988.

In some cases, the endonuclease may comprise a variant having at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% identity to any one of SEQ ID NOs: 1093-1353. In some cases, the endonuclease may be substantially identical to any one of SEQ ID NOs: 1093-1353. In some cases, the endonuclease may comprise a variant having at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% identity to any one of SEQ ID NOs: 1093-1353. In some cases, the endonuclease may be substantially identical to any one of SEQ ID NOs: 1093-1353.

In some cases, the endonuclease may comprise a variant having one or more nuclear localization sequences (NLSs). The NLS may be proximal to the N- or C-terminus of said endonuclease. The NLS may be appended N-terminal or C-terminal to any one of SEQ ID NOs: 1093-1353, or to a variant having at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% identity to any one of SEQ ID NOs: 1093-1353. The NLS may be an SV40 large T antigen NLS. The NLS may be a c-myc NLS. The NLS can comprise a sequence with at least about 80%, at least about 85%, at least about 90%, at least about 95%, at least about 99% identity to any one of SEQ ID NOs: 5593-5608. The NLS can comprise a sequence substantially identical to any one of SEQ ID NOs: 5593-5608. The NLS can comprise any of the sequences in Table 1 or a combination thereof:

In some cases, sequence identity may be determined by the BLASTP, CLUSTALW, MUSCLE, MAFFT, Novafold, or CLUSTALW with the parameters of the Smith-Waterman homology search algorithm. The sequence identity may be determined by the BLASTP algorithm using parameters of a wordlength (W) of 3, an expectation (E) of 10, and using a BLOSUM62 scoring matrix setting gap costs at existence of 11, extension of 1, and using a conditional compositional score matrix adjustment.

In some cases, the system above may comprise (b) at least one engineered synthetic guide ribonucleic acid (sgRNA) capable of forming a complex with the endonuclease bearing a 5' targeting region complementary to a desired cleavage sequence. In some cases, the 5' targeting region may comprises a PAM sequence compatible with the endonuclease. In some cases, the 5' most nucleotide of the targeting region may be G. In some cases, the 5' targeting region may be 15-23 nucleotides in length. The guide sequence and the tracr sequence may be supplied as separate ribonucleic acids (RNAs) or a single ribonucleic acid (RNA). The guide RNA may comprise a crRNA tracrRNA binding sequence 3' to the targeting region. The guide RNA may comprise a tracrRNA sequence preceded by a 4-nucleotide linker 3' to the crRNA tracrRNA binding region. The sgRNA may comprise, from 5' to 3': a non-natural guide nucleic acid sequence capable of hybridizing to a target sequence in a cell; and a tracr sequence. In some cases, the non-natural guide nucleic acid sequence and the tracr sequence are covalently linked.

In some cases, the tracr sequence may have a particular sequence. The tracr sequence may have at least about 80% to at least about 60-100 (e.g., at least about 60, at least about 65, at least about 70, at least about 75, at least about 80, at least about 85, or at least about 90) consecutive nucleotides of a natural tracrRNA sequence. The tracr sequence may have at least about 80% sequence identity to at least about 60-100 (e.g., at least about 60, at least about 65, at least about 70, at least about 75, at least about 80, at least about 85, or at least about 90) consecutive nucleotides of SEQ ID NO: 5507. In some cases, the tracrRNA may have at least about 80%, at least about 85%, at least about 90%, at least about 91%, at least about 92%, at least about 93%, at least about 94%, at least about 95%, at least about 96%, at least about 97%, at least about 98%, or at least about 99% identity to at least about 60-90 (e.g., at least about 60, at least about 65, at least about 70, at least about 75, at least about 80, at least about 85, or at least about 90) consecutive nucleotides of SEQ ID NO: 5507. In some cases, the tracrRNA may be substantially identical to at least about 60-100 (e.g., at least about 60, at least about 65, at least about 70, at least about 75, at least about 80, at least about 85, or at least about 90) consecutive nucleotides of SEQ ID NO: 5507. The tracrRNA may comprise SEQ ID NO: 5507.

In some cases, the at least one engineered synthetic guide ribonucleic acid (sgRNA) capable of forming a complex with the endonuclease may comprise a sequence having at least about 80% identity to SEQ ID NO: 5471. The sgRNA may comprise a sequence having at least about 80%, at least about 85%, at least about 90%, at least about 91%, at least about 92%, at least about 93%, at least about 94%, at least about 95%, at least about 96%, at least about 97%, at least about 98%, or at least about 99% identity to SEQ ID NO: 5471. The sgRNA may comprise a sequence substantially identical to SEQ ID NO: 5471.

In some cases, the system above may comprise two different sgRNAs targeting a first region and a second region for cleavage in a target DNA locus, wherein the second region is 3' to the first region. In some cases, the system above may comprise a single- or double-stranded DNA repair template comprising from 5' to 3': a first homology arm comprising a sequence of at least about 20 (e.g., at least about 40, 80, 120, 150, 200, 300, 500, or 1kb) nucleotides 5' to the first region, a synthetic DNA sequence of at least about 10 nucleotides, and a second homology arm comprising a sequence of at least about 20 (e.g., at least about 40, 80, 120, 150, 200, 300, 500, or 1kb) nucleotides 3' to the second region.

In another aspect, the present disclosure provides a method for modifying a target nucleic acid locus of interest. The method may comprise delivering to the target nucleic acid locus any of the non-natural systems disclosed herein, including an enzyme and at least one synthetic guide RNA (sgRNA) disclosed herein. The enzyme may form a complex with the at least one sgRNA, and upon binding of the complex to the target nucleic acid locus of interest, may modify the target nucleic acid locus of interest. Delivering the enzyme to said locus may comprise transfecting a cell with the system or nucleic acids encoding the system. Delivering the nuclease to said locus may comprise electroporating a cell with the system or nucleic acids encoding the system. Delivering the nuclease to said locus may comprise incubating the system in a buffer with a nucleic acid comprising the locus of interest. In some cases, the target nucleic acid locus comprises deoxyribonucleic acid (DNA) or ribonucleic acid (RNA). The target nucleic acid locus may comprise genomic DNA, viral DNA, viral RNA, or bacterial DNA. The target nucleic acid locus may be within a cell. The target nucleic acid locus may be in vitro. The target nucleic acid locus may be within a eukaryotic cell or a prokaryotic cell. The cell may be an animal cell, a human cell, bacterial cell, archaeal cell, or a plant cell. The enzyme may induce a single or double-stranded break at or proximal to the target locus of interest.

In cases where the target nucleic acid locus may be within a cell, the enzyme may be supplied as a nucleic acid containing an open reading frame encoding the enzyme having a RuvC_III domain having at least about 75% (e.g., at least about 90%, at least about 91%, at least about 92%, at least about 93%, at least about 94%, at least about 95%, at least about 96%, at least about 97%, at least about 98%, at least about 99%) identity to any one of SEQ ID NOs: 2914-3174. The deoxyribonucleic acid (DNA) containing an open reading frame encoding said endonuclease may comprise a sequence substantially identical to SEQ ID NO: 5583 or at variant having at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% identity to SEQ ID NO: 5583. In some cases, the nucleic acid comprises a promoter to which the open reading frame encoding the endonuclease is operably linked. The promoter may be a CMV, EF1a, SV40, PGK1, Ubc, human beta actin, CAG, TRE, or CaMKIIa promoter. The endonuclease may be supplied as a capped mRNA containing said open reading frame encoding said endonuclease. The endonuclease may be supplied as a translated polypeptide. The at least one engineered sgRNA may be supplied as deoxyribonucleic acid (DNA) containing a gene sequence encoding said at least one engineered sgRNA operably linked to a ribonucleic acid (RNA) pol III promoter. In some cases, the organism may be eukaryotic. In some cases, the organism may be fungal. In some cases, the organism may be human.

### MG18 Enzymes

In one aspect, the present disclosure provides for an engineered nuclease system comprising (a) an endonuclease. In some cases, the endonuclease is a Cas endonuclease. In some cases, the endonuclease is a Type II, Class II Cas endonuclease. The endonuclease may comprise a RuvC_III domain, wherein said RuvC_III domain has at least about 70% sequence identity to any one of SEQ ID NOs: 3175-3300. In some cases, the endonuclease may comprise a RuvC_III domain, wherein the RuvC_III domain has at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99% identity to any one of SEQ ID NOs: 3175-3300. In some cases, the endonuclease may comprise a RuvC_III domain, wherein the substantially identical to any one of SEQ ID NOs: 3175-3300. The endonuclease may comprise a RuvC_III domain having at least about 70% sequence identity to any one of SEQ ID NOs: 3175-3300. In some cases, the endonuclease may comprise a RuvC_III domain having at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99% identity to any one of SEQ ID NOs: 3175-3300. In some cases, the endonuclease may comprise a RuvC_III domain substantially identical to any one of SEQ ID NOs: 3175-3300.

The endonuclease may comprise an HNH domain having at least about 70% identity to any one of SEQ ID NOs: 4989-5146. In some cases, the endonuclease may comprise an HNH domain having at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99% identical to any one of SEQ ID NOs: 4989-5146. The endonuclease may comprise an HNH domain substantially identical to any one of SEQ ID NOs: 4989-5146. The endonuclease may comprise an HNH domain having at least about 70% identity to any one of SEQ ID NOs: 4989-5146. In some cases, the endonuclease may comprise an HNH domain having at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99% identical to any one of SEQ ID NOs: 4989-5146. The endonuclease may comprise an HNH domain substantially identical to any one of SEQ ID NOs: 4989-5146.

In some cases, the endonuclease may comprise a variant having at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% identity to any one of SEQ ID NOs: 1354-1511. In some cases, the endonuclease may be substantially identical to any one of SEQ ID NOs: 1354-1511. In some cases, the endonuclease may comprise a variant having at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% identity to any one of SEQ ID NOs: 1354-1511. In some cases, the endonuclease may be substantially identical to any one of SEQ ID NOs: 1354-1511.

In some cases, the endonuclease may comprise a variant having one or more nuclear localization sequences (NLSs). The NLS may be proximal to the N- or C-terminus of said endonuclease. The NLS may be appended N-terminal or C-terminal to any one of SEQ ID NOs: 1354-1511, or to a variant having at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% identity to any one of SEQ ID NOs: 1354-1511. The NLS may be an SV40 large T antigen NLS. The NLS may be a c-myc NLS. The NLS can comprise a sequence with at least about 80%, at least about 85%, at least about 90%, at least about 95%, at least about 99% identity to any one of SEQ ID NOs: 5593-5608. The NLS can comprise a sequence substantially identical to any one of SEQ ID NOs: 5593-5608. The NLS can comprise any of the sequences in Table 1 or a combination thereof:

In some cases, sequence identity may be determined by the BLASTP, CLUSTALW, MUSCLE, MAFFT, Novafold, or CLUSTALW with the parameters of the Smith-Waterman homology search algorithm. The sequence identity may be determined by the BLASTP algorithm using parameters of a wordlength (W) of 3, an expectation (E) of 10, and using a BLOSUM62 scoring matrix setting gap costs at existence of 11, extension of 1, and using a conditional compositional score matrix adjustment.

In some cases, the system above may comprise (b) at least one engineered synthetic guide ribonucleic acid (sgRNA) capable of forming a complex with the endonuclease bearing a 5' targeting region complementary to a desired cleavage sequence. In some cases, the 5' targeting region may comprises a PAM sequence compatible with the endonuclease. In some cases, the 5' most nucleotide of the targeting region may be G. In some cases, the 5' targeting region may be 15-23 nucleotides in length. The guide sequence and the tracr sequence may be supplied as separate ribonucleic acids (RNAs) or a single ribonucleic acid (RNA). The guide RNA may comprise a crRNA tracrRNA binding sequence 3' to the targeting region. The guide RNA may comprise a tracrRNA sequence preceded by a 4-nucleotide linker 3' to the crRNA tracrRNA binding region. The sgRNA may comprise, from 5' to 3': a non-natural guide nucleic acid sequence capable of hybridizing to a target sequence in a cell; and a tracr sequence. In some cases, the non-natural guide nucleic acid sequence and the tracr sequence are covalently linked.

In some cases, the tracr sequence may have a particular sequence. The tracr sequence may have at least about 80% to at least about 60-100 (e.g., at least about 60, at least about 65, at least about 70, at least about 75, at least about 80, at least about 85, or at least about 90) consecutive nucleotides of a natural tracrRNA sequence. The tracr sequence may have at least about 80% sequence identity to at least about 60-100 (e.g., at least about 60, at least about 65, at least about 70, at least about 75, at least about 80, at least about 85, or at least about 90) consecutive nucleotides of SEQ ID NO: 5508. In some cases, the tracrRNA may have at least about 80%, at least about 85%, at least about 90%, at least about 91%, at least about 92%, at least about 93%, at least about 94%, at least about 95%, at least about 96%, at least about 97%, at least about 98%, or at least about 99% identity to at least about 60-90 (e.g., at least about 60, at least about 65, at least about 70, at least about 75, at least about 80, at least about 85, or at least about 90) consecutive nucleotides of SEQ ID NO: 5508. In some cases, the tracrRNA may be substantially identical to at least about 60-100 (e.g., at least about 60, at least about 65, at least about 70, at least about 75, at least about 80, at least about 85, or at least about 90) consecutive nucleotides of SEQ ID NO: 5508. The tracrRNA may comprise SEQ ID NO: 5508.

In some cases, the at least one engineered synthetic guide ribonucleic acid (sgRNA) capable of forming a complex with the endonuclease may comprise a sequence having at least about 80% identity to SEQ ID NO: 5472. The sgRNA may comprise a sequence having at least about 80%, at least about 85%, at least about 90%, at least about 91%, at least about 92%, at least about 93%, at least about 94%, at least about 95%, at least about 96%, at least about 97%, at least about 98%, or at least about 99% identity to SEQ ID NO: 5472. The sgRNA may comprise a sequence substantially identical to SEQ ID NO: 5472.

In some cases, the system above may comprise two different sgRNAs targeting a first region and a second region for cleavage in a target DNA locus, wherein the second region is 3' to the first region. In some cases, the system above may comprise a single- or double-stranded DNA repair template comprising from 5' to 3': a first homology arm comprising a sequence of at least about 20 (e.g., at least about 40, 80, 120, 150, 200, 300, 500, or 1kb) nucleotides 5' to the first region, a synthetic DNA sequence of at least about 10 nucleotides, and a second homology arm comprising a sequence of at least about 20 (e.g., at least about 40, 80, 120, 150, 200, 300, 500, or 1kb) nucleotides 3' to the second region.

In another aspect, the present disclosure provides a method for modifying a target nucleic acid locus of interest. The method may comprise delivering to the target nucleic acid locus any of the non-natural systems disclosed herein, including an enzyme and at least one synthetic guide RNA (sgRNA) disclosed herein. The enzyme may form a complex with the at least one sgRNA, and upon binding of the complex to the target nucleic acid locus of interest, may modify the target nucleic acid locus of interest. Delivering the enzyme to said locus may comprise transfecting a cell with the system or nucleic acids encoding the system. Delivering the nuclease to said locus may comprise electroporating a cell with the system or nucleic acids encoding the system. Delivering the nuclease to said locus may comprise incubating the system in a buffer with a nucleic acid comprising the locus of interest. In some cases, the target nucleic acid locus comprises deoxyribonucleic acid (DNA) or ribonucleic acid (RNA). The target nucleic acid locus may comprise genomic DNA, viral DNA, viral RNA, or bacterial DNA. The target nucleic acid locus may be within a cell. The target nucleic acid locus may be in vitro. The target nucleic acid locus may be within a eukaryotic cell or a prokaryotic cell. The cell may be an animal cell, a human cell, bacterial cell, archaeal cell, or a plant cell. The enzyme may induce a single or double-stranded break at or proximal to the target locus of interest.

In cases where the target nucleic acid locus may be within a cell, the enzyme may be supplied as a nucleic acid containing an open reading frame encoding the enzyme having a RuvC_III domain having at least about 75% (e.g., at least about 90%, at least about 91%, at least about 92%, at least about 93%, at least about 94%, at least about 95%, at least about 96%, at least about 97%, at least about 98%, at least about 99%) identity to any one of SEQ ID NOs: 3175-3300. The deoxyribonucleic acid (DNA) containing an open reading frame encoding said endonuclease may comprise a sequence substantially identical to SEQ ID NOs: 5584 or at variant having at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% identity to SEQ ID NOs: 5584. In some cases, the nucleic acid comprises a promoter to which the open reading frame encoding the endonuclease is operably linked. The promoter may be a CMV, EF1a, SV40, PGK1, Ubc, human beta actin, CAG, TRE, or CaMKIIa promoter. The endonuclease may be supplied as a capped mRNA containing said open reading frame encoding said endonuclease. The endonuclease may be supplied as a translated polypeptide. The at least one engineered sgRNA may be supplied as deoxyribonucleic acid (DNA) containing a gene sequence encoding said at least one engineered sgRNA operably linked to a ribonucleic acid (RNA) pol III promoter. In some cases, the organism may be eukaryotic. In some cases, the organism may be fungal. In some cases, the organism may be human.

### MG21 Enzymes

In one aspect, the present disclosure provides for an engineered nuclease system comprising (a) an endonuclease. In some cases, the endonuclease is a Cas endonuclease. In some cases, the endonuclease is a Type II, Class II Cas endonuclease. The endonuclease may comprise a RuvC_III domain, wherein said RuvC_III domain has at least about 70% sequence identity to any one of SEQ ID NOs: 3331-3474. In some cases, the endonuclease may comprise a RuvC_III domain, wherein the RuvC_III domain has at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99% identity to any one of SEQ ID NOs: 3331-3474. In some cases, the endonuclease may comprise a RuvC_III domain, wherein the substantially identical to any one of SEQ ID NOs: 3331-3474. The endonuclease may comprise a RuvC_III domain having at least about 70% sequence identity to any one of SEQ ID NOs: 3331-3474. In some cases, the endonuclease may comprise a RuvC_III domain having at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99% identity to any one of SEQ ID NOs: 3331-3474. In some cases, the endonuclease may comprise a RuvC_III domain substantially identical to any one of SEQ ID NOs: 3331-3474.

The endonuclease may comprise an HNH domain having at least about 70% identity to any one of SEQ ID NOs: 5147-5290. In some cases, the endonuclease may comprise an HNH domain having at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99% identical to any one of SEQ ID NOs: 5147-5290. The endonuclease may comprise an HNH domain substantially identical to any one of SEQ ID NOs: 5147-5290. The endonuclease may comprise an HNH domain having at least about 70% identity to any one of SEQ ID NOs: 5147-5290. In some cases, the endonuclease may comprise an HNH domain having at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99% identical to any one of SEQ ID NOs: 5147-5290. The endonuclease may comprise an HNH domain substantially identical to any one of SEQ ID NOs: 5147-5290.

In some cases, the endonuclease may comprise a variant having at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% identity to any one of SEQ ID NOs: 1512-1655. In some cases, the endonuclease may be substantially identical to any one of SEQ ID NOs: 1512-1655. In some cases, the endonuclease may comprise a variant having at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% identity to any one of SEQ ID NOs: 1512-1655. In some cases, the endonuclease may be substantially identical to any one of SEQ ID NOs: 1512-1655.

In some cases, the endonuclease may comprise a variant having one or more nuclear localization sequences (NLSs). The NLS may be proximal to the N- or C-terminus of said endonuclease. The NLS may be appended N-terminal or C-terminal to any one of SEQ ID NOs: 1512-1655, or to a variant having at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% identity to any one of SEQ ID NOs: 1512-1655. The NLS may be an SV40 large T antigen NLS. The NLS may be a c-myc NLS. The NLS can comprise a sequence with at least about 80%, at least about 85%, at least about 90%, at least about 95%, at least about 99% identity to any one of SEQ ID NOs: 5593-5608. The NLS can comprise a sequence substantially identical to any one of SEQ ID NOs: 5593-5608. The NLS can comprise any of the sequences in Table 1 or a combination thereof:

In some cases, sequence identity may be determined by the BLASTP, CLUSTALW, MUSCLE, MAFFT, Novafold, or CLUSTALW with the parameters of the Smith-Waterman homology search algorithm. The sequence identity may be determined by the BLASTP algorithm using parameters of a wordlength (W) of 3, an expectation (E) of 10, and using a BLOSUM62 scoring matrix setting gap costs at existence of 11, extension of 1, and using a conditional compositional score matrix adjustment.

In some cases, the system above may comprise (b) at least one engineered synthetic guide ribonucleic acid (sgRNA) capable of forming a complex with the endonuclease bearing a 5' targeting region complementary to a desired cleavage sequence. In some cases, the 5' targeting region may comprises a PAM sequence compatible with the endonuclease. In some cases, the 5' most nucleotide of the targeting region may be G. In some cases, the 5' targeting region may be 15-23 nucleotides in length. The guide sequence and the tracr sequence may be supplied as separate ribonucleic acids (RNAs) or a single ribonucleic acid (RNA). The guide RNA may comprise a crRNA tracrRNA binding sequence 3' to the targeting region. The guide RNA may comprise a tracrRNA sequence preceded by a 4-nucleotide linker 3' to the crRNA tracrRNA binding region. The sgRNA may comprise, from 5' to 3': a non-natural guide nucleic acid sequence capable of hybridizing to a target sequence in a cell; and a tracr sequence. In some cases, the non-natural guide nucleic acid sequence and the tracr sequence are covalently linked.

In some cases, the tracr sequence may have a particular sequence. The tracr sequence may have at least about 80% to at least about 60-100 (e.g., at least about 60, at least about 65, at least about 70, at least about 75, at least about 80, at least about 85, or at least about 90) consecutive nucleotides of a natural tracrRNA sequence. The tracr sequence may have at least about 80% sequence identity to at least about 60-100 (e.g., at least about 60, at least about 65, at least about 70, at least about 75, at least about 80, at least about 85, or at least about 90) consecutive nucleotides of SEQ ID NO: 5509. In some cases, the tracrRNA may have at least about 80%, at least about 85%, at least about 90%, at least about 91%, at least about 92%, at least about 93%, at least about 94%, at least about 95%, at least about 96%, at least about 97%, at least about 98%, or at least about 99% identity to at least about 60-90 (e.g., at least about 60, at least about 65, at least about 70, at least about 75, at least about 80, at least about 85, or at least about 90) consecutive nucleotides of SEQ ID NO: 5509. In some cases, the tracrRNA may be substantially identical to at least about 60-100 (e.g., at least about 60, at least about 65, at least about 70, at least about 75, at least about 80, at least about 85, or at least about 90) consecutive nucleotides of SEQ ID NO: 5509. The tracrRNA may comprise SEQ ID NO: 5509.

In some cases, the at least one engineered synthetic guide ribonucleic acid (sgRNA) capable of forming a complex with the endonuclease may comprise a sequence having at least about 80% identity to SEQ ID NO: 5473. The sgRNA may comprise a sequence having at least about 80%, at least about 85%, at least about 90%, at least about 91%, at least about 92%, at least about 93%, at least about 94%, at least about 95%, at least about 96%, at least about 97%, at least about 98%, or at least about 99% identity to SEQ ID NO: 5473. The sgRNA may comprise a sequence substantially identical to SEQ ID NO: 5473.

In some cases, the system above may comprise two different sgRNAs targeting a first region and a second region for cleavage in a target DNA locus, wherein the second region is 3' to the first region. In some cases, the system above may comprise a single- or double-stranded DNA repair template comprising from 5' to 3': a first homology arm comprising a sequence of at least about 20 (e.g., at least about 40, 80, 120, 150, 200, 300, 500, or 1kb) nucleotides 5' to the first region, a synthetic DNA sequence of at least about 10 nucleotides, and a second homology arm comprising a sequence of at least about 20 (e.g., at least about 40, 80, 120, 150, 200, 300, 500, or 1kb) nucleotides 3' to the second region.

In another aspect, the present disclosure provides a method for modifying a target nucleic acid locus of interest. The method may comprise delivering to the target nucleic acid locus any of the non-natural systems disclosed herein, including an enzyme and at least one synthetic guide RNA (sgRNA) disclosed herein. The enzyme may form a complex with the at least one sgRNA, and upon binding of the complex to the target nucleic acid locus of interest, may modify the target nucleic acid locus of interest. Delivering the enzyme to said locus may comprise transfecting a cell with the system or nucleic acids encoding the system. Delivering the nuclease to said locus may comprise electroporating a cell with the system or nucleic acids encoding the system. Delivering the nuclease to said locus may comprise incubating the system in a buffer with a nucleic acid comprising the locus of interest. In some cases, the target nucleic acid locus comprises deoxyribonucleic acid (DNA) or ribonucleic acid (RNA). The target nucleic acid locus may comprise genomic DNA, viral DNA, viral RNA, or bacterial DNA. The target nucleic acid locus may be within a cell. The target nucleic acid locus may be in vitro. The target nucleic acid locus may be within a eukaryotic cell or a prokaryotic cell. The cell may be an animal cell, a human cell, bacterial cell, archaeal cell, or a plant cell. The enzyme may induce a single or double-stranded break at or proximal to the target locus of interest.

In cases where the target nucleic acid locus may be within a cell, the enzyme may be supplied as a nucleic acid containing an open reading frame encoding the enzyme having a RuvC_III domain having at least about 75% (e.g., at least about 90%, at least about 91%, at least about 92%, at least about 93%, at least about 94%, at least about 95%, at least about 96%, at least about 97%, at least about 98%, at least about 99%) identity to any one of SEQ ID NOs: 3331-3474. The deoxyribonucleic acid (DNA) containing an open reading frame encoding said endonuclease may comprise a sequence substantially identical to SEQ ID NOs: 5585 or at variant having at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% identity to SEQ ID NOs: 5585. In some cases, the nucleic acid comprises a promoter to which the open reading frame encoding the endonuclease is operably linked. The promoter may be a CMV, EF1a, SV40, PGK1, Ubc, human beta actin, CAG, TRE, or CaMKIIa promoter. The endonuclease may be supplied as a capped mRNA containing said open reading frame encoding said endonuclease. The endonuclease may be supplied as a translated polypeptide. The at least one engineered sgRNA may be supplied as deoxyribonucleic acid (DNA) containing a gene sequence encoding said at least one engineered sgRNA operably linked to a ribonucleic acid (RNA) pol III promoter. In some cases, the organism may be eukaryotic. In some cases, the organism may be fungal. In some cases, the organism may be human.

### MG22 Enzymes

In one aspect, the present disclosure provides for an engineered nuclease system comprising (a) an endonuclease. In some cases, the endonuclease is a Cas endonuclease. In some cases, the endonuclease is a Type II, Class II Cas endonuclease. The endonuclease may comprise a RuvC_III domain, wherein said RuvC_III domain has at least about 70% sequence identity to any one of SEQ ID NOs: 3475-3568. In some cases, the endonuclease may comprise a RuvC_III domain, wherein the RuvC_III domain has at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99% identity to any one of SEQ ID NOs: 3475-3568. In some cases, the endonuclease may comprise a RuvC_III domain, wherein the substantially identical to any one of SEQ ID NOs: 3475-3568. The endonuclease may comprise a RuvC_III domain having at least about 70% sequence identity to any one of SEQ ID NOs: 3475-3568. In some cases, the endonuclease may comprise a RuvC_III domain having at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99% identity to any one of SEQ ID NOs: 3475-3568. In some cases, the endonuclease may comprise a RuvC_III domain substantially identical to any one of SEQ ID NOs: 3475-3568.

The endonuclease may comprise an HNH domain having at least about 70% identity to any one of SEQ ID NOs: 5291-5389. In some cases, the endonuclease may comprise an HNH domain having at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99% identical to any one of SEQ ID NOs: 5291-5389. The endonuclease may comprise an HNH domain substantially identical to any one of SEQ ID NOs: 5291-5389. The endonuclease may comprise an HNH domain having at least about 70% identity to any one of SEQ ID NOs: 5291-5389. In some cases, the endonuclease may comprise an HNH domain having at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99% identical to any one of SEQ ID NOs: 5291-5389. The endonuclease may comprise an HNH domain substantially identical to any one of SEQ ID NOs: 5291-5389.

In some cases, the endonuclease may comprise a variant having at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% identity to any one of SEQ ID NOs: 1656-1755. In some cases, the endonuclease may be substantially identical to any one of SEQ ID NOs: 1656-1755. In some cases, the endonuclease may comprise a variant having at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% identity to any one of SEQ ID NOs: 1656-1755. In some cases, the endonuclease may be substantially identical to any one of SEQ ID NOs: 1656-1755.

In some cases, the endonuclease may comprise a variant having one or more nuclear localization sequences (NLSs). The NLS may be proximal to the N- or C-terminus of said endonuclease. The NLS may be appended N-terminal or C-terminal to any one of SEQ ID NOs: 432-660, or to a variant having at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% identity to any one of SEQ ID NOs: 1656-1755. The NLS may be an SV40 large T antigen NLS. The NLS may be a c-myc NLS. The NLS can comprise a sequence with at least about 80%, at least about 85%, at least about 90%, at least about 95%, at least about 99% identity to any one of SEQ ID NOs: 5593-5608. The NLS can comprise a sequence substantially identical to any one of SEQ ID NOs: 5593-5608. The NLS can comprise any of the sequences in Table 1 or a combination thereof:

In some cases, sequence identity may be determined by the BLASTP, CLUSTALW, MUSCLE, MAFFT, Novafold, or CLUSTALW with the parameters of the Smith-Waterman homology search algorithm. The sequence identity may be determined by the BLASTP algorithm using parameters of a wordlength (W) of 3, an expectation (E) of 10, and using a BLOSUM62 scoring matrix setting gap costs at existence of 11, extension of 1, and using a conditional compositional score matrix adjustment.

In some cases, the system above may comprise (b) at least one engineered synthetic guide ribonucleic acid (sgRNA) capable of forming a complex with the endonuclease bearing a 5' targeting region complementary to a desired cleavage sequence. In some cases, the 5' targeting region may comprises a PAM sequence compatible with the endonuclease. In some cases, the 5' most nucleotide of the targeting region may be G. In some cases, the 5' targeting region may be 15-23 nucleotides in length. The guide sequence and the tracr sequence may be supplied as separate ribonucleic acids (RNAs) or a single ribonucleic acid (RNA). The guide RNA may comprise a crRNA tracrRNA binding sequence 3' to the targeting region. The guide RNA may comprise a tracrRNA sequence preceded by a 4-nucleotide linker 3' to the crRNA tracrRNA binding region. The sgRNA may comprise, from 5' to 3': a non-natural guide nucleic acid sequence capable of hybridizing to a target sequence in a cell; and a tracr sequence. In some cases, the non-natural guide nucleic acid sequence and the tracr sequence are covalently linked.

In some cases, the tracr sequence may have a particular sequence. The tracr sequence may have at least about 80% to at least about 60-100 (e.g., at least about 60, at least about 65, at least about 70, at least about 75, at least about 80, at least about 85, or at least about 90) consecutive nucleotides of a natural tracrRNA sequence. The tracr sequence may have at least about 80% sequence identity to at least about 60-100 (e.g., at least about 60, at least about 65, at least about 70, at least about 75, at least about 80, at least about 85, or at least about 90) consecutive nucleotides of SEQ ID NO: 5510. In some cases, the tracrRNA may have at least about 80%, at least about 85%, at least about 90%, at least about 91%, at least about 92%, at least about 93%, at least about 94%, at least about 95%, at least about 96%, at least about 97%, at least about 98%, or at least about 99% identity to at least about 60-90 (e.g., at least about 60, at least about 65, at least about 70, at least about 75, at least about 80, at least about 85, or at least about 90) consecutive nucleotides of SEQ ID NO: 5510. In some cases, the tracrRNA may be substantially identical to at least about 60-100 (e.g., at least about 60, at least about 65, at least about 70, at least about 75, at least about 80, at least about 85, or at least about 90) consecutive nucleotides of SEQ ID NO: 5510. The tracrRNA may comprise SEQ ID NO: 5510.

In some cases, the at least one engineered synthetic guide ribonucleic acid (sgRNA) capable of forming a complex with the endonuclease may comprise a sequence having at least about 80% identity to SEQ ID NO: 5474. The sgRNA may comprise a sequence having at least about 80%, at least about 85%, at least about 90%, at least about 91%, at least about 92%, at least about 93%, at least about 94%, at least about 95%, at least about 96%, at least about 97%, at least about 98%, or at least about 99% identity to SEQ ID NO: 5474. The sgRNA may comprise a sequence substantially identical to SEQ ID NO: 5474.

In some cases, the system above may comprise two different sgRNAs targeting a first region and a second region for cleavage in a target DNA locus, wherein the second region is 3' to the first region. In some cases, the system above may comprise a single- or double-stranded DNA repair template comprising from 5' to 3': a first homology arm comprising a sequence of at least about 20 (e.g., at least about 40, 80, 120, 150, 200, 300, 500, or 1kb) nucleotides 5' to the first region, a synthetic DNA sequence of at least about 10 nucleotides, and a second homology arm comprising a sequence of at least about 20 (e.g., at least about 40, 80, 120, 150, 200, 300, 500, or 1kb) nucleotides 3' to the second region.

In another aspect, the present disclosure provides a method for modifying a target nucleic acid locus of interest. The method may comprise delivering to the target nucleic acid locus any of the non-natural systems disclosed herein, including an enzyme and at least one synthetic guide RNA (sgRNA) disclosed herein. The enzyme may form a complex with the at least one sgRNA, and upon binding of the complex to the target nucleic acid locus of interest, may modify the target nucleic acid locus of interest. Delivering the enzyme to said locus may comprise transfecting a cell with the system or nucleic acids encoding the system. Delivering the nuclease to said locus may comprise electroporating a cell with the system or nucleic acids encoding the system. Delivering the nuclease to said locus may comprise incubating the system in a buffer with a nucleic acid comprising the locus of interest. In some cases, the target nucleic acid locus comprises deoxyribonucleic acid (DNA) or ribonucleic acid (RNA). The target nucleic acid locus may comprise genomic DNA, viral DNA, viral RNA, or bacterial DNA. The target nucleic acid locus may be within a cell. The target nucleic acid locus may be in vitro. The target nucleic acid locus may be within a eukaryotic cell or a prokaryotic cell. The cell may be an animal cell, a human cell, bacterial cell, archaeal cell, or a plant cell. The enzyme may induce a single or double-stranded break at or proximal to the target locus of interest.

In cases where the target nucleic acid locus may be within a cell, the enzyme may be supplied as a nucleic acid containing an open reading frame encoding the enzyme having a RuvC_III domain having at least about 75% (e.g., at least about 90%, at least about 91%, at least about 92%, at least about 93%, at least about 94%, at least about 95%, at least about 96%, at least about 97%, at least about 98%, at least about 99%) identity to any one of SEQ ID NOs: 3475-3568. The deoxyribonucleic acid (DNA) containing an open reading frame encoding said endonuclease may comprise a sequence substantially identical to SEQ ID NOs: 5586 or at variant having at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% identity to SEQ ID NOs: 5586. In some cases, the nucleic acid comprises a promoter to which the open reading frame encoding the endonuclease is operably linked. The promoter may be a CMV, EF1a, SV40, PGK1, Ubc, human beta actin, CAG, TRE, or CaMKIIa promoter. The endonuclease may be supplied as a capped mRNA containing said open reading frame encoding said endonuclease. The endonuclease may be supplied as a translated polypeptide. The at least one engineered sgRNA may be supplied as deoxyribonucleic acid (DNA) containing a gene sequence encoding said at least one engineered sgRNA operably linked to a ribonucleic acid (RNA) pol III promoter. In some cases, the organism may be eukaryotic. In some cases, the organism may be fungal. In some cases, the organism may be human.

### MG23 Enzymes

In one aspect, the present disclosure provides for an engineered nuclease system comprising (a) an endonuclease. In some cases, the endonuclease is a Cas endonuclease. In some cases, the endonuclease is a Type II, Class II Cas endonuclease. The endonuclease may comprise a RuvC_III domain, wherein said RuvC_III domain has at least about 70% sequence identity to any one of SEQ ID NOs: 3569-3637. In some cases, the endonuclease may comprise a RuvC_III domain, wherein the RuvC_III domain has at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99% identity to any one of SEQ ID NOs: 3569-3637. In some cases, the endonuclease may comprise a RuvC_III domain, wherein the substantially identical to any one of SEQ ID NOs: 3569-3637. The endonuclease may comprise a RuvC_III domain having at least about 70% sequence identity to any one of SEQ ID NOs: 3569-3637. In some cases, the endonuclease may comprise a RuvC_III domain having at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99% identity to any one of SEQ ID NOs: 3569-3637. In some cases, the endonuclease may comprise a RuvC_III domain substantially identical to any one of SEQ ID NOs: 3569-3637.

The endonuclease may comprise an HNH domain having at least about 70% identity to any one of SEQ ID NOs: 5390-5460. In some cases, the endonuclease may comprise an HNH domain having at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99% identical to any one of SEQ ID NOs: 5390-5460. The endonuclease may comprise an HNH domain substantially identical to any one of SEQ ID NOs: 5390-5460. The endonuclease may comprise an HNH domain having at least about 70% identity to any one of SEQ ID NOs: 5390-5460. In some cases, the endonuclease may comprise an HNH domain having at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, or at least about 99% identical to any one of SEQ ID NOs: 5390-5460. The endonuclease may comprise an HNH domain substantially identical to any one of SEQ ID NOs: 5390-5460.

In some cases, the endonuclease may comprise a variant having at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% identity to any one of SEQ ID NOs: 1756-1826. In some cases, the endonuclease may be substantially identical to any one of SEQ ID NOs: 1756-1826. In some cases, the endonuclease may comprise a variant having at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% identity to any one of SEQ ID NOs: 1756-1826. In some cases, the endonuclease may be substantially identical to any one of SEQ ID NOs: 1756-1826.

In some cases, the endonuclease may comprise a variant having one or more nuclear localization sequences (NLSs). The NLS may be proximal to the N- or C-terminus of said endonuclease. The NLS may be appended N-terminal or C-terminal to any one of SEQ ID NOs: 1756-1826, or to a variant having at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% identity to any one of SEQ ID NOs: 1756-1826. The NLS may be an SV40 large T antigen NLS. The NLS may be a c-myc NLS. The NLS can comprise a sequence with at least about 80%, at least about 85%, at least about 90%, at least about 95%, at least about 99% identity to any one of SEQ ID NOs: 5593-5608. The NLS can comprise a sequence substantially identical to any one of SEQ ID NOs: 5593-5608. The NLS can comprise any of the sequences in Table 1 or a combination thereof:

In some cases, sequence identity may be determined by the BLASTP, CLUSTALW, MUSCLE, MAFFT, Novafold, or CLUSTALW with the parameters of the Smith-Waterman homology search algorithm. The sequence identity may be determined by the BLASTP algorithm using parameters of a wordlength (W) of 3, an expectation (E) of 10, and using a BLOSUM62 scoring matrix setting gap costs at existence of 11, extension of 1, and using a conditional compositional score matrix adjustment.

In some cases, the system above may comprise (b) at least one engineered synthetic guide ribonucleic acid (sgRNA) capable of forming a complex with the endonuclease bearing a 5' targeting region complementary to a desired cleavage sequence. In some cases, the 5' targeting region may comprises a PAM sequence compatible with the endonuclease. In some cases, the 5' most nucleotide of the targeting region may be G. In some cases, the 5' targeting region may be 15-23 nucleotides in length. The guide sequence and the tracr sequence may be supplied as separate ribonucleic acids (RNAs) or a single ribonucleic acid (RNA). The guide RNA may comprise a crRNA tracrRNA binding sequence 3' to the targeting region. The guide RNA may comprise a tracrRNA sequence preceded by a 4-nucleotide linker 3' to the crRNA tracrRNA binding region. The sgRNA may comprise, from 5' to 3': a non-natural guide nucleic acid sequence capable of hybridizing to a target sequence in a cell; and a tracr sequence. In some cases, the non-natural guide nucleic acid sequence and the tracr sequence are covalently linked.

In some cases, the tracr sequence may have a particular sequence. The tracr sequence may have at least about 80% to at least about 60-100 (e.g., at least about 60, at least about 65, at least about 70, at least about 75, at least about 80, at least about 85, or at least about 90) consecutive nucleotides of a natural tracrRNA sequence. The tracr sequence may have at least about 80% sequence identity to at least about 60-100 (e.g., at least about 60, at least about 65, at least about 70, at least about 75, at least about 80, at least about 85, or at least about 90) consecutive nucleotides of SEQ ID NO: 5511. In some cases, the tracrRNA may have at least about 80%, at least about 85%, at least about 90%, at least about 91%, at least about 92%, at least about 93%, at least about 94%, at least about 95%, at least about 96%, at least about 97%, at least about 98%, or at least about 99% identity to at least about 60-90 (e.g., at least about 60, at least about 65, at least about 70, at least about 75, at least about 80, at least about 85, or at least about 90) consecutive nucleotides of SEQ ID NO: 5511. In some cases, the tracrRNA may be substantially identical to at least about 60-100 (e.g., at least about 60, at least about 65, at least about 70, at least about 75, at least about 80, at least about 85, or at least about 90) consecutive nucleotides of SEQ ID NO: 5511. The tracrRNA may comprise SEQ ID NO: 5511.

In some cases, the at least one engineered synthetic guide ribonucleic acid (sgRNA) capable of forming a complex with the endonuclease may comprise a sequence having at least about 80% identity to SEQ ID NO: 5475. The sgRNA may comprise a sequence having at least about 80%, at least about 85%, at least about 90%, at least about 91%, at least about 92%, at least about 93%, at least about 94%, at least about 95%, at least about 96%, at least about 97%, at least about 98%, or at least about 99% identity to SEQ ID NO: 5475. The sgRNA may comprise a sequence substantially identical to SEQ ID NO: 5475.

In some cases, the system above may comprise two different sgRNAs targeting a first region and a second region for cleavage in a target DNA locus, wherein the second region is 3' to the first region. In some cases, the system above may comprise a single- or double-stranded DNA repair template comprising from 5' to 3': a first homology arm comprising a sequence of at least about 20 (e.g., at least about 40, 80, 120, 150, 200, 300, 500, or 1kb) nucleotides 5' to the first region, a synthetic DNA sequence of at least about 10 nucleotides, and a second homology arm comprising a sequence of at least about 20 (e.g., at least about 40, 80, 120, 150, 200, 300, 500, or 1kb) nucleotides 3' to the second region.

In another aspect, the present disclosure provides a method for modifying a target nucleic acid locus of interest. The method may comprise delivering to the target nucleic acid locus any of the non-natural systems disclosed herein, including an enzyme and at least one synthetic guide RNA (sgRNA) disclosed herein. The enzyme may form a complex with the at least one sgRNA, and upon binding of the complex to the target nucleic acid locus of interest, may modify the target nucleic acid locus of interest. Delivering the enzyme to said locus may comprise transfecting a cell with the system or nucleic acids encoding the system. Delivering the nuclease to said locus may comprise electroporating a cell with the system or nucleic acids encoding the system. Delivering the nuclease to said locus may comprise incubating the system in a buffer with a nucleic acid comprising the locus of interest. In some cases, the target nucleic acid locus comprises deoxyribonucleic acid (DNA) or ribonucleic acid (RNA). The target nucleic acid locus may comprise genomic DNA, viral DNA, viral RNA, or bacterial DNA. The target nucleic acid locus may be within a cell. The target nucleic acid locus may be in vitro. The target nucleic acid locus may be within a eukaryotic cell or a prokaryotic cell. The cell may be an animal cell, a human cell, bacterial cell, archaeal cell, or a plant cell. The enzyme may induce a single or double-stranded break at or proximal to the target locus of interest.

In cases where the target nucleic acid locus may be within a cell, the enzyme may be supplied as a nucleic acid containing an open reading frame encoding the enzyme having a RuvC_III domain having at least about 75% (e.g., at least about 90%, at least about 91%, at least about 92%, at least about 93%, at least about 94%, at least about 95%, at least about 96%, at least about 97%, at least about 98%, at least about 99%) identity to any one of SEQ ID NOs: 3569-3637. The deoxyribonucleic acid (DNA) containing an open reading frame encoding said endonuclease may comprise a sequence substantially identical to SEQ ID NOs: 5587 or at variant having at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, at least about 96%, at least about 97%, at least about 98%, or at least about 99% identity to SEQ ID NOs: 5587. In some cases, the nucleic acid comprises a promoter to which the open reading frame encoding the endonuclease is operably linked. The promoter may be a CMV, EF1a, SV40, PGK1, Ubc, human beta actin, CAG, TRE, or CaMKIIa promoter. The endonuclease may be supplied as a capped mRNA containing said open reading frame encoding said endonuclease. The endonuclease may be supplied as a translated polypeptide. The at least one engineered sgRNA may be supplied as deoxyribonucleic acid (DNA) containing a gene sequence encoding said at least one engineered sgRNA operably linked to a ribonucleic acid (RNA) pol III promoter. In some cases, the organism may be eukaryotic. In some cases, the organism may be fungal. In some cases, the organism may be human.

Systems of the present disclosure may be used for various applications, such as, for example, nucleic acid editing (e.g., gene editing), binding to a nucleic acid molecule (e.g., sequence-specific binding). Such systems may be used, for example, for addressing (e.g., removing or replacing) a genetically inherited mutation that may cause a disease in a subject, inactivating a gene in order to ascertain its function in a cell, as a diagnostic tool to detect disease-causing genetic elements (e.g. via cleavage of reverse-transcribed viral RNA or an amplified DNA sequence encoding a disease-causing mutation), as deactivated enzymes in combination with a probe to target and detect a specific nucleotide sequence (e.g. sequence encoding antibiotic resistance int bacteria), to render viruses inactive or incapable of infecting host cells by targeting viral genomes, to add genes or amend metabolic pathways to engineer organisms to produce valuable small molecules, macromolecules, or secondary metabolites, to establish a gene drive element for evolutionary selection, to detect cell perturbations by foreign small molecules and nucleotides as a biosensor.

### EXAMPLES

### Example 1. -Metagenomic analysis for new proteins

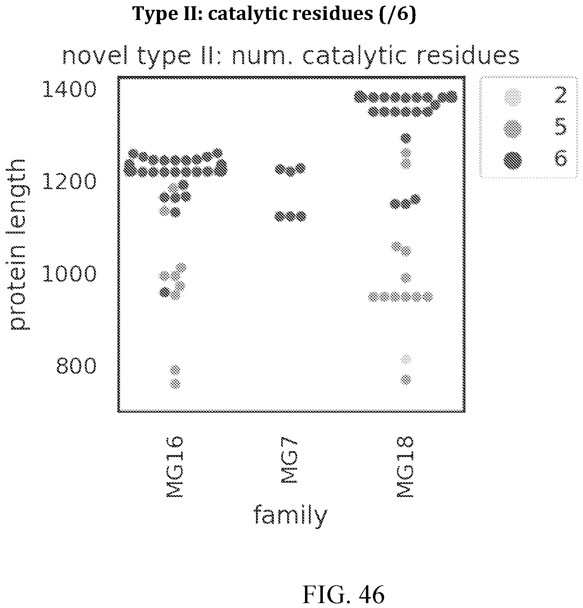

Metagenomic samples were collected from sediment, soil and animal. Deoxyribonucleic acid (DNA) was extracted with a Zymobiomics DNA mini-prep kit and sequenced on an Illumina HiSeq^{®} 2500. Samples were collected with consent of property owners. Additional raw sequence data from public sources included animal microbiomes, sediment, soil, hot springs, hydrothermal vents, marine, peat bogs, permafrost, and sewage sequences. Metagenomic sequence data was searched using Hidden Markov Models generated based on known Cas protein sequences including type II Cas effector proteins to identify new Cas effectors (see Figure 45, which shows distribution of such proteins detected from different sample types). Novel effector proteins identified by the search were aligned to known proteins to identify potential active sites (see Figure 46, which shows distribution of Cas catalytic residues among the enzymes identified from the different sites). This metagenomic workflow resulted in delineation of the MG1, MG2, MG3, MG4, MG6, MG14, MG15, MG16, MG18, MG21, MG22, and MG23 families of class II, type II CRISPR endonucleases described herein.

### Example 2A. - Discovery of an MG1 Family of CRISPR systems

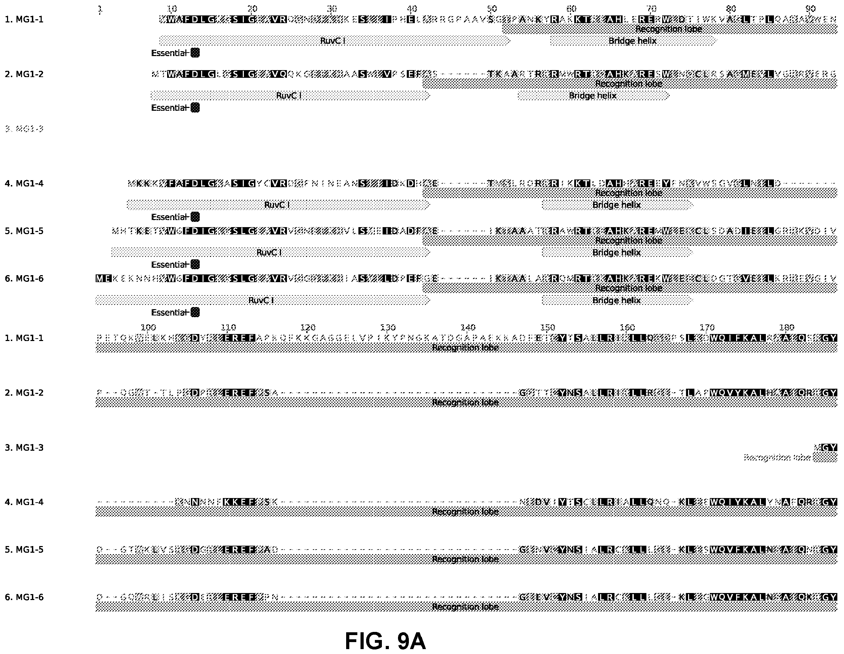

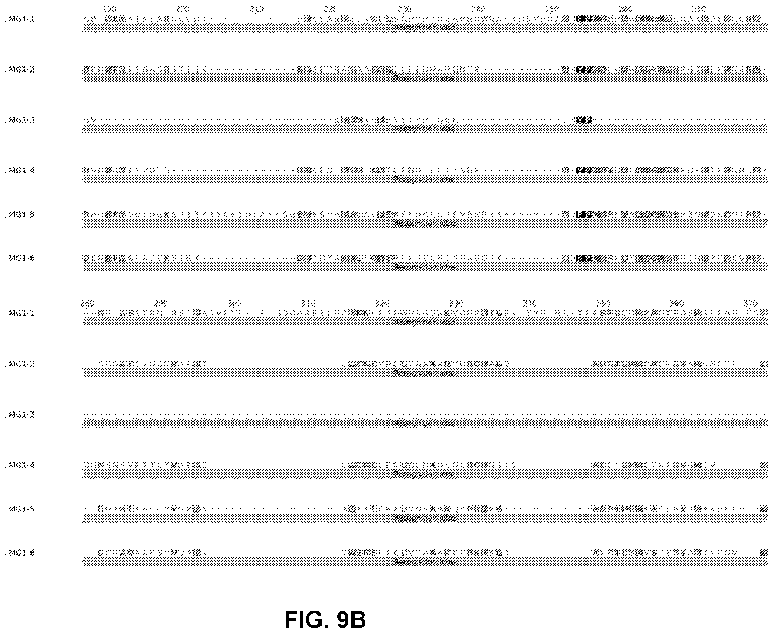

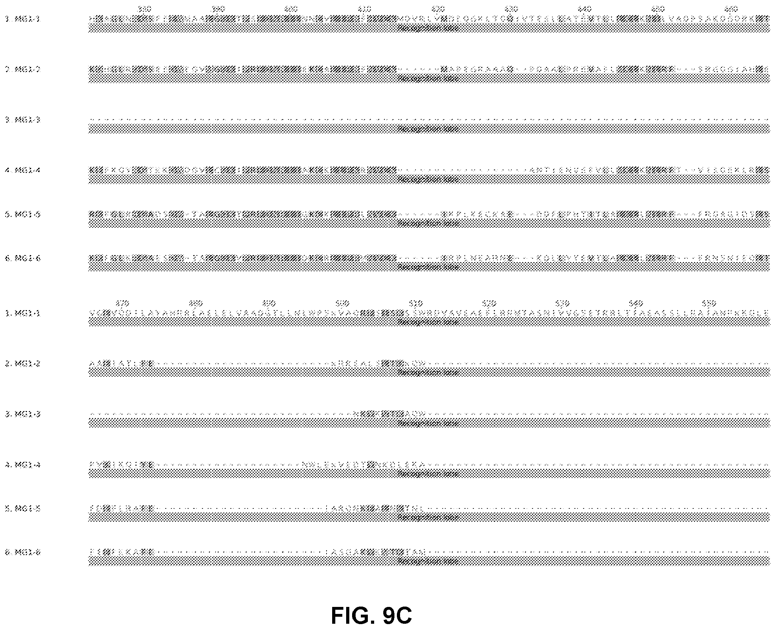

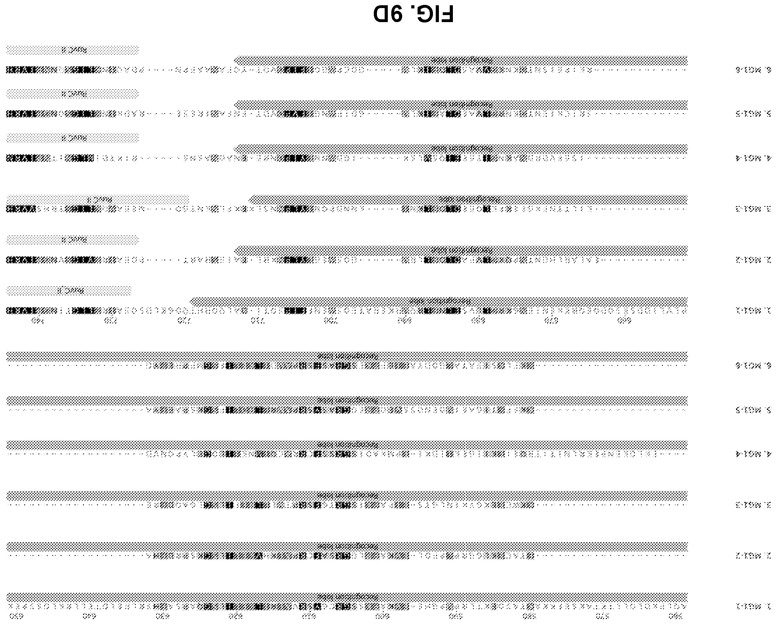

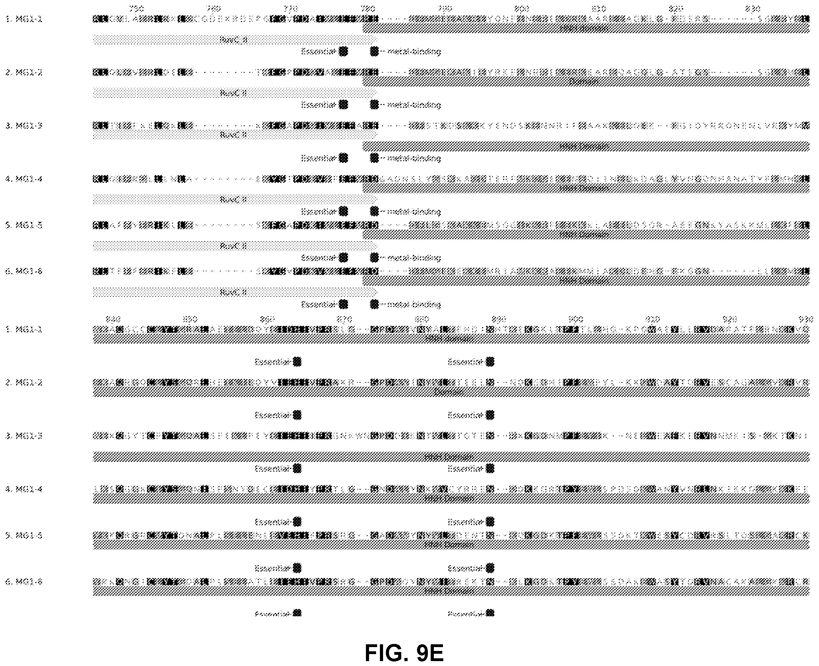

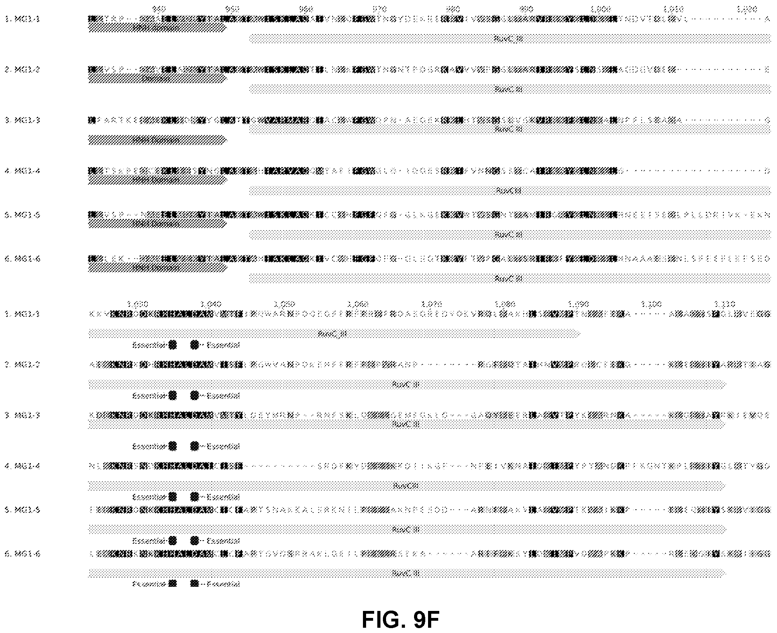

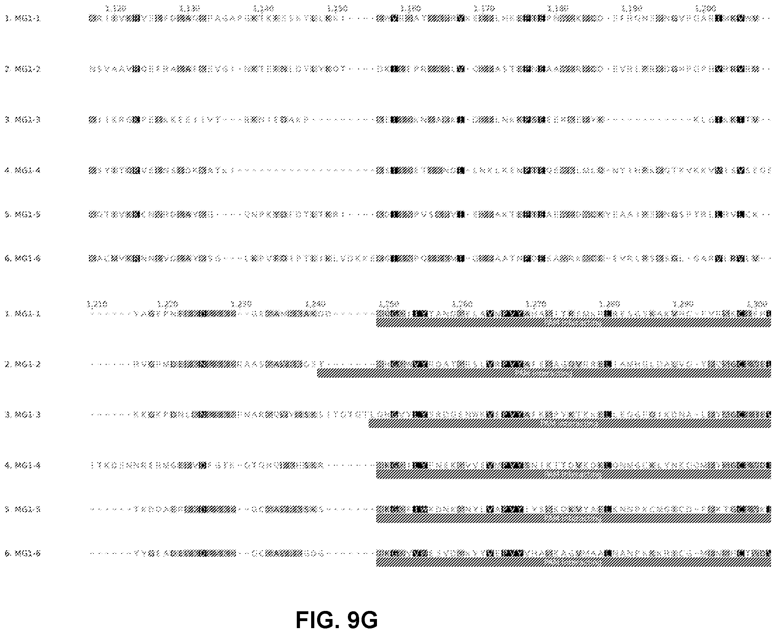

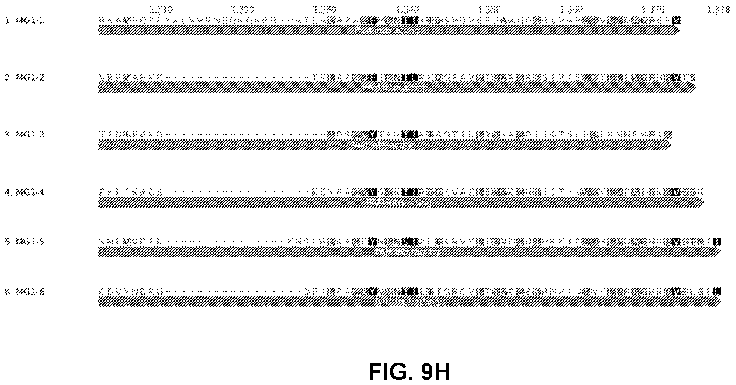

Analysis of the data from the metagenomic analysis of Example 1 revealed a new cluster of previously undescribed putative CRISPR systems initially comprising six members (MG1-1, MG1-2, MG1-3, MG1-4, MG1-5, and MG1-6 recorded as SEQ ID NOs: 5, 6, 1, 2, and 3 respectively). This family is characterized by an enzyme bearing HNH and RuvC domains. The RuvC domains of this family have a RuvC_III portion having low homology to previously described Cas9 family members. Although the initial family members have a maximum of 56.8% identity among them, all 6 enzymes exhibit a divergent RuvC_III portion of the RuvC domain and bear the common motif of RHHALDAMV (SEQ ID NO:5615), KHHALDAMC (SEQ ID NO:5616), or KHHALDAIC (SEQ ID NO:5617). These motifs are not found in other described Cas9-like enzymes. The corresponding protein and nucleic acid sequences for these new enzymes and their relevant subdomains are presented in the sequence listing. Putative tracrRNA sequences were identified based on their location relative to the other genes and are presented as SEQ ID NOs: 5476-5479. The enzyme systems appear to derive from the Phylum Verrucomicrobia, the Phylum Candidatus Peregrinibacteria, or the Phylum Candidatus Melainabacteria based on the sequences of 16S rRNAs from genome bins containing the CRISPR systems. The 16S rRNA sequences are presented as SEQ ID NOs: 5592-5596). A detailed domain-level alignment of the CRISPR system sequences together calling out the features described by Shmakov et al. (Mol Cell. 2015 Nov 5;60(3):385-97), which is entirely incorporated by reference) is depicted in FIGURES 9A, 9B, 9C, 9D, 9E, 9F, 9G, and 9H. A comparison of MG1-1, 1-2, and 1-3 versus additional proprietary protein datasets revealed additional protein sequences with similar architecture, presented as SEQ NOs: 7-319. These MG1 protein sequences led to the discovery of additional MG1 motifs as shown in SEQ ID NOs: 5618-5632.

### Example 2B. - Discovery of an MG2 Family of CRISPR systems

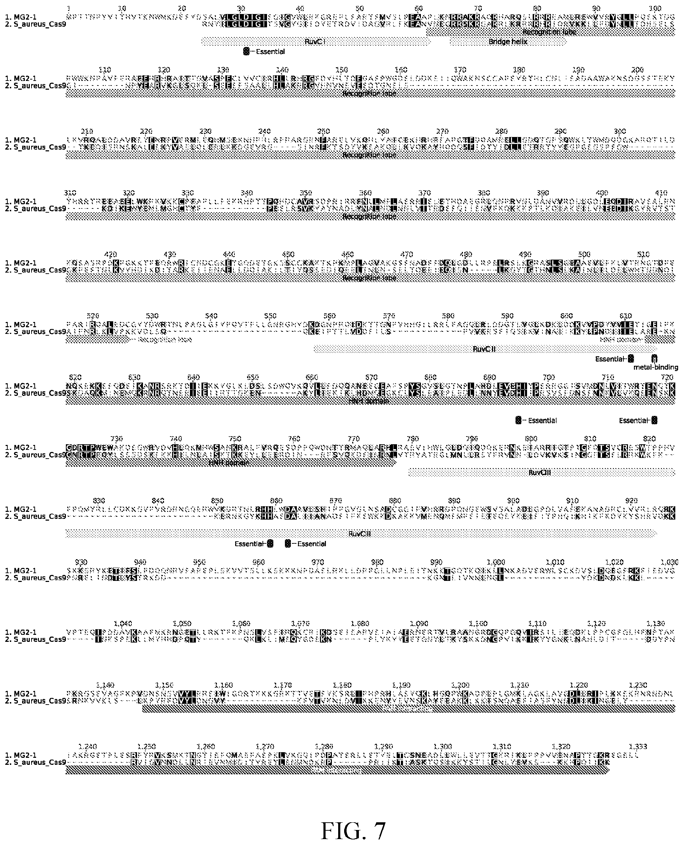

Analysis of data from the metagenomic analysis of Example 1 revealed a new cluster of previously undescribed putative CRISPR systems comprising six members (MG2-1, MG2-2, MG2-3, MG2-5, and MG2-6). The corresponding protein and nucleic acid sequences for these new enzymes and exemplary subdomains are presented as SEQ ID NOs: 320, 322-325. Based on their location relative to the other genes, putative tracrRNA sequences were identified in the operon and are presented as SEQ ID NOs: 5490, 5492-5494, and 5538. A detailed domain-level alignment of these sequences versus Cas9 as outlined in Shmakov et al. (Mol Cell. 2015 Nov 5;60(3):385-97.), is depicted in FIGURE 7.

A comparison of MG2-1, MG2-2, MG2-3, MG2-5, and MG2-6 versus additional proprietary protein datasets revealed additional protein sequences with similar architecture, presented as SEQ NOs: 321 and 326-420. Motifs commonly found in MG2 family members are presented as SEQ ID NOs: 5631-5638.

### Example 2C. - Discovery of an MG3 Family of CRISPR systems

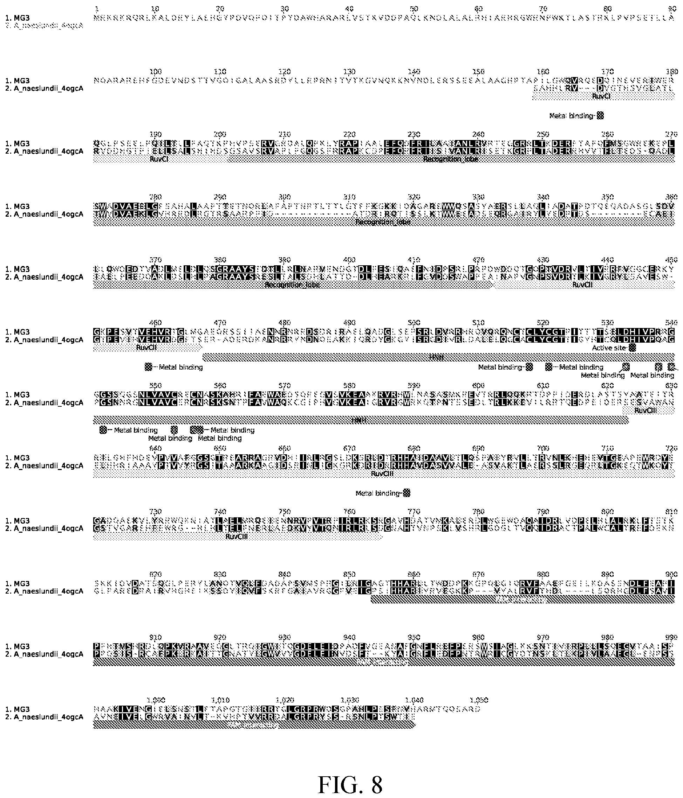

Analysis of the data from the metagenomic analysis of Example 1 revealed a new previously undescribed putative CRISPR system: MG3-1. The corresponding amino acid sequences for this new enzyme and its exemplary subdomains are presented as SEQ ID NOs: 424, 2245, and 4059. Based on proximity to the other elements in the operon, a putative tracrRNA containing sequence was identified and is included as SEQ ID NO: 5498. A detailed domain-level alignment of the sequence versus Cas9 from *Actinomyces naeslundii* is depicted in FIGURE 8.

A comparison of MG3-1 versus additional proprietary protein datasets revealed additional protein sequences with similar architecture, presented as SEQ NOs: 421-423, 425-431.

### Example 2D. - Discovery of MG4, 7, 14, 15, 16, 18, 21, 22, 23 Families of CRISPR systems

Analysis of the data from the metagenomic analysis of Example 1 revealed new clusters of previously undescribed putative CRISPR systems comprising 9 families of one member each (MG 4-5, MG7-2, MG14-1, MG15-1, MG16-2, MG18-1, MG21-1, MG22-1, MG23-1). The corresponding protein and nucleic acid sequences for these new enzymes and their exemplary subdomains are presented as SEQ ID NOs: 432, 669, 678, 930, 1093, 1354, 1512, 1656, 1756. Based on proximity to the other elements in the operon, a putative tracr containing sequence was identified for each family. These sequences are presented in the sequence listing as SEQ ID NOs: 5503-5511, respectively.

A comparison of MG 4-5, MG7-2, MG14-1, MG15-1, MG16-2, MG18-1, MG21-1, MG22-1, MG23-1 versus additional proprietary protein datasets revealed additional protein sequences with similar architecture, presented as SEQ NOs: 433-660, 670-677, 679-929, 931-1092, 1094-1353, 1355-1511, 1513-1655, 1657-1755, and 1757-1826. Motifs common to the nucleases of these sets of CRISPR systems are presented as SEQ ID NO: 5649 for MG4; SEQ ID NOs: 5650-5667 for MG14; 5668-5675 for MG15; SEQ ID NOs: 5676-5678 for MG16; SEQ ID NOs: 5679-5686 for MG18; SEQ ID NOs: 5687-5693 and SEQ ID NOs: 5674-5675 for MG21; SEQ ID NOs: 5694-5699 for MG22; and SEQ ID NOs: 5700-5717 for MG23.

### Example 3.-Prophetic-Determination of Protospacer-Adjacent Motif.

Experiments are performed as in any of the examples in Karvelis et al. Methods. 2017 May 15;121-122:3-8, which is entirely incorporated by reference herein, to identify the protospacer adjacent motif (PAM) sequence specificity for the novel enzymes described herein to allow for optimal synthetic sequence targeting.

In one example (in-vivo screen), cells bearing plasmids encoding any of the enzymes described herein and protospacer-targeting guide RNA are co-transformed with a plasmid library containing an antibiotic resistance gene, and a protospacer sequence flanked by a randomized PAM sequence. Plasmids containing functional PAMs are cleaved by the enzyme, leading to cell death. Deep-sequencing of the enzyme cleavage-resistant plasmid pool isolated from the surviving cells displays a set of depleted plasmids that contain functional cleavage-permitting PAMs.

In another example (in vitro screen), PAM library in the form of DNA plasmid or concatemeric repeats is subjected to cleavage by the RNP complex (e.g., including the enzyme, tracrRNA and crRNA or the enzyme and hybrid sgRNA) assembled in vitro or in cell lysates. Resulting free DNA ends from successful cleavage events are captured by adapter ligation, followed by the PCR amplification of the PAM-sided products. Amplified library of functional PAMs is subjected to deep sequencing and PAMs licensing DNA cleavage are identified.

### Example 4.-Prophetic-Use of synthetic CRISPR system as described herein in a mammalian cell for genome editing

DNA/RNA sequences encoding (i) an ORF encoding codon-optimized enzyme under a cell-compatible promoter with a cell-compatible C-terminal nuclear localization sequence (e.g., SV40 NLS in the case of human cells) and a suitable polyadenylation signal (e.g., TK pA signal in the case of human cells); and (ii) an ORF encoding an sgRNA (having a 5' sequence beginning with G followed by 20 nt of a complementary targeting nucleic acid sequence targeting genomic DNA followed by a corresponding compatible PAM identified via Example 3 and a 3' tracr-binding sequence, a linker, and the tracrRNA sequence) under a suitable Polymerase III promoter (e.g., the U6 promoter in mammalian cells) are prepared. In some embodiments, these sequences are prepared on the same or separate plasmid vectors, which are transfected via a suitable technique into eukaryotic cells. In some embodiments, these sequences are prepared as separate DNA sequences, which are transfected or microinjected into cells. In some embodiments, these sequences are prepared as synthesized RNAs or in-vitro transcribed RNAs which are transfected or microinjected into cells. In some embodiments, these sequences are translated into proteins and transfected or microinjected into cells.

Whichever transfection method is selected, (i) and (ii) are introduced into cells. A period of incubation is allowed to pass so that the enzyme and/or sgRNA can be transcribed and/or translated into active form. After the incubation period, genomic DNA in the vicinity of the targeting sequence is analyzed (e.g., by sequencing). An indel is introduced into the genomic DNA in the vicinity of the targeting sequence as a result of enzyme-mediated cleavage and non-homologous end joining.

In some embodiments, (i) and (ii) are introduced into cells with a third repair nucleotide that encodes regions of the genome flanking the cleavage site of sizes 25 bp or larger, which will facilitate homology directed repair. Containing within these flanking sequences may be a single base pair mutation, a functional gene fragment, a foreign or native gene for expression, or several genes composing a biochemical pathway.

### Example 5.-Prophetic-Use of synthetic CRISPR system as described herein in vitro

Any of the enzymes described herein are cloned into a suitable E. coli expression plasmid containing a purification tag and are recombinantly expressed in E. coli and purified using the recombinant tag. RNAs comprising a 5' G followed by a 20 nt targeting sequence and PAM sequence, a tracrRNA binding region of a compatible crRNA, a GAAA linker, and a compatible tracrRNA are synthesized by suitable solid-phase RNA synthesis methods. Recombinant enzymes and sgRNA are combined in a suitable cleavage buffer containing Mg2+

(e.g., 20 mM HEPES pH 7.5, 100 mM KCl, 5 mM MgClz, 1 mM DTT, 5% glycerol) and the reaction is initiated by introducing a target DNA including a sequence complementary to the targeting sequence and PAM sequence. Cleavage of the DNA is monitored by a suitable assay (e.g., agarose gel electrophoresis followed by ethidium bromide staining (or similarly acting DNA-intercalating agent) and UV visualization).

### Example 6.-(General protocol) PAM Sequence identification/confirmation for the endonucleases described herein

PAM sequences were determined by sequencing plasmids containing randomly-generated PAM sequences that could be cleaved by putative endonucleases expressed in an *E*. *coli* lysate-based expression system (myTXTL, Arbor Biosciences). In this system, an *E*. *coli* codon optimized nucleotide sequence was transcribed and translated from a PCR fragment under control of a T7 promoter. A second PCR fragment with a tracr sequence under a T7 promoter and a minimal CRISPR array composed of a T7 promoter followed by a repeat-spacer-repeat sequence was transcribed in the same reaction. Successful expression of the endonuclease and tracr sequence in the TXTL system followed by CRISPR array processing provided active *in vitro* CRISPR nuclease complexes.

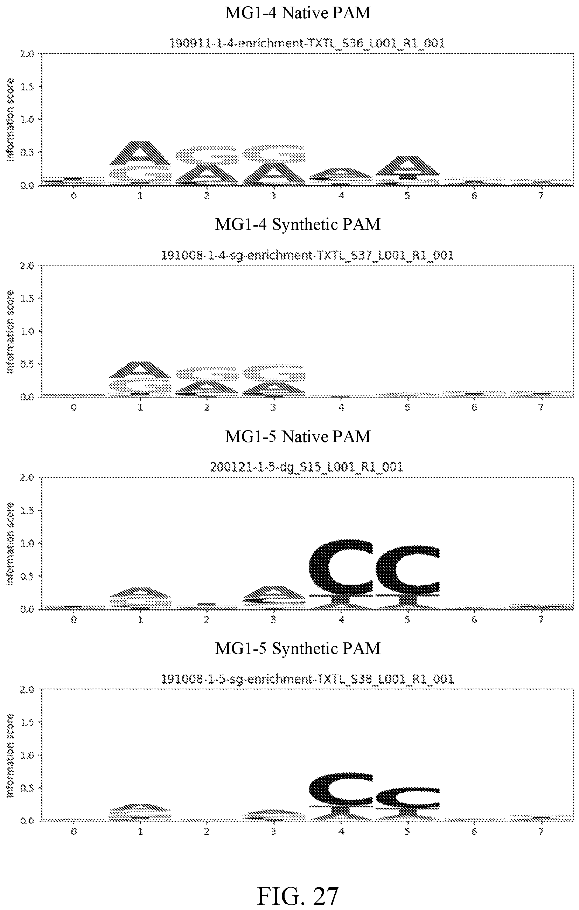

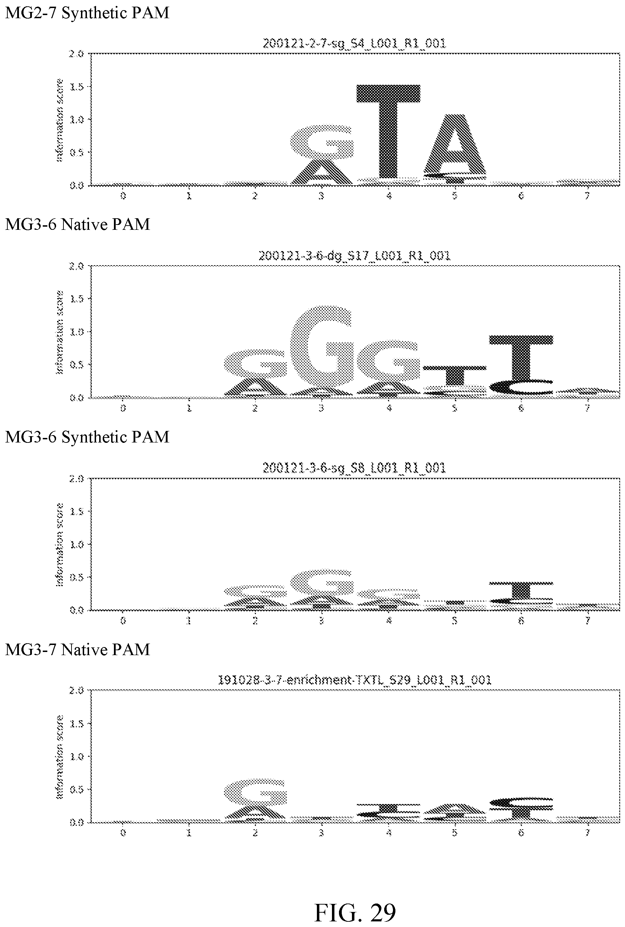

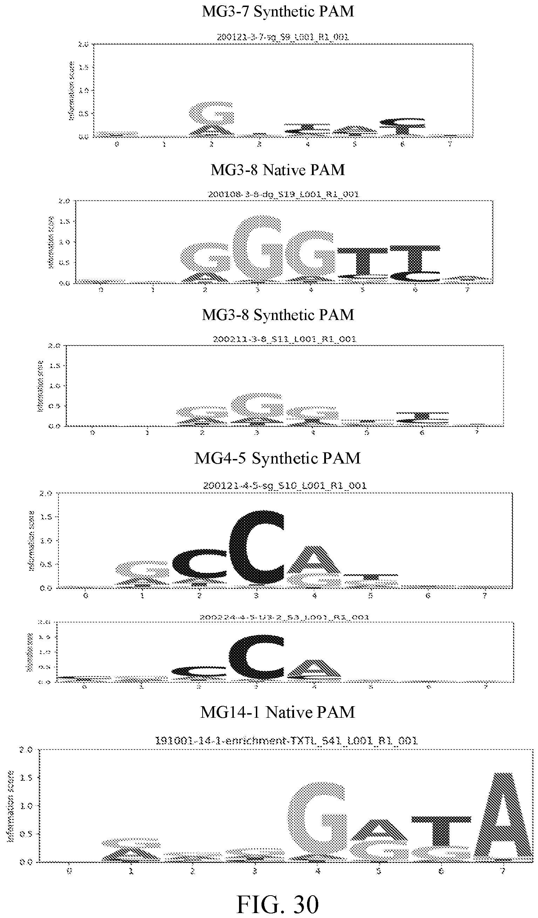

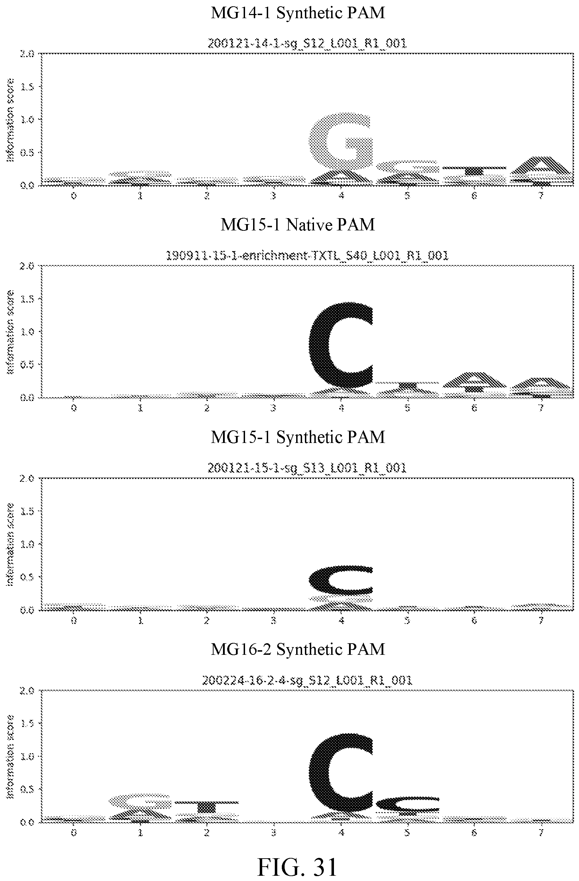

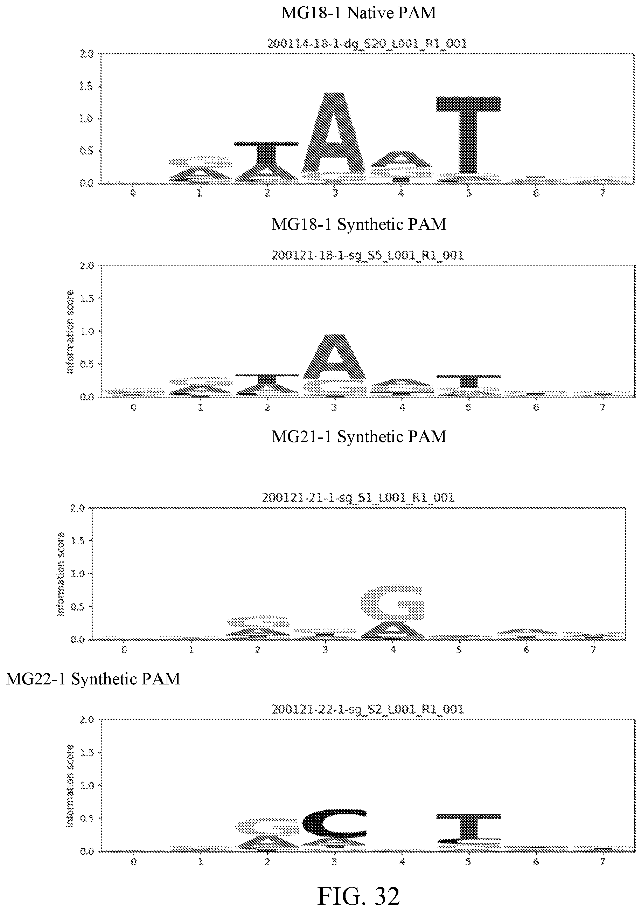

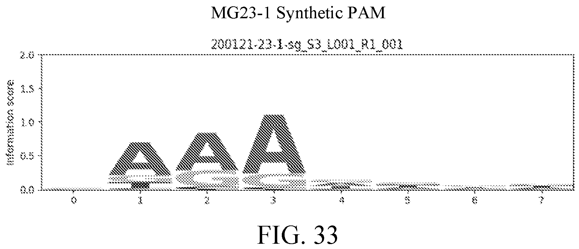

A library of target plasmids containing a spacer sequence matching that in the minimal array followed by 8N mixed bases (putative PAM sequences) was incubated with the output of the TXTL reaction. After 1-3 hr, the reaction was stopped and the DNA was recovered via a DNA clean-up kit, e.g., Zymo DCC, AMPure XP beads, QiaQuick etc. Adapter sequences were blunt-end ligated to DNA with active PAM sequences that had been cleaved by the endonuclease, whereas DNA that had not been cleaved was inaccessible for ligation. DNA segments comprising active PAM sequences were then amplified by PCR with primers specific to the library and the adapter sequence. The PCR amplification products were resolved on a gel to identify amplicons that corresponded to cleavage events. The amplified segments of the cleavage reaction were also used as template for preparation of an NGS library. Sequencing this resulting library, which was a subset of the starting 8N library, revealed the sequences which contain the correct PAM for the active CRISPR complex. For PAM testing with a single RNA construct, the same procedure was repeated except that an *in vitro* transcribed RNA was added along with the plasmid library and the tracr/minimal CRISPR array template was omitted. For endonucleases where NGS libraries were prepared, seqLogo (see e.g., Huber et al. Nat Methods. 2015 Feb;12(2):115-21) representations were constructed and are presented in Figures 27, 38, 29, 30, 31, 32, 33, 34, and 35. The seqLogo module used to construct these representations takes the position weight matrix of a DNA sequence motif (e.g. a PAM sequence) and plots the corresponding sequence logo as introduced by Schneider and Stephens (see e.g. Schneider et al. Nucleic Acids Res. 1990 Oct 25;18(20):6097-100. The characters representing the sequence in the seqLogo representations have been stacked on top of each other for each position in the aligned sequences (e.g. PAM sequences). The height of each letter is proportional to its frequency, and the letters have been sorted so the most common one is on top.

### Example 7.-(General protocol) RNA Folding of tracrRNA and sgRNA structures

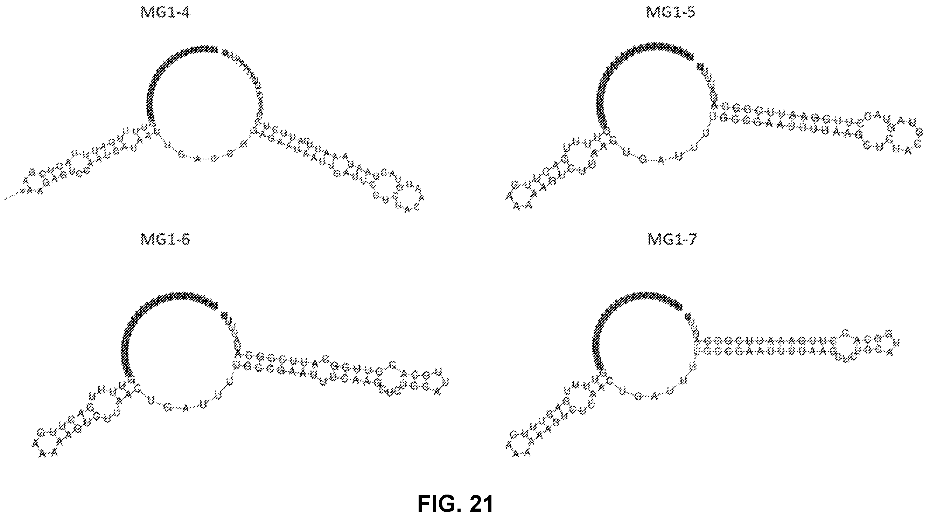

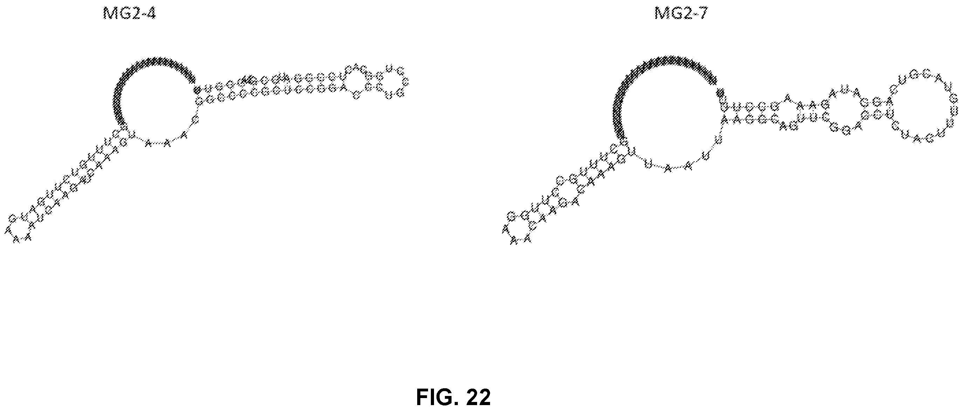

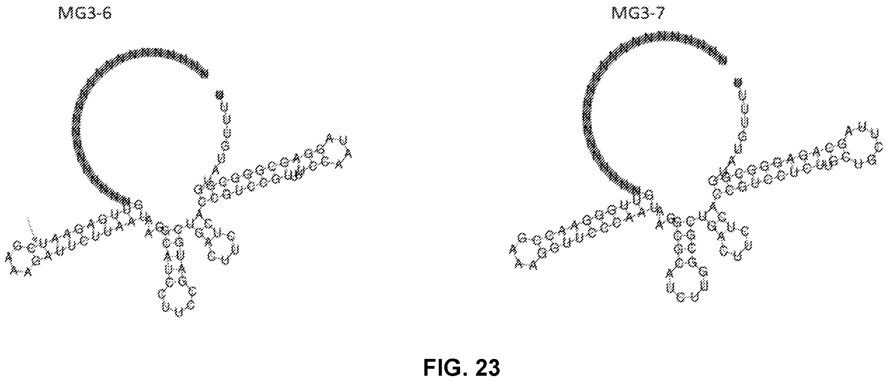

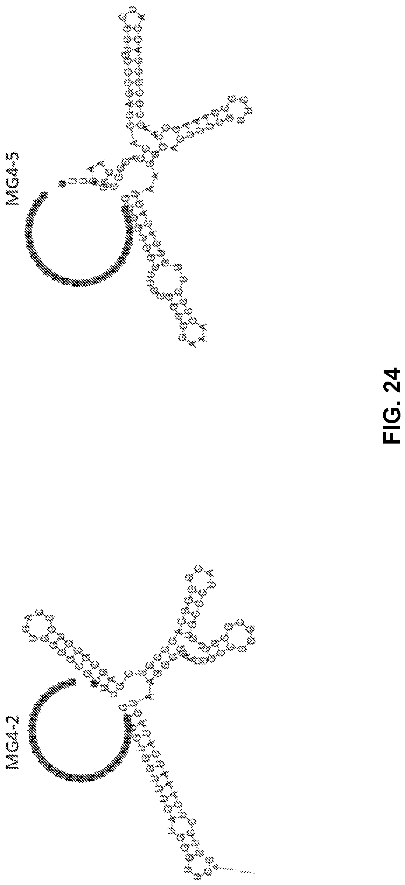

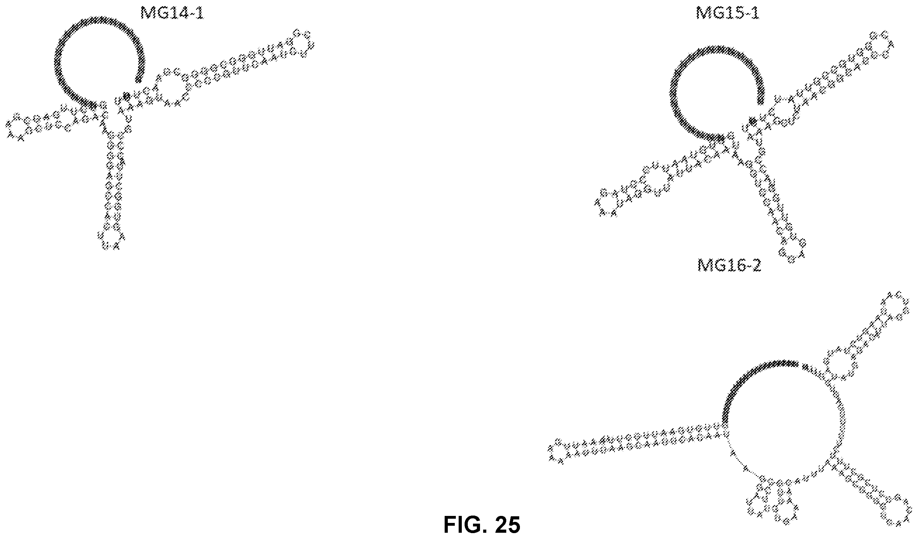

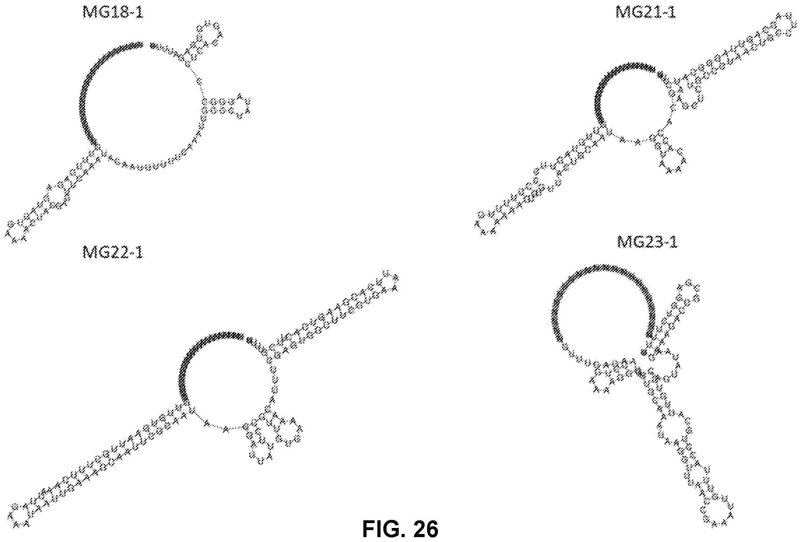

Folded structures of guide RNA sequences at 37 °C were computed using the method of Andronescu et al. Bioinformatics. 2007 Jul 1;23(13):i19-28, which is incorporated by reference herein in its entirety. Predicted structures of exemplary sgRNAs described herein are presented in Figures 21, 22, 23, 24, 25, and 26.

### Example 8.-(General protocol) In vitro cleavage efficiency of MG CRISPR Complexes

Endonucleases were expressed as His-tagged fusion proteins from an inducible T7 promoter in a protease deficient *E. coli* B strain. Cells expressing the His-tagged proteins were lysed by sonication and the His-tagged proteins were purified by Ni-NTA affinity chromatography on a HisTrap FF column (GE Lifescience) on an AKTA Avant FPLC (GE Lifescience). The eluate was resolved by SDS-PAGE on acrylamide gels (Bio-Rad) and stained with InstantBlue Ultrafast coomassie (Sigma-Aldrich). Purity was determined using densitometry of the protein band with ImageLab software (Bio-Rad). Purified endonucleases were dialyzed into a storage buffer composed of 50 mM Tris-HCl, 300 mM NaCl, 1 mM TCEP, 5% glycerol; pH 7.5 and stored at -80°C.

Target DNAs containing spacer sequences and PAM sequences (determined e.g., as in Example 6) were constructed by DNA synthesis. A single representative PAM was chosen for testing when the PAM had degenerate bases . The target DNAs comprised 2200 bp of linear DNA derived from a plasmid via PCR amplification with a PAM and spacer located 700 bp from one end. Successful cleavage resulted in fragments of 700 and 1500 bp. The target DNA, *in vitro* transcribed single RNA, and purified recombinant protein were combined in cleavage buffer (10 mM Tris, 100 mM NaCl, 10 mM MgCh) with an excess of protein and RNA and incubated for 5 minutes to 3 hours, usually 1 hr. The reaction was stopped via addition of RNAse A and incubation at 60 minutes. The reaction was then resolved on a 1.2% TAE agarose gel and the fraction of cleaved target DNA is quantified in ImageLab software.

### Example 9.-(General protocol) Testing of Genome Cleavage Activity of MG CRISPR Complexes in E. coli

*E. coli* lacks the capacity to efficiently repair double-stranded DNA breaks. Thus, cleavage of genomic DNA can be a lethal event. Exploiting this phenomenon, endonuclease activity was tested in *E. coli* by recombinantly expressing an endonuclease and a tracrRNA in a target strain with spacer/target and PAM sequences integrated into its genomic DNA.

In this assay, the PAM sequence is specific for the endonuclease being tested as determined by the methods described in Example 6. sgRNA sequences were determined based upon the sequence and predicted structure of the tracrRNA. Repeat-anti-repeat pairings of 8-12 bp (generally 10bp) were chosen, starting from the 5' end of the repeat. The remaining 3' end of the repeat and 5' end of the tracrRNA were replaced with a tetraloop. Generally, the tetraloop was GAAA, but other tetraloops can be used, particularly if the GAAA sequence is predicted to interfere with folding. In these cases, a TTCG tetraloop was used.

Engineered strains with PAM sequences integrated into their genomic DNA were transformed with DNA encoding the endonuclease. Transformants were then made chemocompetent and transformed with 50 ng of single guide RNAs either specific to the target sequence ("on target"), or non-specific to the target ("non target"). After heat shock, transformations were recovered in SOC for 2 hrs at 37°C. Nuclease efficiency was then determined by a 5-fold dilution series grown on induction media. Colonies were quantified from the dilution series in triplicate.

### Example 10a.-(General protocol) Testing of Genome Cleavage Activity of MG CRISPR Complexes in Mammalian Cells

To show targeting and cleavage activity in mammalian cells, the MG Cas effector protein sequences were tested in two mammalian expression vectors: (a) one with a C-terminal SV40 NLS and a 2A-GFP tag, and (b) one with no GFP tag and two SV40 NLS sequences, one on the N-terminus and one on the C-terminus. In some instances, nucleotide sequences encoding the endonucleases were codon-optimized for expression in mammalian cells.

The corresponding single guide RNA sequence (sgRNA) with targeting sequence attached is cloned into a second mammalian expression vector. The two plasmids are cotransfected into HEK293T cells. 72 hr after co-transfection of the expression plasmid and a sgRNA targeting plasmid into HEK293T cells, the DNA is extracted and used for the preparation of an NGS-library. Percent NHEJ is measured via indels in the sequencing of the target site to demonstrate the targeting efficiency of the enzyme in mammalian cells. At least 10 different target sites were chosen to test each protein's activity.

### Example 10b. (General Protocol) Testing of Genome Cleavage Activity of MG CRISPR Complexes in Mammalian Cells

To show targeting and cleavage activity in mammalian cells, the MG Cas effector protein sequences were cloned into two mammalian expression vector: (a) one with flanking N and C-terminal SV40 NLS sequences, a C-terminal His tag, and a 2A-GFP tag at the C terminus after the His tag (Backbone 1), and (b) one with flanking NLS sequences and C-terminal His tag but no T2A GFP tag (Backbone 2). In some instances, nucleotide sequences encoding the endonucleases were the native sequence, codon-optimized for expression in E. coli, or codon-optimized for expression in mammalian cells.

The corresponding single guide RNA sequence (sgRNA) with targeting sequence attached was cloned into a second mammalian expression vector. The two plasmids were cotransfected into HEK293T cells. 72 hr after co-transfection of the expression plasmid and a sgRNA targeting plasmid into HEK293T cells, the DNA was extracted and used for the preparation of an NGS-library. Percent NHEJ was measured via indels in the sequencing of the target site to demonstrate the targeting efficiency of the enzyme in mammalian cells. About 7-12 different target sites were chosen for testing each protein's activity. An arbitrary threshold of 5% indels was used to identify active candidates.

### Example 11.- Characterization of MG1 family members

### PAM Specificity, tracrRNA/sgRNA Validation

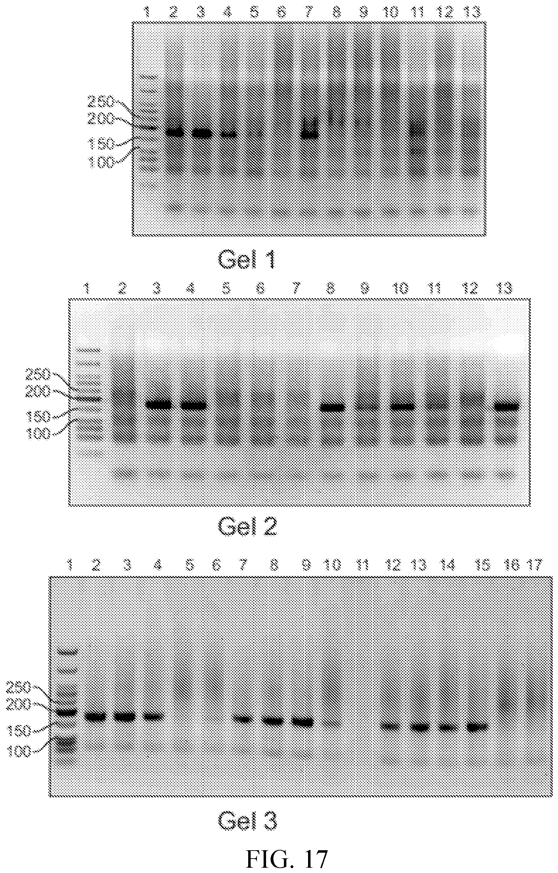

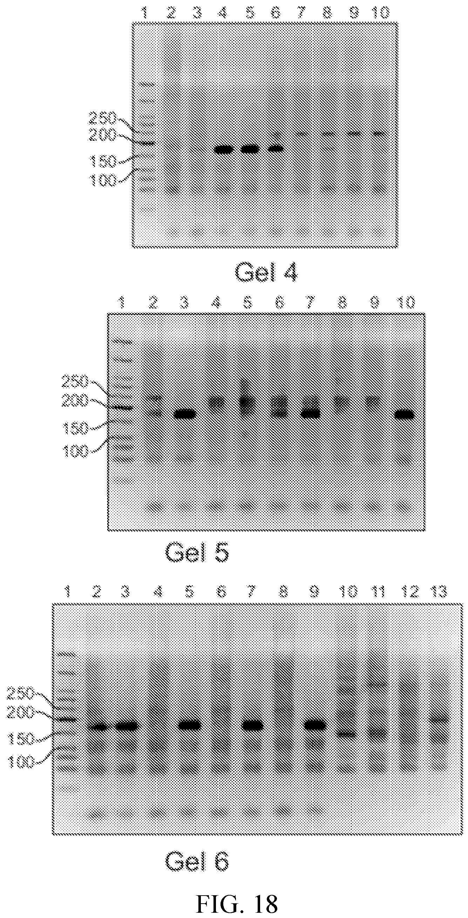

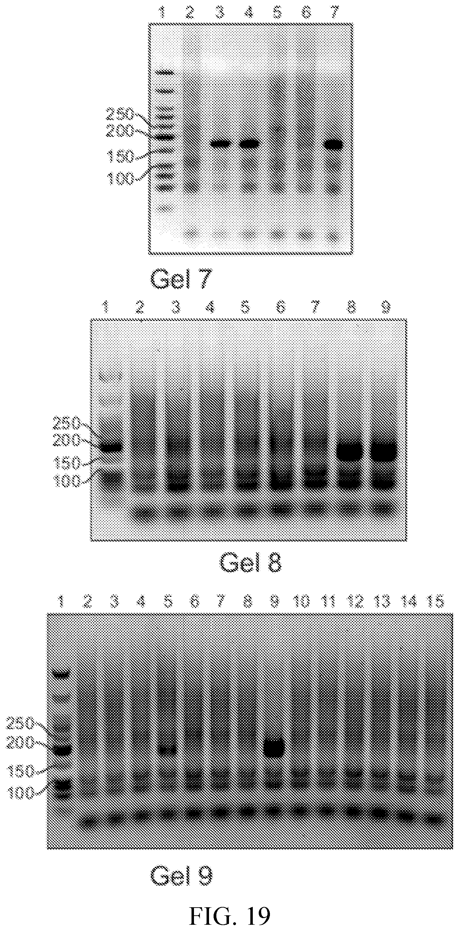

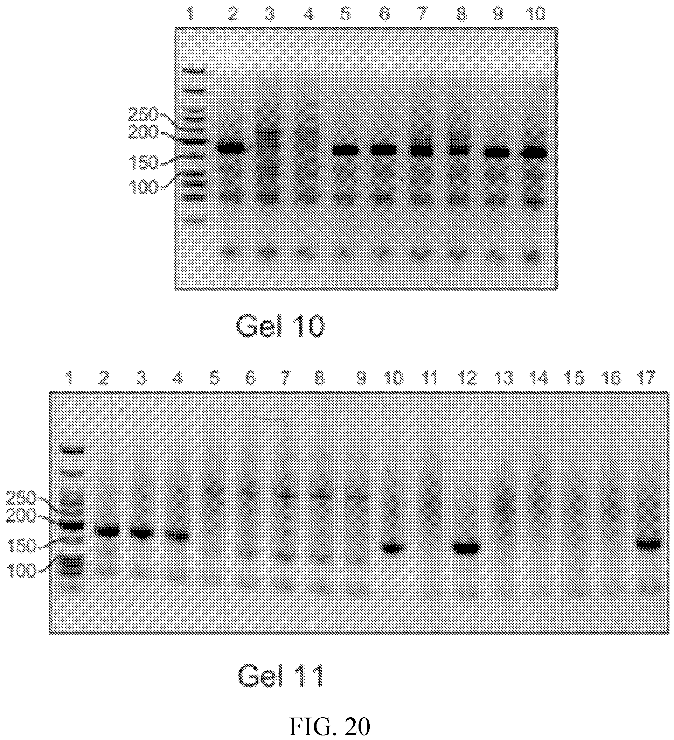

The targeted endonuclease activity of MG1 family endonuclease systems was confirmed using the myTXTL system described in Example 6. In this assay, PCR amplification of cleaved target plasmids yields a product that migrates at approximately 170 bp in the gel, as shown in Figures 17-20. Amplification products were observed for MG1-4 (dual guide: see gel 1, lane 3, single guide: see gel 6 lane 2), MG1-5 (gel 2 lane 10), MG1-6 (dual guide: see gel 5 lane 6, single guide see: gel 6 lane 5), and MG1-7 (dual guide: see gel 3 lane 13, single guide: see gel 3 lane 2) (protein SEQ ID NOs: 1-4, respectively). Sequencing the PCR products revealed active PAM sequences for these enzymes as shown in Table 2.

**Table 2: PAM sequence specificities and related data for MG1 enzymes**

| **Enzyme** | **Enzyme protein SEQ ID NO** | **Native (dual guide) PAM** | **PAM SEQ ID NO:** | **tracrRNA SEQ ID NO:** | **sgRNA SEQ ID NO:** | **Synthetic (single guide) PAM** | **Synthetic (single guide) PAM SEQ ID NO:** |
|---|---|---|---|---|---|---|---|
| MG1-4 | 1 | nRRRAA | 5527 | 5476 | 5461 | nRRR | 5512 |
| MG1-5 | 2 | nnnnCC | 5528 | 5477 | 5462 | nnnnYY | 5513 |
| MG1-6 | 3 | nnRRAC | 5529 | 5478 | 5463 | nnRRAY | 5514 |
| MG1-7 | 4 | nRRRAA | 5530 | 5479 | 5464 | nRRRAAG | 5515 |

Synthetic single guide RNAs (sgRNAs) were designed based on the sequences and predicted structures of the tracrRNAs and are presented as SEQ ID NOs: 5461-5464. The PAM sequence screen of Example 6 was repeated with the sgRNAs. The results of this experiment are also presented in Table 2, which reveals that PAM specificity changed slightly when using sgRNAs.

### Targeted endonuclease activity In Vitro

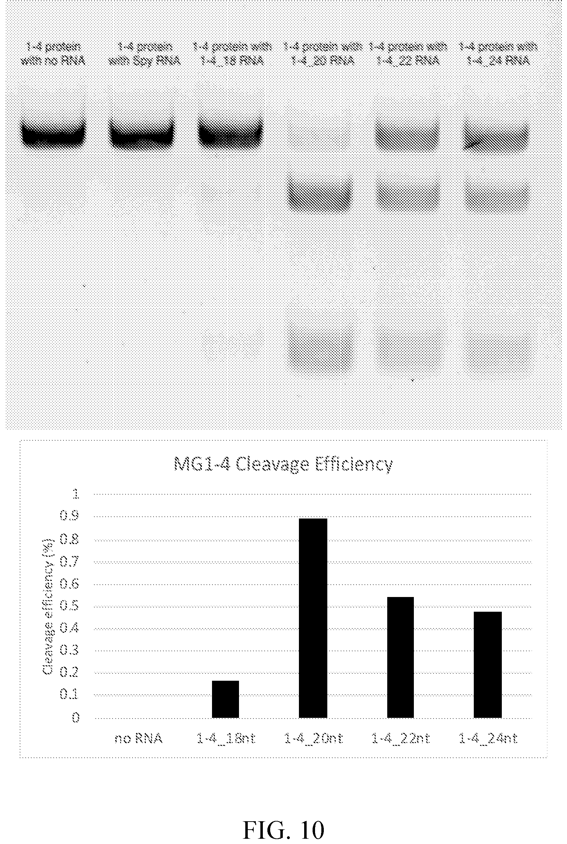

*In vitro* activity of the MG1-4 endonuclease system (protein SEQ ID NO: 1 with sgRNA SEQ ID NO: 5461) on a target DNA with a PAM sequence CAGGAAGG was verified using the method of Example 8. The single guide sequence reported above (SEQ ID NO: 5461) was used, with varying spacer/targeting sequence lengths from 18-24 nt replacing the Ns of the sequence. The results are shown in Figure 10, wherein the left panel shows a gel demonstrating DNA cleavage by MG1-4 in combination with corresponding single guide sgRNAs having different targeting sequence lengths (18-24nt), and the right panel shows the same data quantified as a bar graph. The data demonstrated that targeting sequences from 18-24 nucleotides were functional with the MG1-4/sgRNA system.

### Targeted endonuclease activity in bacterial cells

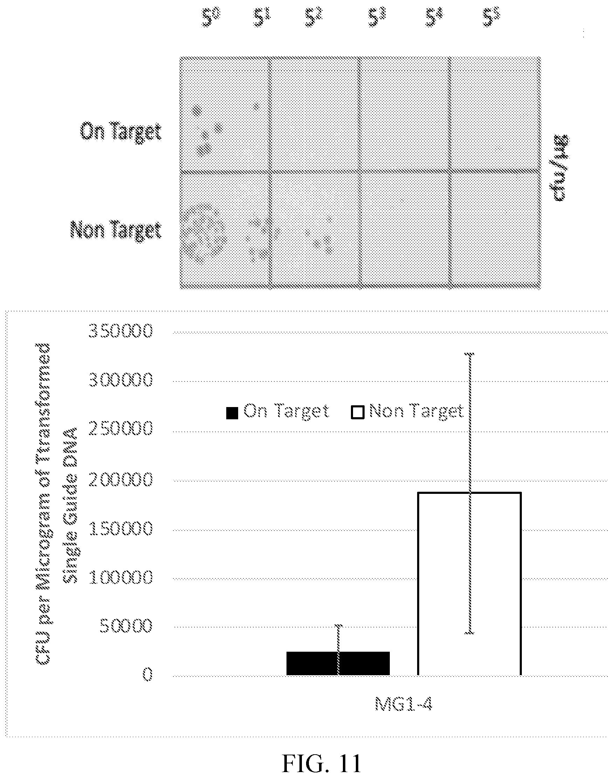

*In vivo* activity of the MG1-4 endonuclease system (protein SEQ ID NO: 1, sgRNA SEQ ID NO: 5461) was tested with the PAM sequence CAGGAAGG as in Example 9. Transformed E. coli were plated in serial dilution, and the results (showing E. coli serial dilutions in the left panel and quantitated growth in the right panel) are presented in Figure 11. A substantial reduction in the growth of E. coli expressing on target sgRNA compared to E. coli expressing non-target sgRNA indicates that genomic DNA was specifically cleaved by the endonuclease in *E. coli* cells.

### Targeted endonuclease activity in mammalian cells (a)

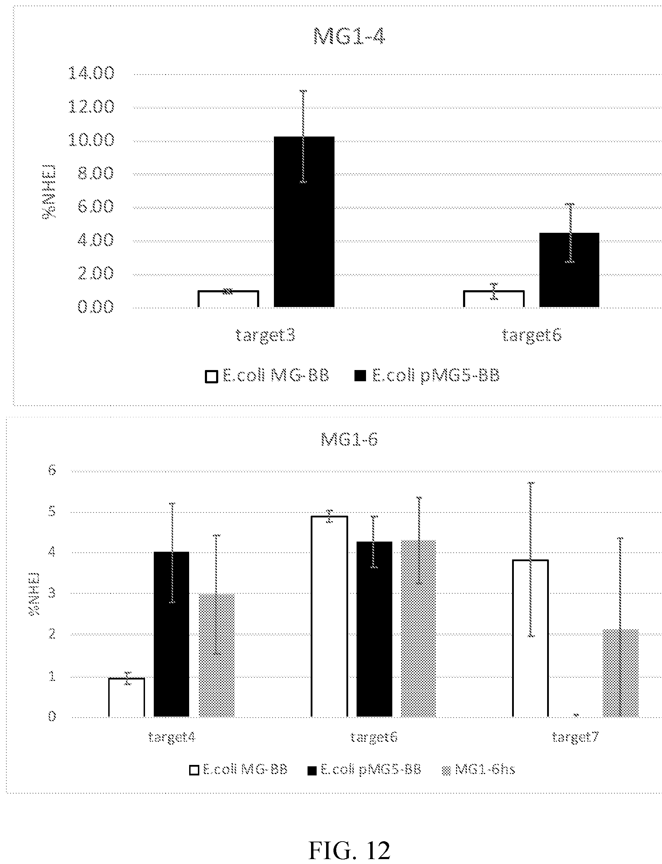

The method of Example 10 was used to demonstrate targeting and cleavage activity in mammalian cells. Open reading frames encoding the MG1-4 (protein SEQ ID NO: 5527) and MG1-6 (protein SEQ ID NO: 5529) sequences were cloned into 2 mammalian expression vectors, one with a C-terminal SV40 NLS and a 2A-GFP tag (E. coli MG-BB) and one with no GFP tag and 2 NLS sequences, one on the N-terminus and one on the C-terminus (E. coli pMG5-BB). For MG1-6, the open reading frame was additionally codon-optimized for mammalian expression (SEQ ID NO: 5589) and cloned into the 2-NLS plasmid backbone (MG-16hs). The results of this experiment are shown in Figure 12. The endonuclease expression vectors were cotransfected into HEK293T cells with a second vector for expressing a sgRNA (e.g., SEQ ID NOs: 5512 or 5515) with a tracr sequence specific for the endonuclease and a guide sequence selected from Tables 3-4. 72 hr after co-transfection the DNA was extracted and used for the preparation of an NGS-library. Cleavage activity was detected by the appearance of internal deletions (NHEJ remnants) proximal to the sequence of the target site. Percent NHEJ was measured via indels in the sequencing of the target site to demonstrate the targeting efficiency of the enzyme in mammalian cells and is presented in Figure 12.

**Table 3: MG1-4 mammalian targeting sequences**

| **MG1-4 Target ID** | **MG1-4 Targeting Sequence** | **Targeting sequence SEQ ID NO:** | **Targeted Gene** |
|---|---|---|---|
| 1 | aatatgtagctgtttgggaggt | 5543 | VEGFA |
| 2 | ctagggggcgctcggccaccac | 5544 | VEGFA |
| 3 | tggctaaagagggaatgggctt | 5545 | VEGFA |
| 4 | cacaccccggctctggctaaag | 5546 | VEGFA |
| 5 | tcggaggagccgtggtccgcgc | 5547 | VEGFA |
| 6 | gcggaccacggctcctccgaag | 5548 | VEGFA |
| 7 | gtacaaacggcagaagctggag | 5549 | EMX1 |
| 8 | gaggaagggcctgagtccgagca | 5550 | EMX1 |
| 9 | aaggcaaacatcctgataatgg | 5551 | Apolipoprotein |

**Table 4: MG1-6 mammalian targeting sequences**

| **MG1-6 Target ID** | **MG1-6 Targeting Sequence** | **Targeting sequence SEQ ID NO:** | **Targeted Gene** |
|---|---|---|---|
| 1 | tctttagccagagccggggtgt | 5552 | VEGFA |
| 2 | tggaccccctatttctgacctc | 5553 | VEGFA |
| 3 | atgggagcccttcttcttctgc | 5554 | EMX1 |
| 4 | tgccacgaagcaggccaatggg | 5555 | EMX1 |
| 5 | tggtgtctgtttgaggttgcta | 5556 | HBB-R01 |
| 6 | gggcaggttggtatcaaggtta | 5557 | HBB-R01 |
| 7 | aggtgctgacgtaggtagtgct | 5558 | FANCF |
| 8 | gccctacttccgctttcacctt | 5559 | FANCF |
| 9 | aatgtatgctggcttttaaggg | 5560 | IVS40 |
| 10 | gctcctttggctagggaagtgt | 5561 | IVS40 |

### Targeted endonuclease activity in mammalian cells (b)

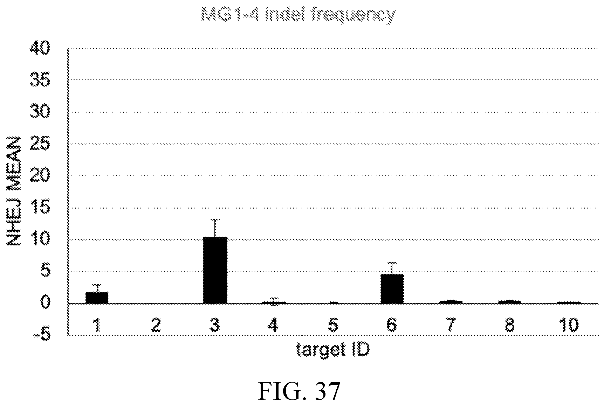

MG1-4 target loci were chosen to test locations in the genome with the PAM nRRRAA (SEQ ID NO: 5527). The spacers corresponding to the chosen target sites were cloned into the sgRNA scaffold in the mammalian vector system Backbone 2 described in Example 10b. The sites are listed in Table 4a below. The activity of MG1-4 at various target sites is shown in Table 4a and Figure 37

**Table 4a: Activity of MG1-4 at various target sites**

| **target ID** | **target sequence** | **PAM** | **locus** | **%NHEJ (mean ± std)** |
|---|---|---|---|---|
| 1 | aatatgtagctgtttgggaggt | CAGAAA | VEGFA | 1.62 ± 1.2 |
| 2 | ctagggggcgctcggccaccac | AGGGAA | VEGFA | -0.07 ± 0 |
| 3 | tggctaaagagggaatgggctt | TGGAAA | VEGFA | 10.32 ± 2.75 |
| 4 | cacaccccggctctggctaaag | AGGGAA | VEGFA | 0.18 ± 0.53 |
| 5 | tcggaggagccgtggtccgcgc | GGGGAA | VEGFA | 0 ± 0.03 |
| 6 | gaagccgagccgagcggagccg | CGAGAA | VEGFA | 4.53 ± 1.73 |
| 7 | gcggaccacggctcctccgaag | GAGGAA | VEGFA | 0.3 ± 0.04 |
| 8 | gtacaaacggcagaagctggag | GAAGAA | EMX1 | 0.23 ± 0.1 |
| 10 | Gaaggcaaacatcctgataatgg | TGAAAA | Apolipoprotein | 0.07 ± 0.06 |

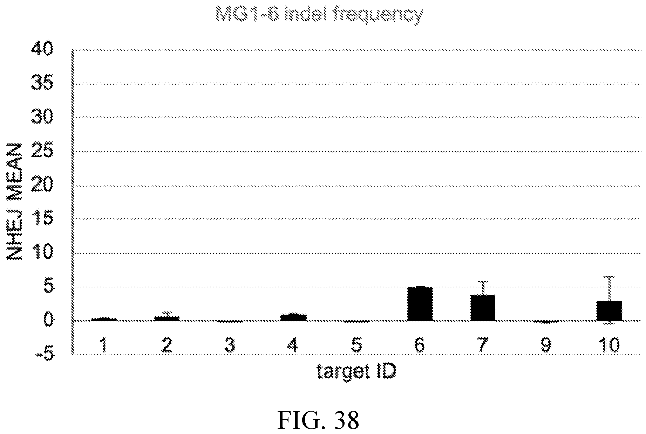

MG1-6 target loci were chosen to test locations in the genome with the PAM nnRRAC (SEQ ID NO: 5529). The spacers corresponding to the chosen target sites were cloned into the sgRNA scaffold in the mammalian vector system backbone 2 described in Example 10b. The sites are listed in Table 4b below. The activity of MG1-6 at various target sites is shown in Table 4b and Figure 38.

**Table 4b: Activity of MG1-6 at various target sites.**

| **target ID** | **target sequence** | **PAM** | **locus** | **%NHEJ (mean ± std)** |
|---|---|---|---|---|
| 1 | tctttagccagagccggggtgt | GCAGAC | VEGFA | 0.385 ± 0.035 |
| 2 | tggaccccctatttctgacctc | CCAAAC | VEGFA | 0.65 ± 0.62 |
| 3 | atgggagcccttcttcttctgc | TCGGAC | EMX1 | -0.045 ±0.105 |
| 4 | tgccacgaagcaggccaatggg | GAGGAC | EMX1 | 0.96 ±0.14 |
| 5 | tggtgtctgtttgaggttgcta | GTGAAC | HBB | -0.09 ±0.09 |
| 6 | gggcaggttggtatcaaggtta | CAAGAC | HBB | 4.915 ±0.135 |
| 7 | aggtgctgacgtaggtagtgct | TGAGAC | FANCF | 3.85 ±1.86 |
| 9 | aatgtatgctggcttttaaggg | GGAAAC | IVS40 | -0.215 ±0.105 |
| 10 | gctcctttggctagggaagtgt | TAAAAC | IVS40 | 3.03 ±3.5 |

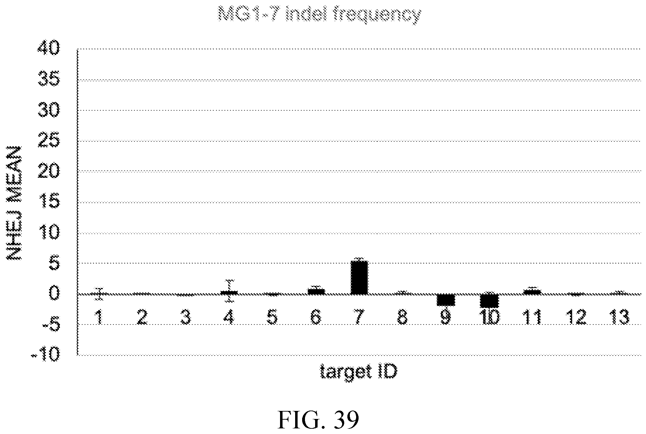

MG1-7 target loci were chosen to test locations in the genome with the PAM nRRRAAG (SEQ ID NO: 5515). The spacers corresponding to the chosen target sites were cloned into the sgRNA scaffold in the mammalian vector system backbone 2 described in Example 10b. The sites are listed in Table 4c below. The activity of MG1-7 at various target sites is shown in Table 4c and Figure 39.

**Table 4c: Activity of MG1-7 at various target sites**

| **target ID** | **target sequence** | **PAM** | **locus** | **%NHEJ (mean ± std)** |
|---|---|---|---|---|
| 1 | atcaaactgtgagcatcttcag | ggaaaag | IVS40 | 0.105 ±0.88 |
| 2 | cagcgaatctactcagccagag | caggaag | IVS40 | 0.075 ±0.02 |
| 3 | ctttaacttgtgtgattctgga | gaggaag | IVS40 | -0.25 |
| 4 | agcttaattagctcctttggct | agggaag | IVS40 | 0.54 ±1.71 |
| 5 | ccatggtgcatctgactcctga | ggagaag | HBB | 0.005 ±0.01 |
| 6 | tgcttgagaccgccagaagctc | ggaaaag | FANCF | 0.91 ±0.38 |
| 7 | cgagaacagcccagaagttgga | cgaaaag | VEGFA | 5.49 ±0.44 |
| 8 | cggaggagccgtggtccgcgcg | ggggaag | VEGFA | 0.27 ±0.20 |
| 9 | atgtgtgagaaaaatttaatca | taagaag | Apolipoprotein | -1.85 ±1.89 |
| 10 | aggtccaagtaaaatttgtctt | agaaaag | Apolipoprotein | -2.17 ±2.55 |
| 11 | gtacaaacggcagaagctggag | gaggaag | EMX1 | 0.77 ±0.33 |
| 12 | aggaagggcctgagtccgagca | gaagaag | EMX1 | 0.01 ±0.01 |
| 13 | aagggcctgagtccgagcagaa | gaagaag | EMX1 | 0.18 ±0.22 |

### Example 12.- Characterization of MG2 family members

### PAM Specificity, tracrRNA/sgRNA Validation

The targeted endonuclease activity of MG2 family members was confirmed in the myTXTL system as described in Example 6. Results of this assay are shown in Figures 17-20. In the assay shown in Figures 17-20, active proteins that successfully cleave the library result in a band around 170 bp in the gel. Amplification products were observed for MG2-1 (see gel 2 lane 11 and gel 4 lane 6) and MG2-7 (see gel 11 lane 10) (SEQ ID NOs: 320 and 321, respectively). Sequencing the PCR products revealed active PAM sequences in Table 5 below:

**Table 5: PAM sequence specificities and related data for MG2 enzymes**

| **Enzyme** | **Enzym e protein SEQ ID NO** | **Native (dual guide) PAM** | **PAM SEQ ID NO:** | **tracrRNA SEQ ID NO:** | **sgRNA SEQ ID NO:** | **Synthetic (single guide) PAM** | **Syntheti c (single guide) PAM SEQ ID NO:** |
|---|---|---|---|---|---|---|---|
| MG2-1 | 320 | nRCGT A | 5531 | 5490 | N/A | N/A | N/A |
| MG2-7 | 321 | N/A | N/A | 5491 | 5465 | NNNRTA | 5516 |

### Targeted endonuclease activity in bacterial cells

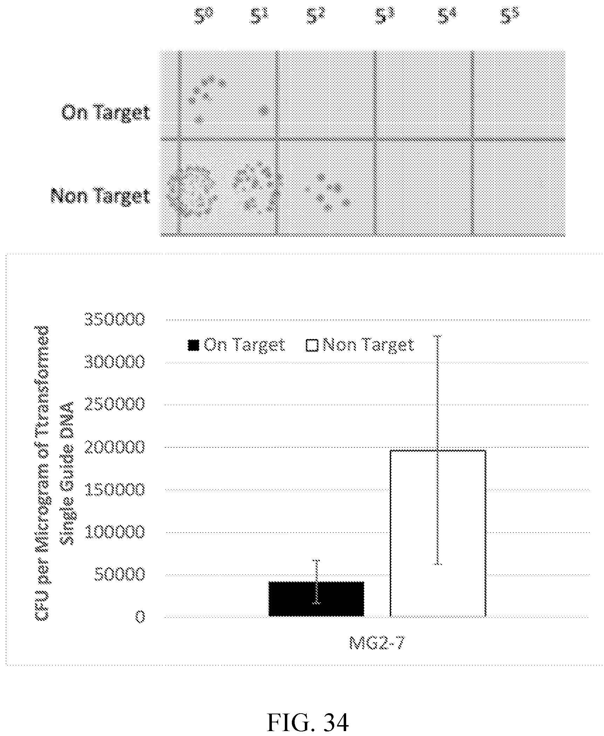

*In vivo* activity of the MG2-7 endonuclease system with a sgRNA (endonuclease SEQ ID NO: 321; sgRNA SEQ ID NO: 5465) and an AGCGTAAG PAM sequence was confirmed using the method described in Example 9. Transformed *E. coli* were plated in serial dilution, and the results (showing *E. coli* serial dilutions in the left panel and quantitated growth in the right panel) are presented in Figure 34. A substantial reduction in the growth of *E. coli* expressing on target sgRNA compared to *E. coli* expressing non-target sgRNA indicates that genomic DNA was specifically cleaved by the MG1-4 endonuclease in *E. coli* cells.

### Example 13.- Characterization of MG3 family members

### PAM Specificity, tracrRNA/sgRNA Validation

The targeted endonuclease activity of MG3 family members was confirmed using the myTXTL system as described in Example 6 using tracr sequences and CRISPR arrays. In this assay, PCR amplification of cleaved target plasmids yields a product that migrates at approximately 170 bp in the gel, as shown in Figures 17-20. Amplification products were observed for MG3-6 (dual guide: see gel 2 lane 8; single guide: see gel 3 lane 3), MG3-7 (dual guide: see gel 2 lane 3, single guide: see gel 3 lane 4), and MG3-8 (dual guide: see gel 9 lane 5) (SEQ ID NOs: 421, 422, and 423, respectively). Sequencing the PCR products revealed active PAM sequences in Table 6 below:

**Table 6: PAM sequence specificities and related data for MG3 enzymes**

| **Enzyme** | **Enzym e protein SEQ ID NO** | **Native (dual guide) PAM** | **PAM SEQ ID NO:** | **tracrRN A SEQ ID NO:** | **sgRNA SEQ ID NO:** | **Synthetic (single guide) PAM** | **Synthetic (single guide) PAM SEQ ID NO:** |
|---|---|---|---|---|---|---|---|
| MG3-6 | 421 | nnRGGTT | 5532 | 5500 | 5466 | nnGGG | 5517 |
| MG3-7 | 422 | nnRnYAY | 5533 | 5501 | 5467 | nnGnTnT | 5518 |
| MG3-8 | 423 | nnRGGTT | 5534 | 5502 | N/A | N/A | N/A |

Synthetic single guide RNAs (sgRNAs) were designed based on the sequences and predicted structures of the tracrRNAs and are presented as SEQ ID NOs: 5466-5467. The PAM sequence screen of Example 6 was repeated with the sgRNAs. The results of this experiment are also presented in Table 6, which reveals that PAM specificity changed slightly when using sgRNAs.

### Targeted endonuclease activity In Vitro

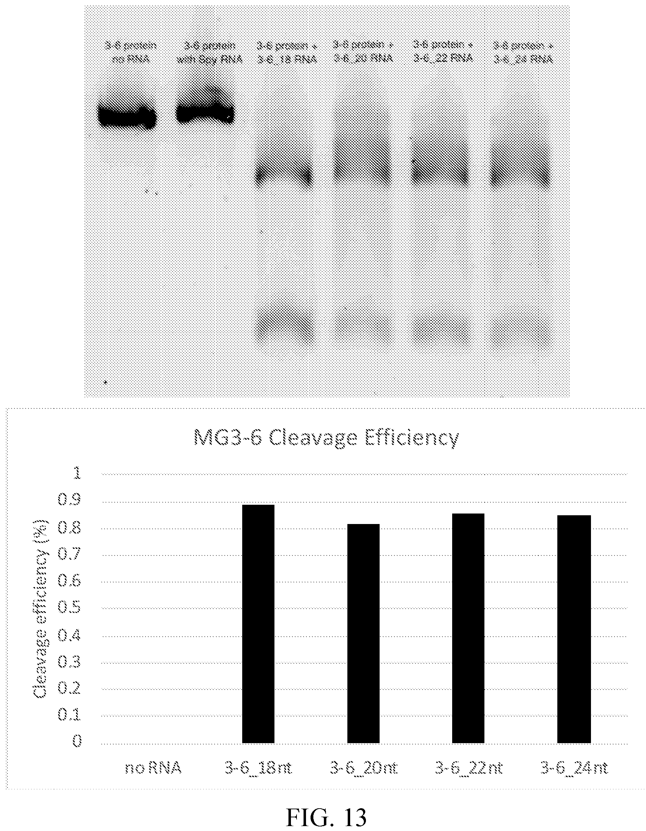

*In vitro* activity of the MG3-6 (endonuclease SEQ ID NO: 421) was verified with the PAM sequence GTGGGTTA using the method of Example 8. The single guide sequence reported above (SEQ ID NO: 5466) was used, with varying spacer/targeting sequence lengths from 18-24 nt replacing the Ns of the sequence. The results are shown in Figure 13, wherein the top panel shows a gel demonstrating DNA cleavage by MG3-6 in combination with different sgRNAs having different targeting sequence lengths (18-24nt), and the bottom panel shows the same data quantified as a bar graph. The data demonstrated that targeting sequences from 18-24 nucleotides were functional with the MG3-6/sgRNA system.

### Targeted endonuclease activity in bacterial cells

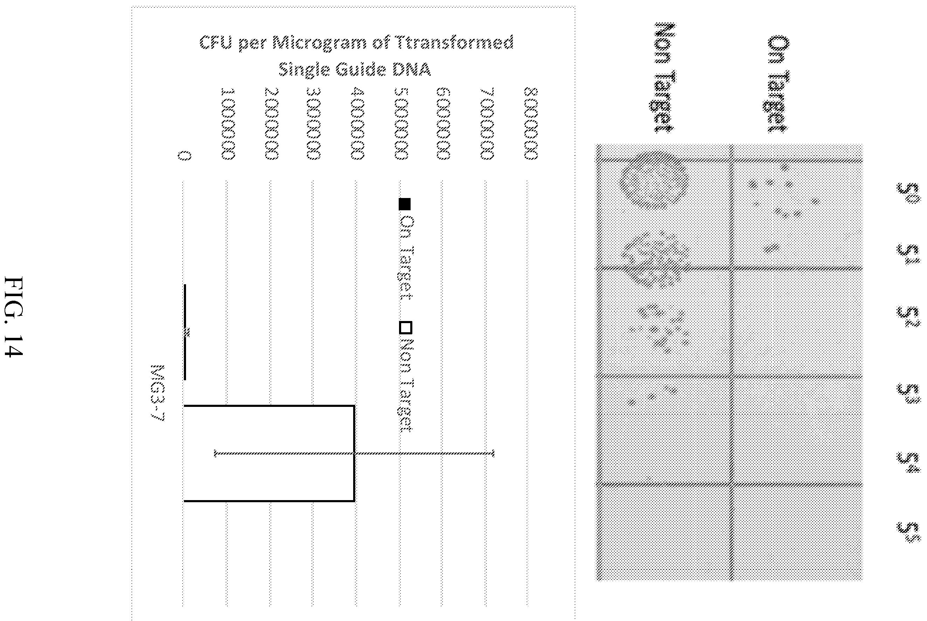

*In vivo* activity of the MG3-7 endonuclease system (protein SEQ ID NO: 422; sgRNA SEQ ID NO: 5467) was tested with the PAM sequence TGGACCTG using the method of Example 9. Transformed E. coli were plated in serial dilution, and the results (showing E. coli serial dilutions in the top panel and quantitated growth in the bottom panel) are presented in Figure 14. A substantial reduction in the growth of *E. coli* expressing on target sgRNA compared to *E. coli* expressing non-target sgRNA indicates that genomic DNA was being specifically cleaved by the MG3-7 endonuclease system.

### Targeted endonuclease activity in mammalian cells (a)

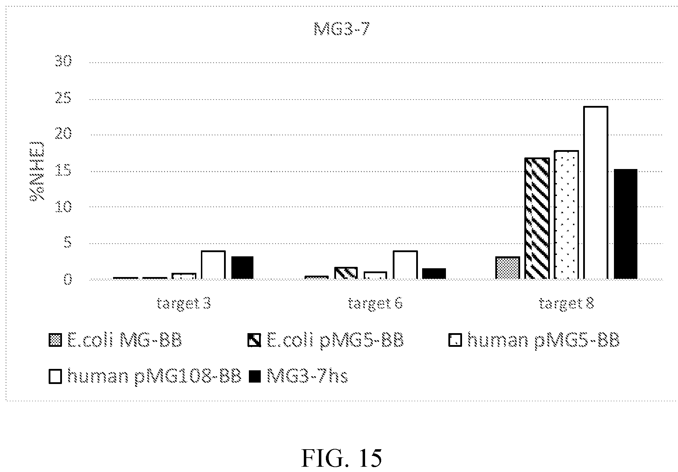

The method of Example 10 was used to demonstrate targeting and cleavage activity in mammalian cells. Open reading frames encoding MG3-7 (protein SEQ ID NO: 422) was cloned into 2 mammalian expression vectors, one with a C-terminal SV40 NLS and a 2A-GFP tag (E. coli MG-BB) and one with no GFP tag and 2 NLS sequences, one on the N-terminus and one on the C-terminus (E. coli pMG5-BB). The endonuclease expression vectors were cotransfected into HEK293T cells with a second vector for expressing the sgRNA above with a guide sequence selected from Table 7. The results of this experiment are shown in Figure 12 . 72 hr after co-transfection DNA was extracted and used for the preparation of an NGS-library. Cleavage activity was detected by the appearance of internal deletions (NHEJ remnants) in the vicinity of the target site. Results are presented in Figure 15.

The target site which were encoded on the sgRNA plasmids are shown in Table 7 below.

**Table 7: MG3-7 mammalian targeting sequences**

| **MG3-7 Target ID** | **MG3-7 Targeting Sequence** | **Targeting sequence SEQ ID NO:** | **Targeted Gene** |
|---|---|---|---|
| 1 | cccctatttctgacctcccaaa | 5563 | VEGFA |
| 2 | tgtggttccagaaccggaggac | 5564 | EMX1 |
| 3 | ggccctgggcaggttggtatca | 5565 | HBB-R01 |
| 4 | tccttaaacctgtcttgtaacc | 5566 | HBB-R01 |
| 5 | ctgactcctgaggagaagtctg | 5567 | HBB-R01 |
| 6 | tccgagcttctggcggtctcaa | 5568 | FANCF |
| 7 | tatcatttcgcggatgttccaa | 5569 | FANCF |
| 8 | tcgggcagagggtgcatcacct | 5570 | Apolipoprotein |
| 9 | ataataagcagaacttttagtg | 5571 | Fibrinogen |
| 10 | gttttctttagttattaatttc | 5572 | Fibrinogen |

### Targeted endonuclease activity in mammalian cells (b)

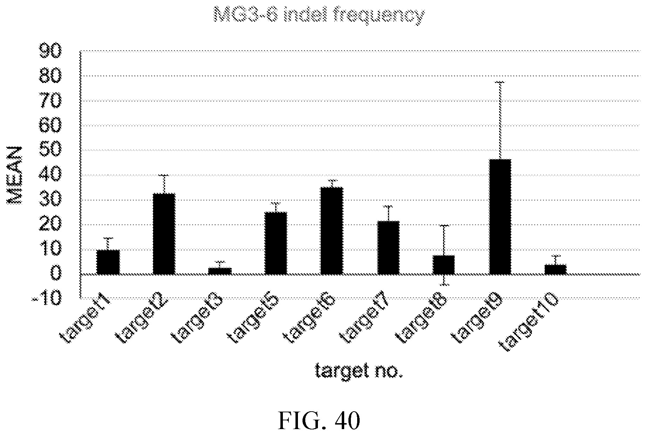

MG3-6 target loci were chosen to test locations in the genome with the PAM nnRGGTT (SEQ ID NO: 5532). The spacers corresponding to the chosen target sites were cloned into the sgRNA scaffold in the mammalian vector system backbone 1 described in Example 10b. The sites are listed in Table 7a below. The activity of MG3-6 at various target sites is shown in Table 7a and Figure 40.

**Table 7a: Activity of MG3-6 at various target sites**

| target ID | target sequence | PAM | locus | %NHEJ (mean ± std) |
|---|---|---|---|---|
| target1 | gagtgactgaaacttcacagaa | tagggtt | Albumin | 9.75 ±4.67 |
| target2 | ttctatttatgagatcaacagc | acaggtt | Albumin | 32.75 ±7.42 |
| targets | cagatgcaccatggtgtctgtt | tgaggt | HBB_R01 | 2.52 ±2.13 |
| targets | gaggccctgggcaggttggtat | caaggtt | HBB_R01 | 25.16 ±3.67 |
| target6 | aggttggtatcaaggttacaag | acaggtt | HBB_R01 | 35.27 ±2.78 |
| target7 | tcaactttcctggcaacttgcg | gtaggtt | Apolipoprotein | 21.57 ±5.70 |
| targets | aggaacattcagttaagatagt | ctaggtt | Fibrinogen | 7.56 ±11.99 |
| target9 | tttaaattatgaatccatctct | aaaggtt | Fibrinogen | 46.32 ±31.46 |
| target10 | gagtgactgaaacttcacagaa | tagggtt | Albumin | 3.93 ±3.53 |

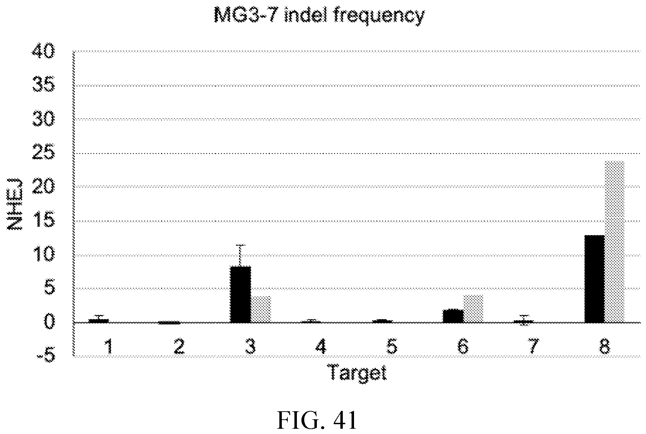

MG3-7 target loci were chosen to test locations in the genome with the PAM nnRnTAC (SEQ ID NO: 6303). The spacers corresponding to the chosen target sites were cloned into the sgRNA scaffold in the mammalian vector systems described in Example 10b. The sites are listed in Table 7b below. The activity of MG3-7 at various target sites is shown in Table 7b and Figure 41.

**Table 7b: Activity of MG3-7 at various target sites**

| **target ID** | **target sequence** | **PAM** | **locus** | **%NHEJ backbone 2 (mean ± std)** | **%NHEJ backbone 1** |
|---|---|---|---|---|---|
| 1 | cccctatttctgacctcccaaa | CAGCTAC | VEGFA | 0.54 ± 0.44 | |
| 2 | tgtggttccagaaccggaggac | AAAGTAC | EMX1 | -0.09 ±0.04 | |
| 3 | ggccctgggcaggttggtatca | AGGTTAC | HBB | 8.275±0.3.12 | 3.87 |
| 4 | tccttaaacctgtcttgtaacc | TTGATAC | HBB | 0.16 ±0.20 | |
| 5 | ctgactcctgaggagaagtctg | CCGTTAC | HBB | 0.25±0.16 | |
| 6 | tccgagcttctggcggtctcaa | GCACTAC | FANCF | 1.84±0.10 | 3.95 |
| 7 | tatcatttcgcggatgttccaa | TCAGTAC | FANCF | 0.32±0.68 | |
| 8 | tcgggcagagggtgcatcacct | GGACTAC | Apolipoprotein | 13 | 23.88 |

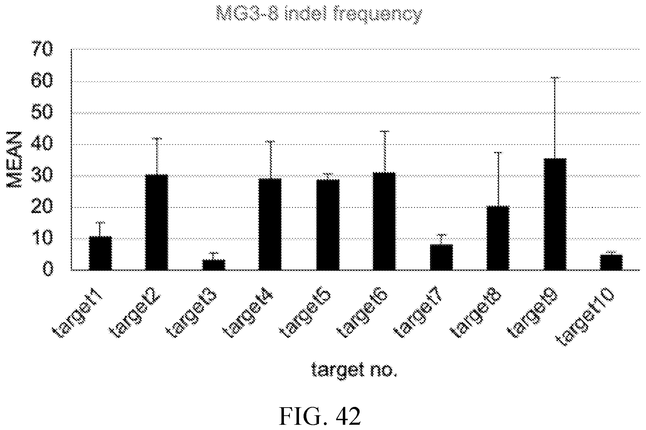

MG3-8 target loci were chosen to test locations in the genome with the PAM nnRGGTT (SEQ ID NO: 5534). The spacers corresponding to the chosen target sites were cloned into the sgRNA scaffold in the mammalian vector system backbone 1 described in Example 10b. The sites are listed in Table 7c below. The activity of MG3-8 at various target sites is shown in Table 7c and Figure 42.

**Table 7c: Activity of MG3-8 at various target sites**

| **target ID** | **target sequence** | **PAM** | **locus** | **%NHEJ (mean ± std)** |
|---|---|---|---|---|
| target1 | gagtgactgaaacttcacagaa | tagggtt | Albumin | 10.81 ± 4.26 |
| target2 | ttctatttatgagatcaacagc | acaggtt | Albumin | 30.48 ± 11.17 |
| target3 | cagatgcaccatggtgtctgtt | tgaggt | HBB | 3.39± 2.13 |
| target4 | gaagttggtggtgaggccctgg | gcaggtt | HBB | 29.00± 11.87 |
| targets | gaggccctgggcaggttggtat | caaggtt | HBB | 28.69± 1.96 |
| target6 | aggttggtatcaaggttacaag | acaggtt | HBB | 30.86±13.24 |
| target7 | tcaactttcctggcaacttgcg | gtaggtt | Apolipoprotein | 7.98±3.23 |
| targets | aggaacattcagttaagatagt | ctaggtt | Fibrinogen | 20.29±17.10 |
| target9 | tttaaattatgaatccatctct | aaaggtt | Fibrinogen | 35.5±25.5 |
| target10 | gagtgactgaaacttcacagaa | tagggtt | Albumin | 4.9±0.71 |

### Example 13.- Characterization of MG4 family members

### PAM Specificity, tracrRNA/sgRNA Validation

The targeted endonuclease activity of MG4 family endonuclease systems was confirmed using the myTXTL system as described in Example 6. In this assay, PCR amplification of cleaved target plasmids yields a product that migrates at approximately 170 bp in the gel, as shown in Figures 17-20. Amplification products were observed for, MG4-2 (dual guide: see gel2 lane 9, single guide: see gel 10 lane 7) (SEQ ID NO: 432). Sequencing the PCR products revealed active PAM sequences shown in Table 8 below.

**Table 8: PAM sequence specificities and related data for MG4 enzymes**

| **Enzyme** | **Enzyme protein SEQ ID NO** | **tracrRN A SEQ ID NO:** | **sgRNA SEQ ID NO:** | **Synthetic (single guide) PAM** | **Synthetic (single guide) PAM SEQ ID NO:** |
|---|---|---|---|---|---|
| MG4-5 | 432 | 5503 | 5468 | nnCCR | 5519 |

### Example 14.- Characterization of MG14 family members

### PAM Specificity, tracrRNA/sgRNA Validation

The targeted endonuclease activity of MG14 family members (was confirmed using the myTXTL system as described in Example 6. In this assay, PCR amplification of cleaved target plasmids yields a product that migrates at approximately 170 bp in the gel, as shown in Figures 17-20. Amplification products were observed for MG14-1 (dual guide: see gel 1 lane 4, single guide: see gel 3 lane 8) (SEQ ID NO: 678). Sequencing the PCR products revealed active PAM sequence specificities shown in Table 9 below.

**Table 9: PAM sequence specificities and related data for MG14 enzymes**

| **Enzyme** | **Enzym e protein SEQ ID NO** | **Native (dual guide) PAM** | **PAM SEQ ID NO:** | **tracrRN A SEQ ID NO:** | **sgRNA SEQ ID NO:** | **Synthetic (single guide) PAM** | **Synthetic (single guide) PAM SEQ ID NO:** |
|---|---|---|---|---|---|---|---|
| MG14-1 | 678 | NNNNG GTA | 5535 | 5505 | 5469 | NNNGGR TA | 5520 |

### Targeted endonuclease activity in bacterial cells

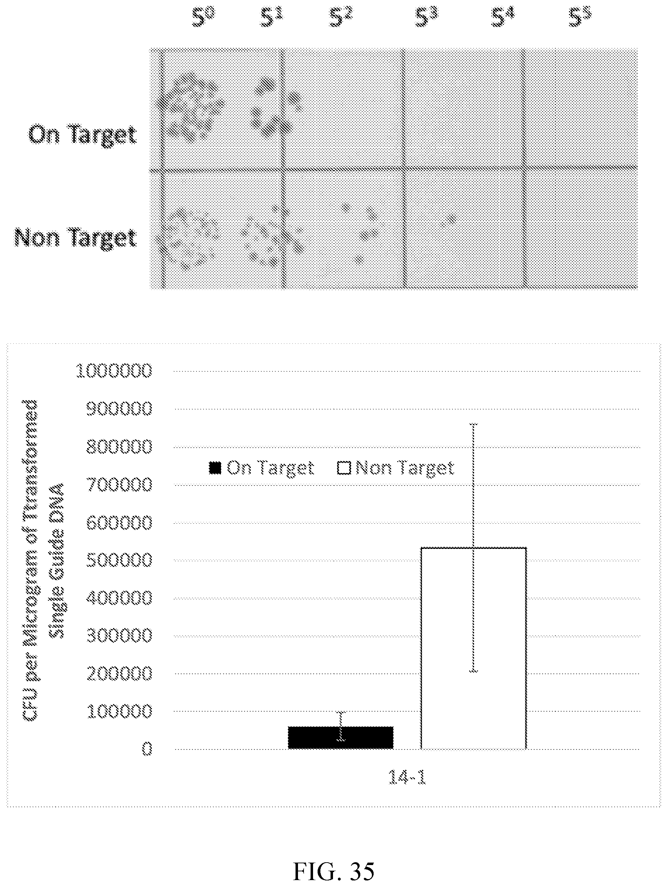

*In vivo* activity of the MG14-1 endonuclease system with a sgRNA (endonuclease SEQ ID NO: 678; sgRNA SEQ ID NO: 5469) and a GGCGGGGA PAM sequence was confirmed using the method described in Example 9. Transformed *E. coli* were plated in serial dilution, and the results (showing *E. coli* serial dilutions in the left panel and quantitated growth in the right panel) are presented in Figure 35. A substantial reduction in the growth of *E. coli* expressing on target sgRNA compared to *E. coli* expressing non-target sgRNA indicates that genomic DNA was specifically cleaved by the MG1-4 endonuclease in *E. coli* cells.

### Targeted endonuclease activity in mammalian cells

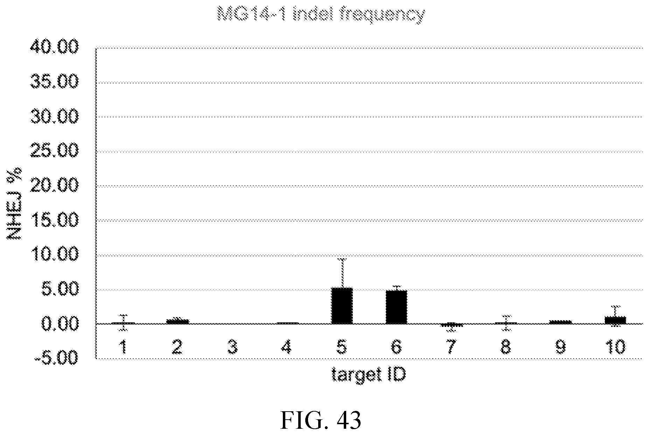

MG14-1 target loci were chosen to test locations in the genome with the PAM nnnnGGTA (SEQ ID NO: 5535). The spacers corresponding to the chosen target sites were cloned into the sgRNA scaffold in the mammalian vector system backbone 2 described in Example 10b. The sites are listed in Table 9a below. The activity of MG14-1 at various target sites is shown in Table 9a and Figure 43.

**Table 9a: Activity of MG14-1 at various target sites**

| **target ID** | **target sequence** | **PAM** | **locus** | **%NHEJ (mean ± std)** |
|---|---|---|---|---|
| 1 | cttattaataaaattcaaacat | CCTAGGTA | Albumin | 0.22± 1.04 |
| 2 | gttggtggtgaggccctgggca | GGTTGGTA | HBB | 0.67±0.16 |
| 3 | ctagttaatggaataaaacatt | TTATGGTA | Fibrinogen | 0.15 |
| 4 | gcattataatgcaccaaggctt | TATAGGTA | Fibrinogen | 0.2 |
| 5 | gtggcggggtcccaggtgctga | CGTAGGTA | FANCF | 5.33±4.13 |
| 6 | tgtccgccgccggccggggagg | AGGTGGTA | VEGFA | 4.88±0.65 |
| 7 | ggtgatgcaccctctgcccgat | GCTTGGTA | Apolipoprotein | -0.35±0.59 |
| 8 | ccacatcaactttcctggcaac | TTGCGGTA | Apolipoprotein | 0.19±0.98 |
| 9 | gctagttaatggaataaaacatt | TTATGGTA | Fibrinogen | 0.6 |
| 10 | gcattataatgcaccaaggctt | TATAGGTA | Fibrinogen | 1.17±1.42 |

### Example 15.- Characterization of MG15 family members

### PAM Specificity, tracrRNA/sgRNA Validation

The targeted endonuclease activity of MG15 family members was confirmed using the myTXTL system as described in Example 6. In this assay, PCR amplification of cleaved target plasmids yields a product that migrates at approximately 170 bp in the gel, as shown in Figures 17-20. Amplification products were observed for MG15-1 (dual guide: see gel 7 lane 7, single guide: see gel 3 lane 9) (SEQ ID NO: 930). Sequencing the PCR products revealed active PAM sequence specificities detailed in Table 10 below.

**Table 10:**

| **Enzyme** | **Enzyme protein SEQ ID NO** | **Native (dual guide) PAM** | **PAM SEQ ID NO:** | **tracrRN A SEQ ID NO:** | **sgRNA SEQ ID NO:** | **Synthetic (single guide) PAM** | **Synthetic (single guide) PAM SEQ ID NO:** |
|---|---|---|---|---|---|---|---|
| MG15-1 | 930 | nnnnC | 5536 | 5506 | 5470 | nnnnC | 5521 |

### In Vitro Activity

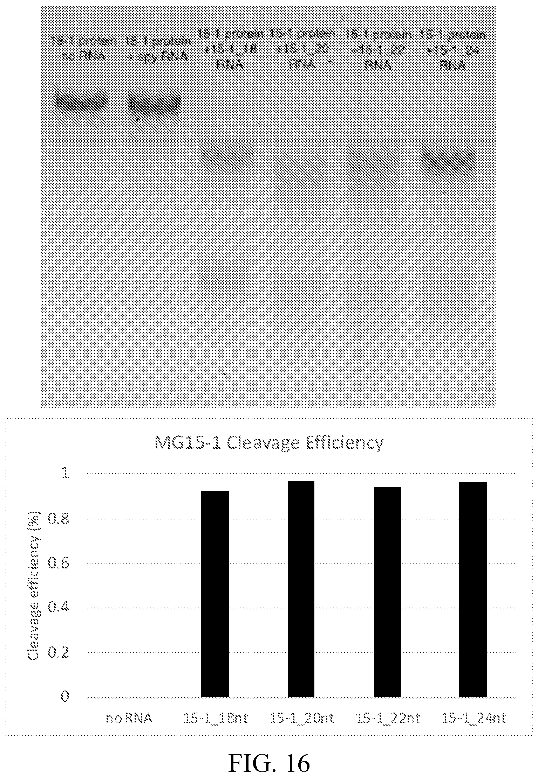

*In vitro* activity of the MG15-1 endonuclease system (protein SEQ ID NO: 930; sgRNA SEQ ID NO:5470) was tested with the PAM sequence GGGTCAAA using the method of Example 8. The single guide sequence reported above (SEQ ID NO: 5470) was used, with varying spacer/targeting sequence lengths from 18-24 nt (replacing the Ns of the sequence). The results are shown in Figure 16, wherein the top panel shows a gel demonstrating DNA cleavage by MG15-1 in combination with different sgRNAs having different targeting sequence lengths (18-24nt), and the bottom panel shows the same data quantified as a bar graph. The data demonstrated that targeting sequences from 18-24 nucleotides were functional with the MG15-1/sgRNA system.

### Targeted endonuclease activity in bacterial cells

*In vivo* activity of the MG15-1 endonuclease system with a sgRNA (endonuclease SEQ ID NO: 930; sgRNA SEQ ID NO: 5470) and a GGGTCAAA PAM sequence was confirmed using the method described in Example 9. Transformed *E. coli* were plated in serial dilution, and the results (showing *E. coli* serial dilutions in the left panel and quantitated growth in the right panel) are presented in Figure 35. A substantial reduction in the growth of *E. coli* expressing on target sgRNA compared to *E. coli* expressing non-target sgRNA indicates that genomic DNA was specifically cleaved by the MG1-4 endonuclease in *E. coli* cells.

### Example 16.- Characterization of MG16 family members

### PAM Specificity, tracrRNA/sgRNA Validation

The targeted endonuclease activity of MG16 family members was confirmed using the myTXTL system as described in Example 6. In this assay, PCR amplification of cleaved target plasmids yields a product that migrates at approximately 170 bp in the gel, as shown in Figures 17-20. Amplification products were observed for MG16-2 (see gel 11, lane 17) (SEQ ID NO: 1093). Sequencing the PCR products revealed active PAM sequence specificities detailed in Table 11 below.

**Table 11: PAM sequence specificities and related data for MG16 enzymes**

| **Enzyme** | **Enzym e protein SEQ ID NO** | **sgRNA SEQ ID NO:** | **Synthetic (single guide) PAM** | **Synthetic (single guide) PAM SEQ ID NO:** |
|---|---|---|---|---|
| MG16-2 | 1093 | 5471 | nRTnCC | 5522 |

### Example 17.- Characterization of MG18 family members

### PAM Specificity, tracrRNA/sgRNA Validation

The targeted endonuclease activity of MG18 family members was confirmed using the myTXTL system as described in Example 6. In this assay, PCR amplification of cleaved target plasmids yields a product that migrates at approximately 170 bp in the gel, as shown in Figures 17-20. Amplification products were observed for MG18-1 (dual guide: see gel 9 lane 9, single guide: see gel 11 lane 12) (SEQ ID NO: 1354). Sequencing the PCR products revealed active PAM sequence specificities detailed in Table 12 below.

**Table 12: PAM sequence specificities and related data for MG18 enzymes**

| **Enzyme** | **Enzym e protein SEQ ID NO** | **Native (dual guide) PAM** | **PAM SEQ ID NO:** | **tracrRN A SEQ ID NO:** | **sgRNA SEQ ID NO:** | **Synthetic (single guide) PAM** | **Synthetic (single guide) PAM SEQ ID NO:** |
|---|---|---|---|---|---|---|---|
| MG18-1 | 1354 | nRWART | 5537 | 5508 | 5472 | nnnRRT | 5523 |

### Targeted endonuclease activity in mammalian cells

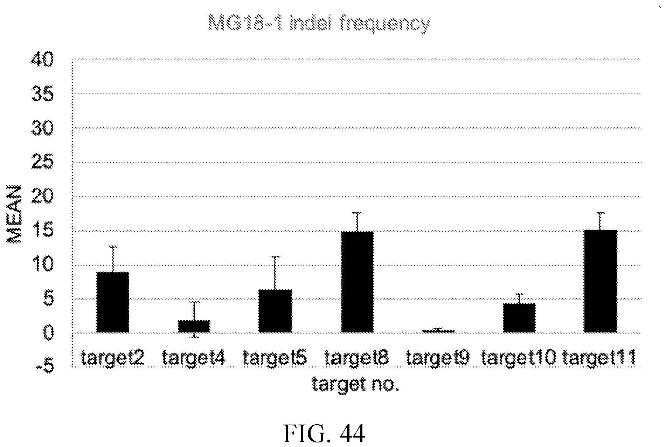

MG18-1 target loci were chosen to test locations in the genome with the PAM nRWART (SEQ ID NO: 5537). The spacers corresponding to the chosen target sites were cloned into the sgRNA scaffold in the mammalian vector system backbone 1 described in Example 10b. The sites are in Table 12a below. The activity of MG18-1 at various target sites is shown in Table 12a and Figure 44.

**Table 12a: Activity of MG18-1 at various target sites**

| **target ID** | **target sequence** | **PAM** | **locus** | **%NHEJ (mean ± std)** |
|---|---|---|---|---|
| target2 | tgcttattatggacaagtagca | agaaat | Fibrinogen | 9.00±3.70 |
| target4 | tccctgaagatgctcacagttt | gatagt | IVS40 | 1.99±2.56 |
| target5 | ttttatttctcctgcatatgat | gatagt | IVS40 | 6.30±4.82 |
| target8 | tgtggggcaaggtgaacgtgga | tgaagt | HBB | 14.90±2.73 |
| target9 | atggtgcatctgactcctgagg | agaagt | HBB | 0.39±0.32 |
| target10 | agaacagcccagaagttggacg | aaaagt | VEGFA | 4.3±1.29 |
| target11 | tcctccgaagcgagaacagccc | agaagt | VEGFA | 15.22±2.40 |

### Example 18.- Characterization of MG21 family members

### PAM Specificity, tracrRNA/sgRNA Validation

The targeted endonuclease activity of MG21 family was confirmed using the myTXTL system as described in Example 6. In this assay, PCR amplification of cleaved target plasmids yields a product that migrates at approximately 170 bp in the gel, as shown in Figures 17-20. Amplification products were observed for MG21-1 (see gel 11 lane 2) (SEQ ID NO: 1512). Sequencing the PCR products revealed active PAM sequence specificities detailed in Table 13 below.

**Table 13:**

| **Enzyme** | **Enzyme protein SEQ ID NO** | **sgRNA SEQ ID NO:** | **Synthetic (single guide) PAM** | **Synthetic (single guide) PAM SEQ ID NO:** |
|---|---|---|---|---|
| MG21-1 | 1512 | 5473 | nnRnR | 5524 |

### Example 19.- Characterization of MG22 family members

### PAM Specificity, tracrRNA/sgRNA Validation

The targeted endonuclease activity of MG22 family members was confirmed using the myTXTL system as described in Example 6. In this assay, PCR amplification of cleaved target plasmids yields a product that migrates at approximately 170 bp in the gel, as shown in Figures 17-20. In the assay shown Figures 17-20, active proteins that successfully cleave the library result in a band around 170 bp in the gel. Amplification products were observed for MG22-1 (see gel 11 lane 3) (protein SEQ ID NO: 1656). Sequencing the PCR products revealed active PAM sequence specificities detailed in Table 14 below.

**Table 14:**

| **Enzyme** | **Enzyme protein SEQ ID NO** | **Native (dual guide) PAM** | **PAM SEQ ID NO:** | **tracrRN A SEQ ID NO:** | **sgRNA SEQ ID NO:** | **Synthetic (single guide) PAM** | **Synthetic (single guide) PAM SEQ ID NO:** |
|---|---|---|---|---|---|---|---|
| MG22-1 | 1656 | N/A | N/A | 5510 | 5474 | nnRCnT | 5525 |

### Example 20.- Characterization of MG23 family members

### PAM Specificity, tracrRNA/sgRNA Validation

The targeted endonuclease activity of MG23 family members was confirmed using the myTXTL system as described in Example 6. In this assay, PCR amplification of cleaved target plasmids yields a product that migrates at approximately 170 bp in the gel, as shown in Figures 17-20. Amplification products were observed for MG23-1 (see gel 11 lane 4) (SEQ ID NO: 1756). Sequencing the PCR products revealed active PAM sequences specificities for these enzymes detailed Table 15 below.

**Table 15:**

| **Enzyme** | **Enzyme protein SEQ ID NO** | **tracrRNA SEQ ID NO:** | **sgRNA SEQ ID NO:** | **Synthetic (single guide) PAM** | **Synthetic (single guide) PAM SEQ ID NO:** |
|---|---|---|---|---|---|
| MG23-1 | 1756 | 5511 | 5475 | nRRA | 5526 |

### Example 21.- Mammalian activity of MG21-MG23 family members

To show targeting and cleavage activity in mammalian cells, the protein sequences were cloned into a mammalian expression vector with flanking N and C-terminal SV40 NLS sequences, a C-terminal His tag, and a 2A-GFP tag at the C terminus after the His tag (backbone 1) or an expression vector with flanking NLS sequences and C-terminal His tag but no 2A GFP tag (backbone 2). The DNA sequence for the protein can be the native sequence, the *E. coli* codon optimized sequence, or the mammalian codon optimized sequence. The single guide RNA sequence with a gene target of interest is also cloned into a mammalian expression vector. The two plasmids are cotransfected into HEK293T cells. 72 hr after co-transfection of the expression plasmid and a sgRNA targeting plasmid into HEK293T cells, the DNA is extracted and used for the preparation of an NGS-library. Percent NHEJ is measured via indels in the sequencing of the target site to demonstrate the targeting efficiency of the enzyme in mammalian cells. 7-12 different target sites were chosen for testing each protein's activity. An arbitrary threshold of 5% indels is used to identify active candidates.

MG21-1 target loci were chosen to test locations with the PAM nnRnR. The spacers corresponding to the chosen target sites were cloned into the sgRNA scaffold in the mammalian vector system backbone 2 described above. The sites are listed below in Table 16.

**Table 16: MG21 target loci and target efficiency demonstrated in mammalian cells**

| **target ID** | **target sequence** | **PAM** | **locus** | **%NHEJ (mean ± std)** |
|---|---|---|---|---|
| 1 | TCCCTCTTTAGCCAGAGCCGGG (SEQ ID NO: 5897) | GTGTG (SEQ ID NO: 5907) | VEGFA_T22 | 25.5±9.5 |
| 2 | CCAAAGCCCATTCCCTCTTTAG (SEQ ID NO: 5898) | CCAGA (SEQ ID NO: 5908) | VEGFA_T22 | 0.79±0.04 |
| 3 | ACGGCAGTCACTAGGGGGCGCT (SEQ ID NO: 5899) | CTGCA (SEQ ID NO: 5909) | VEGFA_T22 | 2.30±0.28 |
| 4 | GGGGGCGCTCGGCCACCACAGG (SEQ ID NO: 5900) | TAGTG (SEQ ID NO: 5910) | VEGFA_T22 | 0.69±0.54 |
| 5 | ACAACTGTGTTCACTAGCAACC (SEQ ID NO: 5901) | TCAAA (SEQ ID NO: 5911) | HBB-R01 | 0.07±0.11 |
| 6 | GACACCATGGTGCATCTGACTC (SEQ ID NO: 5902) | CTGAG (SEQ ID NO: 5912) | HBB-R01 | 0.0±0.03 |
| 7 | TCACTAGCAACCTCAAACAGAC (SEQ ID NO: 5903) | ACACA (SEQ ID NO: 5913) | HBB-R01 | 0.04 ±0.16 |
| 8 | GTATCAAGGTTACAAGACAGGT (SEQ ID NO: 5904) | TTAAG (SEQ ID NO: 5914) | HBB-R01 | 0.±0.028 |
| 9 | GCTGGAGCAGTACTCTAGTAAC (SEQ ID NO: 5905) | CTGGA (SEQ ID NO: 5915) | Gucy-2D_new | 0.0±0.24 |
| 10 | AAGCAGAAGACAGACCGGCTGC (SEQ ID NO: 5906) | TTACA (SEQ ID NO: 5916) | Gucy-2D_new | 0.0±0.21 |

MG22-1 target loci were chosen to test locations with the PAM nnRCnT. The spacers corresponding to the chosen target sites were cloned into the sgRNA scaffold in the mammalian vector system backbone 2 described above. The sites are listed below in Table 17.

**Table 17: MG22 target loci and target efficiency demonstrated in mammalian cells**

| **target ID** | **target sequence** | **PAM** | **locus** | **%NHEJ (mean ± std)** |
|---|---|---|---|---|
| 1 | GAAATAGGGGGTCCAGGAGCAA (SEQ ID NO: 5917) | TGACCT (SEQ ID NO: 5926) | VEGFA-T22 | -0.3±0.47 |
| 2 | ATTCCCTCTTTAGCCAGAGCCG (SEQ ID NO: 5918) | GGGCTT (SEQ ID NO: 5927) | VEGFA-T22 | 0.105±0.007 |
| 3 | GAAGCTGGGTGAATGGAGCGAG (SEQ ID NO: 5919) | CAGCGT (SEQ ID NO: 5928) | VEGFA-T22 | 32.6± 26.7 |
| 4 | CTGACACAACTGTGTTCACTAG (SEQ ID NO: 5920) | CAACCT (SEQ ID NO: 5929) | HBB-R01 | 5.59± 2.03 |
| 6 | GTATCAAGGTTACAAGACAGGT SEQ ID NO: 5921) | CAACCT (SEQ ID NO: 5930) | HBB-R01 | 0.65± 1.65 |
| 7 | GCCGTTACTGCCCTGTGGGGCA (SEQ ID NO: 5922) | AGACTT (SEQ ID NO: 5931) | HBB-R01 | -0.13±0.11 |
| 8 | GGCAGAGGGTGCATCACCTGGA (SEQ ID NO: 5923) | CTACAT (SEQ ID NO: 5932) | Apolipoprotein-T524 | 42.66 |
| 9 | GAATCTGCTATTTGAGCAAGGC (SEQ ID NO: 5924) | AAACAT (SEQ ID NO: 5933) | Apolipoprotein-T524 | 0.68 |
| 10 | ACTACCAAGCATCGGGCAGAGG (SEQ ID NO: 5925) | GTGCAT (SEQ ID NO: 5934) | Apolipoprotein-T524 | 21.59± 19.13 |

MG23-1 target loci were chosen to test locations with the PAM nRRA. The spacers corresponding to the chosen target sites were cloned into the sgRNA scaffold in the mammalian vector system backbone 2 described above. The sites are listed below in Table 18.

**Table 18: MG23 target loci and target efficiency demonstrated in mammalian cells**

| **target ID** | **target sequence** | **PAM** | **locus** | **%NHEJ (mean ± std)** |
|---|---|---|---|---|
| 1 | GGAGGTCAGAAATAGGGGGTCC (SEQ ID NO: 5935) | AGGA (SEQ ID NO: 5942) | VEGFA-T22 | 0.605±0.078 |
| 2 | CAAAGCCCATTCCCTCTTTAGC (SEQ ID NO: 5936) | CAGA (SEQ ID NO: 5943) | VEGFA-T22 | 19.1±8.5 |
| 3 | CGGCCACCACAGGGAAGCTGGG (SEQ ID NO: 5937) | TGAA (SEQ ID NO: 5944) | VEGFA-T22 | 8.3±5.4 |
| 5 | TGTGTTCACTAGCAACCTCAAA (SEQ ID NO: 5938) | CAGA (SEQ ID NO: 5945) | HBB-R01 | 0.37±0.82 |
| 7 | CCGTTACTGCCCTGTGGGGCAA (SEQ ID NO: 5939) | CAGA (SEQ ID NO: 5946) | HBB-R01 | 0.24±0.52 |
| 8 | AAGGAAACTGGAACACAAAGCA (SEQ ID NO: 5940) | TAGA (SEQ ID NO: 5947) | HEKsite2 | 21.64±11.14 |
| 9 | TCCAGCCCGCTGGCCCTGTAAA (SEQ ID NO: 5941) | GGAA (SEQ ID NO: 5948) | HEKsite2 | 34.1±17.6 |

### Example 22.- T cell editing using systems described herein

Having established that various MG systems function in mammalian cells, we sought to test their usefulness for editing human T cell genomes. Seeking to first use MG3-6 (protein sequence SEQ ID NO:421) and MG3-8 (protein sequence SEQ ID NO: 423) for this purpose, we determined that based on guide activity in human cells, the MG3-6 consensus PAM sequence was 5'-NNRGRYY-3' (SEQ ID NO: 5949). The PAM for MG3-8 was previously determined as 5'-NNRGGTT-3' (SEQ ID NO: 5534).

### TRAC locus targeting

To generate edited cells, appropriate spacer sequences targeting the TRAC locus in T-cells were designed for both MG3-6 (e.g. spacers represented in SEQ ID NOs: 5950-5958, referred to in experiments as MG3-6 guides 1-9 ) and MG3-8 (e.g. spacers represented in SEQ ID NOs: 5959-5965, referred to in experiments as "MG3-8 guide 1-7"). Spacer sequences were used in the background of the SEQ ID NO: 5466 sgRNA for MG3-6 and the SEQ ID NO: 6304 sgRNA for MG3-8 (which is listed below).

| **SEQ ID NO:** | **Nucleotide sequence (RNA)** |
|---|---|
| 6304 | (N22)GUUGAGAAUCUUUCGAAAGAAAGAUUCUUAAUAAGGCAUCCUUC CGAUGCUGACUUCUCACCGUCCGGCUCCUCUUAGGAACGGGCGGUAUG UUUU |
| * where (N22) denotes 22 N bases | |

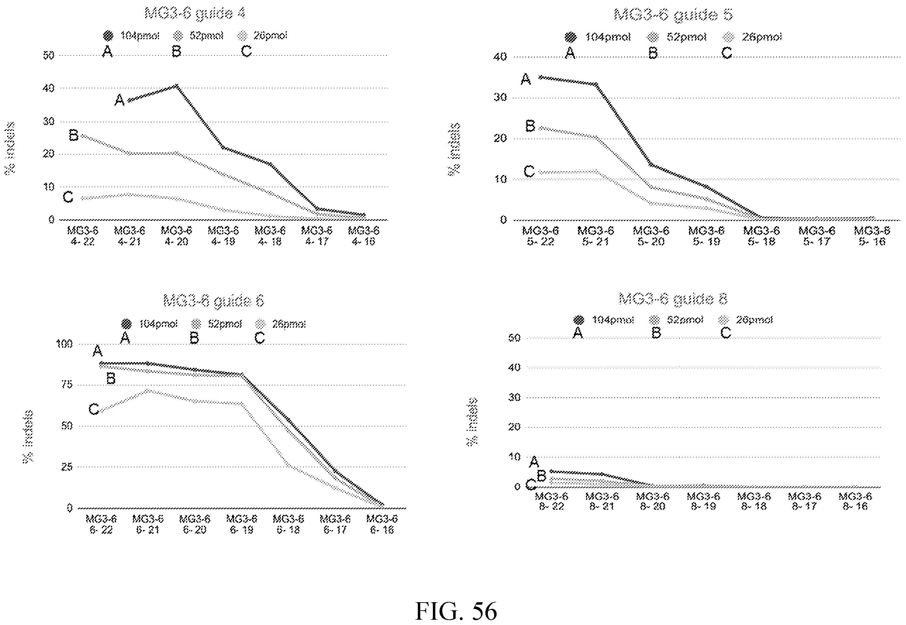

We first sought to determine the optimal length of spacer used to target the TRAC gene compatible with each endonuclease. For MG3-6, we nucleofected sgRNAs with a subset of the spacers above (SEQ ID NOs:5953-5957, referred to as "MG3-6 guide 4-8" in experiments) having lengths ranging from 22-16 nucleotides truncating from the 5' PAM-distal end when the sequence was to be shortened. These spacer-bearing guide RNAs were nucleofected into 200K primary T cells (that had previously been expanded with CD2/3/28 beads) per condition using a Lonza 4D electroporator and solution P3, delivering 26 or 52 or 104 pmol of MG3-6 protein with 32 or 64 or 128 pmol of guide RNAs, respectively. Genomic DNA from the T cells were harvested after 3 days and analyzed by NGS. The data are presented in FIGURE 56, which shows the effect of truncating MG3-6 guides 4-8 from 22-16 nucleotides, demonstrating that lengths from 19-22 nucleotides showed superior performance to shorter spacers for MG3-6.

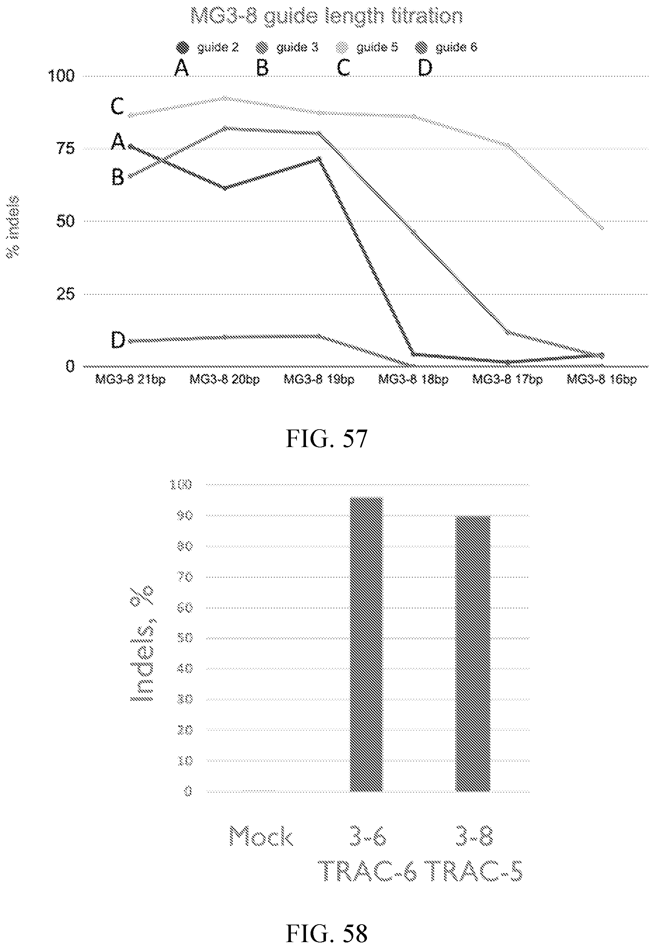

To perform the same spacer-length optimizing experiment with MG3-8, we nucleofected sgRNAs with a subset of the spacers above (SEQ ID NOs: 5960-5961 and 5963-5964, referred to as MG3-8 guides 2, 3, 5, and 8 in experiments) having lengths ranging from 22-16 nucleotides truncating from the 5' PAM-distal end when the sequence was to be shortened. These spacer-bearing guide RNAs were nucleofected into 200K primary T cells (that had previously been expanded with CD2/3/28 beads) per condition) using a Lonza 4D electroporator and solution P3, delivering 104 pmol of MG3-8 protein and 120 pmol of guide RNAs. Genomic DNA from the T cells was harvested after 3 days and analyzed by NGS. The data are presented in FIGURE 57, which shows the effect of truncating MG3-8 guides 2, 3, 5, and 8 from 22-16 nucleotides, demonstrating that lengths from 19-22 nucleotides showed superior performance to shorter spacers for MG3-8.

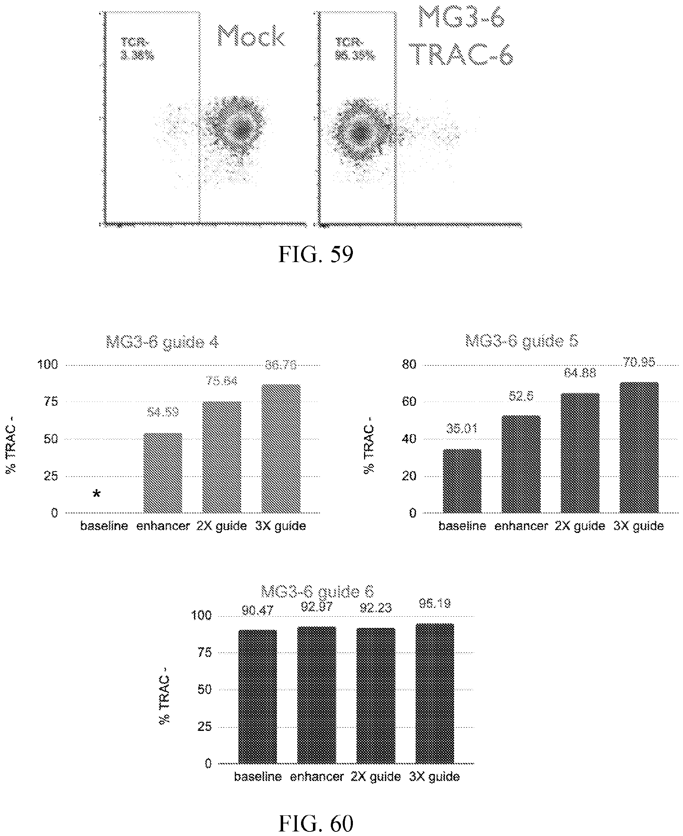

Having identified spacer sequences that appeared to target TRAC, we tested the ability of these spacers to induce indels in the TRAC gene and disrupt TRAC expression in cells. We nucleofected the best-performing 22-nucleotide-spacer-bearing sgRNAs (MG3-6 guide 6, SEQ ID NO: 5955; and MG3-8 guide 5, SEQ ID NO: 5963) into primary T cells as above using 104 pmol of MG3-6 protein and 128 pmol of guide RNAs. Genomic DNA was harvested after 3 days of recovery and analyzed by NGS for appearance of indels or by flow-cytometry using an anti-TCR-alpha chain Ab. The NGS indel analysis is shown in FIGURE 58, which demonstrates that both the MG3-6 and MG3-8 sgRNA/enzyme combinations generate approximately 90% or greater frequency of indels in the TRAC gene. The flow cytometry analysis is shown in in FIGURE 59, which demonstrates that the MG3-6 sgRNA/enzyme combination generates approximately 95% TCR negative cells.

Having observed that the MG3-6/guide 6 combination effectively generated TRAC knockout by flow cytometry, we tested whether the addition of transfection enhancer or higher guide concentrations could improve efficiency of knockout for the lower-performing MG3-6 TRAC spacer-bearing guides. Accordingly we transfected T cells as above, delivering 52 pmol of MG3-6 protein and 60 pmol guide RNA and 1 µL IDT transfection enhancer (if utilized) or 120 pmol guide RNA or 180 pmol guide RNA for each of MG3-6 guides 4 through 6 (spacer sequences SEQ ID NOs: 5953-5955 and were 22 nucleotides in length), and assaying by flow cytometry again using an anti-TCR-alpha chain Ab. The results are shown in FIGURE 60, which demonstrates that for guides 4 and 5, efficiency of TRAC knockout could be increased to -87% or -71% respectively by increasing the guide concentration.

### TRBC locus targeting

Having demonstrated TRAC locus targeting in T cells above, we next designed and screened reagents for targeting the TRBC locus. Accordingly, we again designed corresponding spacers for MG3-6 and MG3-8 as above (see Table 19 for MG3-6 and Table 20 for MG3-8), only directing them against sequences in the TRBC locus. As TRBC has two splice variants (TRBC1 and TRBC2), we designed spacers to target each. We nucleofected each 22-nt spacer bearing guide RNA into T cells alongside the enzyme as described above, and assessed the expression of T cell receptor using anti-TCR Ab. The TCR for each spacer-bearing guide are shown in Tables 19 and 20 below, alongside the % viability of the T cells at the time of flow cytometry. Tables 19 and 20 show that several spacers (5, 6, 18, and 19 for MG3-6, and 2, 6, 7 , and 8 for MG3-8) are moderately to highly effective in inducing TCR knockout in T cells.

**Table 19 - MG3-6 TRBC guide screen**

| **System No.** | **Spacer sequence** | **TRBC (1 or 2)** | **%Viability** | **% TCR-** |
|---|---|---|---|---|
| MG3-6 TRBC guide 1 | ACCAGCTCAGCTCCACGTGG (SEQ ID NO: 5966) | 2 | 86.86 | 7.8 |
| MG3-6 TRBC guide 2 | ACCTGCAGAAGAGAAAGTTT (SEQ ID NO: 5967) | 1 | 73.98 | 3.12 |
| MG3-6 TRBC guide 3 | AGGTCAAGAGAAAGGATTCC (SEQ ID NO: 5968) | 2 | 64.35 | 4.51 |
| MG3-6 TRBC guide 4 | AGGTCCTCTGGAAAGGGAAG (SEQ ID NO: 5969) | 1 | 75.39 | 10.89 |
| MG3-6 TRBC guide 5 | AGTATCTGGAGTCATTGAGG (SEQ ID NO: 5970) | 1 | 84.75 | 25.66 |
| MG3-6 TRBC guide 6 | AGTATCTGGAGTCATTGAGG (SEQ ID NO: 5971) | 2 | 82.68 | 28.72 |
| MG3-6 TRBC guide 7 | ATACAGGGTGGCCTTCCCTA (SEQ ID NO: 5972) | 1 | 83.9 | 4.2 |
| MG3-6 TRBC guide 8 | ATACTGCCTGAGCAGCCGCC (SEQ ID NO: 5973) | 1 | 83.91 | 3.37 |
| MG3-6 TRBC guide 9 | ATACTGCCTGAGCAGCCGCC (SEQ ID NO: 5974) | 2 | 82.22 | 4.21 |
| MG3-6 TRBC guide 10 | CAGATCGTCAGCGCCGAGGC (SEQ ID NO: 5975) | 1 | 84.52 | 11.18 |
| MG3-6 TRBC guide 11 | CAGATCGTCAGCGCCGAGGC (SEQ ID NO: 5976) | 2 | 85.73 | 10.38 |
| MG3-6 TRBC guide 12 | CAGGTCCTCTGGAAAGGGAA (SEQ ID NO: 5977) | 1 | 81.67 | 3.39 |
| MG3-6 TRBC guide 13 | CCACACTGGTGTGCCTGGCC (SEQ ID NO: 5978) | 1 | 86.46 | 9.13 |
| MG3-6 TRBC guide 14 | CCACACTGGTGTGCCTGGCC (SEQ ID NO: 5979) | 2 | 80.45 | 5.11 |
| MG3-6 TRBC guide 15 | CCGCTGTCAAGTCCAGTTCT (SEQ ID NO: 5980) | 1 | 83.96 | 3.89 |
| MG3-6 TRBC guide 16 | CCGCTGTCAAGTCCAGTTCT (SEQ ID NO: 5981) | 2 | 79.26 | 5.13 |
| MG3-6 TRBC guide 17 | CCTGCAGAAGAGAAAGTTTT (SEQ ID NO: 5982) | 1 | 81.71 | 4.79 |
| MG3-6 TRBC guide 18 | CGGCGCTGACGATCTGGGTG (SEQ ID NO: 5983) | 1 | 83.62 | 88.51 |
| MG3-6 TRBC guide 19 | CGGCGCTGACGATCTGGGTG (SEQ ID NO: 5984) | 2 | 86.75 | 89.05 |
| MG3-6 TRBC guide 20 | CTGTGGAAGAGAGAACATTT (SEQ ID NO: 5985) | 2 | 77.22 | 4.04 |
| MG3-6 TRBC guide 21 | CTTCCCTAGCAAGATCTCAT (SEQ ID NO: 5986) | 2 | 0 | 0 |
| MG3-6 TRBC guide 22 | CTTCCCTAGCAGGATCTCAT (SEQ ID NO: 5987) | 1 | 85.64 | 3.42 |
| MG3-6 TRBC guide 23 | CTTGACAGCGGAAGTGGTTG (SEQ ID NO: 5988) | 1 | 77.99 | 4.49 |
| MG3-6 TRBC guide 24 | CTTGACAGCGGAAGTGGTTG (SEQ ID NO: 5989) | 2 | 83.42 | 4.86 |
| MG3-6 TRBC guide 25 | GAATGGGAAGGAGGTGCACA (SEQ ID NO: 5990) | 1 | 81.42 | 8.25 |
| MG3-6 TRBC guide 26 | GAATGGGAAGGAGGTGCACA (SEQ ID NO: 5991) | 2 | 56.57 | 4.22 |
| MG3-6 TRBC guide 27 | GCCAACAGTGTCCTACCAGC (SEQ ID NO: 5992) | 1 | 83.36 | 3.51 |
| MG3-6 TRBC guide 28 | GCTGATGGCCATGGTAAGGA (SEQ ID NO: 5993) | 2 | 0 | 0 |
| MG3-6 TRBC guide 29 | GGCGCTGACCAGCACAGCAT (SEQ ID NO: 5994) | 1 | 71.58 | 3.89 |
| MG3-6 TRBC guide 30 | GTCAACAGAGTCTTACCAGC (SEQ ID NO: 5995) | 2 | 72.87 | 6.24 |
| MG3-6 TRBC guide 31 | TCGGAGAATGACGAGTGGAC (SEQ ID NO: 5996) | 1 | 84.3 | 8.5 |
| MG3-6 TRBC guide 32 | TCGGAGAATGACGAGTGGAC (SEQ ID NO: 5997) | 2 | 84.37 | 8.92 |
| MG3-6 TRBC guide 33 | TCTCTTCCACAGGTCAAGAG (SEQ ID NO: 5998) | 2 | 72.63 | 4.46 |
| MG3-6 TRBC guide 34 | TCTCTTCTGCAGGTCAAGAG (SEQ ID NO: 5999) | 1 | 81.88 | 3.55 |
| MG3-6 TRBC guide 35 | TGAGGGCGGGCTGCTCCTTG (SEQ ID NO: 6000) | 1 | 50.37 | 4.52 |
| MG3-6 TRBC guide 36 | TGAGGGCGGGCTGCTCCTTG (SEQ ID NO: 6001) | 2 | 78.44 | 5.76 |
| MG3-6 TRBC guide 37 | TGTGGAAGAGAGAACATTTT (SEQ ID NO: 6002) | 2 | 75.46 | 8.69 |
| MG3-6 TRBC guide 38 | TTGACAGCGGAAGTGGTTGC (SEQ ID NO: 6003) | 1 | 80.06 | 4.62 |
| MG3-6 TRBC guide 39 | TTGACAGCGGAAGTGGTTGC (SEQ ID NO: 6004) | 2 | 81.85 | 4.44 |

**Table 20 - MG3-8 TRBC guide screen**

| **System** | **Spacer sequence** | **TRBC (1 or 2)** | **% Viability** | **% TCR-** |
|---|---|---|---|---|
| MG3-8 TRBC Guide 1 | AATGGGAAGGAGGTGCACAG (SEQ ID NO: 6005) | 1 | 83.9 | 3.74 |
| MG3-8 TRBC Guide 2 | AATGGGAAGGAGGTGCACAG (SEQ ID NO: 6006) | 2 | 82.55 | 40.52 |
| MG3-8 TRBC Guide 3 | AGGTCCTCTGGAAAGGGAAG (SEQ ID NO: 6007) | 1 | 89.35 | 3.95 |
| MG3-8 TRBC Guide 4 | CCAACAGTGTCCTACCAGCA (SEQ ID NO: 6008) | 1 | 82.02 | 6.91 |
| MG3-8 TRBC Guide 5 | CCTGCAGAAGAGAAAGTTTT (SEQ ID NO: 6009) | 1 | 81.37 | 5.34 |
| MG3-8 TRBC Guide 6 | CGGCGCTGACGATCTGGGTG (SEQ ID NO: 6010) | 1 | 84.53 | 75.35 |
| MG3-8 TRBC Guide 7 | CGGCGCTGACGATCTGGGTG (SEQ ID NO: 6011) | 2 | 85.77 | 87.58 |
| MG3-8 TRBC Guide 8 | CTCGGGTGGGAACACCTTGT (SEQ ID NO: 6012) | 1 | 85.95 | 48.69 |
| MG3-8 TRBC Guide 9 | CTGAAAAACGTGTTCCCACC (SEQ ID NO: 6013) | 2 | 85.32 | 4.18 |
| MG3-8 TRBC Guide 10 | CTGAACAAGGTGTTCCCACC (SEQ ID NO: 6014) | 1 | 86.84 | 3.9 |
| MG3-8 TRBC Guide 11 | CTTGGGTGGGAACACGTTTT (SEQ ID NO: 6015) | 2 | 82.04 | 4.63 |
| MG3-8 TRBC Guide 12 | GGGCTGCTCCTTGAGGGGCT (SEQ ID NO: 6016) | 1 | 82.64 | 7.12 |
| MG3-8 TRBC Guide 13 | GGGCTGCTCCTTGAGGGGCT (SEQ ID NO: 6017) | 2 | 80.1 | 3.38 |
| MG3-8 TRBC Guide 14 | GTGACAGGTTTGGCCCTATC (SEQ ID NO: 6018) | 2 | 82.46 | 3.65 |
| MG3-8 TRBC Guide 15 | GTGACGGGTTTGGCCCTATC (SEQ ID NO: 6019) | 1 | 85.63 | 3.7 |
| MG3-8 TRBC Guide 16 | TACTGCCTGAGCAGCCGCCT (SEQ ID NO: 6020) | 1 | 83.6 | 4.24 |
| MG3-8 TRBC Guide 17 | TACTGCCTGAGCAGCCGCCT (SEQ ID NO: 6021) | 2 | 82.5 | 10.84 |
| MG3-8 TRBC Guide 18 | TCAACAGAGTCTTACCAGCA (SEQ ID NO: 6022) | 2 | 82.09 | 6.91 |
| MG3-8 TRBC Guide 19 | TGTGGAAGAGAGAACATTTT (SEQ ID NO: 6023) | 2 | 82.55 | 6.95 |
| MG3-8 TRBC Guide 20 | TTGACAGCGGAAGTGGTTGC (SEQ ID NO: 6024) | 1 | 74.67 | 4.26 |
| MG3-8 TRBC Guide 21 | TTGACAGCGGAAGTGGTTGC (SEQ ID NO: 6025) | 2 | 70.69 | 3.83 |

### TCR ablation alongside CAR-T expression

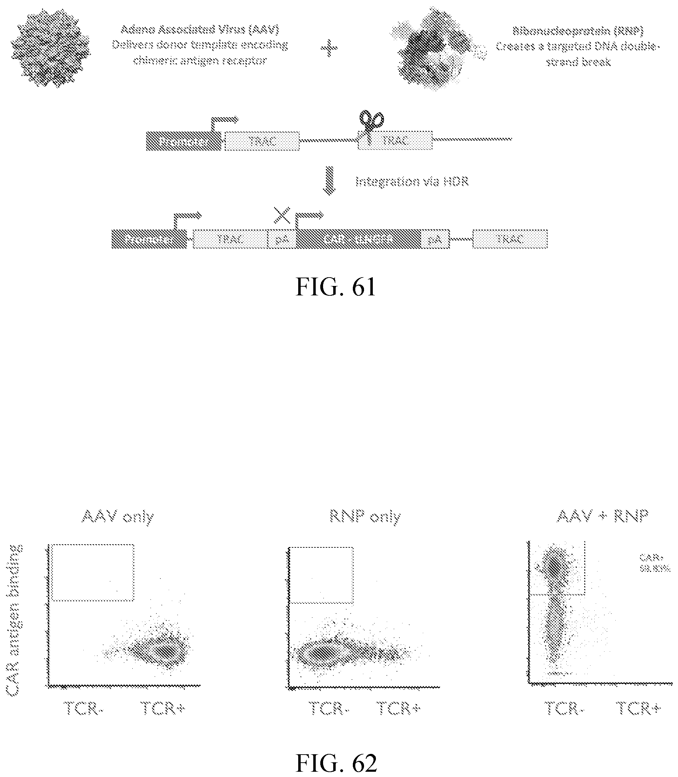

Having demonstrated that we could effectively knock out TCR expression in T-cells using our endonuclease/guide combinations, we next asked if we could generate allogeneic CAR-T cells by knocking out TCR and expression a heterologous CAR (chimeric antigen receptor) in the same cells. The scheme we used for this experiment is depicted in FIGURE 61, which shows the proposed targeting of the TCR locus followed by integration of a CAR at the same locus by homologous recombination..

Accordingly, we nucleofected T cells as described above using the high performing MG3-6 enzyme with TRAC-targeting MG3-6 guide 6, SEQ ID NO: 5955, only infecting the same T cells with AAV #3029 bearing a heterologous CAR sequence with TRAC homology arms at an MOI of 300K. After observing the transfected cells alongside controls using flow cytometry against the TCR receptor and the heterologous CAR antigen, we observed that the MG3-6 transfection condition could generate CAR+/TCR- cells at an approximate frequency of -60%.

### GR (Glucocorticoid Receptor) Locus Targeting

Having demonstrated that we could effectively knock out TCR expression in T-cells using our endonuclease/guide combinations, we next asked if we could target the GR (Glucocorticoid receptor) locus to modulate other characteristics of T cells (e.g. response to glucocorticoids). We again designed appropriate spacers for MG3-6 and MG3-8, but this time targeted against sequences in the GR locus (see Table 21 below and Table 22 below).

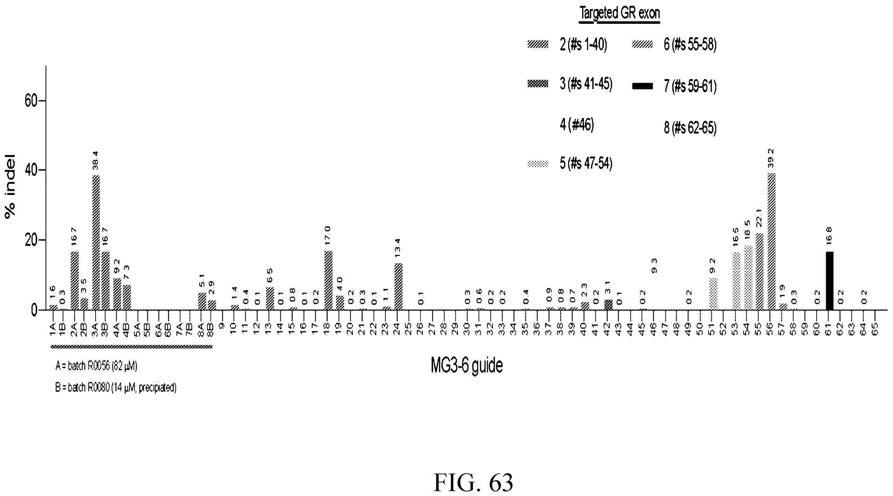

For the MG3-6 targeting sequences (Table 21), sequences 1-40 were designed to target GR exon 2, sequences 41-45 were designed to target GR exon 3, sequence 46 was designed to target GR exon 4, sequences 47-54 were designed to target GR exon 5, sequences55-58 were designed to target GR exon 6, sequences 59-61 were designed to target GR exon 7, and sequences 62-65 were designed to target GR exon 8. Sequences were screened by nucleofection into primary T cells above using 126 pmol MG3-6 protein and 160 pmol guide, analyzing as before by NGS. The results of screening are depicted in FIGURE 63, which depicts % indels generated by the numbered guides in Table 21. The results indicated that several spacer sequences (2, 3, 4, 13, 18, 24, 51, 55, 56, and 61 in Table 21 below) were moderately effective at generating indels in the GR gene using MG3-6..

**Table 21 - MG3-6 GR guides**

| **Number** | **System** | **Name** | **DNA sequence of spacer** |
|---|---|---|---|
| 1 | MG3-6 | A1 | CTCAGGAGAGGGGAGATGTG (SEQ ID NO: 6026) |
| 2 | MG3-6 | B1 | CTCCTGAGCAAGCACACTGC (SEQ ID NO: 6027) |
| 3 | MG3-6 | C1 | CTAAGAGGAGGAGCTACTGT (SEQ ID NO: 6028) |
| 4 | MG3-6 | D1 | TTCACAGTAGCTCCTCCTCT (SEQ ID NO: 6029) |
| 5 | MG3-6 | E1 | CACTGGCTGTCGCTTCTCAA (SEQ ID NO: 6030) |
| 6 | MG3-6 | F1 | TCAATCAGACTCCAAGCAGC (SEQ ID NO: 6031) |
| 7 | MG3-6 | G1 | GACTTTTGGTTGATTTTCCA (SEQ ID NO: 6032) |
| 8 | MG3-6 | H1 | CAGTAAGCAATGCGCAGCAG (SEQ ID NO: 6033) |
| 9 | MG3-6 | A2 | CAAAGCAGTTTCACTCTCAA (SEQ ID NO: 6034) |
| 10 | MG3-6 | B2 | TGAGAGTGAAACTGCTTTGG (SEQ ID NO: 6035) |
| 11 | MG3-6 | C2 | AAAAGTGATGGGAAATGACC (SEQ ID NO: 6036) |
| 12 | MG3-6 | D2 | ATGACCTGGGATTCCCACAG (SEQ ID NO: 6037) |
| 13 | MG3-6 | E2 | TTGGCCCTGCTGTGGGAATC (SEQ ID NO: 6038) |
| 14 | MG3-6 | F2 | TCAGCCTTTCCTCGGGGGAA (SEQ ID NO: 6039) |
| 15 | MG3-6 | G2 | TAAGTCTGTTTCCCCCGAGG (SEQ ID NO: 6040) |
| 16 | MG3-6 | H2 | AGAAAGCATTGCAAACCTCA (SEQ ID NO: 6041) |
| 17 | MG3-6 | A3 | TGGAACACTGGTCGACCTAT (SEQ ID NO: 6042) |
| 18 | MG3-6 | B3 | AGCAGTGGATGCTGAACTCT (SEQ ID NO: 6043) |
| 19 | MG3-6 | C3 | CTTCAGAACAGCAACATTTG (SEQ ID NO: 6044) |
| 20 | MG3-6 | D3 | GAACAGCAACATTTGAAGGG (SEQ ID NO: 6045) |
| 21 | MG3-6 | E3 | GCAATGTGAAATTGTATACC (SEQ ID NO: 6046) |
| 22 | MG3-6 | F3 | AAAGCACCTTTGACATTTTG (SEQ ID NO: 6047) |
| 23 | MG3-6 | G3 | GCAGGATTTGGAGTTTTCTT (SEQ ID NO: 6048) |
| 24 | MG3-6 | H3 | AGAGACGAATGAGAGTCCTT (SEQ ID NO: 6049) |
| 25 | MG3-6 | A4 | TCTCATTCGTCTCTTTACCT (SEQ ID NO: 6050) |
| 26 | MG3-6 | B4 | TATCAACAGGTCTGATCTCC (SEQ ID NO: 6051) |
| 27 | MG3-6 | C4 | CAAACAGTTTTCATCTATCA (SEQ ID NO: 6052) |
| 28 | MG3-6 | D4 | TTTTGGAAGGAAACTCGAAT (SEQ ID NO: 6053) |
| 29 | MG3-6 | E4 | AACCCAAAATTAAGGATAAT (SEQ ID NO: 6054) |
| 30 | MG3-6 | F4 | GTTTAGTGTCCGGTAAAATG (SEQ ID NO: 6055) |
| 31 | MG3-6 | G4 | GATCTCCATTATCCTTAATT (SEQ ID NO: 6056) |
| 32 | MG3-6 | H4 | ATTACTGGGGCTTGACAAAA (SEQ ID NO: 6057) |
| 33 | MG3-6 | A5 | CCCAAGTGAAAACAGAAAAA (SEQ ID NO: 6058) |
| 34 | MG3-6 | B5 | TTGGGGCAGTGTTACATTAC (SEQ ID NO: 6059) |
| 35 | MG3-6 | C5 | CAGCATCCCTTTCTCAACAG (SEQ ID NO: 6060) |
| 36 | MG3-6 | D5 | TTGGTGGAATGACATTAAAA (SEQ ID NO: 6061) |
| 37 | MG3-6 | E5 | CGAAAATTGGAATAGGTGCC (SEQ ID NO: 6062) |
| 38 | MG3-6 | F5 | GATGACAACTTGACTTCTCT (SEQ ID NO: 6063) |
| 39 | MG3-6 | G5 | GAGAAGTCAAGTTGTCATCT (SEQ ID NO: 6064) |
| 40 | MG3-6 | H5 | TTTCTAATGGCTATTCAAGG (SEQ ID NO: 6065) |
| 41 | MG3-6 | A6 | CCCCTTCTTTAGCCCCAGCA (SEQ ID NO: 6066) |
| 42 | MG3-6 | B6 | GCTTACATCTGGTCTCATGC (SEQ ID NO: 6067) |
| 43 | MG3-6 | C6 | CAGCTCCTCAACAGCAACAA (SEQ ID NO: 6068) |
| 44 | MG3-6 | D6 | ACTTTACAGCTTCCACAAGT (SEQ ID NO: 6069) |
| 45 | MG3-6 | E6 | TGCCGCTATCGAAAATGTCT (SEQ ID NO: 6070) |
| 46 | MG3-6 | F6 | TCAGATATTTATATTACCTT (SEQ ID NO: 6071) |
| 47 | MG3-6 | G6 | AAAAAAATAAAAGGAATTCA (SEQ ID NO: 6072) |
| 48 | MG3-6 | H6 | GGATTTTCAGAGGTTTCTTG (SEQ ID NO: 6073) |
| 49 | MG3-6 | A7 | GTTTTGTTACCAGGATTTTC (SEQ ID NO: 6074) |
| 50 | MG3-6 | B7 | AGGAACTATTGTTTTGTTAC (SEQ ID NO: 6075) |
| 51 | MG3-6 | C7 | CCTACCCTGGTGTCACTGTT (SEQ ID NO: 6076) |
| 52 | MG3-6 | D7 | CAGGATATGATAGCTCTGTT (SEQ ID NO: 6077) |
| 53 | MG3-6 | E7 | CATATCCTGCATATAACACT (SEQ ID NO: 6078) |
| 54 | MG3-6 | F7 | TCTGTTCCAGACTCAACTTG (SEQ ID NO: 6079) |
| 55 | MG3-6 | G7 | AACATCCAGGAGTACTGCAG (SEQ ID NO: 6080) |
| 56 | MG3-6 | H7 | TATGGCATTTGCTCTGGGGT (SEQ ID NO: 6081) |
| 57 | MG3-6 | A8 | CAGATCAGGAGCAAAACACA (SEQ ID NO: 6082) |
| 58 | MG3-6 | B8 | TACAACTTACTCATTAATAA (SEQ ID NO: 6083) |
| 59 | MG3-6 | C8 | GTATGTTTCCTCTGAGTTAC (SEQ ID NO: 6084) |
| 60 | MG3-6 | D8 | AAACCTTACTGCTTCTCTCT (SEQ ID NO: 6085) |
| 61 | MG3-6 | E8 | CAACCTGAAGAGAGAAGCAG (SEQ ID NO: 6086) |
| 62 | MG3-6 | F8 | CAGACCGTCCTTAGGAACTA (SEQ ID NO: 6087) |
| 63 | MG3-6 | G8 | ATCAAATAGCTCTTGGCTCT (SEQ ID NO: 6088) |
| 64 | MG3-6 | H8 | GCTTTTCCTAGCTCTTTGAT (SEQ ID NO: 6089) |
| 65 | MG3-6 | A9 | TTTATCAACTGACAAAACTC (SEQ ID NO: 6090) |

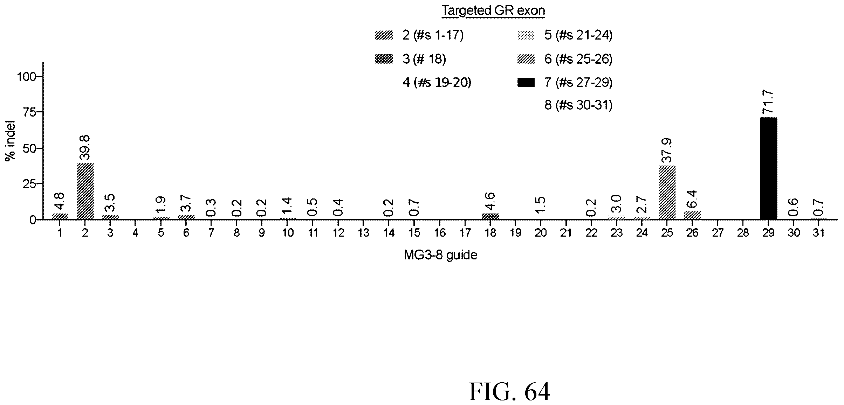

For the MG3-8 targeting sequences (Table 22), sequences 1-17 were designed to target GR exon 2, sequence 18 was designed to target GR exon 3, sequences 19-20 were designed to target GR exon 4, sequences 21-24 were designed to target GR exon 5, sequences 25-26 were designed to target GR exon 6, sequences 27-29 were designed to target GR exon 7, and sequences 30-31 were designed to target GR exon 8. Sequences were screened by nucleofection into primary T cells as above using 52 pmol MG3-8 protein and 60 pmol guide, analyzing as before by NGS. The results of screening are depicted in FIGURE 64, which depicts % indels generated by the numbered guides in Table 22. The results indicated that some spacer sequences (2, 25, and 29 in Table 22 below) were moderately effective at generating indels in the GR gene using MG3-8.

**Table 22 - GR (NR3Cl) guide screen for MG3-8 guides**

| **Number** | **System** | **Name** | **DNA sequence of spacer** |
|---|---|---|---|
| 1 | MG3-8 | B9 | CTCCTGAGCAAGCACACTGC (SEQ ID NO: 6091) |
| 2 | MG3-8 | C9 | CTAAGAGGAGGAGCTACTGT (SEQ ID NO: 6092) |
| 3 | MG3-8 | D9 | TTCACAGTAGCTCCTCCTCT (SEQ ID NO: 6093) |
| 4 | MG3-8 | E9 | GACTTTTGGTTGATTTTCCA (SEQ ID NO: 6094) |
| 5 | MG3-8 | F9 | ATGACCTGGGATTCCCACAG (SEQ ID NO: 6095) |
| 6 | MG3-8 | G9 | TTGGCCCTGCTGTGGGAATC (SEQ ID NO: 6096) |
| 7 | MG3-8 | H9 | TAAGTCTGTTTCCCCCGAGG (SEQ ID NO: 6097) |
| 8 | MG3-8 | A10 | AGAAAGCATTGCAAACCTCA (SEQ ID NO: 6098) |
| 9 | MG3-8 | B10 | TGGAACACTGGTCGACCTAT (SEQ ID NO: 6099) |
| 10 | MG3-8 | C10 | AGCAGTGGATGCTGAACTCT (SEQ ID NO: 6100) |
| 11 | MG3-8 | D10 | CTTCAGAACAGCAACATTTG (SEQ ID NO: 6101) |
| 12 | MG3-8 | E10 | GCAGGATTTGGAGTTTTCTT (SEQ ID NO: 6102) |
| 13 | MG3-8 | F10 | CAAACAGTTTTCATCTATCA (SEQ ID NO: 6103) |
| 14 | MG3-8 | G10 | GTTTAGTGTCCGGTAAAATG (SEQ ID NO: 6104) |
| 15 | MG3-8 | H10 | GATCTCCATTATCCTTAATT (SEQ ID NO: 6105) |
| 16 | MG3-8 | A11 | TTGGGGCAGTGTTACATTAC (SEQ ID NO: 6106) |
| 17 | MG3-8 | B11 | TTGGTGGAATGACATTAAAA (SEQ ID NO: 6107) |
| 18 | MG3-8 | C11 | GCTTACATCTGGTCTCATGC (SEQ ID NO: 6108) |
| 19 | MG3-8 | D11 | TGCCGCTATCGAAAATGTCT (SEQ ID NO: 6109) |
| 20 | MG3-8 | E11 | TCAGATATTTATATTACCTT (SEQ ID NO: 6110) |
| 21 | MG3-8 | F11 | AAAAAAATAAAAGGAATTCA (SEQ ID NO: 6111) |
| 22 | MG3-8 | G11 | GTTTTGTTACCAGGATTTTC (SEQ ID NO: 6112) |
| 23 | MG3-8 | H11 | CCTACCCTGGTGTCACTGTT (SEQ ID NO: 6113) |
| 24 | MG3-8 | A12 | CATATCCTGCATATAACACT (SEQ ID NO: 6114) |
| 25 | MG3-8 | B12 | AACATCCAGGAGTACTGCAG (SEQ ID NO: 6115) |
| 26 | MG3-8 | C12 | CAGATCAGGAGCAAAACACA (SEQ ID NO: 6116) |
| 27 | MG3-8 | D12 | GTATGTTTCCTCTGAGTTAC (SEQ ID NO: 6117) |
| 28 | MG3-8 | E12 | AAACCTTACTGCTTCTCTCT (SEQ ID NO: 6118) |
| 29 | MG3-8 | F12 | CAACCTGAAGAGAGAAGCAG (SEQ ID NO: 6119) |
| 30 | MG3-8 | G12 | CAGACCGTCCTTAGGAACTA (SEQ ID NO: 6120) |
| 31 | MG3-8 | H12 | GCTTTTCCTAGCTCTTTGAT (SEQ ID NO: 6121) |

| | | | |
|---|---|---|---|
| *AAVS1 safe-harbor Locus Targeting* | | | |

Having demonstrated that we could effectively knock out TCR expression in T-cells using our endonuclease/guide combinations, we next asked if we could target the AAVS 1 safe-harbor locus in T cells. We designed appropriate spacers for MG3-6, but this time targeted against sequences in the AAVS1 locus (see Table 23 below). Sequences were screened by nucleofection into primary T cells as above using 126 pmol MG3-6 protein and 160 pmol guide and analyzed by NGS for indel formation in the AAVS1 locus. Table 23 shows the percentage of indels generated alongside each AAVS 1-targeting spacer sequence in the transfected T cells, demonstrating that several sequences (A1, D1, E1, G1, B2, D2, G2, D3, F3, and C4) generate indels with moderate to high frequency in the AAVS1 locus with MG3-6.

**Table 23 - AAVS1 guide screen with MG3-6**

| System | Name | DNA sequence of spacer | Indels |
|---|---|---|---|
| MG3-6 | A1 | CCTCACCACAGCCCTGCCAGGA (SEQ ID NO: 6122) | 54.84 |
| MG3-6 | B1 | GAAGTGTAAGGAAGCTGCAGCA (SEQ ID NO: 6123) | 0.08 |
| MG3-6 | C1 | CAGACGGCCGCGTCAGAGCAGC (SEQ ID NO: 6124) | 20.19 |
| MG3-6 | D1 | TCAGAGCAGCTCAGGTTCTGGG (SEQ ID NO: 6125) | 61.3 |
| MG3-6 | E1 | TCAGGTTCTGGGAGAGGGTAGC (SEQ ID NO: 6126) | 32.56 |
| MG3-6 | F1 | ACCCCCTGCCAAGCTCTCCCTC (SEQ ID NO: 6127) | N/A |
| MG3-6 | G1 | GGCTCCATCGTAAGCAAACCTT (SEQ ID NO: 6128) | 100 |
| MG3-6 | H1 | GGAGAGAGATGGCTCCAGGAAA (SEQ ID NO: 6129) | 0 |
| MG3-6 | A2 | AAGGAATCTGCCTAACAGGAGG (SEQ ID NO: 6130) | 21.8 |
| MG3-6 | B2 | AGGAATCTGCCTAACAGGAGGT (SEQ ID NO: 6131) | 87.48 |
| MG3-6 | C2 | GGAGACTAGGAAGGAGGAGGCC (SEQ ID NO: 6132) | 0.16 |
| MG3-6 | D2 | TAGGAAGGAGGAGGCCTAAGGA (SEQ ID NO: 6133) | 75.26 |
| MG3-6 | E2 | TCCTGTCCCTAGTGGCCCCACT (SEQ ID NO: 6134) | 5.1 |
| MG3-6 | F2 | AAGGTGGTCCCAGCTCGGGGAC (SEQ ID NO: 6135) | 0.04 |
| MG3-6 | G2 | GTCCTGAAGTGGACATAGGGGC (SEQ ID NO: 6136) | 78.2 |
| MG3-6 | H2 | ACCCCTGGAAGATGCCATGACA (SEQ ID NO: 6137) | 2.37 |
| MG3-6 | A3 | CTAGCACAGACTAGAGAGGTAA (SEQ ID NO: 6138) | 19.22 |
| MG3-6 | B3 | GGTGACCCGAATCCACAGGAGA (SEQ ID NO: 6139) | 0.9 |
| MG3-6 | C3 | GGGTGTCCAGGCAAAGAAAGCA (SEQ ID NO: 6140) | 0.14 |
| MG3-6 | D3 | AGAGGTGGCTAAAGCCAGGGAG (SEQ ID NO: 6141) | 67.24 |
| MG3-6 | E3 | AAAGCCAGGGAGACGGGGTACT (SEQ ID NO: 6142) | 1.14 |
| MG3-6 | F3 | AAGCCAGGGAGACGGGGTACTT (SEQ ID NO: 6143) | 67.85 |
| MG3-6 | G3 | GGCCTGTGCCATCTCTCGTTTC (SEQ ID NO: 6144) | 0.81 |
| MG3-6 | H3 | AGCCCCCTGTCATGGCATCTTC (SEQ ID NO: 6145) | 0.37 |
| MG3-6 | A4 | AGGACAGCATGTTTGCTGCCTC (SEQ ID NO: 6146) | 0.05 |
| MG3-6 | B4 | CCCCACAGTGGGGCCACTAGGG (SEQ ID NO: 6147) | 2.32 |
| MG3-6 | C4 | TCTCTCTCCTTGCCAGAACCTC (SEQ ID NO: 6148) | 82.66 |
| MG3-6 | D4 | GGTTTGCTTACGATGGAGCCAG (SEQ ID NO: 6149) | 0.57 |
| MG3-6 | E4 | ATCCTGGGAGGGAGAGCTTGGC (SEQ ID NO: 6150) | N/A |
| MG3-6 | F4 | TGGCAGGGGGTGGGAGGGAAGG (SEQ ID NO: 6151) | 0 |
| MG3-6 | G4 | CCAGTAGCCAGCCCCGTCCTGG (SEQ ID NO: 6152) | N/A |

### TIGIT locus targeting

Having demonstrated that we could effectively knock out TCR expression in T-cells using our endonuclease/guide combinations, we next asked if we could target the TIGIT locus in T cells. We designed appropriate spacers for MG3-6, but this time targeted against sequences in the TIGIT locus (see Table 24 below). Sequences were screened by nucleofection into primary T cells as above using 126 pmol MG3-6 protein and 160 pmol guide and analyzed by NGS as above. Table 24 shows the percentage of indels generated alongside each TIGIT-targeting spacer sequence in the transfected T cells, demonstrating that several sequences (C1, G1, H1, D3) have high indel-generating activity with MG3-6. Genomic DNA was harvested after 3 days and analyzed by NGS (see table 24 below).

**Table 24 - TIGIT guide screen with MG3-6**

| System | Name | DNA sequence of spacer | % indels |
|---|---|---|---|
| MG3-6 | A1 | TGCGCTGGTGTCTCCTCCTGAT (SEQ ID NO: 6153) | 0 |
| MG3-6 | B1 | TGTCTCCTCCTGATCTGGGCCC (SEQ ID NO: 6154) | 17 |
| MG3-6 | C1 | TGATCTGGGCCCAGGGGCTGAG (SEQ ID NO: 6155) | 81 |
| MG3-6 | D1 | GGCAGGCTCCCCTCGCCTCAGG (SEQ ID NO: 6156) | 30 |
| MG3-6 | E1 | CGAGGGGAGCCTGCCTCAGCCC (SEQ ID NO: 6157) | 18 |
| MG3-6 | F1 | GAGCCTGCCTCAGCCCCTGGGC (SEQ ID NO: 6158) | 7 |
| MG3-6 | G1 | CCTCCACCACGGCACAAGTGAC (SEQ ID NO: 6159) | 75 |
| MG3-6 | H1 | GACCCAGGTCAACTGGGAGCAG (SEQ ID NO: 6160) | 73 |
| MG3-6 | A2 | GTGGCACATCTCCCCATCCTTC (SEQ ID NO: 6161) | 3 |
| MG3-6 | B2 | ATCCTTCAAGGATCGAGTGGCC (SEQ ID NO: 6162) | 4 |
| MG3-6 | C2 | TCGAGTGGCCCCAGGTCCCGGC (SEQ ID NO: 6163) | 1 |
| MG3-6 | D2 | CGTACACTGGGAGAATCTTCCT (SEQ ID NO: 6164) | 0 |
| MG3-6 | E2 | CAGCAGGAATACCTGAGCTTTC (SEQ ID NO: 6165) | 0 |
| MG3-6 | F2 | CTGAGCTTTCTAGGACCTCCAG (SEQ ID NO: 6166) | 0 |
| MG3-6 | G2 | TTCACGGTCAGCGACTGGAGGG (SEQ ID NO: 6167) | 3 |
| MG3-6 | H2 | GTCAGCGACTGGAGGGTGAGGC (SEQ ID NO: 6168) | 1 |
| MG3-6 | A3 | GACTGGAGGGTGAGGCCCAGGC (SEQ ID NO: 6169) | 0 |
| MG3-6 | B3 | GGGTGAGGCCCAGGCCGGGACC (SEQ ID NO: 6170) | 0 |
| MG3-6 | C3 | GGTCCTGCTGCTCCCAGTTGAC (SEQ ID NO: 6171) | 0 |
| MG3-6 | D3 | CCTCTAGTGGCTGAGCACGGTG (SEQ ID NO: 6172) | 79 |
| MG3-6 | E3 | TGGCTGAGCACGGTGCCAGGTT (SEQ ID NO: 6173) | 42 |
| MG3-6 | F3 | GTCCAGGCAGAAGCTGCACCTG (SEQ ID NO: 6174) (data not available) | |
| MG3-6 | G3 | GCTCTGTGGAGAGCAGCGGGGA (SEQ ID NO: 6175) (data not available) | |
| MG3-6 | H3 | TGGGTAACTGCAGCTTCTTCAC (SEQ ID NO: 6176) | 0 |
| MG3-6 | A4 | TCTGTGAAGAAGCTGCAGTTAC (SEQ ID NO: 6177) | 0 |
| MG3-6 | B4 | CTTCCTGGGGGTGAGGGAGCAC (SEQ ID NO: 6178) | 16 |
| MG3-6 | C4 | TCCTGTCCAGCTGATTTTCTCC (SEQ ID NO: 6179) | 6 |
| MG3-6 | D4 | TCCTGAGGTCACCTTCCACAGA (SEQ ID NO: 6180) | 10 |
| MG3-6 | E4 | CACCTTCCACAGAATGGATTCT (SEQ ID NO: 6181) | 10 |

### Example 23.- NK cell editing using systems described herein

### TCR ablation combined with CAR expression

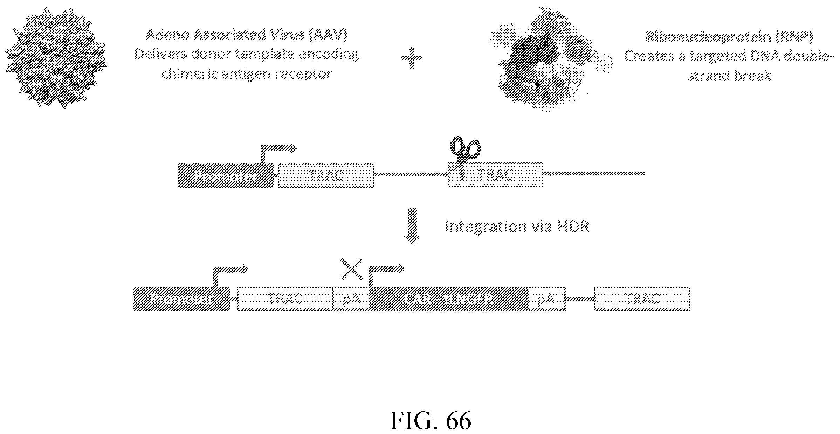

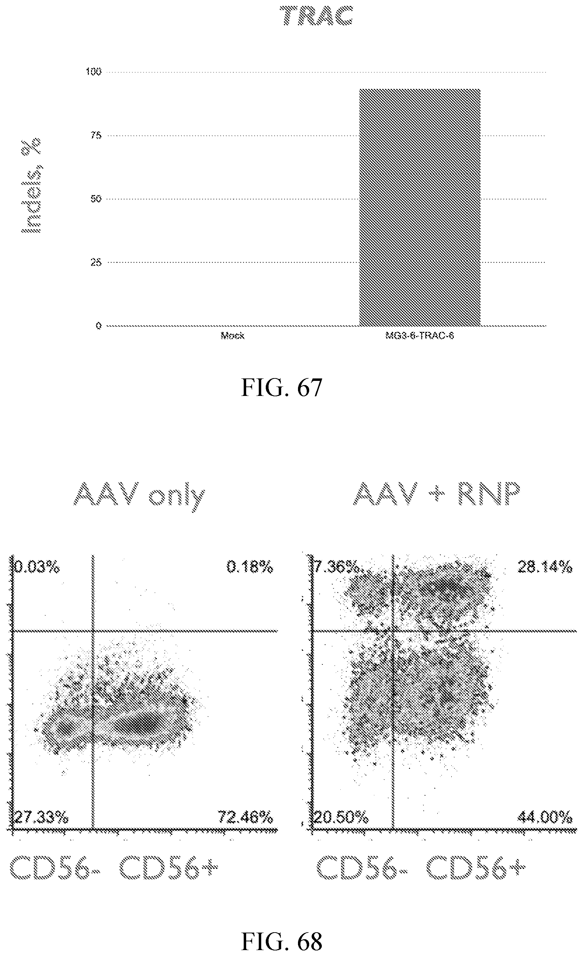

Having observed we could efficiently knock out TCR expression in T cells, we next asked if we could edit TRAC in NK cells, for example disrupting TCR alongside introduction of a CAR with TRAC homology arms to create an allogeneic CAR-NK cell (see FIG. 66 for an example scheme). Accordingly, we cultured NK cells using the Cloudz Human NK cell Expansion Kit. NK cells were transfected with the Lonza 4D system using the mannitol-containing buffer in Rautela et al. (see https://doi.org/10.1101/406934, which is incorporated by reference herein) with 104 pmol MG3-6 protein and 180 pmol guide RNA (using the high performing MG3-6 enzyme with TRAC-targeting MG3-6 guide 6, SEQ ID NO: 5955), followed by infection using 300K MOI with AAV #3029 bearing a CAR sequence with TRAC homology arms. Genomic DNA was harvested from the cells after 5 days recovery and analyzed by NGS. Simultaneously, CAR expression assayed by flow cytometry using biotinylated BCMA protein in parallel with an antibody to the NK cell marker CD56. The results are presented in FIGURE 67 (which shows TRAC indel formation) and FIGURE 68 (which shows flow cytometry for CD56 expression on the x-axis and CAR expression on the y-axis). The results demonstrate that the MG3-6/guide RNA combination alongside CAR expression was effective for generating CAR-positive NK cells.

### CD38 targeting

A CD38 guide screen was then conducted in primary NK cells using spacer sequences designed for MG3-6 (Table 24) and MG3-8 (Table 25) to target the CD38 gene in NK cells. Results are presented alongside the sequences in Tables 24 and 25, demonstrating that several sequences (A1, B1, H1, B2, C4, E4, F4, B5, D5, for MG3-6 and C1 for MG3-8) have moderate to high activity for generating indels in the CD38 locus when introduced to cells alongside their respective endonucleases.

**Table 24 - CD38 guide screen using MG3-6**

| System | Name | DNA sequence of spacer | Indels |
|---|---|---|---|
| MG3-6 | A1 | CTGAAATTCATCCTGAGATGAG (SEQ ID NO: 6182) | 54.32 |
| MG3-6 | B1 | TTAGTCGCCAACCCACCTCATC (SEQ ID NO: 6183) | 95.45 |
| MG3-6 | C1 | ACGACCACCGCGAGCACCACGA (SEQ ID NO: 6184) | 0.4 |
| MG3-6 | D1 | AGCACCACGACGAGGATCAGGA (SEQ ID NO: 6185) | 6.79 |
| MG3-6 | E1 | GGATACTGACGCCAAGACAGAG (SEQ ID NO: 6186) | 14.35 |
| MG3-6 | F1 | GGGCTCTCCTAGAGAGCCGGCA (SEQ ID NO: 6187) | 0.16 |
| MG3-6 | G1 | GGCTCTCCTAGAGAGCCGGCAG (SEQ ID NO: 6188) | 31.35 |
| MG3-6 | H1 | CAGCAGGGTTTGTCCCCGGACA (SEQ ID NO: 6189) | 53.03 |
| MG3-6 | A2 | GGGCTGAACTCGCAGTTGGCCA (SEQ ID NO: 6190) | 0.62 |
| MG3-6 | B2 | GCAGTTGGCCATAGGGCTCCAG (SEQ ID NO: 6191) | 41.92 |
| MG3-6 | C2 | CAGTTGGCCATAGGGCTCCAGG (SEQ ID NO: 6192) | 70 |
| MG3-6 | D2 | GACATGTAGACTGCCAAAGTGT (SEQ ID NO: 6193) | 0.19 |
| MG3-6 | E2 | GCCAAAGTGTATGGGATGCTTT (SEQ ID NO: 6194) | 0.02 |
| MG3-6 | F2 | ATAGTCTTCTTCAGTAATGTTG (SEQ ID NO: 6195) | 0.2 |
| MG3-6 | G2 | GTTCACCCTGGAGGACACGCTG (SEQ ID NO: 6196) | 9.82 |
| MG3-6 | H2 | GTGGTGAATTCAACACTTCCAG (SEQ ID NO: 6197) | 0.21 |
| MG3-6 | A3 | AGGTAGCCTAGCAGCGTGTCCT (SEQ ID NO: 6198) | 0.04 |
| MG3-6 | B3 | GTATTCTGGAAAACGGTTTCCC (SEQ ID NO: 6199) | 0.17 |
| MG3-6 | C3 | AACCGTTTTCCAGAATACTGAA (SEQ ID NO: 6200) | 0.01 |
| MG3-6 | D3 | ACCGTTTTCCAGAATACTGAAA (SEQ ID NO: 6201) | 0.11 |
| MG3-6 | E3 | TGTTTTTGTCAAAGATTTTACT (SEQ ID NO: 6202) | 0.16 |
| MG3-6 | F3 | AAGTCCATAATTTGCAACCAGA (SEQ ID NO: 6203) | 0.17 |
| MG3-6 | G3 | CTTTCTTCCCCAGAGACTTATG (SEQ ID NO: 6204) | 0.12 |
| MG3-6 | H3 | AATCGATTCCAGCTCTTTTATG (SEQ ID NO: 6205) | 0.29 |
| MG3-6 | A4 | TTCTTCAGTGTGTGAAAAATCC (SEQ ID NO: 6206) | 0.6 |
| MG3-6 | B4 | CTCAGATGTGCAAGATGAATCC (SEQ ID NO: 6207) | 0.06 |
| MG3-6 | C4 | CGGGCACCACCAAGCGCTTTCC (SEQ ID NO: 6208) | 98.21 |
| MG3-6 | D4 | CTGAAATTCATCCTGAGATGAG (SEQ ID NO: 6209) | 100 |
| MG3-6 | E4 | TCGGGAAAGCGCTTGGTGGTGC (SEQ ID NO: 6210) | 99.59 |
| MG3-6 | F4 | CGACCACCGCGAGCACCACGAC (SEQ ID NO: 6211) | 95.73 |
| MG3-6 | G4 | CCGCGAGCACCACGACGAGGAT (SEQ ID NO: 6212) | 2.08 |
| MG3-6 | H4 | GGATACTGACGCCAAGACAGAG (SEQ ID NO: 6213) | 9 |
| MG3-6 | A5 | GGCTCTCCTAGAGAGCCGGCAG (SEQ ID NO: 6214) | 87.5 |
| MG3-6 | B5 | CAGCAGGGTTTGTCCCCGGACA (SEQ ID NO: 6215) | 98.07 |
| MG3-6 | C5 | GGGCTGAACTCGCAGTTGGCCA (SEQ ID NO: 6216) | 0.55 |
| MG3-6 | D5 | CAGTTGGCCATAGGGCTCCAGG (SEQ ID NO: 6217) | 92.34 |
| MG3-6 | E5 | TTCTGTGTTTTATCTCAGACAT (SEQ ID NO: 6218) | 0.01 |
| MG3-6 | F5 | ACATCCTTGCAACATTACTGAA (SEQ ID NO: 6219) | 0.02 |
| MG3-6 | G5 | AGCCACTAATGAAGTTGGGAAC (SEQ ID NO: 6220) | 0.02 |
| MG3-6 | H5 | GATTCTTCTTTGGAGCAGAATA (SEQ ID NO: 6221) | 0.07 |
| MG3-6 | A6 | AAGATCTGGCCCATCAGTTCAC (SEQ ID NO: 6222) | 0 |
| MG3-6 | B6 | GTTCACCCTGGAGGACACGCTG (SEQ ID NO: 6223) | 8.42 |
| MG3-6 | C6 | GTGGTGAATTCAACACTTCCAG (SEQ ID NO: 6224) | 0.14 |
| MG3-6 | D6 | ATTCAACACTTCCAGTGAGGCT (SEQ ID NO: 6225) | 0.41 |
| MG3-6 | E6 | GTGTTGAATTCACCACACCATG (SEQ ID NO: 6226) | 3.84 |
| MG3-6 | F6 | CCTCCAGGGTGAACATGTCCCG (SEQ ID NO: 6227) | 0.66 |
| MG3-6 | G6 | CCCGCTGGACCTGTGTGAACTG (SEQ ID NO: 6228) | 2.42 |
| MG3-6 | H6 | TGGACCTGTGTGAACTGATGGG (SEQ ID NO: 6229) | 26.83 |
| MG3-6 | A7 | AGAAATAAACTATCAATCTTGC (SEQ ID NO: 6230) | 0 |
| MG3-6 | B7 | TCAATCTTGCCCAGACTGGAGA (SEQ ID NO: 6231) | 0.06 |
| MG3-6 | C7 | ACCGTTTTCCAGAATACTGAAA (SEQ ID NO: 6232) | 0.11 |
| MG3-6 | D7 | GCAGTCCTTTCTCCAGTCTGGG (SEQ ID NO: 6233) | 0.11 |
| MG3-6 | E7 | GATGTGGTCCATGTGATGCTCA (SEQ ID NO: 6234) | 0.02 |
| MG3-6 | F7 | TAAATGTGTACCTGTTTTTGTC (SEQ ID NO: 6235) | 0.18 |
| MG3-6 | G7 | GTTTTTGTCAAAGATTTTACTG (SEQ ID NO: 6236) | 0.53 |
| MG3-6 | H7 | TGCGGGATCCATTGAGCATCAC (SEQ ID NO: 6237) | 1.72 |
| MG3-6 | A8 | AAGTCCATAATTTGCAACCAGA (SEQ ID NO: 6238) | 0 |
| MG3-6 | B8 | AACCAGAGAAGGTTCAGACACT (SEQ ID NO: 6239) | 0.3 |
| MG3-6 | C8 | CTGGGTGATACATGGTGGAAGA (SEQ ID NO: 6240) | 0.15 |
| MG3-6 | D8 | CTTCTCTTCCACCATGTATCAC (SEQ ID NO: 6241) | 0.08 |
| MG3-6 | E8 | GAACCTTCTCTGGTTGCAAATT (SEQ ID NO: 6242) | 0.12 |
| MG3-6 | F8 | TTTCTTCCCCAGAGACTTATGC (SEQ ID NO: 6243) | 0.06 |
| MG3-6 | G8 | ATCGATTCCAGCTCTTTTATGG (SEQ ID NO: 6244) | 0.08 |
| MG3-6 | H8 | TCTTCAGTGTGTGAAAAATCCT (SEQ ID NO: 6245) | 0.18 |
| MG3-6 | A9 | CTGAGGATTCATCTTGCACATC (SEQ ID NO: 6246) | 0 |
| MG3-6 | B9 | TCAGATGTGCAAGATGAATCCT (SEQ ID NO: 6247) | 2.73 |
| MG3-6 | C9 | TTTCACACACTGAAGAAACTTG (SEQ ID NO: 6248) | 22.95 |

**Table 25 - CD38 guide screen using MG3-8**

| System | Name | DNA sequence of spacer | Indels |
|---|---|---|---|
| MG3-8 | A1 | CTGAAATTCATCCTGAGATGAG (SEQ ID NO: 6249) | N/A |
| MG3-8 | B1 | GGCTCTCCTAGAGAGCCGGCAG (SEQ ID NO: 6250) | N/A |
| MG3-8 | C1 | CAGTTGGCCATAGGGCTCCAGG (SEQ ID NO: 6251) | 83.37 |
| MG3-8 | D1 | AAGATCTGGCCCATCAGTTCAC (SEQ ID NO: 6252) | 0.02 |
| MG3-8 | E1 | GTGTTGAATTCACCACACCATG (SEQ ID NO: 6253) | 0.13 |
| MG3-8 | F1 | ACCGTTTTCCAGAATACTGAAA (SEQ ID NO: 6254) | 0.07 |
| MG3-8 | G1 | AAGTCCATAATTTGCAACCAGA (SEQ ID NO: 6255) | 1.06 |
| MG3-8 | H1 | TTTCACACACTGAAGAAACTTG (SEQ ID NO: 6256) | 2.2 |

### Example 23.- Gene editing in hematopoietic stem cells using systems described herein

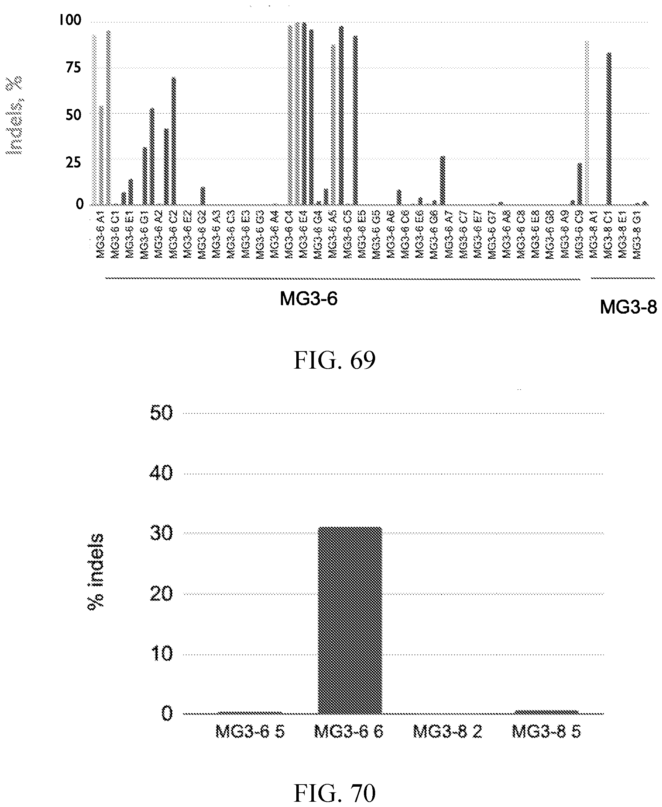

For Hematopoietic stem cell (HSC) editing, HSCs were thawed at 37 per Allcells instructions, washed in DMEM + 10% FBS, resuspended in Stemspan II medium plus CC110 cytokines. 200K cells were nucleofected using a Lonza 4D electroporator and solution P3. Genomic DNA was harvested three days post-transfection and analyzed by NGS (see FIGURE 70). MG3-6 was tested with TRAC guide 5 (SEQ ID NO: 5954) and TRAC guide 6 (SEQ ID NO: 5955). MG3-8 was tested with TRAC guide 2 (SEQ ID NO: 5960) and TRAC guide 5 (SEQ ID NO: 5963).

### Example 24.- Gene editing in B-cells using systems described herein

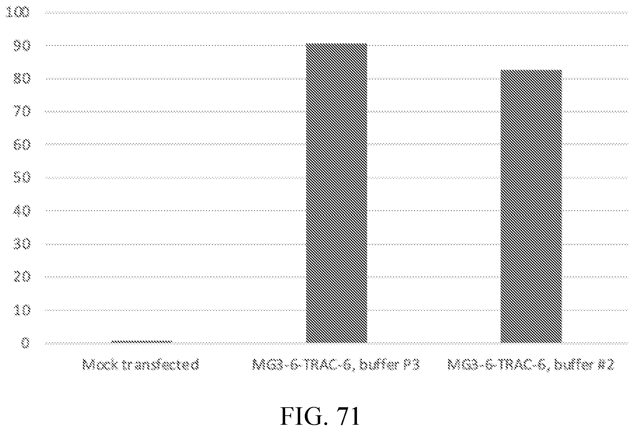

For B-cell editing, B cells were transfected with the Lonza 4D system using buffer P3 or buffer #2 (the mannitol-containing buffer described in Rautela et al., "Efficient genome editing of human natural killer cells by CRISPS RNP," (2021) (available at https://doi.org/10.1101/406934)) with 104 pmol MG3-6 protein and 180 pmol guide. Genomic DNA was harvested three days post-transfection and analyzed by NGS (see FIGURE 71). MG3-6 was tested with TRAC guide 6 (SEQ ID NO: 5955).

### Example 25.- Characterization of MG48 Family Members

### Template DNA for Transcription/Translation

*E. coli* codon-optimized sequences of MG48-1 (protein sequence SEQ ID NO: 5769) and MG48-3 (protein sequence SEQ ID NO: 5771) were ordered (Twist Biosciences) with a T7 promoter. Linear templates were amplified from the plasmids by PCR to include the T7 and nuclease sequence. Minimal array linear templates were amplified from sequences composed of a T7 promoter, native repeat, universal spacer targeting our plasmid library, native repeat, flanked by adapter sequences for amplification. Three intergenic sequences near the ORF or CRISPR array were identified from the metagenomic contigs and ordered as gBlocks with flanking adapter sequences for amplification (Integrated DNA Technologies).

### Transcription/Translation and Cleavage Reactions

MG48-1 and MG48-3 nucleases, intergenic sequences and minimal arrays were expressed in transcription-translation reaction mixtures using myTXTL^{®}Sigma 70 Master Mix Kit (Arbor Biosciences). The final reaction mixtures contained 5 nM nuclease DNA template, 12 nM intergenic DNA template, 15 nM minimal array DNA template, 0.1 nM pTXTL-P70aT7rnap and 1X of myTXTL^{®}Sigma 70 Master Mix. The reactions were incubated at 29 °C for 16 hours then stored at 4 °C.

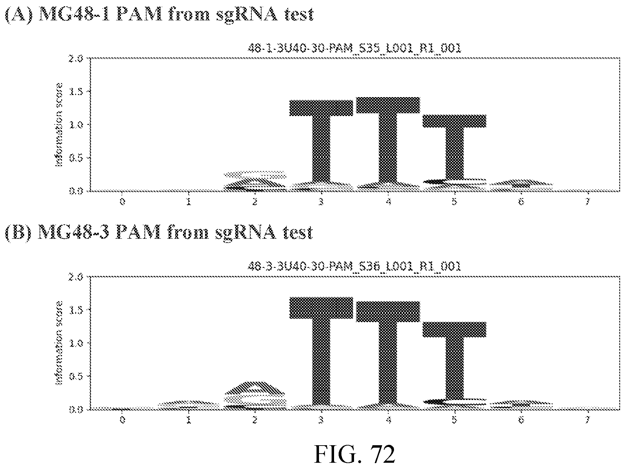

Plasmid library DNA cleavage reactions were carried out by mixing 5 nM of the target library, a 5-fold dilution of the TXTL expressions, 10 nM Tris-HCl, 10 nM MgCl2 and, 100 mM NaCl at 37 °C for 2 hours. The reactions were stopped and cleaned with SPRIselect beads (Beckman Coulter, Inc.) and eluted in Tris EDTA pH 8.0 buffer. 1.5 nM of the cleavage products were ligated with 150 nM adapters, 1 X T4 ligase buffer (New England Biolabs), 20 U/µL T4 DNA ligase (New England Biolabs) at room temperature for 20 minutes. The ligated products were amplified by PCR with NGS primers and sequenced by NGS to obtain the PAM. The results of this experiment are shown in FIGURE 72, which show consensus PAM sequences for MG48-1 (panel A, SEQ ID NO: 5855) and MG48-3 (panel B, SEQ ID NO:5856) obtained from NGS.

### RNAseq Library Prep of Intergenic Enrichment from Transcription/Translation

RNA was extracted from TXTL expressions following the Quick-RNA^{™} Miniprep Kit (Zymo Research) and eluted in 50 µL of water. The total concentration of the transcripts were measured on a Nanodrop and Tapestation.

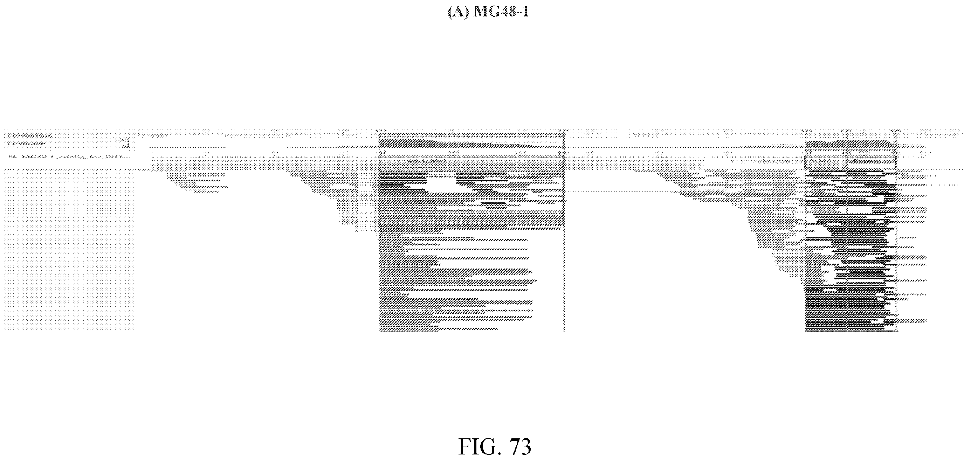

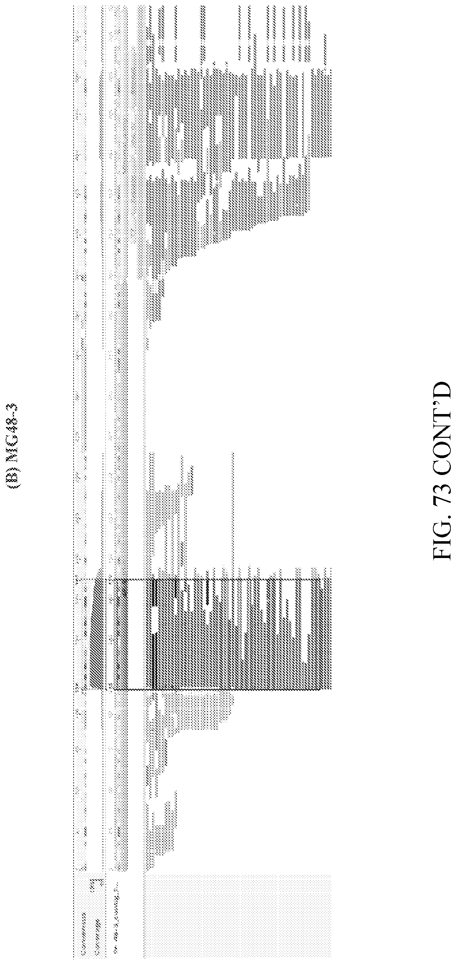

100 ng of total RNA from each sample were prepped for RNA sequencing using the RealSeq-AC miRNA Library Kit (Somagenics). Amplicons between 162-163 bp were quantified with Tapestation and pooled to a final concentration of 20 nM. A final concentration of 6 pM was loaded into a Nano MiSeq V2 kit and sequenced in a Miseq system (Illumina). The RNAseq reads were used to identify the tracr sequence (SEQ ID NO:5886 for MG48-1 and SEQ ID NO: 5893 for MG48-3) of the genes (see FIGURE 73, which illustrates RNAseq mapping with the sequenced tracr region highlighted). Using the tracr sequence we designed sgRNAs (SEQ ID NO: 5888 for MG48-1 and SEQ ID NO: 5895 for MG48-3), which was in vitro transcribed from a dsDNA template using the HiScribe T7 kit (New England Biolabs). The sgRNAs were tested in vitro using the same protocol as the examples above to verify activity and were verified as functional.

### EMBODIMENTS

The following embodiments are illustrative in nature and are not intended to be limiting in any way:
1. An engineered nuclease system, comprising:
   (a) an endonuclease comprising a RuvC_III domain and an HNH domain, wherein said endonuclease is derived from an uncultivated microorganism, wherein said endonuclease is a class 2, type II Cas endonuclease; and
   (b) an engineered guide ribonucleic acid structure configured to form a complex with said endonuclease comprising:
      (i) a guide ribonucleic acid sequence configured to hybridize to a target deoxyribonucleic acid sequence; and
      (ii) a tracr ribonucleic acid sequence configured to bind to said endonuclease.
2. The engineered nuclease system of embodiment 1, wherein said RuvC_III domain comprises a sequence with at least 70%, at least 75%, at least 80% or at least 90% sequence identity to any one of SEQ ID NOs: 1827-3637.
3. An engineered nuclease system comprising:
   (a) an endonuclease comprising a RuvC_III domain having at least 75% sequence identity to any one of SEQ ID NOs: 1827-3637; and
   (b) an engineered guide ribonucleic acid structure configured to form a complex with said endonuclease comprising:
      (i) a guide ribonucleic acid sequence configured to hybridize to a target deoxyribonucleic acid sequence; and
      (ii) a tracr ribonucleic acid sequence configured to bind to said endonuclease.
4. An engineered nuclease system comprising:
   (a) an endonuclease configured to bind to a protospacer adjacent motif (PAM) sequence comprising SEQ ID NOs: 5512-5537, wherein said endonuclease is a class 2, type II Cas endonuclease; and
   (b) an engineered guide ribonucleic acid structure configured to form a complex with said endonuclease comprising:
      (i) a guide ribonucleic acid sequence configured to hybridize to a target deoxyribonucleic acid sequence; and
      (ii) a tracr ribonucleic acid sequence configured to bind to said endonuclease.
5. The engineered nuclease system of embodiment 2, wherein said endonuclease is derived from an uncultivated microorganism.
6. The engineered nuclease system of any one of embodiments 2-3, wherein said endonuclease has not been engineered to bind to a different PAM sequence.
7. The engineered nuclease system of embodiment 2, wherein said endonuclease is not a Cas9 endonuclease, a Cas14 endonuclease, a Cas12a endonuclease, a Cas12b endonuclease, a Cas 12c endonuclease, a Cas12d endonuclease, a Cas12e endonuclease, a Cas13a endonuclease, a Cas13b endonuclease, a Cas13c endonuclease, or a Cas 13d endonuclease.
8. The engineered nuclease system of embodiment 2, wherein said endonuclease has less than 80% identity to a Cas9 endonuclease.
9. The engineered nuclease system of any one of embodiments 1-6, wherein said endonuclease further comprises an HNH domain.
10. The engineered nuclease system of any one of embodiments 1-9, wherein said tracr ribonucleic acid sequence comprises a sequence with at least 80% sequence identity to about 60 to 90 consecutive nucleotides selected from any one of SEQ ID NOs: 5476-5511 and SEQ ID NO: 5538.
11. An engineered nuclease system comprising,
   (a) an engineered guide ribonucleic acid structure comprising:
      (i) a guide ribonucleic acid sequence configured to hybridize to a target deoxyribonucleic acid sequence; and
      (ii) a tracr ribonucleic acid sequence configured to bind to an endonuclease,
      wherein said tracr ribonucleic acid sequence comprises a sequence with at least 80% sequence identity to about 60 to 90 consecutive nucleotides selected from any one of SEQ ID NOs: 5476-5511 and SEQ ID NO: 5538; and
   (b) a class 2, type II Cas endonuclease configured to bind to said engineered guide ribonucleic acid.
12. The engineered nuclease system of any of embodiments 1-1 or 8, wherein said endonuclease is configured to bind to a protospacer adjacent motif (PAM) sequence selected from the group comprising SEQ ID NOs: 5512-5537.
13. The engineered nuclease system of any one of embodiments 1-8, wherein said engineered guide ribonucleic acid structure comprises at least two ribonucleic acid polynucleotides.
14. The engineered nuclease system of any one of embodiments 1-8, wherein said engineered guide ribonucleic acid structure comprises one ribonucleic acid polynucleotide comprising said guide ribonucleic acid sequence and said tracr ribonucleic acid sequence.
15. The engineered nuclease system of any one of embodiments 1-14, wherein said guide ribonucleic acid sequence is complementary to a prokaryotic, bacterial, archaeal, eukaryotic, fungal, plant, mammalian, or human genomic sequence.
16. The engineered nuclease system of any one of embodiments 1-15, wherein said guide ribonucleic acid sequence is 15-24 nucleotides in length.
17. The engineered nuclease system of any one of embodiments 1-10, wherein said endonuclease comprises one or more nuclear localization sequences (NLSs) proximal to an Nor C-terminus of said endonuclease.
18. The engineered nuclease system of any one of embodiments 1-11, wherein said NLS comprises a sequence selected from SEQ ID NOs: 5597-5612.
19. The engineered nuclease system of any one of embodiments 1-12, further comprising a single- or double-stranded DNA repair template comprising from 5' to 3': a first homology arm comprising a sequence of at least 20 nucleotides 5' to said target deoxyribonucleic acid sequence, a synthetic DNA sequence of at least 10 nucleotides, and a second homology arm comprising a sequence of at least 20 nucleotides 3' to said target sequence.
20. The engineered nuclease system of embodiment 13, wherein said first or second homology arm comprises a sequence of at least 40, 80, 120, 150, 200, 300, 500, or 1,000 nucleotides.
21. The engineered nuclease system of any one of embodiments 1-14, wherein said system further comprises a source of Mg2+.
22. The engineered nuclease system of any one of embodiments 1-21, wherein said endonuclease and said tracr ribonucleic acid sequence are derived from distinct bacterial species within a same phylum.
23. The engineered nuclease system of any one of embodiments 1-22, wherein said endonuclease is derived from a bacterium belonging to a genus Dermabacter.
24. The engineered nuclease system of any one of embodiments 1-22, wherein said endonuclease is derived from a bacterium belonging to Phylum Verrucomicrobia, Phylum Candidatus Peregrinibacteria, or Phylum Candidatus Melainabacteria.
25. The engineered nuclease system of any one of embodiments 1-22, wherein said endonuclease is derived from a bacterium comprising a 16S rRNA gene having at least 90% identity to any one of SEQ ID NOs: 5592-5595 .
26. The engineered nuclease system of any one of embodiments 1-25, wherein said HNH domain comprises a sequence with at least 70% or at least 80% identity to any one of SEQ ID NOs: 3638-3955.
27. The engineered nuclease system of any one of embodiments 1-26, wherein said endonuclease comprises SEQ ID NOs: 1-1826 or a variant thereof having at least 55% identity thereto.
28. The engineered nuclease system of any one of embodiments 1-27, wherein said endonuclease comprises a sequence at least 70%, 80%, or 90% identical to a sequence selected from the group consisting of SEQ ID NOs: 1827-1830 or SEQ ID NOs: 1827-2140.
29. The engineered nuclease system of any one of embodiments 1-28, wherein said endonuclease comprises a sequence at least 70%, 80%, or 90% identical to a sequence selected from the group consisting of SEQ ID NOs: 3638-3641 or SEQ ID NOs: 3638-3954.
30. The engineered nuclease system of any one of embodiments 1-29, wherein said endonuclease comprises at least 1, at least 2, at least 3, at least 4, or at least 5 peptide motifs selected from the group consisting of SEQ ID NOs: 5615-5632.
31. The engineered nuclease system of any one of embodiments 1-30, wherein said endonuclease comprises a sequence at least 70%, 80%, or 90% identical to a sequence selected from the group consisting of SEQ ID NOs: 1-4 or SEQ ID NOs: 1-319.
32. The engineered nuclease system of any one of embodiments 1-31, wherein said guide RNA structure comprises a sequence at least 70%, 80%, or 90% identical to a sequence selected from the group consisting of SEQ ID NOs: 5461-5464, SEQ ID NOs: 5476-5479, or SEQ ID NOs: 5476-5489.
33. The engineered nuclease system of any one of embodiments 1-32, wherein said guide RNA structure comprises an RNA sequence predicted to comprise a hairpin consisting of a stem and a loop, wherein the stem comprises at least 10, at least 12 or at least 14 base-paired ribonucleotides, and an asymmetric bulge within 4 base pairs of the loop.
34. The engineered nuclease system of any one of embodiments 1-33, wherein said endonuclease is configured to bind to a PAM comprising a sequence selected from the group consisting of SEQ ID NOs: 5512-5515 or SEQ ID NOs: 5527-5530.
35. The engineered nuclease system of any one of embodiments 1-34, wherein:
   a) said endonuclease comprises a sequence at least 70%, at least 80%, or at least 90% identical to SEQ ID NO: 1827;
   b) said guide RNA structure comprises a sequence at least 70%, at least 80%, or at least 90% identical to at least one of SEQ ID NO: 5461 or SEQ ID NO: 5476; and
   c) said endonuclease is configured to bind to a PAM comprising SEQ ID NO: 5512 or SEQ ID NO: 5527.
36. The engineered nuclease system of any one of embodiments 1-34, wherein:
   a) said endonuclease comprises a sequence at least 70%, at least 80%, or at least 90% identical to SEQ ID NO: 1828;
   b) said guide RNA structure comprises a sequence at least 70%, at least 80%, or at least 90% identical to at least one of SEQ ID NO: 5462 or SEQ ID NO: 5477; and
   c) said endonuclease is configured to bind to a PAM comprising SEQ ID NO: 5513 or SEQ ID NO: 5528.
37. The engineered nuclease system of any one of embodiments 1-34, wherein:
   a) said endonuclease comprises a sequence at least 70%, at least 80%, or at least 90% identical to SEQ ID NO: 1829;
   b) said guide RNA structure comprises a sequence at least 70%, at least 80%, or at least 90% identical to at least one of SEQ ID NO: 5463 or SEQ ID NO: 5478; and
   c) said endonuclease is configured to bind to a PAM comprising SEQ ID NO: 5514 or SEQ ID NO: 5529.
38. The engineered nuclease system of any one of embodiments 1-34, wherein:
   a) said endonuclease comprises a sequence at least 70%, at least 80%, or at least 90% identical to SEQ ID NO: 1830;
   b) said guide RNA structure comprises a sequence at least 70%, at least 80%, or at least 90% identical to at least one of SEQ ID NO: 5464 or SEQ ID NO: 5479; and
   c) said endonuclease is configured to bind to a PAM comprising SEQ ID NO: 5515 or SEQ ID NO: 5530.
39. The engineered nuclease system of any one of embodiments 1-27, wherein said endonuclease comprises a sequence at least 70%, 80%, or 90% identical to a sequence selected from the group consisting of SEQ ID NOs: 2141-2142 or SEQ ID NOs: 2141-2241.
40. The engineered nuclease system of any one of embodiments 1-27 or embodiment 39, wherein said endonuclease comprises a sequence at least 70%, 80%, or 90% identical to a sequence selected from the group consisting of SEQ ID NOs: 3955-3956 or SEQ ID NOs: 3955-4055.
41. The engineered nuclease system of any one of embodiments 1-27 or embodiments 39-40, wherein said endonuclease comprises at least 1, at least 2, at least 3, at least 4, or at least 5 peptide motifs selected from the group consisting of SEQ ID NOs: 5632-5638.
42. The engineered nuclease system of any one of embodiments 1-27 or embodiments 39-41, wherein said endonuclease comprises a sequence at least 70%, 80%, or 90% identical to a sequence selected from the group consisting of SEQ ID NOs: 320-321 or SEQ ID NOs: 320-420.
43. The engineered nuclease system of any one of embodiments 1-27 or embodiments 39-42, wherein said guide RNA structure comprises a sequence at least 70%, 80%, or 90% identical to a sequence selected from the group consisting of SEQ ID NO: 5465, SEQ ID NOs: 5490-5491 or SEQ ID NOs: 5490-5494.
44. The engineered nuclease system of any one of embodiments 1-27 or embodiments 39-43, wherein said guide RNA structure comprises a tracr ribonucleic acid sequence comprising a hairpin comprising at least 8, at least 10, or at least 12 base-paired ribonucleotides.
45. The engineered nuclease system of any one of embodiments 1-27 or embodiments 39-44, wherein said endonuclease is configured to bind to a PAM comprising a sequence selected from the group consisting of SEQ ID NOs: 5516 and SEQ ID NOs: 5531.
46. The engineered nuclease system of any one of embodiments 1-27 or embodiment 39-45, wherein:
   a) said endonuclease comprises a sequence at least 70%, 80%, or 90% identical to SEQ ID NO: 2141;
   b) said guide RNA structure comprises a sequence at least 70%, 80%, or 90% identical to SEQ ID NO: 5490; and
   c) said endonuclease is configured to binding to a PAM comprising SEQ ID NO: 5531.
47. The engineered nuclease system of any one of embodiments 1-27 or embodiment 39-45, wherein
   a) said endonuclease comprises a sequence at least 70%, 80%, or 90% identical to SEQ ID NO: 2142;
   b) said guide RNA structure comprises a sequence at least 70%, 80%, or 90% identical to SEQ ID NO: 5465 or SEQ ID NO: 5491; and
   c) said endonuclease is configured to binding to a PAM comprising SEQ ID NO: 5516.
48. The engineered nuclease system of any one of embodiments 1-27, wherein said endonuclease comprises a sequence at least 70%, 80%, or 90% identical to a sequence selected from the group consisting of SEQ ID NOs: 2245-2246.
49. The engineered nuclease system of any one of embodiments 1-27 or embodiment 48, wherein said endonuclease comprises a sequence at least 70%, 80%, or 90% identical to a sequence selected from the group consisting of SEQ ID NOs: 4059-4060.
50. The engineered nuclease system of any one of embodiments 1-27 or embodiments 48-49, wherein said endonuclease comprises at least 1, at least 2, at least 3, at least 4, or at least 5 peptide motifs selected from the group consisting of SEQ ID NOs: 5639-5648.
51. The engineered nuclease system of any one of embodiments 1-27 or embodiments 48-50, wherein said endonuclease comprises a sequence at least 70%, 80%, or 90% identical to a sequence selected from the group consisting of SEQ ID NOs: 424-425.
52. The engineered nuclease system of any one of embodiments 1-27 or embodiments 48-51, wherein said guide RNA structure comprises a sequence at least 70%, 80%, or 90% identical to a sequence selected from the group consisting of SEQ ID NOs: 5498-5499 and SEQ ID NO: 5539.
53. The engineered nuclease system of any one of embodiments 1-27 or embodiments 48-52, wherein said guide RNA structure comprises a guide ribonucleic acid sequence predicted to comprise a hairpin with an uninterrupted base-paired region comprising at least 8 nucleotides of a guide ribonucleic acid sequence and at least 8 nucleotides of a tracr ribonucleic acid sequence, and wherein said tracr ribonucleic acid sequence comprises, from 5' to 3', a first hairpin and a second hairpin, wherein said first hairpin has a longer stem than said second hairpin.
54. The engineered nuclease system of any one of embodiments 1-27, wherein said endonuclease comprises a sequence at least 70%, 80%, or 90% identical to a sequence selected from the group consisting of SEQ ID NOs: 2242-2244 or SEQ ID NOs: 2247-2249.
55. The engineered nuclease system of any one of embodiments 1-27 or embodiment 54, wherein said endonuclease comprises a sequence at least 70%, 80%, or 90% identical to a sequence selected from the group consisting of SEQ ID NOs: 4056-4058 and SEQ ID NOs 4061-4063.
56. The engineered nuclease system of any one of embodiments 1-27 or embodiments 54-55, wherein said endonuclease comprises at least 1, at least 2, at least 3, at least 4, or at least 5 peptide motifs selected from the group consisting of SEQ ID NOs: 5639-5648.
57. The engineered nuclease system of any one of embodiments 1-27 or embodiments 54-56, wherein said endonuclease comprises a sequence at least 70%, 80%, or 90% identical to a sequence selected from the group consisting of SEQ ID NOs: 421-423 or SEQ ID NOs: 426-428.
58. The engineered nuclease system of any one of embodiments 1-27 or embodiments 54-57, wherein said guide RNA structure comprises a sequence at least 70%, 80%, or 90% identical to a sequence selected from the group consisting of SEQ ID NOs: 5466-5467, SEQ ID NOs: 5495-5497, SEQ ID NO: 5500-5502, and SEQ ID NO: 5539.
59. The engineered nuclease system of any one of embodiments 1-27 or embodiments 54-58, wherein said guide RNA structure comprises a guide ribonucleic acid sequence predicted to comprise a hairpin with an uninterrupted base-paired region comprising at least 8 nucleotides of a guide ribonucleic acid sequence and at least 8 nucleotides of a tracr ribonucleic acid sequence, and wherein said tracr ribonucleic acid sequence comprises, from 5' to 3', a first hairpin and a second hairpin, wherein said first hairpin has a longer stem than said second hairpin.
60. The engineered nuclease system of any one of embodiments 1-27 or embodiments 54-59, wherein said endonuclease is configured to binding to a PAM comprising a sequence selected from the group consisting of SEQ ID NOs: 5517-5518 or SEQ ID NOs: 5532-5534.
61. The engineered nuclease system of any one of embodiments 1-27 or embodiments 54-60, wherein:
   a) said endonuclease comprises a sequence at least 70%, 80%, or 90% identical to SEQ ID NO: 2247;
   b) said guide RNA structure comprises a sequence at least 70%, 80%, or 90% identical to SEQ ID NO: 5500; and
   c) said endonuclease is configured to binding to a PAM comprising SEQ ID NO: 5517 or SEQ ID NO: 5532.
62. The engineered nuclease system of any one of embodiments 1-27 or embodiments 54-60, wherein:
   a) said endonuclease comprises a sequence at least 70%, 80%, or 90% identical to SEQ ID NO: 2248;
   b) said guide RNA structure comprises a sequence at least 70%, 80%, or 90% identical to SEQ ID NO: 5501; and
   c) said endonuclease is configured to binding to a PAM comprising SEQ ID NO: 5518 or SEQ ID NOs: 5533.
63. The engineered nuclease system of any one of embodiments 1-27 or embodiments 54-60, wherein:
   a) said endonuclease comprises a sequence at least 70%, 80%, or 90% identical to SEQ ID NO: 2249;
   b) said guide RNA structure comprises a sequence at least 70%, 80%, or 90% identical to SEQ ID NO: 5502; and
   c) said endonuclease is configured to binding to a PAM comprising SEQ ID NO: 5534.
64. The engineered nuclease system of any one of embodiments 1-27, wherein said endonuclease comprises a sequence at least 70%, 80%, or 90% identical to a sequence selected from the group consisting of SEQ ID NO: 2253 or SEQ ID NOs: 2253-2481.
65. The engineered nuclease system of any one of embodiments 1-27 or embodiment 64, wherein said endonuclease comprises a sequence at least 70%, 80%, or 90% identical to a sequence selected from the group consisting of SEQ ID NO: 4067 or SEQ ID NOs: 4067-4295.
66. The engineered nuclease system of any one of embodiments 1-27 or embodiments 64-65, wherein said endonuclease comprises a peptide motif according to SEQ ID NO: 5649.
67. The engineered nuclease system of any one of embodiments 1-27 or embodiments 64-66, wherein said endonuclease comprises a sequence at least 70%, 80%, or 90% identical to a sequence selected from the group consisting of SEQ ID NO: 432 or SEQ ID NOs: 432-660.
68. The engineered nuclease system of any one of embodiments 1-27 or embodiments 64-67, wherein said guide RNA structure comprises a sequence at least 70%, 80%, or 90% identical to a sequence selected from the group consisting of SEQ ID NO: 5468 or SEQ ID NO: 5503.
69. The engineered nuclease system of any one of embodiments 1-27 or embodiments 64-68, wherein said endonuclease is configured to binding to a PAM comprising a sequence selected from the group consisting of SEQ ID NOs: 5519.
70. The engineered nuclease system of any one of embodiments 1-27 or embodiments 64-69, wherein:
   a) said endonuclease comprises a sequence at least 70%, 80%, or 90% identical to SEQ ID NO: 2253;
   b) said guide RNA structure comprises a sequence at least 70%, 80%, or 90% identical to SEQ ID NO: 5468 or SEQ ID NO: 5503; and
   c) said endonuclease is configured to binding to a PAM comprising SEQ ID NO: 5519.
71. The engineered nuclease system of any one of embodiments 1-27, wherein said endonuclease comprises a sequence at least 70%, 80%, or 90% identical to a sequence selected from the group consisting of SEQ ID NOs: 2482-2489.
72. The engineered nuclease system of any one of embodiments 1-27 or embodiment 71, wherein said endonuclease comprises a sequence at least 70%, 80%, or 90% identical to a sequence selected from the group consisting of SEQ ID NOs: 4296-4303.
73. The engineered nuclease system of any one of embodiments 1-27 or embodiments 71-72, wherein said endonuclease comprises a sequence at least 70%, 80%, or 90% identical to a sequence selected from the group consisting of or SEQ ID NOs: 661-668.
74. The engineered nuclease system of any one of embodiments 1-27, wherein said endonuclease comprises a sequence at least 70%, 80%, or 90% identical to a sequence selected from the group consisting of or SEQ ID NOs: 2490-2498.
75. The engineered nuclease system of any one of embodiments 1-27 or embodiment 74, wherein said endonuclease comprises a sequence at least 70%, 80%, or 90% identical to a sequence selected from the group consisting of SEQ ID NOs: 4304-4312.
76. The engineered nuclease system of any one of embodiments 1-27 or embodiments 74-75, wherein said endonuclease comprises a sequence at least 70%, 80%, or 90% identical to a sequence selected from the group consisting of SEQ ID NOs: 669-677.
77. The engineered nuclease system of any one of embodiments 1-27 or embodiments 74-76, wherein said guide RNA structure comprises a sequence at least 70%, 80%, or 90% identical to a sequence selected from the group consisting of SEQ ID NO: 5504.
78. The engineered nuclease system of any one of embodiments 1-27, wherein said endonuclease comprises a sequence at least 70%, 80%, or 90% identical to a sequence selected from the group consisting of SEQ ID NO: 2499 or SEQ ID NOs: 2499-2750.
79. The engineered nuclease system of any one of embodiments 1-27 or embodiment 78, wherein said endonuclease comprises a sequence at least 70%, 80%, or 90% identical to a sequence selected from the group consisting of SEQ ID NO: 4313 or SEQ ID NOs: 4313-4564.
80. The engineered nuclease system of any one of embodiments 1-27 or embodiments 78-79, wherein said endonuclease comprises at least 1, at least 2, at least 3, at least 4, or at least 5 peptide motifs selected from the group consisting of SEQ ID NOs: 5650-5667.
81. The engineered nuclease system of any one of embodiments 1-27 or embodiments 78-80, wherein said endonuclease comprises a sequence at least 70%, 80%, or 90% identical to a sequence selected from the group consisting of SEQ ID NO: 678 or SEQ ID NOs: 678-929
82. The engineered nuclease system of any one of embodiments 1-27 or embodiments 78-81, wherein said guide RNA structure comprises a sequence at least 70%, 80%, or 90% identical to SEQ ID NO: 5469 or SEQ ID NO: 5505.
83. The engineered nuclease system of any one of embodiments 1-27 or embodiments 78-82, wherein said endonuclease is configured to binding to a PAM comprising SEQ ID NOs: 5520 or SEQ ID NOs: 5535.
84. The engineered nuclease system of any one of embodiments 1-27 or embodiments 78-83, wherein:
   a) said endonuclease comprises a sequence at least 70%, 80%, or 90% identical to SEQ ID NO: 2499;
   b) said guide RNA structure comprises a sequence at least 70%, 80%, or 90% identical to SEQ ID NO: 5469 or SEQ ID NO: 5505; and
   c) said endonuclease is configured to binding to a PAM comprising SEQ ID NO: 5520 or SEQ ID NO: 5535.
85. The engineered nuclease system of any one of embodiments 1-27, wherein said endonuclease comprises a sequence at least 70%, 80%, or 90% identical to a sequence selected from the group consisting of SEQ ID NO: 2751 or SEQ ID NOs: 2751-2913.
86. The engineered nuclease system of any one of embodiments 1-27 or embodiment 85, wherein said endonuclease comprises a sequence at least 70%, 80%, or 90% identical to a sequence selected from the group consisting of SEQ ID NO: 4565 or SEQ ID NOs: 4565-4727.
87. The engineered nuclease system of any one of embodiments 1-27 or embodiments 85-86, wherein said endonuclease comprises at least 1, at least 2, at least 3, at least 4, or at least 5 peptide motifs selected from the group consisting of SEQ ID NOs: 5668-5678.
88. The engineered nuclease system of any one of embodiments 1-27 or embodiments 85-87, wherein said endonuclease comprises a sequence at least 70%, 80%, or 90% identical to a sequence selected from the group consisting of SEQ ID NO: 930 or SEQ ID NOs: 930-1092.
89. The engineered nuclease system of any one of embodiments 1-27 or embodiments 85-88, wherein said guide RNA structure comprises a sequence at least 70%, 80%, or 90% identical to SEQ ID NO: 5470 or SEQ ID NOs: 5506.
90. The engineered nuclease system of any one of embodiments 1-27 or embodiments 85-89, wherein said endonuclease is configured to binding to a PAM comprising a sequence selected from the group consisting of SEQ ID NOs: 5521 or SEQ ID NOs: 5536.
91. The engineered nuclease system of any one of embodiments 1-27 or embodiments 85-90, wherein:
   a) said endonuclease comprises a sequence at least 70%, 80%, or 90% identical to SEQ ID NO: 2751;
   b) said guide RNA structure comprises a sequence at least 70%, 80%, or 90% identical to SEQ ID NO: 5470 or SEQ ID NO: 5506; and
   c) said endonuclease is configured to binding to a PAM comprising SEQ ID NO: 5521 or SEQ ID NO: 5536.
92. The engineered nuclease system of any one of embodiments 1-27, wherein said endonuclease comprises a sequence at least 70%, 80%, or 90% identical to a sequence selected from the group consisting of SEQ ID NO: 2914 or SEQ ID NOs: 2914-3174.
93. The engineered nuclease system of any one of embodiments 1-27 or embodiment 92, wherein said endonuclease comprises a sequence at least 70%, 80%, or 90% identical to a sequence selected from the group consisting of SEQ ID NO: 4728 or SEQ ID NOs: 4728-4988.
94. The engineered nuclease system of any one of embodiments 1-27 or embodiments 92-93, wherein said endonuclease comprises at least 1, at least 2, or at least 3 peptide motifs selected from the group consisting of SEQ ID NOs: 5676-5678.
95. The engineered nuclease system of any one of embodiments 1-27 or embodiments 92-94, wherein said endonuclease comprises a sequence at least 70%, 80%, or 90% identical to a sequence selected from the group consisting of SEQ ID NO: 1093 or SEQ ID NOs: 1093-1353.
96. The engineered nuclease system of any one of embodiments 1-27 or embodiments 92-95, wherein said guide RNA structure comprises a sequence at least 70%, 80%, or 90% identical to a sequence selected from the group consisting of SEQ ID NO: 5471, SEQ ID NO: 5507, and SEQ ID NOs: 5540-5542.
97. The engineered nuclease system of any one of embodiments 1-27 or embodiments 92-96, wherein said guide RNA structure comprises a tracr ribonucleic acid sequence predicted to comprise at least two hairpins comprising less than 5 base-paired ribonucleotides.
98. The engineered nuclease system of any one of embodiments 1-27 or embodiments 92-97, wherein said endonuclease is configured to binding to a PAM comprising SEQ ID NO: 5522.
99. The engineered nuclease system of any one of embodiments 1-27 or embodiments 92-98, wherein:
   a) said endonuclease comprises a sequence at least 70%, 80%, or 90% identical to SEQ ID NO: 2914;
   b) said guide RNA structure comprises a sequence at least 70%, 80%, or 90% identical to SEQ ID NO: 5471 or SEQ ID NO: 5507; and
   c) said endonuclease is configured to binding to a PAM comprising SEQ ID NO: 5522.
100. The engineered nuclease system of any one of embodiments 1-27, wherein said endonuclease comprises a sequence at least 70%, 80%, or 90% identical to a sequence selected from the group consisting of SEQ ID NO: 3175 or SEQ ID NOs: 3175-3330.
101. The engineered nuclease system of any one of embodiments 1-27 or embodiment 100, wherein said endonuclease comprises a sequence at least 70%, 80%, or 90% identical to a sequence selected from the group consisting of SEQ ID NO: 4989 or SEQ ID NOs: 4989-5146.
102. The engineered nuclease system of any one of embodiments 1-27 or embodiments 100-101, wherein said endonuclease comprises at least 1, at least 2, at least 3, at least 4, or at least 5 peptide motifs selected from the group consisting of SEQ ID NOs: 5679-5686.
103. The engineered nuclease system of any one of embodiments 1-27 or embodiments 100-102, wherein said endonuclease comprises a sequence at least 70%, 80%, or 90% identical to a sequence selected from the group consisting of SEQ ID NO: 1354 or SEQ ID NOs: 1354-1511.
104. The engineered nuclease system of any one of embodiments 1-27 or embodiments 100-103, wherein said guide RNA structure comprises a sequence at least 70%, 80%, or 90% identical to a sequence selected from the group consisting of SEQ ID NOs: 5472 or SEQ ID NOs: 5508.
105. The engineered nuclease system of any one of embodiments 1-27 or embodiments 100-104, wherein said endonuclease is configured to binding to a PAM comprising a sequence selected from the group consisting of SEQ ID NO: 5523 or SEQ ID NO: 5537.
106. The engineered nuclease system of any one of embodiments 1-27 or embodiments 100-105, wherein:
   a) said endonuclease comprises a sequence at least 70%, 80%, or 90% identical to SEQ ID NO: 3175;
   b) said guide RNA structure comprises a sequence at least 70%, 80%, or 90% identical to SEQ ID NO: 5472 or SEQ ID NO: 5508; and
   c) said endonuclease is configured to binding to a PAM comprising SEQ ID NO: 5523 or SEQ ID NO: 5537.
107. The engineered nuclease system of any one of embodiments 1-27, wherein said endonuclease comprises a sequence at least 70%, 80%, or 90% identical to a sequence selected from the group consisting of SEQ ID NOs: 3331 or SEQ ID NOs: 3331-3474.
108. The engineered nuclease system of any one of embodiments 1-27 or embodiment 107, wherein said endonuclease comprises a sequence at least 70%, 80%, or 90% identical to a sequence selected from the group consisting of SEQ ID NOs: 5147 or SEQ ID NOs: 5147-5290.
109. The engineered nuclease system of any one of embodiments 1-27 or embodiments 107-108, wherein said endonuclease comprises at least 1, at least 2, at least 3, at least 4, or at least 5 peptide motifs selected from the group consisting of SEQ ID NOs: 5674-5675 and SEQ ID NOs: 5687-5693.
110. The engineered nuclease system of any one of embodiments 1-27 or embodiments 107-109, wherein said endonuclease comprises a sequence at least 70%, 80%, or 90% identical to a sequence selected from the group consisting of SEQ ID NO: 1512 or SEQ ID NOs: 1512-1655.
111. The engineered nuclease system of any one of embodiments 1-27 or embodiments 107-110, wherein said guide RNA structure comprises a sequence at least 70%, 80%, or 90% identical to a sequence selected from the group consisting of SEQ ID NO: 5473 or SEQ ID NO: 5509.
112. The engineered nuclease system of any one of embodiments 1-27 or embodiments 107-111, wherein said endonuclease is configured to binding to a PAM comprising SEQ ID NO: 5524.
113. The engineered nuclease system of any one of embodiments 1-27 or embodiments 107-112, wherein:
   a) said endonuclease comprises a sequence at least 70%, 80%, or 90% identical to SEQ ID NO: 3331;
   b) said guide RNA structure comprises a sequence at least 70%, 80%, or 90% identical to SEQ ID NO: 5473 or SEQ ID NO: 5509; and
   c) said endonuclease is configured to binding to a PAM comprising SEQ ID NO: 5524.
114. The engineered nuclease system of any one of embodiments 1-27, wherein said endonuclease comprises a sequence at least 70%, 80%, or 90% identical to a sequence selected from the group consisting of SEQ ID NO: 3475 or SEQ ID NOs: 3475-3568.
115. The engineered nuclease system of any one of embodiments 1-27 or embodiment 114, wherein said endonuclease comprises a sequence at least 70%, 80%, or 90% identical to a sequence selected from the group consisting of SEQ ID NO: 5291 or SEQ ID NOs: 5291-5389.
116. The engineered nuclease system of any one of embodiments 1-27 or embodiments 114-115, wherein said endonuclease comprises at least 1, at least 2, at least 3, at least 4, or at least 5 peptide motifs selected from the group consisting of SEQ ID NOs: 5694-5699.
117. The engineered nuclease system of any one of embodiments 1-27 or embodiments 114-116, wherein said endonuclease comprises a sequence at least 70%, 80%, or 90% identical to a sequence selected from the group consisting of SEQ ID NO: 1656 or SEQ ID NOs: 1656-1755.
118. The engineered nuclease system of any one of embodiments 1-27 or embodiments 114-117, wherein said guide RNA structure comprises a sequence at least 70%, 80%, or 90% identical to SEQ ID NO: 5474 or SEQ ID NO: 5510.
119. The engineered nuclease system of any one of embodiments 1-27 or embodiments 114-118, wherein said endonuclease is configured to binding to a PAM comprising SEQ ID NOs: 5525.
120. The engineered nuclease system of any one of embodiments 1-27 or embodiments 114-119, wherein:
   a) said endonuclease comprises a sequence at least 70%, 80%, or 90% identical to SEQ ID NO: 3475;
   b) said guide RNA structure comprises a sequence at least 70%, 80%, or 90% identical to SEQ ID NO: 5474 or SEQ ID NO: 5510; and
   c) said endonuclease is configured to binding to a PAM comprising SEQ ID NO: 5525.
121. The engineered nuclease system of any one of embodiments 1-27, wherein said endonuclease comprises a sequence at least 70%, 80%, or 90% identical to a sequence selected from the group consisting of SEQ ID NO: 3569 or SEQ ID NOs: 3569-3637.
122. The engineered nuclease system of any one of embodiments 1-27 or embodiment 121, wherein said endonuclease comprises a sequence at least 70%, 80%, or 90% identical to a sequence selected from the group consisting of SEQ ID NO: 5390 or SEQ ID NOs: 5390-5460.
123. The engineered nuclease system of any one of embodiments 1-27 or embodiments 121-122, wherein said endonuclease comprises at least 1, at least 2, at least 3, at least 4, or at least 5 peptide motifs selected from the group consisting of SEQ ID NOs: 5700-5717.
124. The engineered nuclease system of any one of embodiments 1-27 or embodiments 121-123, wherein said endonuclease comprises a sequence at least 70%, 80%, or 90% identical to a sequence selected from the group consisting of SEQ ID NO: 1756 or SEQ ID NOs: 1756-1826.
125. The engineered nuclease system of any one of embodiments 1-27 or embodiments 121-124, wherein said guide RNA structure comprises a sequence at least 70%, 80%, or 90% identical to SEQ ID NO: 5475 or SEQ ID NOs: 5511.
126. The engineered nuclease system of any one of embodiments 1-27 or embodiments 121-125, wherein said endonuclease is configured to binding to a PAM comprising SEQ ID NO: 5526.
127. The engineered nuclease system of any one of embodiments 1-27 or embodiments 121-126, wherein:
   a) said endonuclease comprises a sequence at least 70%, 80%, or 90% identical to SEQ ID NO: 3569;
   b) said guide RNA structure comprises a sequence at least 70%, 80%, or 90% identical to SEQ ID NO: 5475 or SEQ ID NO: 5511; and
   c) said endonuclease is configured to binding to a PAM comprising SEQ ID NO: 5526.
128. The engineered nuclease system of any one of embodiments 1-127, wherein said sequence identity is determined by a BLASTP, CLUSTALW, MUSCLE, MAFFT, or Smith-Waterman homology search algorithm.
129. The engineered nuclease system of embodiment 15, wherein said sequence identity is determined by said BLASTP homology search algorithm using parameters of a wordlength (W) of 3, an expectation (E) of 10, and a BLOSUM62 scoring matrix setting gap costs at existence of 11, extension of 1, and using a conditional compositional score matrix adjustment.
130. An engineered guide ribonucleic acid polynucleotide comprising:
   a) a DNA-targeting segment comprising a nucleotide sequence that is complementary to a target sequence in a target DNA molecule; and
   b) a protein-binding segment comprising two complementary stretches of nucleotides that hybridize to form a double-stranded RNA (dsRNA) duplex,
      wherein said two complementary stretches of nucleotides are covalently linked to one another with intervening nucleotides, and
      wherein said engineered guide ribonucleic acid polynucleotide is configured to forming a complex with an endonuclease comprising a RuvC_III domain having at least 75% sequence identity to any one of SEQ ID NOs: 1827-3637 and targeting said complex to said target sequence of said target DNA molecule.
131. The engineered guide ribonucleic acid polynucleotide of embodiment 17, wherein said DNA-targeting segment is positioned 5' of both of said two complementary stretches of nucleotides.
132. The engineered guide ribonucleic acid polynucleotide of any of embodiments 17-18, wherein:
   a) said protein binding segment comprises a sequence having at least 70%, at least 80%, or at least 90% identity to a sequence selected from the group consisting of SEQ ID NOs: 5476-5479 or SEQ ID NOs: 5476-5489;
   b) said protein binding segment comprises a sequence having at least 70%, at least 80%, or at least 90% identity to a sequence selected from the group consisting of (SEQ ID NOs: 5490-5491 or SEQ ID NOs: 5490-5494) and SEQ ID NO: 5538;
   c) said protein binding segment comprises a sequence having at least 70%, at least 80%, or at least 90% identity to a sequence selected from the group consisting of SEQ ID NOs: 5498-5499;
   d) said protein binding segment comprises a sequence having at least 70%, at least 80%, or at least 90% identity to a sequence selected from the group consisting of SEQ ID NOs: 5495-5497 and SEQ ID NOs: 5500-5502;
   e) said protein binding segment comprises a sequence having at least 70%, at least 80%, or at least 90% identity to SEQ ID NO: 5503;
   f) said protein binding segment comprises a sequence having at least 70%, at least 80%, or at least 90% identity to SEQ ID NO: 5504;
   g) said protein binding segment comprises a sequence having at least 70%, at least 80%, or at least 90% identity to SEQ ID NOs: 5505;
   h) protein binding segment comprises a sequence having at least 70%, at least 80%, or at least 90% identity to SEQ ID NO: 5506;
   i) protein binding segment comprises a sequence having at least 70%, at least 80%, or at least 90% identity to SEQ ID NO: 5507;
   j) said protein binding segment comprises a sequence having at least 70%, at least 80%, or at least 90% identity to SEQ ID NO: 5508;
   k) said protein binding segment comprises a sequence having at least 70%, at least 80%, or at least 90% identity to SEQ ID NO: 5509;
   l) said protein binding segment comprises a sequence having at least 70%, at least 80%, or at least 90% identity to SEQ ID NO: 5510; or
   m) said protein binding segment comprises a sequence having at least 70%, at least 80%, or at least 90% identity to SEQ ID NO: 5511.
133. The engineered guide ribonucleic acid polynucleotide of any of embodiment 17-132, wherein:
   a) said guide ribonucleic acid polynucleotide comprises an RNA sequence comprising a hairpin comprising a stem and a loop, wherein said stem comprises at least 10, at least 12, or at least 14 base-paired ribonucleotides, and an asymmetric bulge within 4 base pairs of the loop;
   b) said guide ribonucleic acid polynucleotide comprises a tracr ribonucleic acid sequence predicted to comprise a hairpin comprising at least 8, at least 10, or at least 12 base-paired ribonucleotides;
   c) said guide ribonucleic acid polynucleotide comprises a guide ribonucleic acid sequence predicted to comprise a hairpin with an uninterrupted base-paired region comprising at least 8 nucleotides of a guide ribonucleic acid sequence and at least 8 nucleotides of a tracr ribonucleic acid sequence, and wherein said tracr ribonucleic acid sequence comprises, from 5' to 3', a first hairpin and a second hairpin, wherein said first hairpin has a longer stem than said second hairpin; or
   d) said guide ribonucleic acid polynucleotide comprises a tracr ribonucleic acid sequence predicted to comprise at least two hairpins comprising less than 5 base-paired ribonucleotides.
134. A deoxyribonucleic acid polynucleotide encoding the engineered guide ribonucleic acid polynucleotide of any one of embodiments 17-133.
135. A nucleic acid comprising an engineered nucleic acid sequence optimized for expression in an organism, wherein said nucleic acid encodes a class 2, type II Cas endonuclease comprising a RuvC_III domain and an HNH domain, and wherein said endonuclease is derived from an uncultivated microorganism.
136. A nucleic acid comprising an engineered nucleic acid sequence optimized for expression in an organism, wherein said nucleic acid encodes an endonuclease comprising a RuvC_III domain having at least 70% sequence identity to any one of SEQ ID NOs: 1827-3637.
137. The nucleic acid of any one of embodiments 135-20, wherein said endonuclease comprises an HNH domain having at least 70% or at least 80% sequence identity to any one of SEQ ID NOs: 3638-5460.
138. The nucleic acid of any one of embodiments 135-137, wherein said endonuclease comprises SEQ ID NOs: 5572-5591 or a variant thereof having at least 70% sequence identity thereto.
139. The nucleic acid of any one of embodiments 135-138, wherein said endonuclease comprises a sequence encoding one or more nuclear localization sequences (NLSs) proximal to an N- or C-terminus of said endonuclease.
140. The nucleic acid of embodiment 21, wherein said NLS comprises a sequence selected from SEQ ID NOs: 5597-5612.
141. The nucleic acid of any one of embodiments 135-22, wherein said organism is prokaryotic, bacterial, eukaryotic, fungal, plant, mammalian, rodent, or human.
142. The nucleic acid of embodiment 23, wherein said organism is E. coli, and wherein:
   a) said nucleic acid sequence has at least 70%, 80%, or 90% identity to a sequence selected from the group consisting of SEQ ID NOs: 5572-5575;
   b) said nucleic acid sequence has at least 70%, 80%, or 90% identity to a sequence selected from the group consisting of SEQ ID NOs: 5576-5577;
   c) said nucleic acid sequence has at least 70%, 80%, or 90% identity to a sequence selected from the group consisting of SEQ ID NOs: 5578-5580;
   d) said nucleic acid sequence has at least 70%, 80%, or 90% identity to SEQ ID NO: 5581;
   e) said nucleic acid sequence has at least 70%, 80%, or 90% identity to SEQ ID NO: 5582;
   f) said nucleic acid sequence has at least 70%, 80%, or 90% identity to SEQ ID NO: 5583;
   g) said nucleic acid sequence has at least 70%, 80%, or 90% identity to SEQ ID NO: 5584;
   h) said nucleic acid sequence has at least 70%, 80%, or 90% identity to SEQ ID NO: 5585;
   i) said nucleic acid sequence has at least 70%, 80%, or 90% identity to SEQ ID NO: 5586; or
   j) said nucleic acid sequence has at least 70%, 80%, or 90% identity to SEQ ID NO: 5587.
143. The nucleic acid of embodiment 23, wherein said organism is human, and wherein:
   a) said nucleic acid sequence has at least 70%, 80%, or 90% identity to SEQ ID NO: 5588 or SEQ ID NO: 5589; or
   b) said nucleic acid sequence has at least 70%, 80%, or 90% identity to SEQ ID NO: 5590 or SEQ ID NO: 5591.
144. A vector comprising a nucleic acid sequence encoding a class 2, type II Cas endonuclease comprising a RuvC_III domain and an HNH domain, wherein said endonuclease is derived from an uncultivated microorganism.
145. A vector comprising the nucleic acid of any of embodiments 135-143.
146. The vector of any of embodiments 144-24, further comprising a nucleic acid encoding an engineered guide ribonucleic acid structure configured to form a complex with said endonuclease comprising:
   a) a guide ribonucleic acid sequence configured to hybridize to a target deoxyribonucleic acid sequence; and
   b) a tracr ribonucleic acid sequence configured to binding to said endonuclease.
147. The vector of any of embodiments 144-25, wherein the vector is a plasmid, a minicircle, a CELiD, an adeno-associated virus (AAV) derived virion, or a lentivirus.
148. A cell comprising the vector of any of embodiments 144-26.
149. A method of manufacturing an endonuclease, comprising cultivating said cell of embodiment 146.
150. A method for binding, cleaving, marking, or modifying a double-stranded deoxyribonucleic acid polynucleotide, comprising:
   (a) contacting said double-stranded deoxyribonucleic acid polynucleotide with a class 2, type II Cas endonuclease in complex with an engineered guide ribonucleic acid structure configured to bind to said endonuclease and said double-stranded deoxyribonucleic acid polynucleotide;
   (b) wherein said double-stranded deoxyribonucleic acid polynucleotide comprises a protospacer adjacent motif (PAM); and
   (c) wherein said PAM comprises a sequence selected from the group consisting of SEQ ID NOs: 5512-5526 or SEQ ID NOs: 5527-5537.
151. A method of embodiment 28, wherein said double-stranded deoxyribonucleic acid polynucleotide comprises a first strand comprising a sequence complementary to a sequence of said engineered guide ribonucleic acid structure and a second strand comprising said PAM.
152. A method of embodiment 30, wherein said PAM is directly adjacent to the 3' end of said sequence complementary to said sequence of said engineered guide ribonucleic acid structure.
153. A method of any one of embodiments 28-31, wherein said class 2, type II Cas endonuclease is not a Cas9 endonuclease, a Cas14 endonuclease, a Cas12a endonuclease, a Cas12b endonuclease, a Cas 12c endonuclease, a Cas12d endonuclease, a Cas12e endonuclease, a Cas13a endonuclease, a Cas13b endonuclease, a Cas13c endonuclease, or a Cas 13d endonuclease.
154. A method of any one of embodiments 28-32, wherein said class 2, type II Cas endonuclease is derived from an uncultivated microorganism.
155. A method of any one of embodiments 28-154, wherein said double-stranded deoxyribonucleic acid polynucleotide is a eukaryotic, plant, fungal, mammalian, rodent, or human double-stranded deoxyribonucleic acid polynucleotide.
156. A method of any one of embodiments 28-33, wherein:
   a) said PAM comprises a sequence selected from the group consisting of SEQ ID NOs: 5512-5515 and SEQ ID NOs: 5527-5530;
   b) said PAM comprises SEQ ID NO: 5516 or SEQ ID NO: 5531;
   c) said PAM comprises SEQ ID NO: 5539;
   d) said PAM comprises SEQ ID NO: 5517 or SEQ ID NO: 5518;
   e) said PAM comprises SEQ ID NO: 5519;
   f) said PAM comprises SEQ ID NO: 5520 or SEQ ID NO: 5535;
   g) said PAM comprises SEQ ID NO: 5521 or SEQ ID NO: 5536;
   h) said PAM comprises SEQ ID NO: 5522;
   i) said PAM comprises SEQ ID NO: 5523 or SEQ ID NO: 5537;
   j) said PAM comprises SEQ ID NO: 5524;
   k) said PAM comprises SEQ ID NO: 5525; or
   l) said PAM comprises SEQ ID NO: 5526.
157. A method of modifying a target nucleic acid locus, said method comprising delivering to said target nucleic acid locus said engineered nuclease system of any one of embodiments 1-16, wherein said endonuclease is configured to form a complex with said engineered guide ribonucleic acid structure, and wherein said complex is configured such that upon binding of said complex to said target nucleic acid locus, said complex modifies said target nucleic locus.
158. A method of embodiment 34, wherein modifying said target nucleic acid locus comprises binding, nicking, cleaving, or marking said target nucleic acid locus.
159. A method of any of embodiments 34-35, wherein said target nucleic acid locus comprises deoxyribonucleic acid (DNA) or ribonucleic acid (RNA).
160. A method of embodiment 36, wherein said target nucleic acid comprises genomic DNA, viral DNA, viral RNA, or bacterial DNA.
161. A method of any one of embodiments 34-37, wherein said target nucleic acid locus is in vitro.
162. A method of any one of embodiments 34-37, wherein said target nucleic acid locus is within a cell.
163. A method of embodiment 39, wherein said cell is a prokaryotic cell, a bacterial cell, a eukaryotic cell, a fungal cell, a plant cell, an animal cell, a mammalian cell, a rodent cell, a primate cell, or a human cell.
164. A method of any one of embodiments 39-40, wherein delivering said engineered nuclease system to said target nucleic acid locus comprises delivering the nucleic acid of any of embodiments 135-22 or the vector of any of embodiments 142-25.
165. A method of any one of embodiments 39-40, wherein delivering said engineered nuclease system to said target nucleic acid locus comprises delivering a nucleic acid comprising an open reading frame encoding said endonuclease.
166. A method of embodiment 41, wherein said nucleic acid comprises a promoter to which said open reading frame encoding said endonuclease is operably linked.
167. A method of any one of embodiments 39-40, wherein delivering said engineered nuclease system to said target nucleic acid locus comprises delivering a capped mRNA containing said open reading frame encoding said endonuclease.
168. A method of any one of embodiments 39-40, wherein delivering said engineered nuclease system to said target nucleic acid locus comprises delivering a translated polypeptide.
169. A method of any one of embodiments 39-40, wherein delivering said engineered nuclease system to said target nucleic acid locus comprises delivering a deoxyribonucleic acid (DNA) encoding said engineered guide ribonucleic acid structure operably linked to a ribonucleic acid (RNA) pol III promoter.
170. A method of any one of embodiments 34-46, wherein said endonuclease induces a single-stranded break or a double-stranded break at or proximal to said target locus.

While preferred embodiments of the present invention have been shown and described herein, it will be obvious to those skilled in the art that such embodiments are provided by way of example only. It is not intended that the invention be limited by the specific examples provided within the specification. While the invention has been described with reference to the aforementioned specification, the descriptions and illustrations of the embodiments herein are not meant to be construed in a limiting sense. Numerous variations, changes, and substitutions will now occur to those skilled in the art without departing from the invention. Furthermore, it shall be understood that all aspects of the invention are not limited to the specific depictions, configurations or relative proportions set forth herein which depend upon a variety of conditions and variables. It should be understood that various alternatives to the embodiments of the invention described herein may be employed in practicing the invention. It is therefore contemplated that the invention shall also cover any such alternatives, modifications, variations or equivalents. It is intended that the following claims define the scope of the invention and that methods and structures within the scope of these claims and their equivalents be covered thereby.

The following clauses, describing aspects of the invention, are part of the description
1. An engineered nuclease system comprising:
   (a) an endonuclease comprising a sequence having at least 75% sequence identity to any one of SEQ ID NOs: 5718-5846 or 6257; and
   (b) an engineered guide ribonucleic acid structure configured to form a complex with said endonuclease comprising:
      (i) a ribonucleic acid sequence configured to hybridize to a target deoxyribonucleic acid sequence; and
      (ii) a ribonucleic acid sequence configured to bind to said endonuclease.
2. An engineered nuclease system comprising:
   (a) an endonuclease configured to bind to a protospacer adjacent motif (PAM) sequence comprising SEQ ID NOs: 5847-5861 or 6258-6278, wherein said endonuclease is a class 2, type II Cas endonuclease; and
   (b) an engineered guide ribonucleic acid structure configured to form a complex with said endonuclease comprising:
      (i) a ribonucleic acid sequence configured to hybridize to a target deoxyribonucleic acid sequence; and
      (ii) a ribonucleic acid sequence configured to bind to said endonuclease.
3. The engineered nuclease system of clause 1 or 2, wherein said endonuclease is derived from an uncultivated microorganism.
4. The engineered nuclease system of any one of clauses 1-3, wherein said endonuclease has not been engineered to bind to a different PAM sequence.
5. The engineered nuclease system any one of clauses 1-4, wherein said endonuclease is not a Cas9 endonuclease, a Cas14 endonuclease, a Cas12a endonuclease, a Cas12b endonuclease, a Cas 12c endonuclease, a Cas12d endonuclease, a Cas12e endonuclease, a Cas13a endonuclease, a Cas13b endonuclease, a Cas13c endonuclease, or a Cas 13d endonuclease.
6. The engineered nuclease system of any one of clauses 1-5, wherein said endonuclease has less than 80% identity to a Cas9 endonuclease.
7. The engineered nuclease system of any one of clauses 1-6, wherein said ribonucleic acid sequence comprises a sequence with at least 80% sequence identity to (a) any one of SEQ ID NOs: 5886-5887, 5891, 5893, or 5894; or (b) the non-degenerate nucleotides of any one of SEQ ID NOs: 5862-5885, 5888-5890, 5892, 5895-5896, or 6279-6301.
8. An engineered nuclease system comprising,
   (a) an engineered guide ribonucleic acid structure comprising:
      (i) a ribonucleic acid sequence configured to hybridize to a target deoxyribonucleic acid sequence; and
      (ii) a ribonucleic acid sequence configured to bind to an endonuclease, wherein said ribonucleic acid sequence comprises a sequence with at least 80% sequence identity (a) any one of SEQ ID NOs: 5886-5887, 5891, 5893, or 5894; or (b) the non-degenerate nucleotides of any one of SEQ ID NOs: 5862-5885, 5888-5890, 5892, 5895-5896, or 6279-6301; and
   (b) a class 2, type II Cas endonuclease configured to bind to said engineered guide ribonucleic acid.
9. The engineered nuclease system of clause 8, wherein said endonuclease is configured to bind to a protospacer adjacent motif (PAM) sequence selected from the group comprising SEQ ID NOs: 5847-5861 or 6258-6278.
10. The engineered nuclease system of any one of clauses 8-9, wherein said guide ribonucleic acid sequence is 15-24 nucleotides in length or 19-24 nucleotides in length.
11. The engineered nuclease system of any one of clauses 1-10, wherein said endonuclease comprises one or more nuclear localization sequences (NLSs) proximal to an Nor C-terminus of said endonuclease.
12. The engineered nuclease system of any one of clauses 1-11, wherein said NLS comprises a sequence selected from SEQ ID NOs: 5597-5612.
13. The engineered nuclease system of any one of clauses 1-12, further comprising a single- or double-stranded DNA repair template comprising from 5' to 3': a first homology arm comprising a sequence of at least 20 nucleotides 5' to said target deoxyribonucleic acid sequence, a synthetic DNA sequence of at least 10 nucleotides, and a second homology arm comprising a sequence of at least 20 nucleotides 3' to said target sequence.
14. The engineered nuclease system of clause 13, wherein said first or second homology arm comprises a sequence of at least 40, 80, 120, 150, 200, 300, 500, or 1,000 nucleotides.
15. The engineered nuclease system of any one of clauses 1-14, wherein said sequence identity is determined by a BLASTP, CLUSTALW, MUSCLE, MAFFT, or CLUSTALW with the parameters of the Smith-Waterman homology search algorithm.
16. The engineered nuclease system of clause 15, wherein said sequence identity is determined by said BLASTP homology search algorithm using parameters of a wordlength (W) of 3, an expectation (E) of 10, and a BLOSUM62 scoring matrix setting gap costs at existence of 11, extension of 1, and using a conditional compositional score matrix adjustment.
17. An engineered guide ribonucleic acid polynucleotide comprising:
   a) a DNA-targeting segment comprising a nucleotide sequence that is complementary to a target sequence in a target DNA molecule; and
   b) a protein-binding segment comprising two complementary stretches of nucleotides that hybridize to form a double-stranded RNA (dsRNA) duplex,
      wherein said two complementary stretches of nucleotides are covalently linked to one another with intervening nucleotides, and
      wherein said engineered guide ribonucleic acid polynucleotide is configured to form a complex with an endonuclease comprising sequence having at least 75% sequence identity to any one of SEQ ID NOs: 5718-5846 or 6257 and target said complex to said target sequence of said target DNA molecule.
18. The engineered guide ribonucleic acid polynucleotide of clause 17, wherein said DNA-targeting segment is positioned 5' of both of said two complementary stretches of nucleotides.
19. A deoxyribonucleic acid polynucleotide encoding the engineered guide ribonucleic acid polynucleotide or structure of any one of clauses 17-18.
20. A nucleic acid comprising an engineered nucleic acid sequence optimized for expression in an organism, wherein said nucleic acid encodes an endonuclease comprising a sequence having at least 75% sequence identity to any one of SEQ ID NOs: 5718-5846 or 6257.
21. The nucleic acid of clause 20, wherein said endonuclease comprises a sequence encoding one or more nuclear localization sequences (NLSs) proximal to an N- or C-terminus of said endonuclease.
22. The nucleic acid of clause 21, wherein said NLS comprises a sequence selected from SEQ ID NOs: 5597-5612.
23. The nucleic acid of any one of clauses 20-22, wherein said organism is prokaryotic, bacterial, eukaryotic, fungal, plant, mammalian, rodent, or human.
24. A vector comprising the nucleic acid of any of clauses 20-23.
25. The vector of clause 24, further comprising a nucleic acid encoding an engineered guide ribonucleic acid structure configured to form a complex with said endonuclease comprising:
   a) a ribonucleic acid sequence configured to hybridize to a target deoxyribonucleic acid sequence; and
   b) a ribonucleic acid sequence configured to bind to said endonuclease.
26. The vector of any one of clauses 24-25, wherein the vector is a plasmid, a minicircle, a CELiD, an adeno-associated virus (AAV) derived virion, or a lentivirus.
27. A cell comprising the vector of any of clauses 24-26.
28. A method of manufacturing an endonuclease, comprising cultivating said cell of clause 27.
29. A method for binding, cleaving, marking, or modifying a double-stranded deoxyribonucleic acid polynucleotide, comprising:
   contacting said double-stranded deoxyribonucleic acid polynucleotide with a class 2, type II Cas endonuclease in complex with an engineered guide ribonucleic acid structure configured to bind to said endonuclease and said double-stranded deoxyribonucleic acid polynucleotide;
   wherein said double-stranded deoxyribonucleic acid polynucleotide comprises a protospacer adjacent motif (PAM); and
   wherein said PAM comprises a sequence selected from the group consisting of SEQ ID NOs: 5847-5861 or 6258-6278.
30. The method of clause 29, wherein said double-stranded deoxyribonucleic acid polynucleotide comprises a first strand comprising a sequence complementary to a sequence of said engineered guide ribonucleic acid structure and a second strand comprising said PAM.
31. The method of clause 30, wherein said PAM is directly adjacent to the 3' end of said sequence complementary to said sequence of said engineered guide ribonucleic acid structure.
32. A method of any one of clauses 29-31, wherein said class 2, type II Cas endonuclease is not a Cas9 endonuclease, a Cas14 endonuclease, a Cas12a endonuclease, a Cas12b endonuclease, a Cas 12c endonuclease, a Cas12d endonuclease, a Cas12e endonuclease, a Cas13a endonuclease, a Cas13b endonuclease, a Cas13c endonuclease, or a Cas 13d endonuclease.
33. The method of any one of clauses 29-32, wherein said double-stranded deoxyribonucleic acid polynucleotide is a eukaryotic, plant, fungal, mammalian, rodent, or human double-stranded deoxyribonucleic acid polynucleotide.
34. A method of modifying a target nucleic acid locus, said method comprising delivering to said target nucleic acid locus said engineered nuclease system of any one of clauses 1-16, wherein said endonuclease is configured to form a complex with said engineered guide ribonucleic acid structure, and wherein said complex is configured such that upon binding of said complex to said target nucleic acid locus, said complex modifies said target nucleic locus.
35. The method of clause 34, wherein modifying said target nucleic acid locus comprises binding, nicking, cleaving, or marking said target nucleic acid locus.
36. The method of any one of clauses 34-35, wherein said target nucleic acid locus comprises deoxyribonucleic acid (DNA) or ribonucleic acid (RNA).
37. The method of clause 36, wherein said target nucleic acid comprises genomic DNA, viral DNA, viral RNA, or bacterial DNA.
38. The method of any one of clauses 34-37, wherein said target nucleic acid locus is in vitro.
39. The method of any one of clauses 34-37, wherein said target nucleic acid locus is within a cell.
40. The method of clause 39, wherein said cell is a prokaryotic cell, a bacterial cell, a eukaryotic cell, a fungal cell, a plant cell, an animal cell, a mammalian cell, a rodent cell, a primate cell, or a human cell.
41. The method of any one of clauses 34-40, wherein delivering said engineered nuclease system to said target nucleic acid locus comprises delivering the nucleic acid of any of clauses 20-23 or the vector of any of clauses 24-26.
42. The method of any one of clauses 34-40, wherein delivering said engineered nuclease system to said target nucleic acid locus comprises delivering a nucleic acid comprising an open reading frame encoding said endonuclease.
43. The method of clause 41, wherein said nucleic acid comprises a promoter to which said open reading frame encoding said endonuclease is operably linked.
44. The method of any one of clauses 34-41, wherein delivering said engineered nuclease system to said target nucleic acid locus comprises delivering a capped mRNA containing said open reading frame encoding said endonuclease.
45. The method of any one of clauses 34-41, wherein delivering said engineered nuclease system to said target nucleic acid locus comprises delivering a translated polypeptide.
46. The method of any one of clauses 34-41, wherein delivering said engineered nuclease system to said target nucleic acid locus comprises delivering a deoxyribonucleic acid (DNA) encoding said engineered guide ribonucleic acid structure operably linked to a ribonucleic acid (RNA) pol III promoter.
47. A method of any one of clauses 34-46, wherein said endonuclease induces a single-stranded break or a double-stranded break at or proximal to said target locus.
48. A method of editing a TRAC locus in a cell, comprising contacting to said cell
   (a) an RNA-guided endonuclease; and
   (b) an engineered guide RNA, wherein said engineered guide RNA is configured to form a complex with said endonuclease and said engineered guide RNA comprises a spacer sequence configured to hybridize to a region of said TRAC locus,
   wherein said engineered guide RNA comprises a targeting sequence having at least 85% identity to at least 18 consecutive nucleotides of any one of SEQ ID NOs: 5950-5958 or 5959-5965.
49. The method of clause 48, wherein said RNA-guided endonuclease is a class II, type II Cas endonuclease.
50. The method of clause 48 or clause 49, wherein said RNA-guided endonuclease comprises a RuvCIII domain comprising a sequence having at least 75% identity to SEQ ID NO: 2242 or SEQ ID NO: 2244.
51. The method of clause 50, wherein said RNA-guided endonuclease further comprises an HNH domain.
52. The method of any one of clauses 48-51, wherein said RNA-guided endonuclease comprises a sequence having at least 75% identity to SEQ ID NO: 421 or SEQ ID NO: 423.
53. The method of any one of clauses 48-52, wherein said engineered guide RNA comprises a targeting sequence having at least 85% identity to at least 18 consecutive nucleotides of any one of SEQ ID NOs: 5950-5958 and said endonuclease comprises a sequence having at least 75% identity to SEQ ID NO:421.
54. The method of any one of clauses 48-52, wherein said engineered guide RNA comprises a targeting sequence having at least 85% identity to at least 18 consecutive nucleotides of any one of SEQ ID NOs: 5959-5965 and said endonuclease comprises a sequence having at least 75% identity to SEQ ID NO: 423.
55. The method of any one of clauses 48-52, wherein said engineered guide RNA comprises a targeting sequence having at least 85% identity to at least 18 consecutive nucleotides of any one of SEQ ID NOs: 5953-5957.
56. The method of any one of clauses 48-52, wherein said engineered guide RNA comprises a targeting sequence having at least 85% identity to at least 18 consecutive nucleotides of any one of SEQ ID NOs: 5960-5961 or 5963-5964.
57. A method of editing a TRBC locus in a cell, comprising contacting to said cell
   (a) an RNA-guided endonuclease; and
   (b) an engineered guide RNA, wherein said engineered guide RNA is configured to form a complex with said endonuclease and said engineered guide RNA comprises a spacer sequence configured to hybridize to a region of said TRBC locus,
   wherein said engineered guide RNA comprises a targeting sequence having at least 85% identity to at least 18 consecutive nucleotides of any one of SEQ ID NOs: 5966-6004 or 6005-6025.
58. The method of clause 57, wherein said RNA-guided endonuclease is a class II, type II Cas endonuclease.
59. The method of clause 57 or clause 58, wherein said RNA-guided endonuclease comprises a RuvCIII domain comprising a sequence having at least 75% identity to SEQ ID NO: 2242 or SEQ ID NO: 2244.
60. The method of clause 59, wherein said RNA-guided endonuclease further comprises an HNH domain.
61. The method of any one of clauses 57-60, wherein said RNA-guided endonuclease comprises a sequence having at least 75% identity to SEQ ID NO: 421 or SEQ ID NO: 423.
62. The method of any one of clauses 57-61, wherein said engineered guide RNA comprises a targeting sequence having at least 85% identity to at least 18 consecutive nucleotides of any one of SEQ ID NOs: 5966-6004 and said endonuclease comprises a sequence having at least 75% identity to SEQ ID NO: 421.
63. The method of any one of clauses 57-61, wherein said engineered guide RNA comprises a targeting sequence having at least 85% identity to at least 18 consecutive nucleotides of any one of SEQ ID NOs: 6005-6025 and said endonuclease comprises a sequence having at least 75% identity to SEQ ID NO: 423.
64. The method of any one of clauses 57-61, wherein said engineered guide RNA comprises a targeting sequence having at least 85% identity to at least 18 consecutive nucleotides of any one of SEQ ID NOs: 5970, 5971, 5983, or 5984.
65. The method of any one of clauses 57-61, wherein said engineered guide RNA comprises a targeting sequence having at least 85% identity to at least 18 consecutive nucleotides of any one of SEQ ID NOs: 6006, 6010, 6011, or 6012.
66. A method of editing a GR (NR3C1) locus in a cell, comprising contacting to said cell
   (a) an RNA-guided endonuclease; and
   (b) an engineered guide RNA, wherein said engineered guide RNA is configured to form a complex with said endonuclease and said engineered guide RNA comprises a spacer sequence configured to hybridize to a region of said GR (NR3C1) locus,
   wherein said engineered guide RNA comprises a targeting sequence having at least 85% identity to at least 18 consecutive nucleotides of any one of SEQ ID NOs: 6026-6090 or 6091-6121.
67. The method of clause 66, wherein said RNA-guided endonuclease is a class II, type II Cas endonuclease.
68. The method of clause 66 or clause 67, wherein said RNA-guided endonuclease comprises a RuvCIII domain comprising a sequence having at least 75% identity to SEQ ID NO: 2242 or SEQ ID NO: 2244.
69. The method of clause 68, wherein said RNA-guided endonuclease further comprises an HNH domain.
70. The method of any one of clauses 66-69, wherein said RNA-guided endonuclease comprises a sequence having at least 75% identity to SEQ ID NO: 421 or SEQ ID NO: 423.
71. The method of any one of clauses 66-70, wherein said engineered guide RNA comprises a targeting sequence having at least 85% identity to at least 18 consecutive nucleotides of any one of SEQ ID NOs: 6026-6090 and said endonuclease comprises a sequence having at least 75% identity to SEQ ID NO: 421.
72. The method of any one of clauses 66-70, wherein said engineered guide RNA comprises a targeting sequence having at least 85% identity to at least 18 consecutive nucleotides of any one of SEQ ID NOs: 6091-6121 and said endonuclease comprises a sequence having at least 75% identity to SEQ ID NO: 423.
73. The method of any one of clauses 66-70, wherein said engineered guide RNA comprises a targeting sequence having at least 85% identity to at least 18 consecutive nucleotides of any one of SEQ ID NOs: 6027-6028, 6029, 6038, 6043, 6049, 6076, 6080, 6081, or 6086.
74. The method of any one of clauses 66-70, wherein said engineered guide RNA comprises a targeting sequence having at least 85% identity to at least 18 consecutive nucleotides of any one of SEQ ID NOs: 6092, 6115, or 6119.
75. A method of editing an AAVS1 locus in a cell, comprising contacting to said cell
   (a) an RNA-guided endonuclease; and
   (b) an engineered guide RNA, wherein said engineered guide RNA is configured to form a complex with said endonuclease and said engineered guide RNA comprises a spacer sequence configured to hybridize to a region of said AAVS1 locus,
   wherein said engineered guide RNA comprises a targeting sequence having at least 85% identity to at least 18 consecutive nucleotides of any one of SEQ ID NOs: 6122-6152.
76. The method of clause 75, wherein said RNA-guided endonuclease is a class II, type II Cas endonuclease.
77. The method of clause 75 or clause 76, wherein said RNA-guided endonuclease comprises a RuvCIII domain comprising a sequence having at least 75% identity to SEQ ID NO: 2242 or SEQ ID NO: 2244.
78. The method of clause 68, wherein said RNA-guided endonuclease further comprises an HNH domain.
79. The method of any one of clauses 75-78, wherein said RNA-guided endonuclease comprises a sequence having at least 75% identity to SEQ ID NO: 421 or SEQ ID NO: 423.
80. The method of any one of clauses 75-79, wherein said engineered guide RNA comprises a targeting sequence having at least 85% identity to at least 18 consecutive nucleotides of any one of SEQ ID NOs: 6122, 6125-6126, 6128, 6131, 6133, 6136, 6141, 6143, or 6148.
81. A method of editing an TIGIT locus in a cell, comprising contacting to said cell
   (a) an RNA-guided endonuclease; and
   (b) an engineered guide RNA, wherein said engineered guide RNA is configured to form a complex with said endonuclease and said engineered guide RNA comprises a spacer sequence configured to hybridize to a region of said TIGIT locus,
   wherein said engineered guide RNA comprises a targeting sequence having at least 85% identity to at least 18 consecutive nucleotides of any one of SEQ ID NOs: 6153-6181.
82. The method of clause 81, wherein said RNA-guided endonuclease is a class II, type II Cas endonuclease.
83. The method of clause 81 or clause 82, wherein said RNA-guided endonuclease comprises a sequence having at least 75% identity to SEQ ID NO: 421 or SEQ ID NO: 423.
84. The method of any one of clauses 81-83, wherein said RNA-guided endonuclease comprises a RuvCIII domain comprising a sequence having at least 75% identity to SEQ ID NO: 2242 or SEQ ID NO: 2244.
85. The method of clause 84, wherein said RNA-guided endonuclease further comprises an HNH domain.
86. The method of any one of clauses 81-85, wherein said engineered guide RNA comprises a targeting sequence having at least 85% identity to at least 18 consecutive nucleotides of any one of SEQ ID NOs: 66155, 6159, 616, or 6172.
87. A method of editing an CD38 locus in a cell, comprising contacting to said cell
   (a) an RNA-guided endonuclease; and
   (b) an engineered guide RNA, wherein said engineered guide RNA is configured to form a complex with said endonuclease and said engineered guide RNA comprises a spacer sequence configured to hybridize to a region of said CD38 locus,
   wherein said engineered guide RNA comprises a targeting sequence having at least 85% identity to at least 18 consecutive nucleotides of any one of SEQ ID NOs: 6182-6248 or 6249-6256.
88. The method of clause 87, wherein said RNA-guided endonuclease is a class II, type II Cas endonuclease.
89. The method of clause 87 or 88, wherein said RNA-guided endonuclease comprises a RuvCIII domain comprising a sequence having at least 75% identity to SEQ ID NO: 2242 or SEQ ID NO: 2244.
90. The method of clause 89, wherein said RNA-guided endonuclease further comprises an HNH domain.
91. The method of any one of clauses 87-90, wherein said RNA-guided endonuclease comprises a sequence having at least 75% identity to SEQ ID NO: 421 or SEQ ID NO: 423.
92. The method of any one of clauses 87-91, wherein said engineered guide RNA comprises a targeting sequence having at least 85% identity to at least 18 consecutive nucleotides of any one of SEQ ID NOs: 6182-6248 and said endonuclease comprises a sequence having at least 75% identity to SEQ ID NO: 421.
93. The method of any one of clauses 87-91, wherein said engineered guide RNA comprises a targeting sequence having at least 85% identity to at least 18 consecutive nucleotides of any one of SEQ ID NOs: 6249-6256 and said endonuclease comprises a sequence having at least 75% identity to SEQ ID NO: 423.
94. The method of any one of clauses 87-91, wherein said engineered guide RNA comprises a targeting sequence having at least 85% identity to at least 18 consecutive nucleotides of any one of SEQ ID NOs: 6182-6183, 6189, 6191, 6208, 6210, 6211, or 6215.
95. The method of any one of clauses 87-91, wherein said engineered guide RNA comprises a targeting sequence having at least 85% identity to at least 18 consecutive nucleotides of SEQ ID NO: 6251.
96. The method of any one of clauses 48-95, wherein said cell is a peripheral blood mononuclear cell, a T-cell, an NK cell, a hematopoietic stem cell (HSCT), or a B-cell.
97. An engineered guide ribonucleic acid polynucleotide comprising:
   a) a DNA-targeting segment comprising a nucleotide sequence that is complementary to a target sequence in a target DNA molecule; and
   b) a protein-binding segment comprising two complementary stretches of nucleotides that hybridize to form a double-stranded RNA (dsRNA) duplex,
   wherein said two complementary stretches of nucleotides are covalently linked to one another with intervening nucleotides, and
   wherein said engineered guide ribonucleic acid polynucleotide is configured to form a complex with a class 2, type II Cas endonuclease and target said complex to said target sequence of said target DNA molecule, wherein said DNA-targeting segment comprises a sequence having at least 85% identity to any one of SEQ ID NOs: 5950-5965, 5966-6025, 6026-6121, 6122-6152, 6153-6181, or 6182-6256.
98. The engineered guide ribonucleic acid polynucleotide of clause 97, wherein said protein-binding segment comprises a sequence having at least 85% identity to any one of SEQ ID NOs: 5466 or 6304.
99. A system for generating an edited immune cell, comprising:
   (a) an RNA-guided endonuclease;
   (b) an engineered guide ribonucleic acid polynucleotide according to clause 97 configured to bind said RNA-guided endonuclease; and
   (c) a single- or double-stranded DNA repair template comprising first and second homology arms flanking a sequence encoding a chimeric antigen receptor (CAR).
100. The system of clause 99, wherein said cell is a peripheral blood mononuclear cell, a T-cell, an NK cell, a hematopoietic stem cell (HSCT), or a B-cell.
101. The system of clause 99 or 100, wherein said wherein said RNA-guided endonuclease is a class II, type II Cas endonuclease.
102. The method of any one of clauses 99-101, wherein said RNA-guided endonuclease comprises a RuvCIII domain comprising a sequence having at least 75% identity to SEQ ID NO: 2242 or SEQ ID NO: 2244.
103. The method of clause 102, wherein said RNA-guided endonuclease further comprises an HNH domain.
104. The method of any one of clauses 99-103, wherein said RNA-guided endonuclease comprises a sequence having at least 75% identity to SEQ ID NO: 421 or SEQ ID NO: 423.

## Claims

1. A method of editing a TRAC locus in a cell, comprising contacting to said cell:
(a) a ribonucleic acid-guided endonuclease or a nucleic acid encoding said ribonucleic acid-guided endonuclease; wherein said ribonucleic acid-guided endonuclease comprises a sequence having at least 90% sequence identity to SEQ ID NO: 421 or SEQ ID NO: 423; and
(b) an engineered guide ribonucleic acid or a nucleic acid encoding said engineered guide ribonucleic acid, wherein said engineered guide ribonucleic acid is configured to form a complex with said ribonucleic acid-guided endonuclease and said engineered guide ribonucleic acid comprises a spacer sequence configured to hybridize to a region of said TRAC locus,
wherein said spacer sequence comprises a sequence having at least 85% sequence identity to at least 18 consecutive nucleotides of any one of SEQ ID NOs: 5955, 5950-5954, or 5956-5965.

2. The method of claim 1, wherein said ribonucleic acid-guided endonuclease comprises a RuvCIII domain comprising a sequence having at least 95% sequence identity to SEQ ID NO: 2242 or SEQ ID NO: 2244.

3. The method of claim 2, wherein said RuvCIII domain comprises the sequence of SEQ ID NO: 2242 or SEQ ID NO: 2244.

4. The method of any one of claims 1-3, wherein said ribonucleic acid-guided endonuclease comprises an HNH domain comprising a sequence having at least 90% sequence identity to SEQ ID NO: 4056 or SEQ ID NO: 4063.

5. The method of claim 4, wherein said HNH domain comprises the sequence of SEQ ID NO: 4056 or SEQ ID NO: 4063.

6. The method of any one of claims 1-5, wherein said ribonucleic acid-guided endonuclease comprises a sequence having at least 95% sequence identity to SEQ ID NO: 421 or SEQ ID NO: 423.

7. The method of claim 6, wherein said ribonucleic acid-guided endonuclease comprises a sequence of SEQ ID NO: 421.

8. The method of claim 6, wherein said ribonucleic acid-guide endonuclease comprises a sequence of SEQ ID NO: 423.

9. The method of any one of claim 1-8, wherein said spacer sequence comprises the sequence of any one of SEQ ID NOs: 5955, 5954, 5953, 5960, 5961, or 5963.

10. The method of any one of claims 1-9, wherein said engineered guide ribonucleic acid comprises a sequence configured to bind said ribonucleic acid-guided endonuclease.

11. The method of claim 10, wherein said sequence configured to bind said ribonucleic acid-guided endonuclease comprises a sequence having at least 80% sequence identity to SEQ ID NOs: 5466 or SEQ ID NO: 6304.

12. The method of any one of claims 1-11, wherein said cell is a human cell and optionally wherein said cell is a peripheral blood mononuclear cell (PBMC), a T-cell, a natural killer (NK) cell, a hematopoietic stem cell (HSCT), or a B-cell, or any combination thereof.

13. The method of any one of claims 1-12, wherein said contacting further comprises transfecting said cell with said nucleic acid encoding said ribonucleic acid-guided endonuclease and said nucleic acid encoding said engineered guide ribonucleic acid.

14. The method of any one of claims 1-13, further comprising contacting said cell with a single- or double-stranded deoxyribonucleic acid repair template.

15. The method of any one of claims 1-14, wherein said ribonucleic acid-guided endonuclease comprises the sequence of SEQ ID NO: 421 and wherein said spacer sequence comprises the sequence of SEQ ID NO: 5955.
